# EUROPEAN PATENT SPECIFICATION

(11) **EP 2 604 701 B1**
(45) Date of publication and mention of the grant of the patent: **04.11.2015**
(21) Application number: 12179588.4
(22) Date of filing: 03.07.2008
(51) Int. Cl.: C12Q 1/68

(54) **Recurrent gene fusions in prostate cancer**
Rezidivierende Genfusionen bei Prostatakrebs
Fusions de gène récurrentes dans le cancer de la prostate

(30) Priority: 06.07.2007 US 825552
(43) Date of publication of application: 19.06.2013
(62) Divisional of application: 08772418.3
(73) Proprietor: The Regents of The University of Michigan, Ann Arbor, MI 48109-2590 (US)
(72) Inventor: Chinnaiyan, Arul, M., Plymouth, MI Michigan 48170 (US); Tomlins, Scott, Ann Arbor, MI 48105 (US)
(74) Representative: Walton, Seán Malcolm

(56) References cited:
- WO-A-2007/033187
- MEHRA ROHIT ET AL: "Comprehensive assessment of TMPRSS2 and ETS family gene aberrations in clinically localized prostate cancer.", MODERN PATHOLOGY : AN OFFICIAL JOURNAL OF THE UNITED STATES AND CANADIAN ACADEMY OF PATHOLOGY, INC MAY 2007, vol. 20, no. 5, May 2007 (2007-05), pages 538-544, XP007906804, ISSN: 0893-3952
- TOMLINS SCOTT A ET AL: "Recurrent fusion of TMPRSS2 and ETS transcription factor genes in prostate cancer", SCIENCE, AMERICAN ASSOCIATION FOR THE ADVANCEMENT OF SCIENCE, US, WASHINGTON, DC, vol. 310, no. 5748, 28 October 2005 (2005-10-28), pages 644-648, XP002464140, ISSN: 0036-8075
- LAXMAN BHARATHI ET AL: "Noninvasive detection of TMPRSS2:ERG fusion transcripts in the urine of men with prostate cancer.", NEOPLASIA (NEW YORK, N.Y.) OCT 2006, vol. 8, no. 10, October 2006 (2006-10), pages 885-888, XP007906803, ISSN: 1476-5586
- PERNER SVEN ET AL: "TMPRSS2-ERG fusion prostate cancer: an early molecular event associated with invasion.", THE AMERICAN JOURNAL OF SURGICAL PATHOLOGY JUN 2007, vol. 31, no. 6, June 2007 (2007-06), pages 882-888, XP009110691, ISSN: 0147-5185

## Description

### FIELD OF THE INVENTION

The present invention relates to compositions and methods for cancer diagnosis and research. In particular, the present invention relates to recurrent gene fusions as diagnostic markers and clinical targets for prostate cancer.

### BACKGROUND OF THE INVENTION

A central aim in cancer research is to identify altered genes that are causally implicated in oncogenesis. Several types of somatic mutations have been identified including base substitutions, insertions, deletions, translocations, and chromosomal gains and losses, all of which result in altered activity of an oncogene or tumor suppressor gene. First hypothesized in the early 1900's, there is now compelling evidence for a causal role for chromosomal rearrangements in cancer (Rowley, Nat Rev Cancer 1: 245 (2001)). Recurrent chromosomal aberrations were thought to be primarily characteristic of leukemias, lymphomas, and sarcomas. Epithelial tumors (carcinomas), which are much more common and contribute to a relatively large fraction of the morbidity and mortality associated with human cancer, comprise less than 1% of the known, disease-specific chromosomal rearrangements (Mitelman, Mutat Res 462: 247 (2000)). While hematological malignancies are often characterized by balanced, disease-specific chromosomal rearrangements, most solid tumors have a plethora of non-specific chromosomal aberrations. It is thought that the karyotypic complexity of solid tumors is due to secondary alterations acquired through cancer evolution or progression.

Two primary mechanisms of chromosomal rearrangements have been described. In one mechanism, promoter/enhancer elements of one gene are rearranged adjacent to a proto-oncogene, thus causing altered expression of an oncogenic protein. This type of translocation is exemplified by the apposition of immunoglobulin (IG) and T-cell receptor (TCR) genes to MYC leading to activation of this oncogene in B- and T-cell malignancies, respectively (Rabbitts, Nature 372: 143 (1994)). In the second mechanism, rearrangement results in the fusion of two genes, which produces a fusion protein that may have a new function or altered activity. The prototypic example of this translocation is the BCR-ABL gene fusion in chronic myelogenous leukemia (CML) (Rowley, Nature 243: 290 (1973); de Klein et al., Nature 300: 765 (1982). Importantly, this finding led to the rational development of imatinib mesylate (Gleevec), which successfully targets the BCR-ABL kinase (Deininger, et al., Blood 105: 2640 (2005)). Thus, identifying recurrent gene rearrangements in common epithelial tumors may have profound implications for cancer drug discovery efforts as well as patient treatment.

Mehra Rohit et al., MODERN PATHOLOGY : AN OFFICIAL JOURNAL OF THE UNITED STATES AND CANADIAN ACADEMY OF PATHOLOGY, INC MAY 2007, vol. 20, no. 5, May 2007 (2007-05), pages 538-544, XP007906804, ISSN: 0893-3952, discloses a comprehensive assessment of TMPRSS2 and ETS family gene aberrations in clinically localized prostate cancer.

WO2007/033187A discloses recurrent gene fusions of androgen regulated genes and ETS family member genes in prostate cancer.

### SUMMARY OF THE INVENTION

The invention is as defined in the claims. Disclosure outside of the claims is for reference.

In some embodiments, the present invention provides a method for diagnosing prostate cancer in a patient comprising: providing a sample from the patient; and, detecting the presence or absence in the sample of a gene fusion having a 5' portion from a transcription regulatory region of androgen regulated gene (ARG) SLC45A3 and a 3' portion from an ETS family member gene (e.g., ETV1), wherein the presence in the sample of the gene fusion is indicative of prostate cancer in the patient. In some embodiments, the 5' portion from the transcriptional regulatory region of the ARG or HG comprises a promoter region of the ARG or HG. In some embodiments, the detecting step comprises detecting chromosomal rearrangements of genomic DNA having a 5' portion from a transcriptional regulatory region of SLC45A3 and a 3' portion from an ETS family member gene. In some embodiments, detecting chromosomal rearrangements of genomic DNA comprises using a nucleic acid sequencing technique, a nucleic acid hybridization technique (e.g., *in situ* hybridization (ISH), microarray or Southern blot), or a nucleic acid amplification method (e.g., polymerase chain reaction (PCR), reverse transcription polymerase chain reaction (RT-PCR), transcription-mediated amplification (TMA), ligase chain reaction (LCR), strand displacement amplification (SDA), or nucleic acid sequence based amplification (NASBA)). In some embodiments, the detecting step comprises detecting chimeric mRNA transcripts having a 5' portion from a transcriptional regulatory region of SLC45A3 and a 3' portion from an ETS family member gene. In some embodiments, detecting chimeric mRNA transcripts comprises using a nucleic acid sequencing technique, a nucleic acid hybridization technique (e.g., *in situ* hybridization (ISH), microarray or Northern blot), or a nucleic acid amplification method (e.g., polymerase chain reaction (PCR), reverse transcription polymerase chain reaction (RT-PCR), transcription-mediated amplification (TMA), ligase chain reaction (LCR), strand displacement amplification (SDA), or nucleic acid sequence based amplification (NASBA)). In some embodiments, the sample is tissue, blood, plasma, serum, urine, urine supernatant, urine cell pellet, semen, prostatic secretions or prostate cells. Described herein is a method for diagnosing prostate cancer in a patient comprising: providing a sample from the patient; and, detecting the presence or absence in the sample of a gene fusion having a 5' portion from an androgen regulated gene (e.g., TMPRSS2 or SLC45A3) and a 3' portion from ETV5, wherein the presence in the sample of the gene fusion is indicative of prostate cancer in the patient. In some embodiments, the 5' portion from the transcriptional regulatory region of the ARG comprises a promoter region of the ARG. In some embodiments, the detecting step comprises detecting chromosomal rearrangements of genomic DNA having a 5' portion from a transcriptional regulatory region of an ARG and a 3' portion from ETV5. In some embodiments, detecting chromosomal rearrangements of genomic DNA comprises using a nucleic acid sequencing technique, a nucleic acid hybridization technique (e.g., *in situ* hybridization (ISH), microarray or Southern blot), or a nucleic acid amplification method (e.g., polymerase chain reaction (PCR), reverse transcription polymerase chain reaction (RT-PCR), transcription-mediated amplification (TMA), ligase chain reaction (LCR), strand displacement amplification (SDA), or nucleic acid sequence based amplification (NASBA)). In some embodiments, the detecting step comprises detecting chimeric mRNA transcripts having a 5' portion from a transcriptional regulatory region of an ARG and a 3' portion from ETV5. In some embodiments, detecting chimeric mRNA transcripts comprises using a nucleic acid sequencing technique, a nucleic acid hybridization technique (e.g., *in situ* hybridization (ISH), microarray or a Northern blot), or a nucleic acid amplification method (e.g., polymerase chain reaction (PCR), reverse transcription polymerase chain reaction (RT-PCR), transcription-mediated amplification (TMA), ligase chain reaction (LCR), strand displacement amplification (SDA), or nucleic acid sequence based amplification (NASBA)). In some embodiments, the sample is tissue, blood, plasma, serum, urine, urine supernatant, urine cell pellet, semen, prostatic secretions or prostate cells.

Additional embodiments are provided in the description and examples below.

### DESCRIPTION OF THE FIGURES

Figure 1 shows the Cancer Outlier Profile Analysis (COPA) of microarray data. (A) ETV1 (left panels) and ERG (middle panels) expression (normalized expression units) are shown from all profiled samples in two large scale gene expression studies. (B) As in (A), except data from laser capture microdissected samples were used. (C) As in (A), except oncogenes (FGFR3 and CCND1) with known translocations to the immunoglobulin heavy chain promoter (IgH) in multiple myeloma were examined.

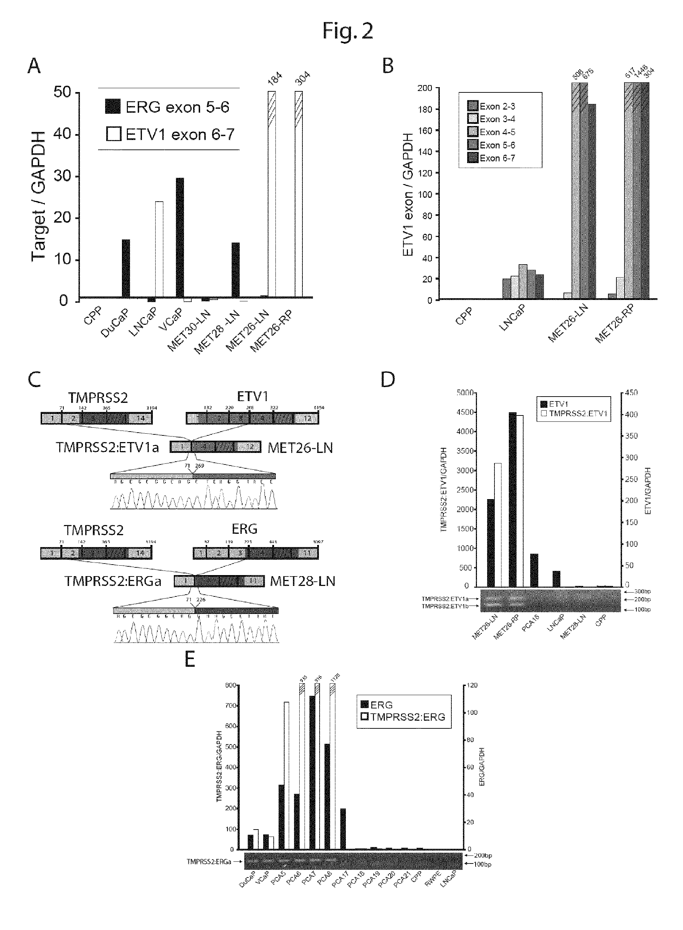

Figure 2 shows the identification and characterization of TMPRSS2:ETV1 and TMPRSS2:ERG gene fusions in prostate cancer (PCA). (A) Prostate cancer cell lines (DuCaP, LnCaP and VCaP) and hormone refractory metastatic (MET) prostate cancer tissues were analyzed for ERG (■) and ETV1 (□) mRNA expression by quantitative PCR (QPCR). (B) Loss of over-expression of ETV1 exons 2 and 3 in MET26 compared to LNCaP cells. (C) Schematic of 5' RNA ligase-mediated rapid amplification of cDNA ends (RLM-RACE) results for ETV1 in MET26-LN and ERG in MET28-LN revealing gene fusions with TMPRSS2. (D) Validation of TMPRSS2:ETV1 expression using translocation-specific QPCR in MET26-LN and MET26-RP. (E) Validation of TMPRSS2:ERG expression using translocation-specific QPCR in cell lines and PCA specimens.

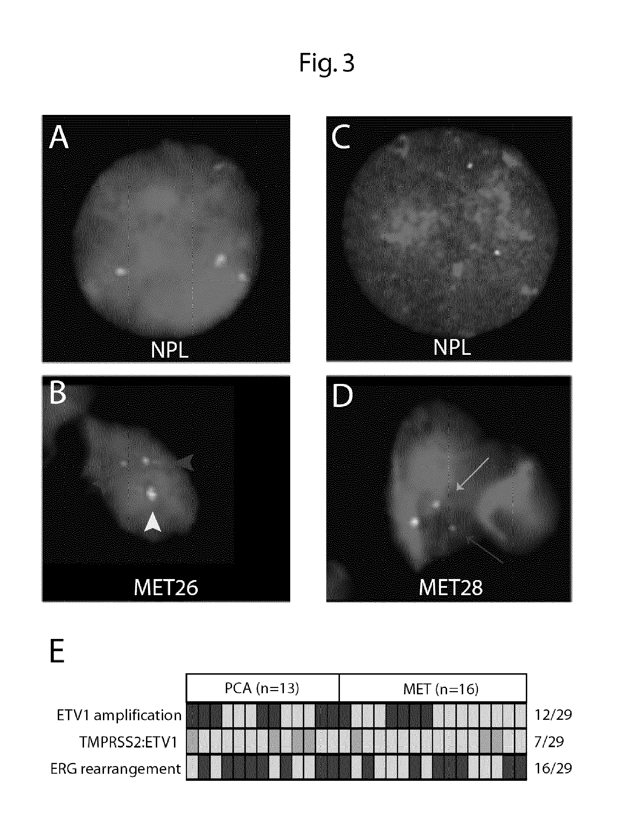

Figure 3 shows interphase fluorescence *in situ* hybridization (FISH) on formalin- fixed paraffin embedded tissue sections that confirms TMPRSS2:ETV1 gene fusion and ERG gene rearrangement. (A and B) show two-color, fusion-signal approach to detect the fusion of TMPRSS2 (green signal) and ETV1 (red signal). (C and D) Detection of ERG gene rearrangements using a two-color split-signal approach with two probes spanning the 5' (green signal) and 3' (red signal) regions of ERG. (E) Matrix representation of FISH results using the same probes as (A-D) on an independent tissue microarray containing cores from 13 cases of clinically localized prostate cancer (PCA) and 16 cases of metastatic prostate cancer (MET).
Figure 4 shows androgen regulation of ERG in prostate cancer cells carrying the TMPRSS2:ERG translocation.
Figure 5 shows Cancer Outlier Profile Analysis (COPA). Figure 5A shows a schematic of COPA analysis. Figure 5B shows that RUNX1T1 (ETO) had the highest scoring outlier profile at the 90th percentile in the Valk et al. acute myeloid leukemia dataset (n = 293).
Figure 6 shows a schematic of RNA ligase-mediated rapid amplification of cDNA ends (RLM-RACE) results for ETV1 in MET26-LN and ERG in PCA4 revealing gene fusions with TMPRSS2 (TMPRSS2:ERGb fusion).
Figure 7 shows over-expression of ETS family members in prostate cancer. Expression of all monitored ETS family members in profiled benign prostate, prostatic intraepithelial neoplasia (PIN), clinically localized prostate cancer and metastatic prostate cancer from grossly dissected tissue (A) or tissue isolated by laser capture microdissection (B) was visualized using Oncomine.

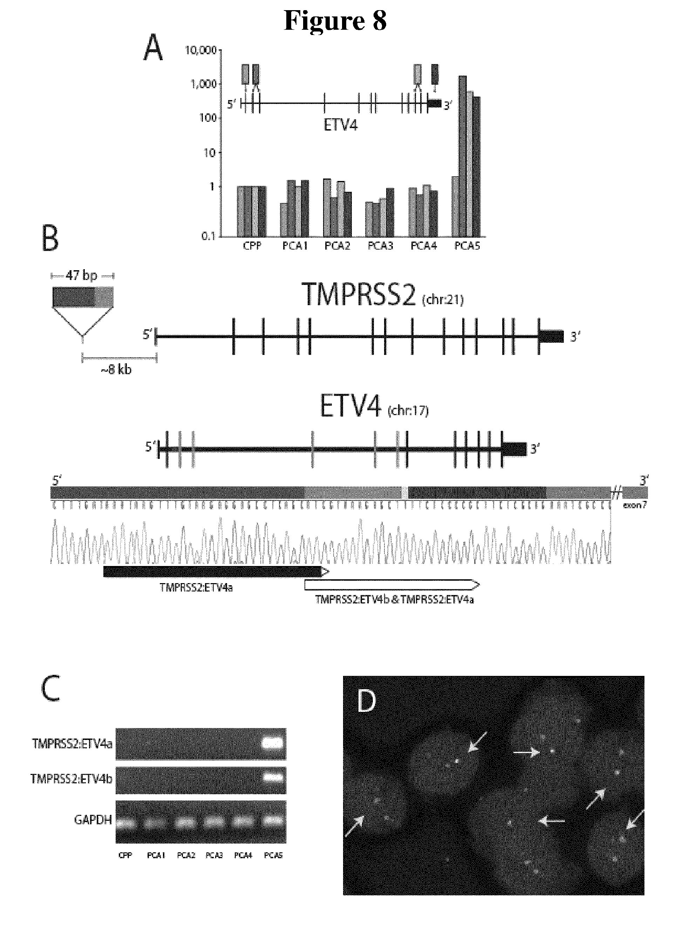

Figure 8 shows over expression of TMPRSS2 and ETV4 loci in a prostate cancer case that over-expresses ETV4. A. Expression of the indicated exons or region of ETV4 in pooled benign prostate tissue (CPP), prostate cancers that did not over-express ETV4 and were either TMPRSS2:ERG positive (PCA1-2) or negative (PCA3-4), and the prostate cancer case from our LCM cohort with ETV4 over-overexpression (PCA5). B. RLM-RACE reveals fusion of sequences upstream of TMPRSS2 with ETV4 in PCA5. C. Expression of TMPRSS2:ETV4a and TMPRSS2:ETV4b in PCA5 by QPCR. D. Interphase fluorescence *in situ* hybridization on formalin-fixed paraffin-embedded tissue confirms fusion of TMPRSS2 and ETV4 loci in PCA5.
Figure 9 shows mRNA sequences of exemplary ETS family genes.

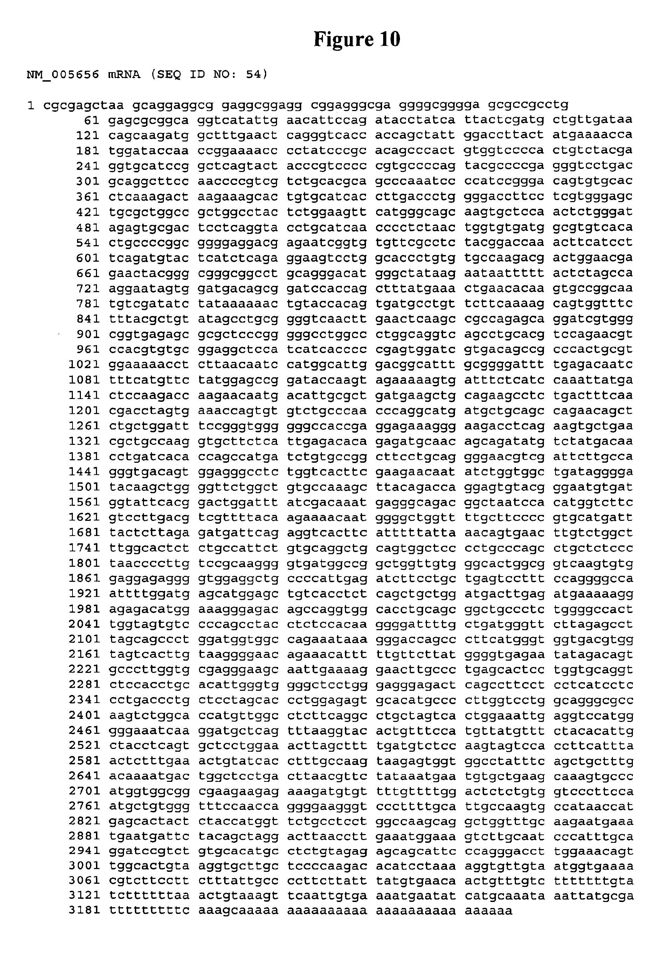

Figure 10 shows the mRNA sequence of TMPRSS2.

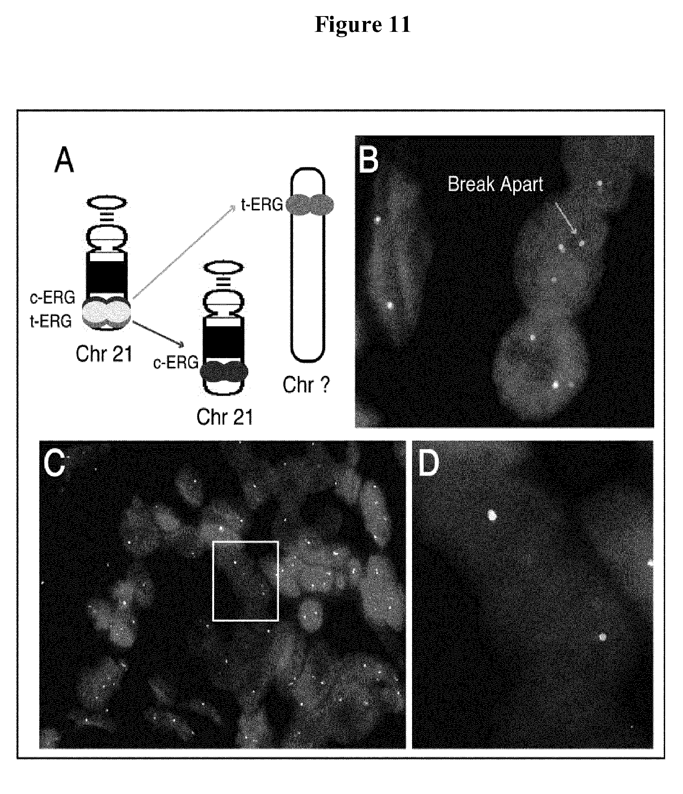

Figure 11 shows *TMPRSS2:ERG* gene fusion analysis by FISH. Panel A: Ideogram, depicting a break apart assay for the indirect detection of *TMPRSS2:ERG* fusion. Panel B: Interphase nuclei of a stromal cell (left) and a prostate cancer gland (right). Panel C: Interphase nuclei of prostate cancer glands showing break apart and simultaneous deletion as indicated by loss of the telomeric probe (100x oil immersion objective magnification). Panel D. Magnified view of boxed area in C demonstrating two nuclei with break apart and loss of the telomeric probe. (60x oil immersion objective magnification).
Figure 12 shows genomic deletions on chromosome 21 between *ERG* and *TMPRSS2.* Panel A: Samples, including 6 cell lines, 13 xenografts and 11 metastatic PCA samples, were characterized for *TMPRSS2:ERG* and *TMPRSS2:ETV1* status (gray bars for negative and blue bar for positive status), by qPCR and/or by FISH. Panel B: Magnification of the green framed box in A. Panel C: Magnification of the black framed box in A.
Figure 13 shows *TMPRSS2:ERG* rearrangement in clinically localized prostate cancer and association with pathological parameters. Panel A. The *TMPRSS2:ERG* rearrangement was identified in 49.2% of the primary PCA samples and 41.2% in the hormone naive metastatic LN samples. Panel B. *TMPRSS2:ERG* rearranged tumors with deletions tended to be observed in a higher percentage of PCA cases with advanced tumor stage (p=0.03).
Figure 14 shows known genes located on 21q22-23 between *ERG* (centromeric) and *TMPRSS2* (telomeric). Genes above the black line are oriented 5'-centromeric to 3'-telomeric and genes below the black line are oriented 5'-telomeric to 3'-centromeric. In the lower half of the image, a magnification of the ERG locus is depicted with FISH probes.

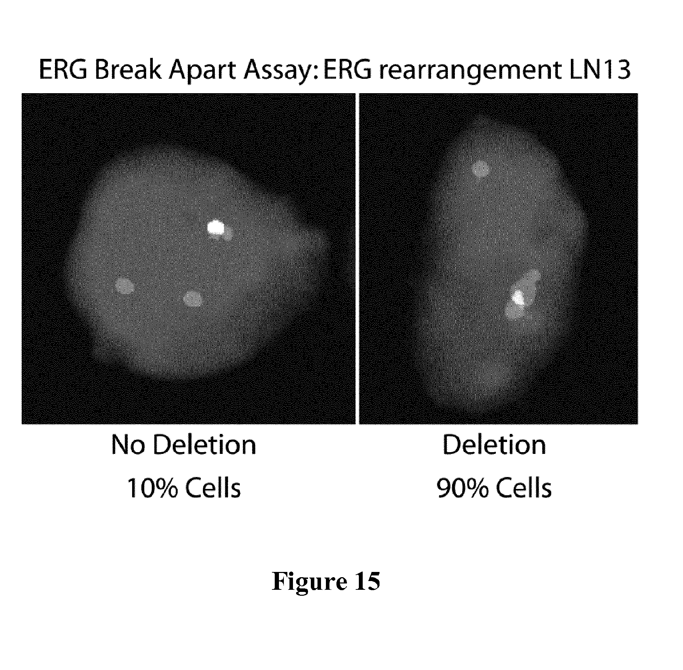

Figure 15 shows 'heterogenous' prostate cancer case predominantly showing *TMPRSS2:ERG* rearrangement with the deletion (nucleus on the right) and only small areas showing the *TMPRSS2:ERG* rearrangement without the deletion (nucleus on the left).
Figure 16 shows meta-analysis of genes located between TMPRSS2 and ERG across 8 published expression array datasets.

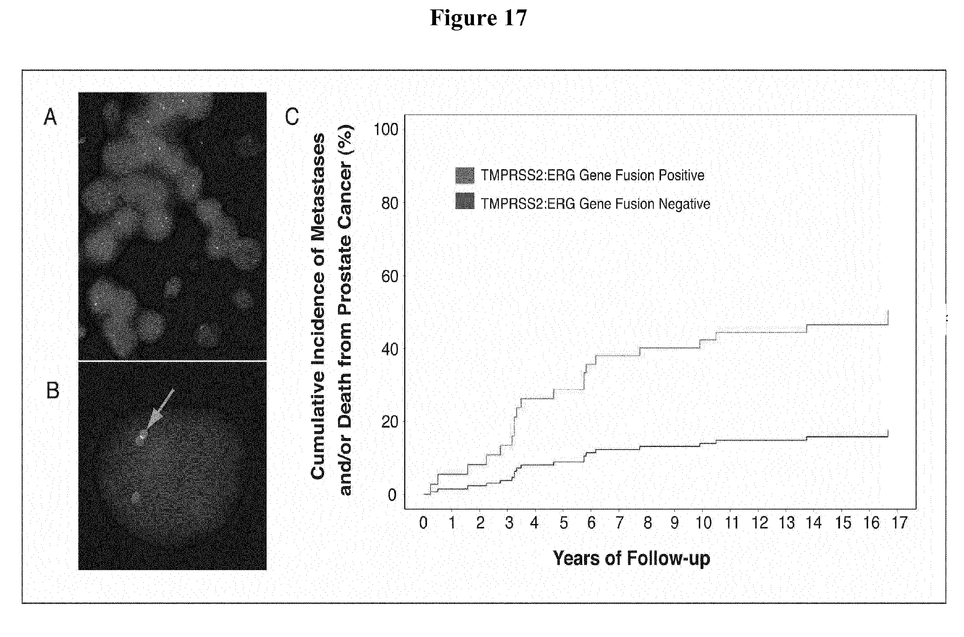

Figure 17 shows that the FISH assay detects the characteristic deletion associated with TMPRSS2:ERG gene fusion, which is associated with disease progression. Panels A and B: For analyzing the ERG rearrangement on chromosome 21q22.2, a break apart probe system was applied, consisting of the Biotin-14-dCTP labeled BAC clone RP11-24A11 (eventually conjugated to produce a red signal) and the Digoxigenin-dUTP labeled BAC clone RP11-137J13 (eventually conjugated to produce a green signal), spanning the neighboring centromeric and telomeric region of the ERG locus, respectively. Using this break apart probe system, a nucleus without ERG rearrangement exhibits two pairs of juxtaposed red and green signals. Juxtaposed red-green signals form a yellow fusion signal (Panel B, arrow). Panel C: In a cumulative incidence regression model, TMPRSS2:ERG was evaluated as a determinant for the cumulative incidence or metastases or prostate cancer-specific death.
Figure 18 shows FLI1 overexpression without fusion transcript.
Figure 19 shows induction of ERG protein expression by androgen in TMPRSS2-ERG+ cells.
Figure 20 shows a schematic of the endogenous and fusion ERG polypeptides.
Figure 21 shows nuclear interactors for ERG2.
Figure 22 shows sequences for peptide antibody and aqua probe generation against ERG1.
Figure 23 shows sequences for peptide antibody and aqua probe generation against ETV1.
Figure 24 shows sequences for peptide antibody and aqua probe generation against FLI1.
Figure 25 shows sequences for peptide antibody and aqua probe generation against ETV4.
Figure 26 shows the over-expression and androgen regulation of ETV1 in the LNCaP prostate cancer cell line. Figure 26A shows expression signature of androgen-regulated genes in VCaP and LNCaP prostate cancer cell lines. Figure 26B shows confirmation of PSA induction by androgen in both VCaP and LNCaP cells by quantitative PCR (QPCR). Figure 26C shows ETV1 induction by androgen in LNCaP cells. Figure 26D shows that ETV1 is markedly over-expressed in LNCaP cells.
Figure 27 shows rearrangement of ETV1 in LNCaP cells. Figure 27A shows a schematic of BACs used as probes for fluorescence *in situ* hybridization (FISH). Figure 27B shows that RP11-124L22 and RP11-1149J13 co-localize to chromosome 7 in normal peripheral lymphocytes (NPLs). Figure 27C shows localization of BAC #1 and BAC #4 on metaphase spreads (top panel) and interphase cells (bottom panel) was determined in the near tetraploid LNCaP cell line. Figure 27D shows signal from RP11-124L22 localizes to chromosome 14 in LNCaP cells.
Figure 28 shows that the entire ETV1 locus is inserted into chromosome 14 in LNCaP cells. Figure 28A shows a schematic of BACs used in this experiment. Figure 28B shows localization of RP11-124L22 (BAC #1) and RP11-313C20 (BAC #2) on metaphase spreads (top panel) and interphase cells (bottom panel) was determined by FISH in LNCaP cells.
Figure 29 shows siRNA knockdown of ETV1 in LnCaP.
Figure 30 shows siRNA knockdown of ERG in VCaP.
Figure 31 shows viral overexpression systems.
Figure 32 shows a schematic of transgenic mice.
Figure 33 shows detection of ERG and ETV1 transcripts in urine. Figure 33A shows detection of ERG and ETV1 in LNCaP (high *ETV1* expression) or VCaP (high *ERG* and *TMPRSS2:ERG* expression) prostate cancer cells. Figure 33B shows detection of ERG and ETV1 in urine of patients suspected of having prostate cancer.
Figure 34 shows assays used to detect TMPRSS2:ETS gene fusions in prostate cancer.
Figure 34A shows break apart assays for TMPRSS2 and ERG. An ERG rearrangement positive case (without deletion), as indicated by one pair of split 5' and 3' signals, is shown in the left panel. A TMPRSS2 rearranegment positive case (with deletion), as indicated by a loss of one 3' signal, is shown in the right panel. Figure 34B shows a fusion assay for TMPRSS2:ETV1 gene fusions. Figure 34C shows a break apart assay for ETV4.
Figure 35 shows TMPRSS2, ERG, ETV1 and ETV4 rearrangements as detected by FISH.
Figure 35A shows a Table of results for rearrangements in TMPRSS2, ERG, ETV1 and ETV4 as detected by the assays shown in Figure 34. Figure 34B shows a heat map representation of the TMPRSS2, ERG, ETV1 and ETV4 status from the 38 cases where all four assays were evaluable as described in A. Figure 34C shows a heat map representation of cases with discordant TMPRSS2 and ETS rearrangement status.
Figure 36 shows the sequences of gene fusions.
Figure 37 shows primers and probes for FLI-1 expression analysis.
Figure 38 shows identification of prostate-specific or ubiquitously active regulatory elements fused to ETV1 in outlier tumor specimens. a. Identification of ETV1 outlier cases. b. Structure of novel 5' partners fused to ETV1 in outlier cases. c. FISH confirmation of ETV1 fusions using probes 5' to the indicated partner and 3' to ETV1. d. Tissue specificity of 5' fusion partners. e. Assessment of androgen regulation of 5' fusion partners.
Figure 39 shows the confirmation of novel ETV1 gene fusions by qPCR.
Figure 40 shows confirmation of novel ETV1 gene fusions by FISH
   a. Schematic of BACs located 5' and 3' to ETV1, HNRPA2B1, HERV-K_22q11.23, C15ORF21 and SLC45A3 used as probes for interphase FISH. b-d. FISH was performed using BACs as indicated with the corresponding fluorescent label on formalin-fixed paraffin-embedded tissue sections for b) split signal assay of the 5' fusion partner, c) fusion of the 5' partner and ETV1, and d) split signal assay of ETV1.

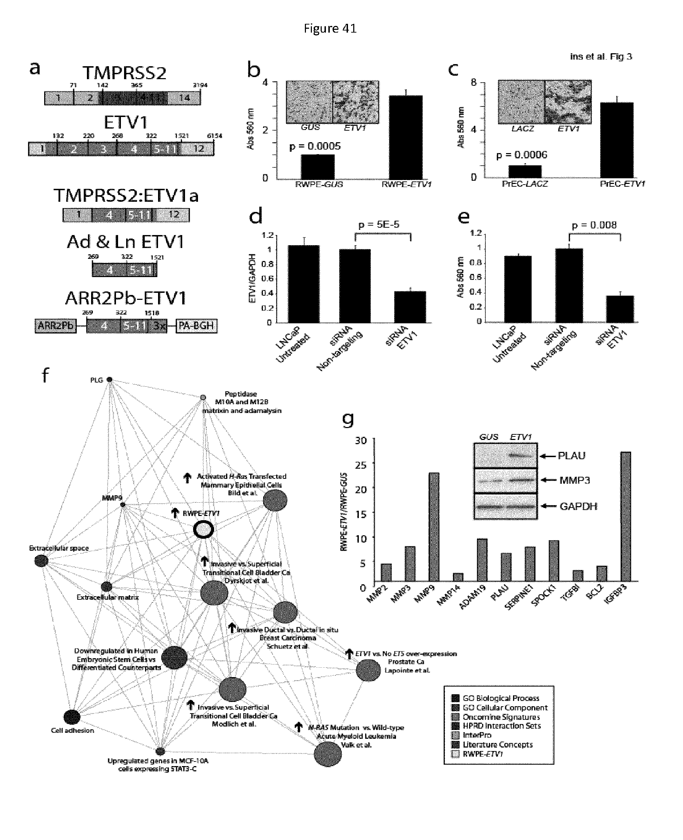

Figure 41 shows that ETV1 over-expression in prostate cells confers invasiveness.
   a. Adenoviruses and lentiviruses expressing the ETV1 gene fusion product (exons 4- through the reported stop codon). b. The benign immortalized prostate cell line RWPE was infected with ETV1 or control (GUS) lentivirus as indicated, and stable clones were generated and assayed for invasion through a modified basement membrane. c. Primary prostatic epithelial cells (PrEC) were infected with ETV1 or LACZ adenovirus as indicated and assayed for invasion. Mean (n = 3) + S.E. are shown. Photomicrographs of invaded cells are indicated in the insets. d-e. siRNA knockdown of ETV1 in LNCaP cells inhibits invasion. LNCaP cells were treated with transfection reagent alone (Untreated), or transfected with non-targeting or ETV1 siRNA as indicated. d. ETV1 knockdown was confirmed by qPCR (Mean (n = 4) + S.E.). e. Cells were assessed for invasion as in b and c (Mean (n = 3) + S.E.). f. RWPE-ETV1 and RWPE-GUS cells were profiled on Agilent Whole Genome microarrays. Network view of the molecular concept analysis of genes over-expressed in RWPE-ETV1 compared to RWPE-GUS cells is shown. Each node represents a molecular concept, or set of biologically related genes. The node size is proportional to the number of genes in the concept (as examples, the "RWPE-ETV1" and "Extracellular matrix" concepts contain 527 and 186 genes, respectively). The concept color indicates the concept type according to the legend. Each edge represents a significant enrichment (P < 0.005). g. qPCR confirmation of selected genes involved in invasion over-expressed in RWPE-ETV1 cells.

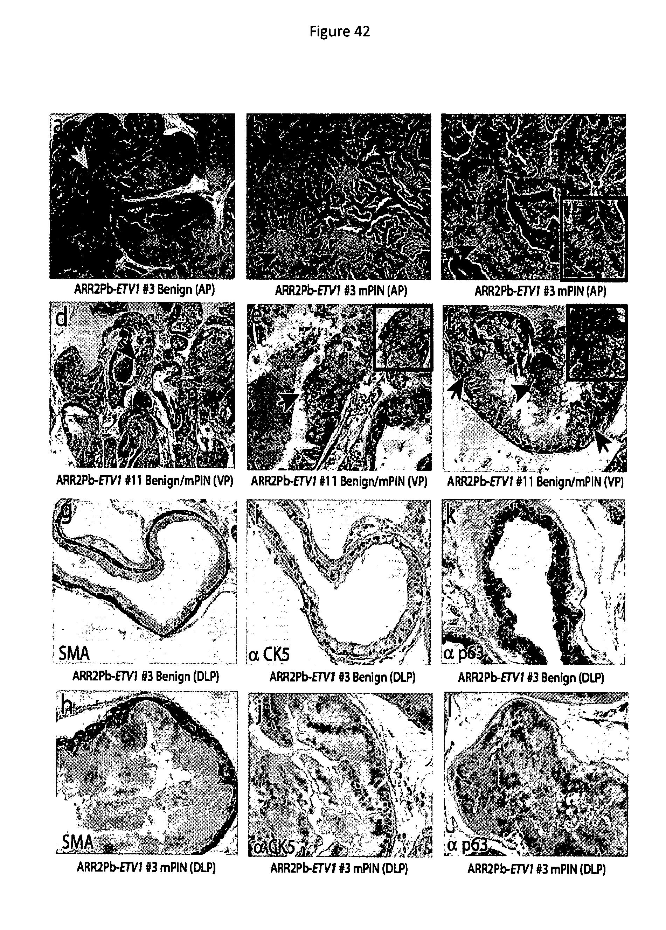

Figure 42 shows that transgenic mice expressing an ETV1 gene fusion product in the prostate develop mouse prostatic intraepithelial neoplasia (mPIN). a-f. Hematoxylin and eosin staining of ARR2Pb-ETV1 prostates for morphological assessment. c. High power view of b, with the inset showing prominent nucleoli in the mPIN lesion. d. Normal glands and foci of mPIN as observed in the ventral prostate (VP) of ARR2Pb-ETV1 mouse #3 (33 weeks). e. High power view of d. f. A single gland showing normal epithelial architecture as well as mPIN. The inset shows the foci of mPIN indicated by the arrowhead. g-l. Immunohistochemistry with smooth muscle actin (SMA) demonstrates a continuous fibromuscular layer around g) benign glands and h) all mPIN lesions, while the basal cell markers i-j) cytokeratin 5 (CK5) and k-l) p63 demonstrate loss of circumferential basal cells in mPIN foci (j,l) compared to normal glands (i,k) in the dorsolateral prostate (DLP) of ARR2Pb-ETV1 mouse #3. Original magnification for a, b & d is 100x and c & e-1 is 400x.

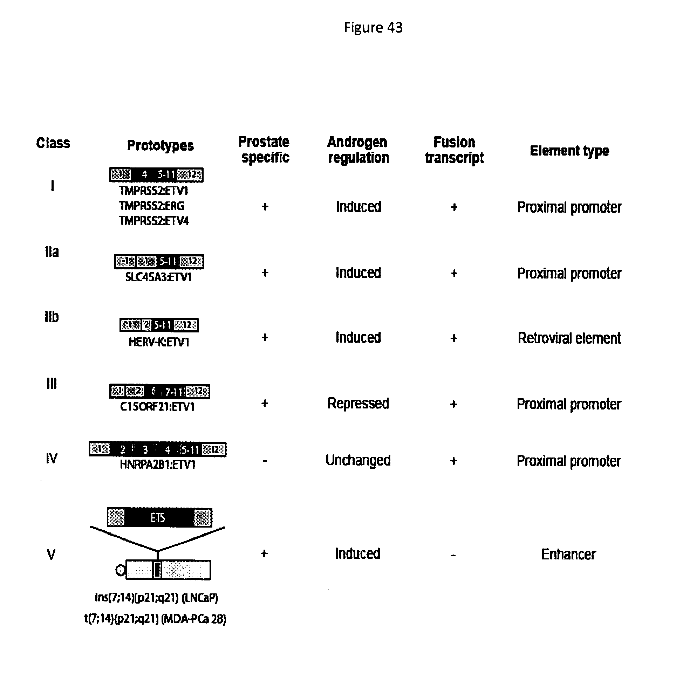

Figure 43 shows that distinct classes of chromosomal rearrangements activate ETS oncogenes in prostate cancer.

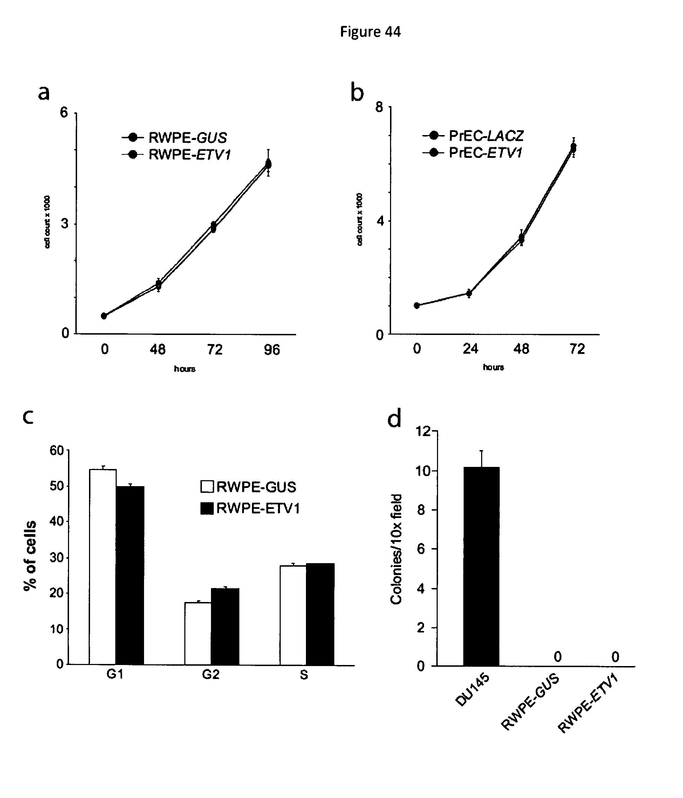

Figure 44 shows that over-expression of ETV1 does not effect proliferation or transform benign prostatic epithelial cells. a. The benign immortalized prostate cell line RWPE was infected with ETV1 or control (GUS) lentivirus as indicated, and stable clones were generated and assayed for proliferation. b. Primary prostatic epithelial cells (PrEC) were infected with ETV1 or LACZ adenovirus as indicated and assayed proliferation. Mean (n = 3) + S.E. are shown. Results are representative of three independent experiments. c. ETV1 overexpression does not increase the percentage of RWPE cells in S phase. d. ETV1 overexpression does not enhance the anchorage independent growth of RWPE cells.

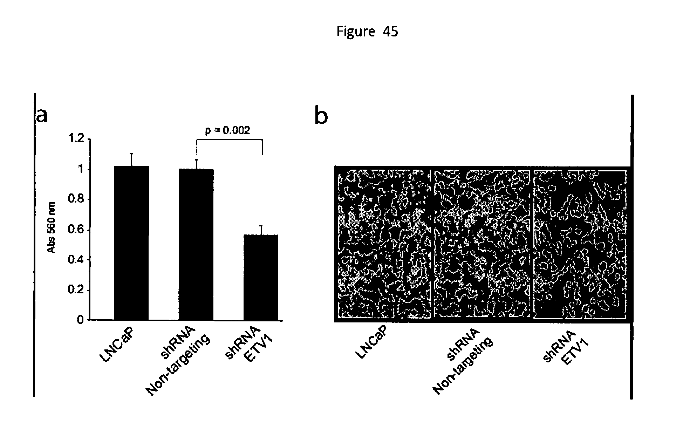

Figure 45 shows that shRNA knockdown of ETV1 inhibits invasion in LNCaP cells. a. Control LNCaP cells, or LNCaP cells infected with lentiviruses expressing a nontargeting or ETV1 shRNAmir, as indicated, were assessed for invasion as in Fig 41e. Mean (n = 3) + S.E. are shown. b. Photomicrographs of invaded cells from a.

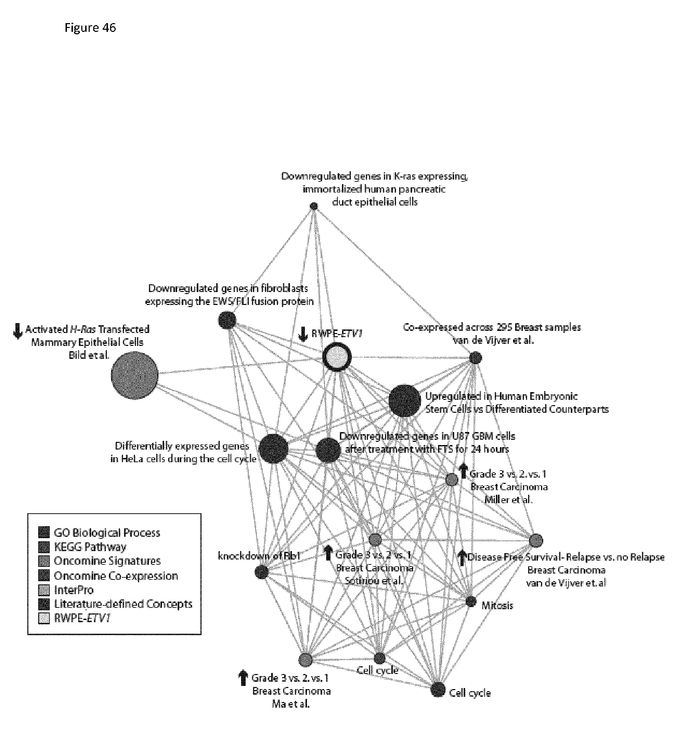

Figure 46 shows that over-expression of ETV1 results in decreased expression of proliferation related concepts.

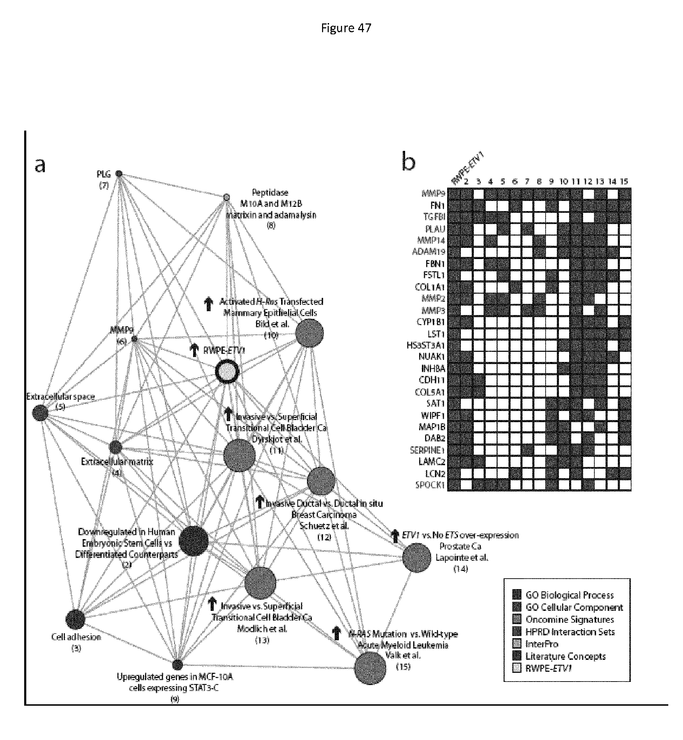

Figure 47 shows the identification of invasion related genes over-expressed in RWPE-ETV1 cells. a. RWPE-ETV1 and RWPE-GUS cells were profiled on Agilent Whole Genome microarrays. b. Overlay map identifying genes present in the RWPE-ETV1 concept and at least 5 of the 14 other concepts in the enrichment network (indicated by number).

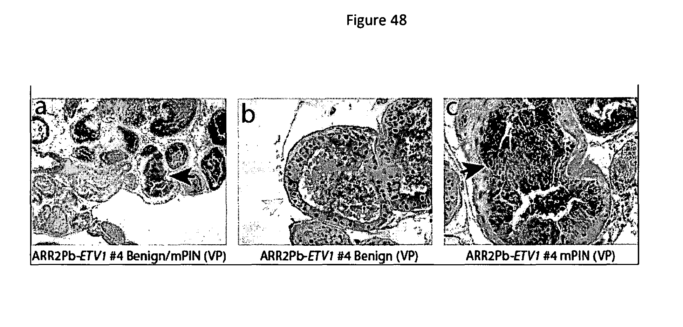

Figure 48 shows confirmation of ARR2Pb-ETV1-FLAG expression in regions of mPIN in the prostates of ARR2Pb-ETV1 mice. a. Low power view of the ventral prostate (VP) of ARR2Pb-ETV1 mouse #4, with benign areas and mPIN foci indicated by yellow and black arrows, respectively. b. High power view of the benign gland indicated in a, showing a lack of ETV1-FLAG expression. c. High power view of the mPIN gland indicated in a, demonstrating ETV1-FLAG expression.

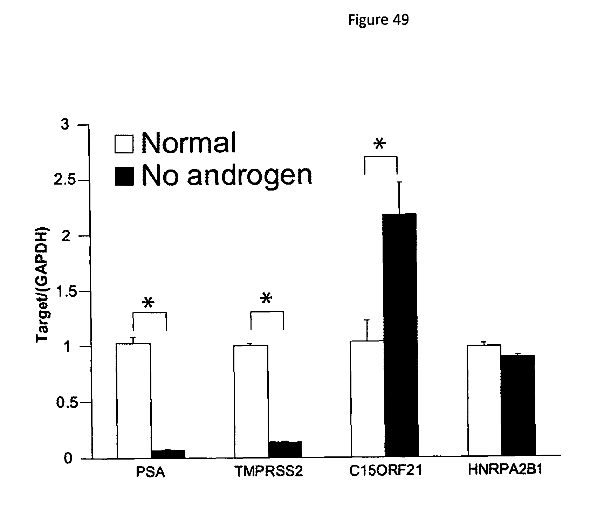

Figure 49 shows androgen deprivation of LNCaP cells modulates the expression of androgen regulated genes.

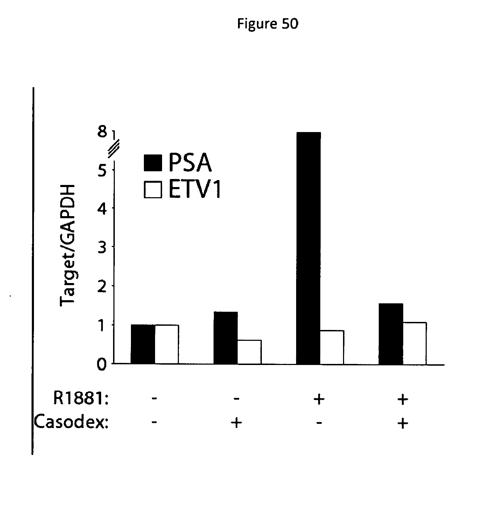

Figure 50 shows that R1881 does not induce ETV1 expression in the androgen-responsive VCaP cell line.

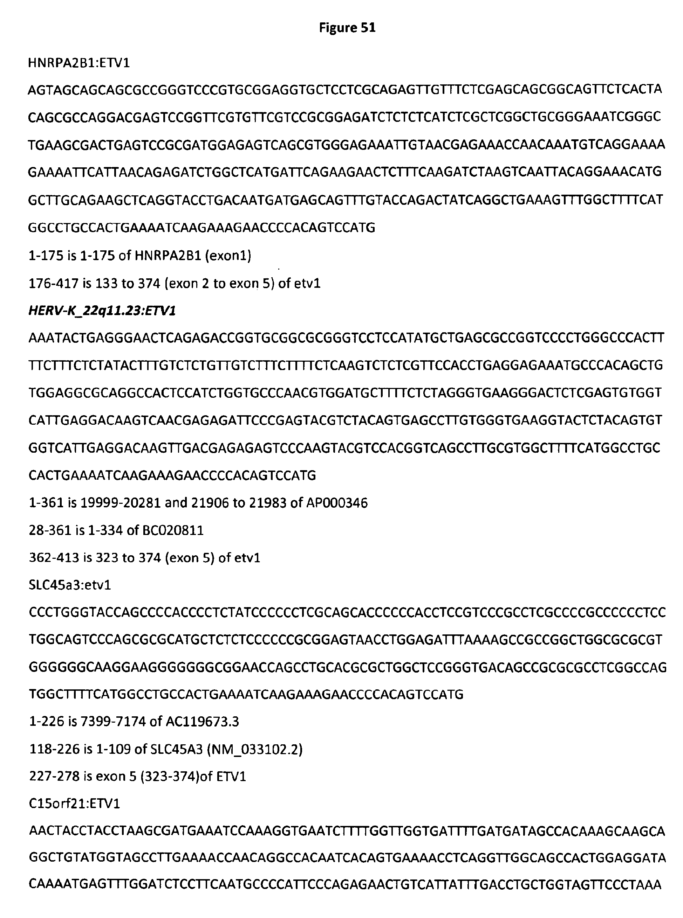

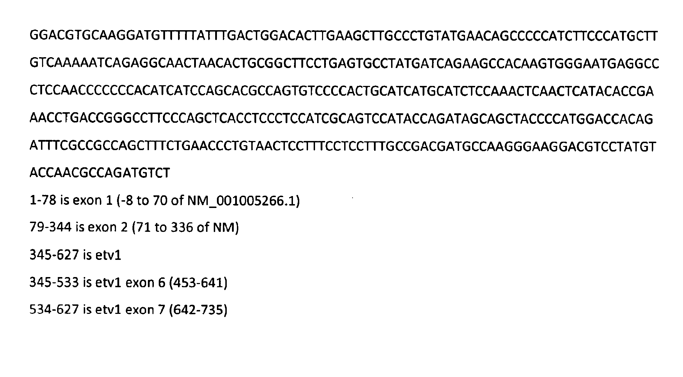

Figure 51 shows sequences of additional gene fusions.

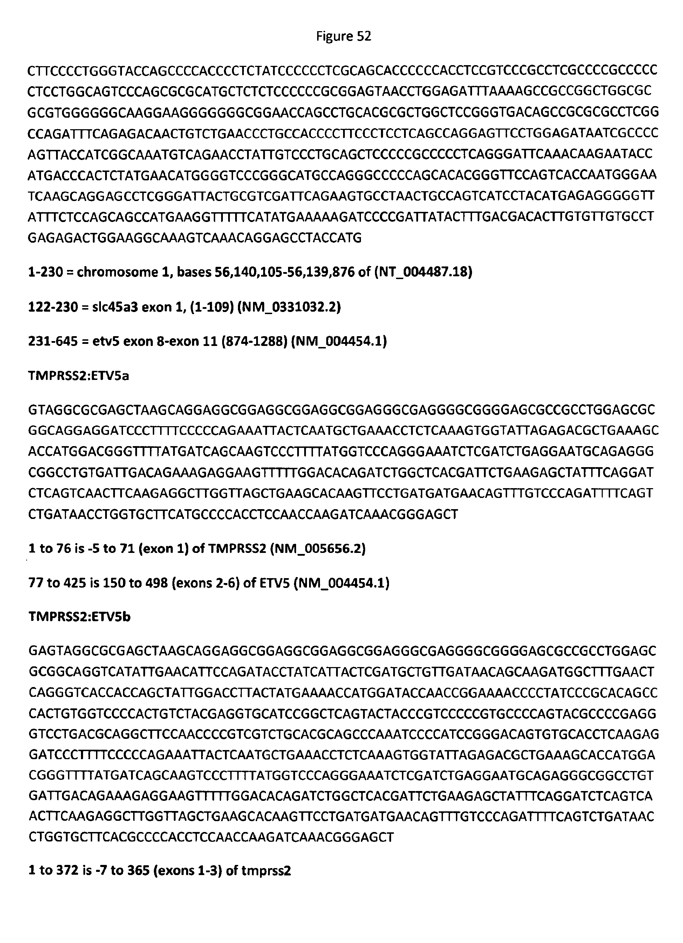

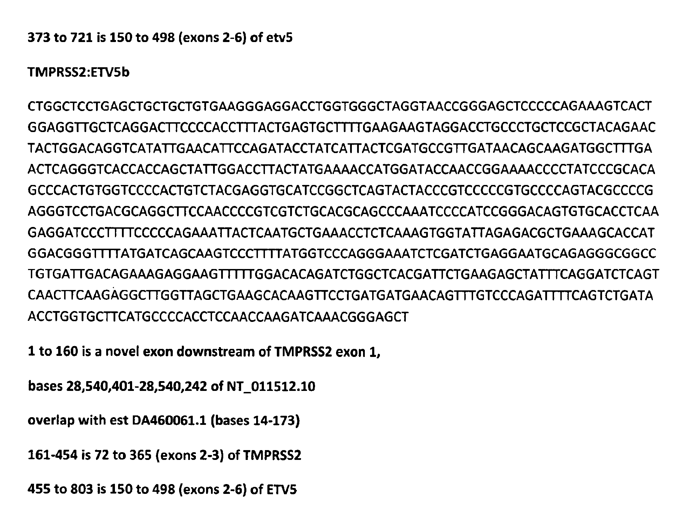

Figure 52 shows sequenes of further gene fusions.

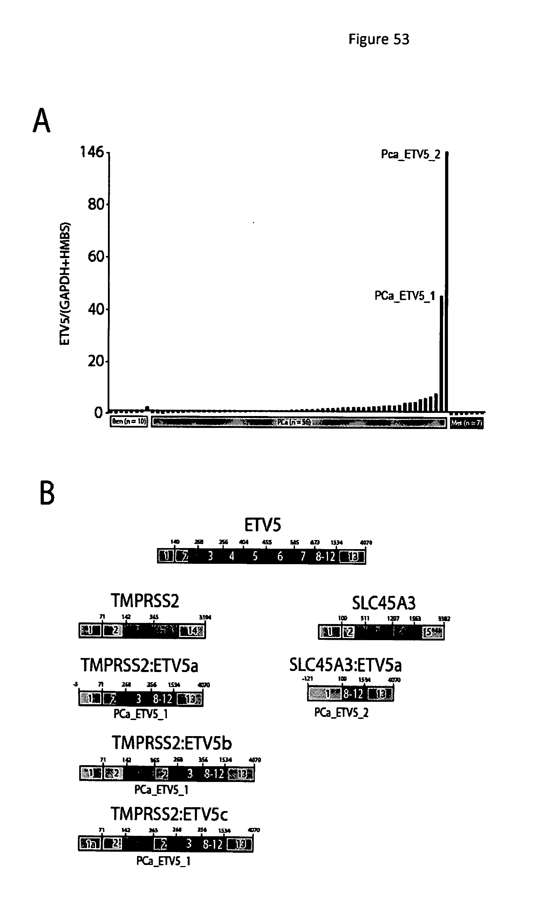

Figure 53 shows the identification of two prostate cancer (PCa) cases as showing ETV5 outlier expression by QPCR. Panel B shows the structure of the fusion transcripts for both cases, as determined by RACE.

### DEFINITIONS

To facilitate an understanding of the present invention, a number of terms and phrases are defined below:
As used herein, the term "gene fusion" refers to a chimeric genomic DNA, a chimeric messenger RNA, a truncated protein or a chimeric protein resulting from the fusion of at least a portion of a first gene to at least a portion of a second gene. The gene fusion need not include entire genes or exons of genes.

As used herein, the term "gene upregulated in cancer" refers to a gene that is expressed (e.g., mRNA or protein expression) at a higher level in cancer (e.g., prostate cancer) relative to the level in other tissues. In some embodiments, genes upregulated in cancer are expressed at a level at least 10%, preferably at least 25%, even more preferably at least 50%, still more preferably at least 100%, yet more preferably at least 200%, and most preferably at least 300% higher than the level of expression in other tissues. In some embodiments, genes upregulated in prostate cancer are "androgen regulated genes."

As used herein, the term "gene upregulated in prostate tissue" refers to a gene that is expressed (e.g., mRNA or protein expression) at a higher level in prostate tissue relative to the level in other tissue. In some embodiments, genes upregulated in prostate tissue are expressed at a level at least 10%, preferably at least 25%, even more preferably at least 50%, still more preferably at least 100%, yet more preferably at least 200%, and most preferably at least 300% higher than the level of expression in other tissues. In some embodiments, genes upregulated in prostate tissue are exclusively expressed in prostate tissue.

As used herein, the term "high expression promoter" refers to a promoter that when fused to a gene causes the gene to be expressed in a particular tissue (e.g., prostate) at a higher level (e.g, at a level at least 10%, preferably at least 25%, even more preferably at least 50%, still more preferably at least 100%, yet more preferably at least 200%, and most preferably at least 300% higher) than the level of expression of the gene when not fused to the high expression promoter. In some embodiments, high expression promoters are promoters from an androgen regulated gene or a housekeeping gene (e.g., HNRPA2B1).

As used herein, the term "transcriptional regulatory region" refers to the region of a gene comprising sequences that modulate (e.g., upregulate or downregulate) expression of the gene. In some embodiments, the transcriptional regulatory region of a gene comprises non-coding upstream sequence of a gene, also called the 5' untranslated region (5'UTR). In other embodiments, the transcriptional regulatory region contains sequences located within the coding region of a gene or within an intron (e.g., enhancers).

As used herein, the term "androgen regulated gene" refers to a gene or portion of a gene whose expression is induced or repressed by an androgen (*e.g.,* testosterone). The promoter region of an androgen regulated gene may contain an "androgen response element" that interacts with androgens or androgen signaling molecules (*e.g*., downstream signaling molecules).

As used herein, the terms "detect", "detecting" or "detection" may describe either the general act of discovering or discerning or the specific observation of a detectably labeled composition.

As used herein, the term "inhibits at least one biological activity of a gene fusion" refers to any agent that decreases any activity of a gene fusion of the present invention (*e.g*., including, but not limited to, the activities described herein), via directly contacting gene fusion protein, contacting gene fusion mRNA or genomic DNA, causing conformational changes of gene fusion polypeptides, decreasing gene fusion protein levels, or interfering with gene fusion interactions with signaling partners, and affecting the expression of gene fusion target genes. Inhibitors also include molecules that indirectly regulate gene fusion biological activity by intercepting upstream signaling molecules.

As used herein, the term "siRNAs" refers to small interfering RNAs. In some embodiments, siRNAs comprise a duplex, or double-stranded region, of about 18-25 nucleotides long; often siRNAs contain from about two to four unpaired nucleotides at the 3' end of each strand. At least one strand of the duplex or double-stranded region of a siRNA is substantially homologous to, or substantially complementary to, a target RNA molecule. The strand complementary to a target RNA molecule is the "antisense strand;" the strand homologous to the target RNA molecule is the "sense strand," and is also complementary to the siRNA antisense strand. siRNAs may also contain additional sequences; non-limiting examples of such sequences include linking sequences, or loops, as well as stem and other folded structures. siRNAs appear to function as key intermediaries in triggering RNA interference in invertebrates and in vertebrates, and in triggering sequence-specific RNA degradation during posttranscriptional gene silencing in plants.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

As used herein, the term "stage of cancer" refers to a qualitative or quantitative assessment of the level of advancement of a cancer. Criteria used to determine the stage of a cancer include, but are not limited to, the size of the tumor and the extent of metastases (*e.g*., localized or distant). technetium-99m, and indium-111 are preferable for gamma camera imaging, gallium-68 is preferable for posistron emission tomography.

A useful methods of labeling antibodies with such radiometals is by means of a bifunctional chelating agent, such as diethylenetriaminepentaacetic acid (DTPA), as described, for example, by Khaw et al. (Science 209;295 [1980]) for In-111 and Tc-99m, and by Scheinberg et al. (Science 215:1511 [1982]). Other chelating agents may also be used, but the 1-(p-carboxymethoxybenzyl)EDTA and the carboxycarbonic anhydride of DTPA are advantageous because their use permits conjugation without affecting the antibody's immunoreactivity substantially.

Another method for coupling DPTA to proteins is by use of the cyclic anhydride of DTPA, as described by Hnatowich et al. (Int J. Appl. Radiat. Isot. 35:327 [1982]) for labeling of albumin with In-111, but which can be adapted for labeling of antibodies. A suitable method of labeling antibodies with Tc-99m which does not use chelation with DPTA is the pretinning method of Crockford et al., (U.S. Pat. No. 4,323,546).

A preferred method of labeling immunoglobulins with Tc-99m is that described by Wong et al. (Int. J. Appl. Radiat. Isot., 29:251 [1978]) for plasma protein, and recently applied successfully by Wong et al. (J. Nucl. Med., 23:229 [1981]) for labeling anttbodies.

In the case of the radiometals conjugated to the specific antibody, it is likewise desirable to introduce as high a proportion of the radiolabel as possible into the antibody molecule without destroying its immunospecificity. A further improvement may be achieved by effecting radiolabeling in the presence of the specific cancer marker of the present invention, to insure that the antigen binding site on the antibody will be protected. The antigen is separated after labeling.

In still further embodiments, *in vivo* biophotonic imaging (Xenogen, Almeda, CA) is utilized for *in vivo* imaging. This real-time *in vivo* imaging utilizes luciferase. The luciferase gene is incorporated into cells, microorganisms, and animals (*e.g*., as a fusion protein with a cancer marker of the present invention). When active, it leads to a reaction that emits light. A CCD camera and software is used to capture the image and analyze it.

### F. Compositions & Kits

Compositions for use in the diagnostic methods of the present invention include, but are not limited to probes, amplification oligonucleotides, and antibodies. Particularly preferred composition detect a product only when an ARG or HG fuses to an ETS family member gene, These compositions include: a single tabeled probe comprising a sequence that hybridizes to the junction at which a 5' portion from a transcriptional regulatory region of an ARG or HG fuses to a 3' portion from an ETS family member gene (i.e., spans the gene fusion junction); a pair of amplification oligonucleotides wherein the first amplification oligonucleotide comprises a located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "heterologous gene" refers to a gene that is not in its natural environment. For example, a heterologous gene includes a gene from one species introduced into another species. A heterologous gene also includes a gene native to an organism that has been altered in some way (*e.g.,* mutated, added in multiple copies, linked to non-native regulatory sequences, etc). Heterologous genes are distinguished from endogenous genes in that the heterologous gene sequences are typically joined to DNA sequences that are not found naturally associated with the gene sequences in the chromosome or are associated with portions of the chromosome not found in nature (*e.g.,* genes expressed in loci where the gene is not normally expressed).

As used herein, the term "oligonucleotide," refers to a short length of single-stranded polynucleotide chain. Oligonucleotides are typically less than 200 residues long (*e.g.,* between 15 and 100), however, as used herein, the term is also intended to encompass longer polynucleotide chains. Oligonucleotides are often referred to by their length. For example a 24 residue oligonucleotide is referred to as a "24-mer". Oligonucleotides can form secondary and tertiary structures by self-hybridizing or by hybridizing to other polynucleotides. Such structures can include, but are not limited to, duplexes, hairpins, cruciforms, bends, and triplexes.

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "homology" refers to a degree of complementarity. There may be partial homology or complete homology (*i.e.,* identity). A partially complementary sequence is a nucleic acid molecule that at least partially inhibits a completely complementary nucleic acid molecule from hybridizing to a target nucleic acid is "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.,* the hybridization) of a completely homologous nucleic acid molecule to a target under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target that is substantially non-complementary (*e.g.,* less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

When used in reference to a single-stranded nucleic acid sequence, the term "substantially homologous" refers to any probe that can hybridize (*i.e.,* it is the complement of) the single-stranded nucleic acid sequence under conditions of low stringency as described above.

As used herein, the term "hybridization" is used in reference to the pairing of complementary nucleic acids. Hybridization and the strength of hybridization (*i.e.,* the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein the term "stringency" is used in reference to the conditions of temperature, ionic strength, and the presence of other compounds such as organic solvents, under which nucleic acid hybridizations are conducted. Under "low stringency conditions" a nucleic acid sequence of interest will hybridize to its exact complement, sequences with single base mismatches, closely related sequences (*e.g.,* sequences with 90% or greater homology), and sequences having only partial homology (*e.g.,* sequences with 50-90% homology). Under 'medium stringency conditions," a nucleic acid sequence of interest will hybridize only to its exact complement, sequences with single base mismatches, and closely relation sequences (*e.g*., 90% or greater homology). Under "high stringency conditions," a nucleic acid sequence of interest will hybridize only to its exact complement, and (depending on conditions such a temperature) sequences with single base mismatches. In other words, under conditions of high stringency the temperature can be raised so as to exclude hybridization to sequences with single base mismatches.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1% SDS at 42°C when a probe of about 500 nucleotides in length is employed.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.,* the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g.,* increasing the temperature of the hybridization and/or wash steps, the use of formamide in the hybridization solution, etc.) (see definition above for "stringency").

As used herein, the term "amplification oligonucleotide" refers to an oligonucleotide that hybridizes to a target nucleic acid, or its complement, and participates in a nucleic acid amplification reaction. An example of an amplification oligonucleotide is a "primer" that hybridizes to a template nucleic acid and contains a 3' OH end that is extended by a polymerase in an amplification process. Another example of an amplification oligonucleotide is an oligonucleotide that is not extended by a polymerase (*e.g.,* because it has a 3' blocked end) but participates in or facilitates amplification. Amplification oligonucleotides may optionally include modified nucleotides or analogs, or additional nucleotides that participate in an amplification reaction but are not complementary to or contained in the target nucleic acid. Amplification oligonucleotides may contain a sequence that is not complementary to the target or template sequence. For example, the 5' region of a primer may include a promoter sequence that is non-complementary to the target nucleic acid (referred to as a "promoter-primer"). Those skilled in the art will understand that an amplification oligonucleotide that functions as a primer may be modified to include a 5' promoter sequence, and thus function as a promoter-primer. Similarly, a promoter-primer may be modified by removal of, or synthesis without, a promoter sequence and still function as a primer. A 3' blocked amplification oligonucleotide may provide a promoter sequence and serve as a template for polymerization (referred to as a "promoter-provider").

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e*., a sequence of nucleotides), whether occurring naturally as in a purified restriction digest or produced synthetically, recombinantly or by PCR amplification, that is capable of hybridizing to at least a portion of another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g.,* ELISA, as well as enzyme-based histochemical assays), fluorescent, radioactive, and luminescent systems. It is not intended that the present invention be limited to any particular detection system or label.

The term "isolated" when used in relation to a nucleic acid, as in "an isolated oligonucleotide" or "isolated polynucleotide" refers to a nucleic acid sequence that is identified and separated from at least one component or contaminant with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. For example, a given DNA sequence (*e.g.,* a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid encoding a given protein includes, by way of example, such nucleic acid in cells ordinarily expressing the given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand (*i.e.,* the oligonucleotide or polynucleotide may be single-stranded), but may contain both the sense and anti-sense strands (*i.e.,* the oligonucleotide or polynucleotide may be double-stranded).

As used herein, the term "purified" or "to purify" refers to the removal of components (*e.g*., contaminants) from a sample. For example, antibodies are purified by removal of contaminating non-immunoglobulin proteins; they are also purified by the removal of immunoglobulin that does not bind to the target molecule. The removal of non-immunoglobulin proteins and/or the removal of immunoglobulins that do not bind to the target molecule results in an increase in the percent of target-reactive immunoglobulins in the sample. In another example, recombinant polypeptides are expressed in bacterial host cells and the polypeptides are purified by the removal of host cell proteins; the percent of recombinant polypeptides is thereby increased in the sample.

### DETAILED DESCRIPTION

The present invention is based on the discovery of recurrent gene fusions in prostate cancer. The present invention provides diagnostic, research methods that either directly or indirectly detect or target the gene fusions. The present invention also provides compositions for diagnostic, research, and therapeutic purposes.

### I. Gene Fusions

The gene fusions are the result of a chromosomal rearrangement of an androgen regulated gene (ARG) or housekeeping gene (HG) and an ETS family member gene. Despite their recurrence, the junction where the ARG or HG fuses to the ETS family member gene varies. The gene fusions typically comprise a 5' portion from a transcriptional regulatory region of an ARG or HG and a 3' portion from an ETS family member gene. The recurrent gene fusions have use as diagnostic markers and clinical targets for prostate cancer.

### A. Androgen Regulated Genes

Genes regulated by androgenic hormones are of critical importance for the normal physiological function of the human prostate gland. They also contribute to the development and progression of prostate carcinoma. Recognized ARGs include, but are not limited to: TMPRSS2; SLC45A3; HERV-K_22q11.23; C150RF21; PSA; PSMA; KLK2; SNRK; Seladin-1; and, FKBP51 (Paoloni-Giacobino et al., Genomics 44: 309 (1997); Velasco et al., Endocrinology 145(8): 3913 (2004)).

TMPRSS2 (NM_005656) has been demonstrated to be highly expressed in prostate epithelium relative to other normal human tissues (Lin et al., Cancer Research 59: 4180 (1999)). The TMPRSS2 gene is located on chromosome 21. This gene is located at 41,750,797-41,801,948 bp from the pter (51,151 total bp; minus strand orientation). The human TMPRSS2 protein sequence may be found at GenBank accession no. AAC51784 (Swiss Protein accession no. 015393) and the corresponding cDNA at GenBank accession no. U75329 (see also, Paoloni-Giacobino, et al., Genomics 44: 309 (1997)).

SLC45A3, also known as prostein or P501S, has been shown to be exclusively expressed in normal prostate and prostate cancer at both the transcript and protein level (Kalos et al., Prostate 60, 246-56 (2004); Xu et al., Cancer Res 61, 1563-8 (2001)).

HERV-K_22q11.23, by EST analysis and massively parallel sequencing, was found to be the second most strongly expressed member of the HERV-K family of human endogenous retroviral elements and was most highly expressed in the prostate compared to other normal tissues (Stauffer et al., Cancer Immun 4, 2 (2004)). While androgen regulation of HERV-K elements has not been described, endogenous retroviral elements have been shown to confer androgen responsiveness to the mouse sex-linked protein gene C4A (Stavenhagen et al., Cell 55, 247-54 (1988)). Other HERV-K family members have been shown to be both highly expressed and estrogen-regulated in breast cancer and breast cancer cell lines (Ono et al., J Virol 61, 2059-62 (1987); Patience et al., J Virol 70, 2654-7 (1996); Wang-Johanning et al., Oncogene 22, 1528-35 (2003)), and sequence from a HERV-K3 element on chromosome 19 was fused to FGFR1 in a case of stem cell myeloproliferative disorder with t(8;19)(p12;q13.3) (Guasch et al., Blood 101, 286-8 (2003)).

C15ORF21, also known as D-PCA-2, was originally isolated based on its exclusive over-expression in normal prostate and prostate cancer (Weigle et al., Int J Cancer 109, 882-92 (2004)).

Gene fusions of the present invention may comprise transcriptional regulatory regions. The transcriptional regulatory region of an ARG may contain coding or non-coding regions of the ARG, including the promoter region. The promoter region of the ARG may further comprise an androgen response element (ARE) of the ARG. The promoter region for TMPRSS2, in particular, is provided by GenBank accession number AJ276404.

### B. Housekeeping Genes

Housekeeping genes are constitutively expressed and are generally ubiquitously expressed in all tissues. These genes encode proteins that provide the basic, essential functions that all cells need to survive. Housekeeping genes are usually expressed at the same level in all cells and tissues, but with some variances, especially during cell growth and organism development. It is unknown exactly how many housekeeping genes human cells have, but most estimates are in the range from 300-500.

Many of the hundreds of housekeeping genes have been identified. The most commonly known gene, GAPDH (glyceraldehyde-3-phosphate dehydrogenase), codes for an enzyme that is vital to the glycolytic pathway. Another important housekeeping gene is albumin, which assists in transporting compounds throughout the body. Several housekeeping genes code for structural proteins that make up the cytoskeleton such as beta-actin and tubulin. Others code for 18S or 28S rRNA subunits of the ribosome. HNRPA2B1 is a member of the ubiquitously expressed heteronuclear ribonuclear proteins. Its promoter has been shown to be unmetheylated and prevents transcriptional silencing of the CMV promoter in transgenes (Williams et al., BMC Biotechnol 5, 17 (2005)). An exemplary listing of housekeeping genes can be found, for example, in Trends in Genetics, 19, 362-365 (2003).

### C. ETS Family Member Genes

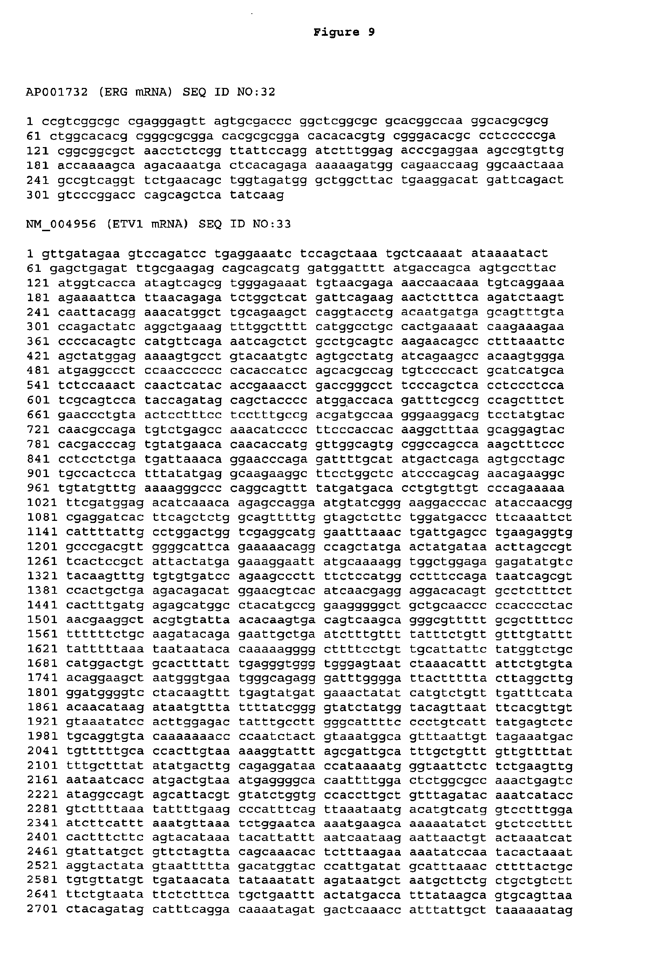

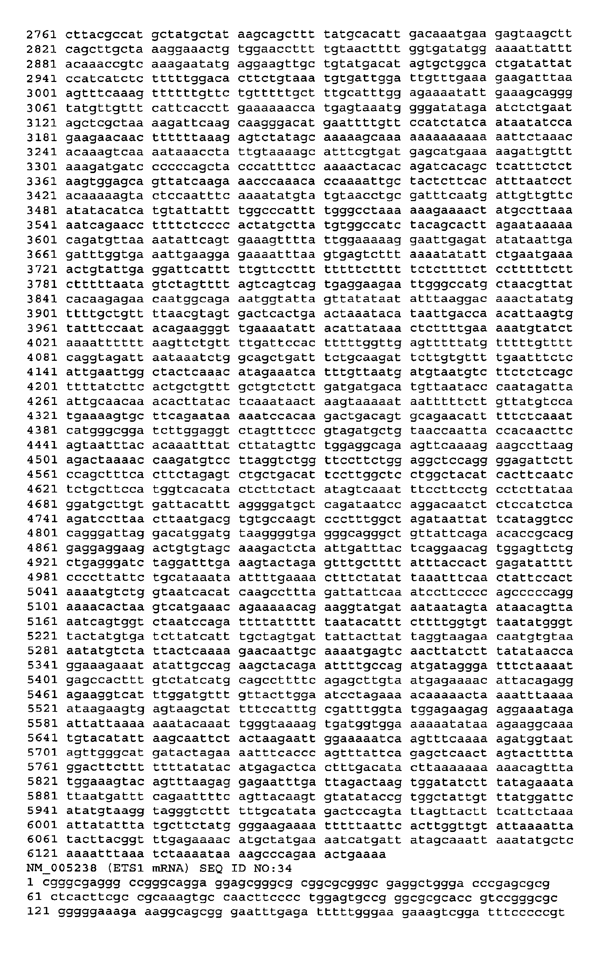

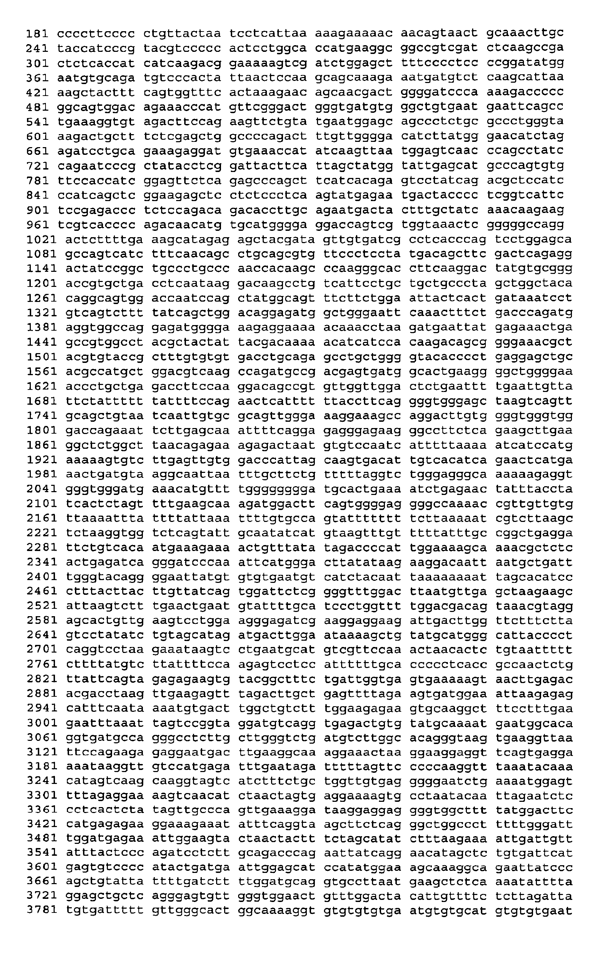

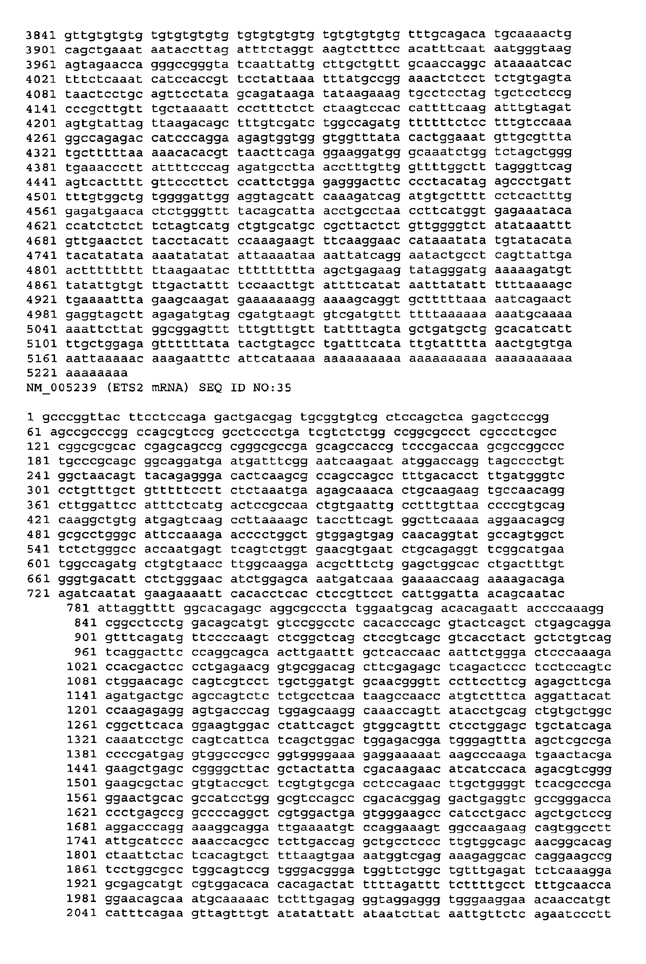

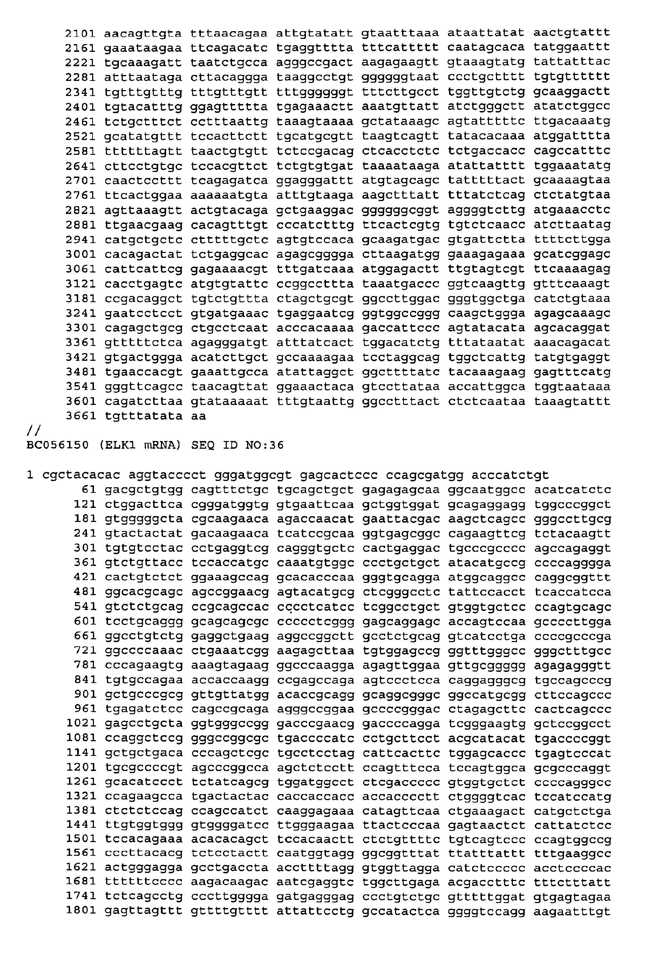

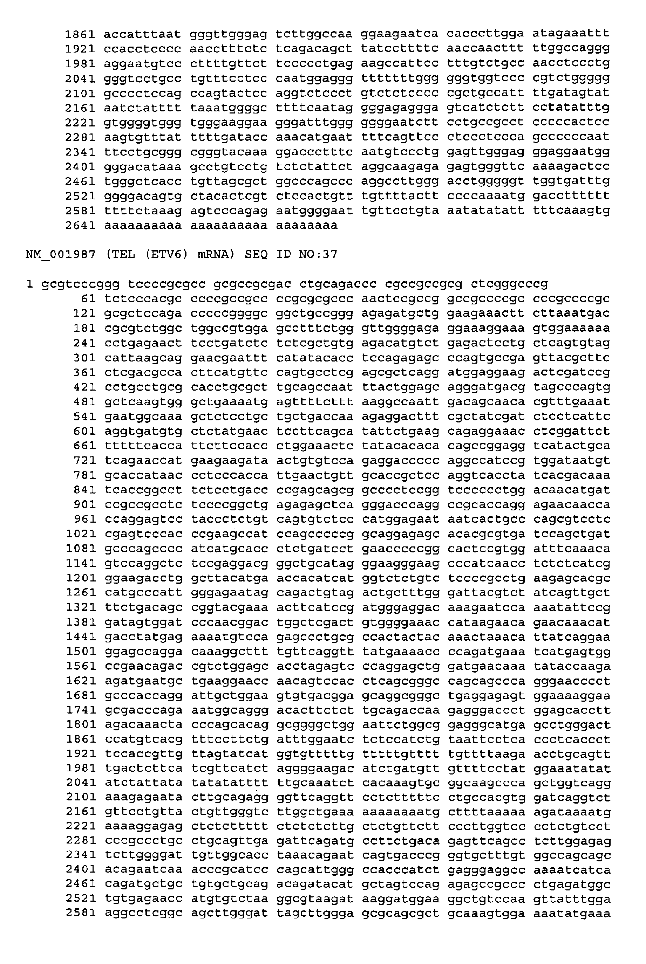

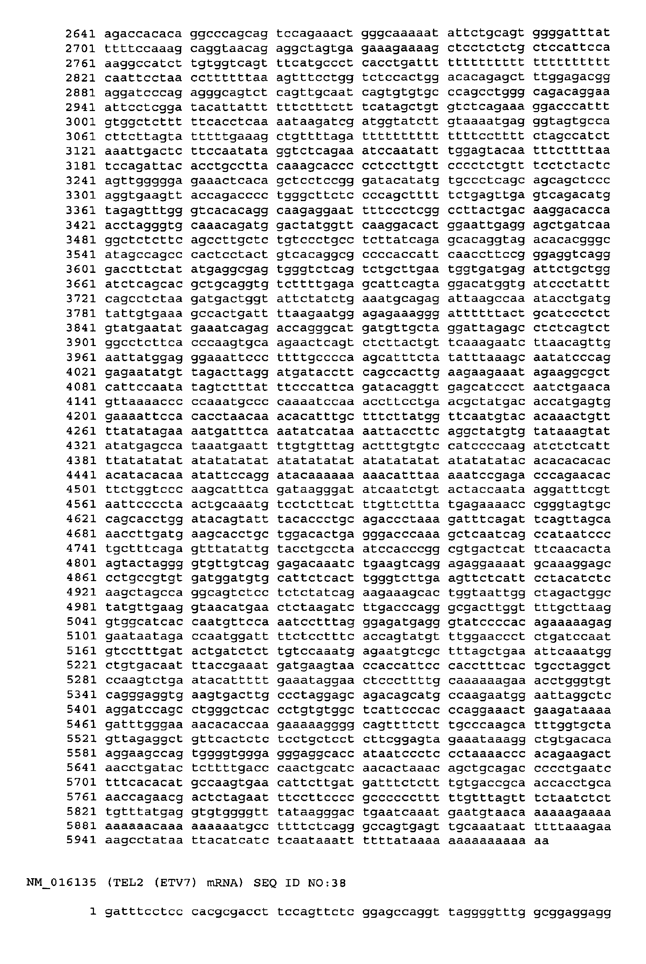

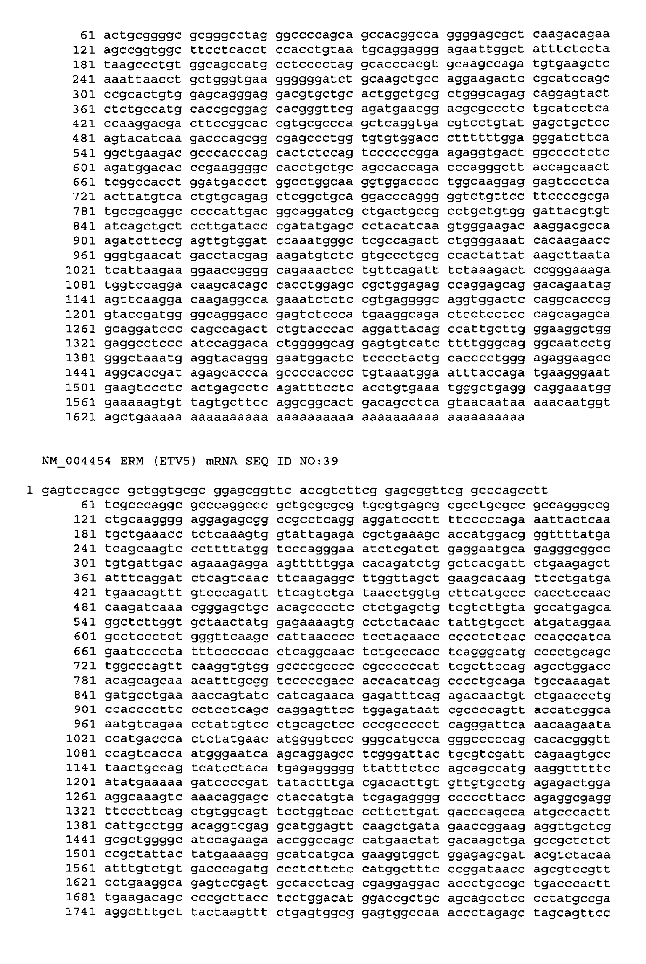

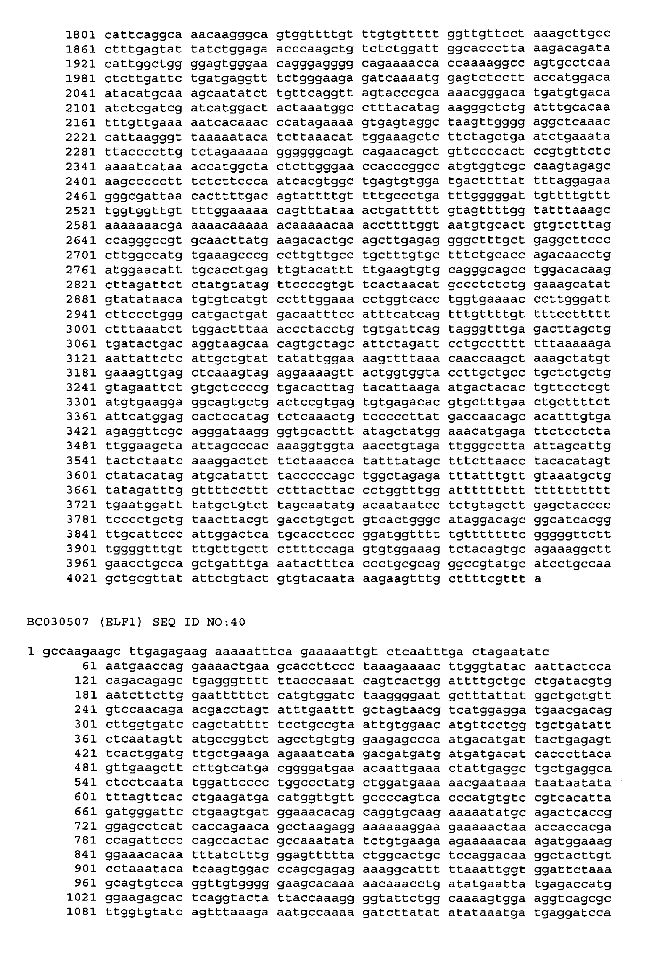

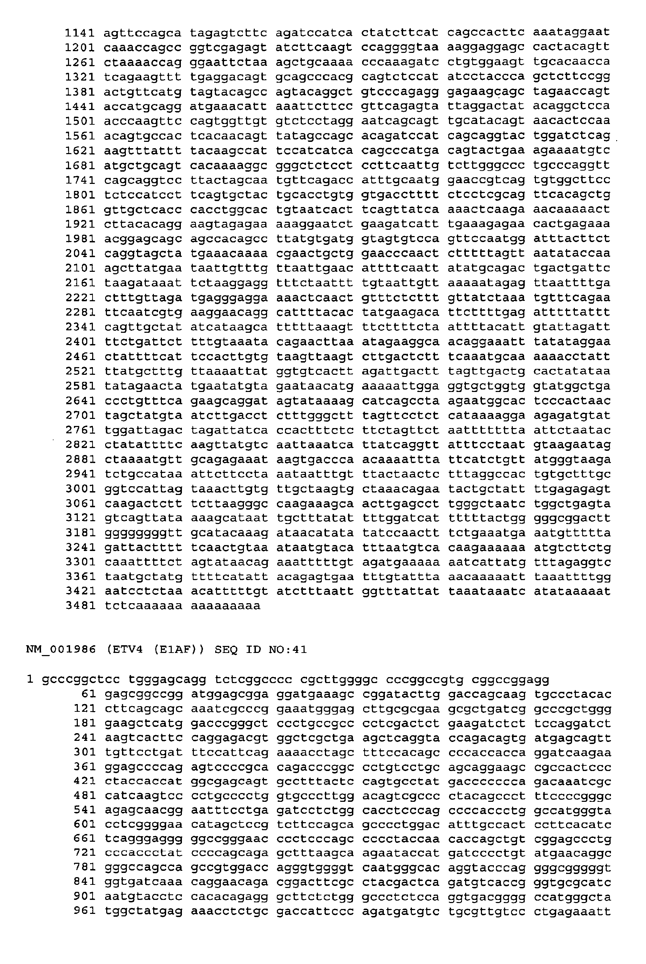

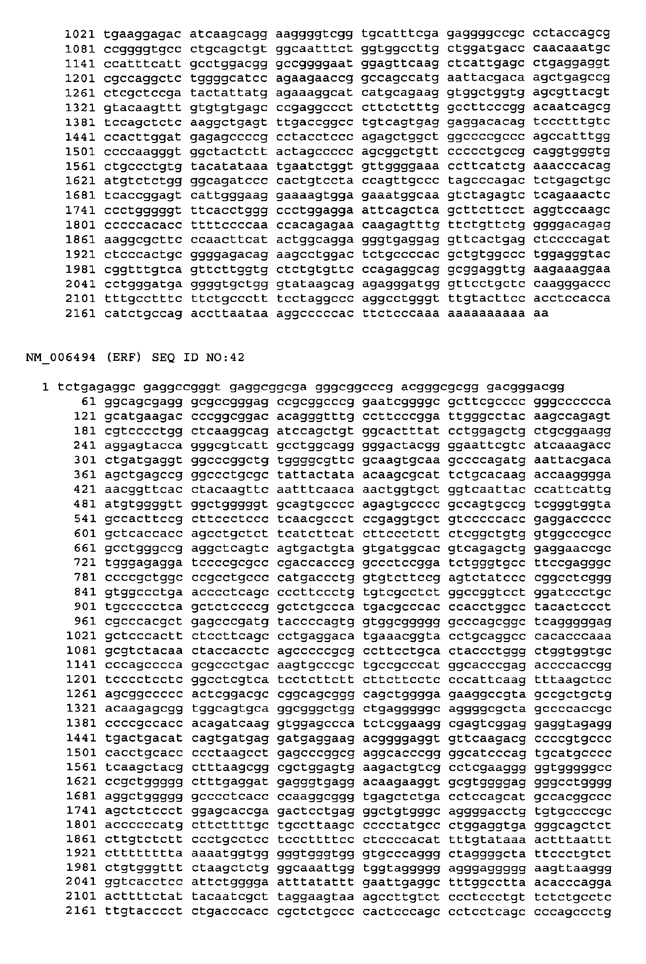

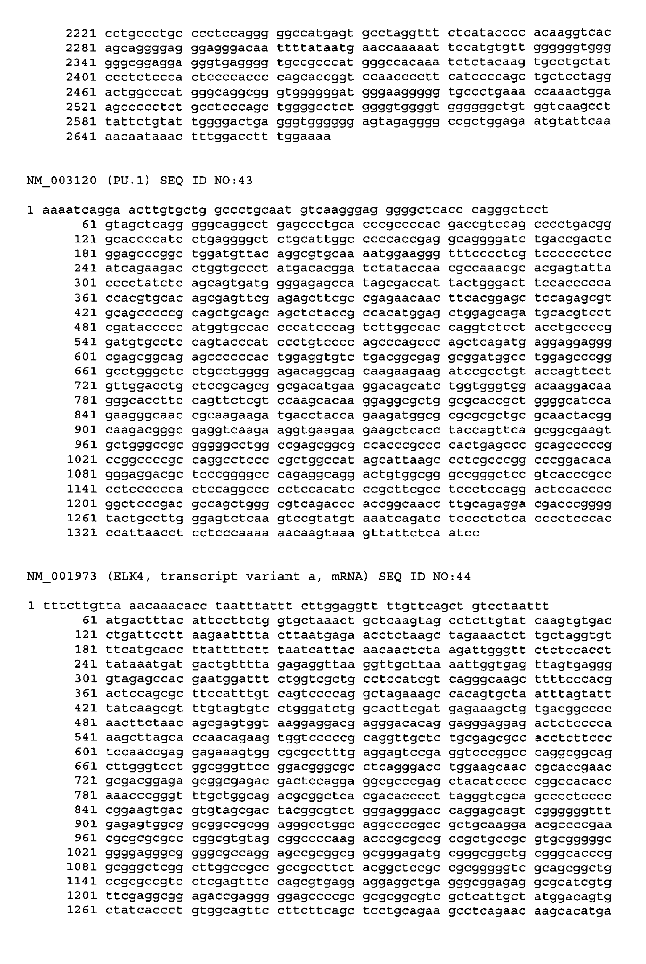

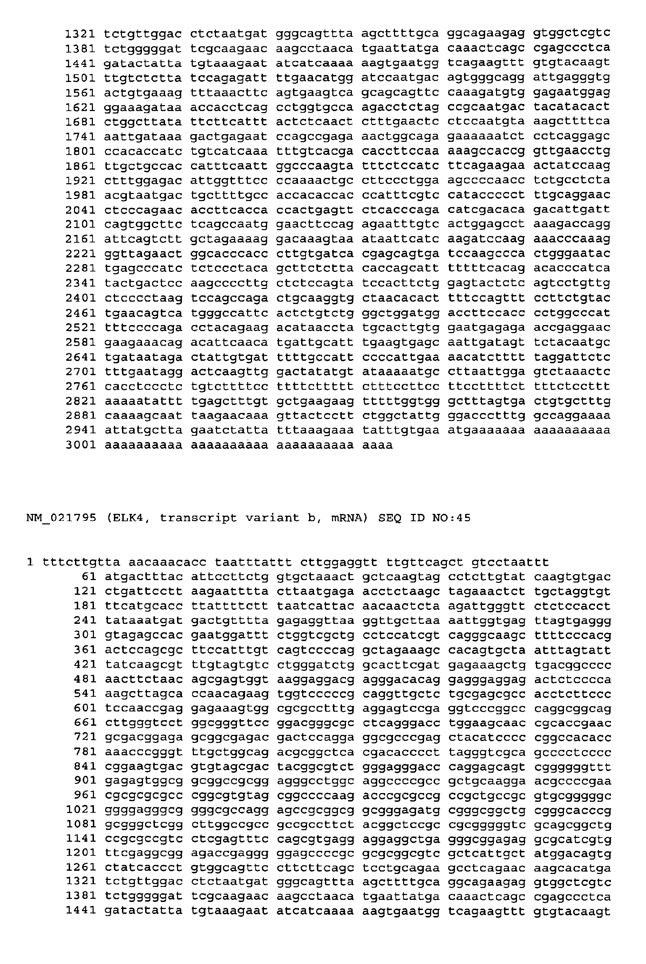

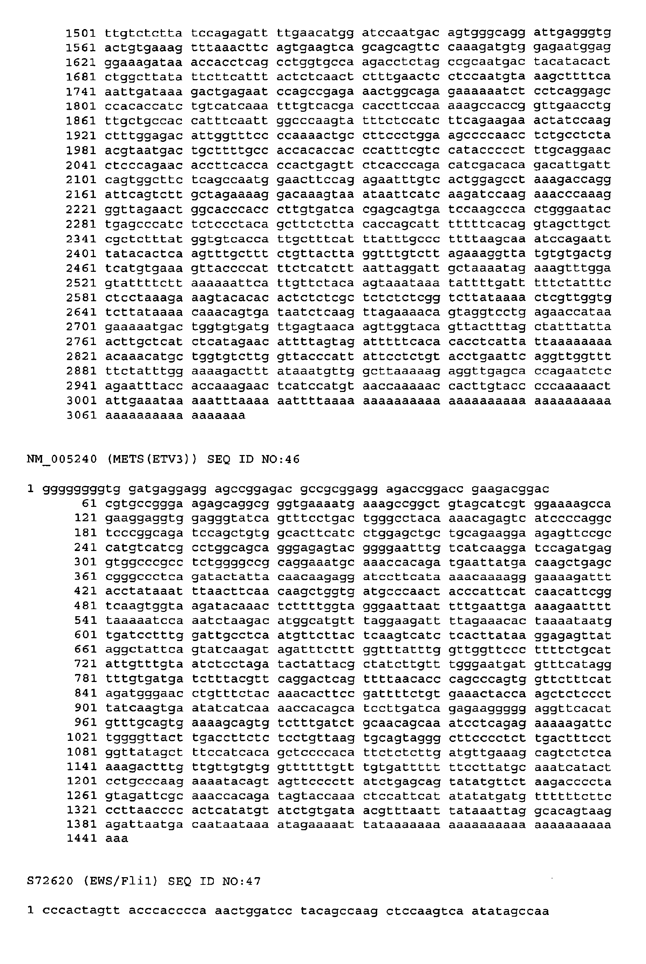

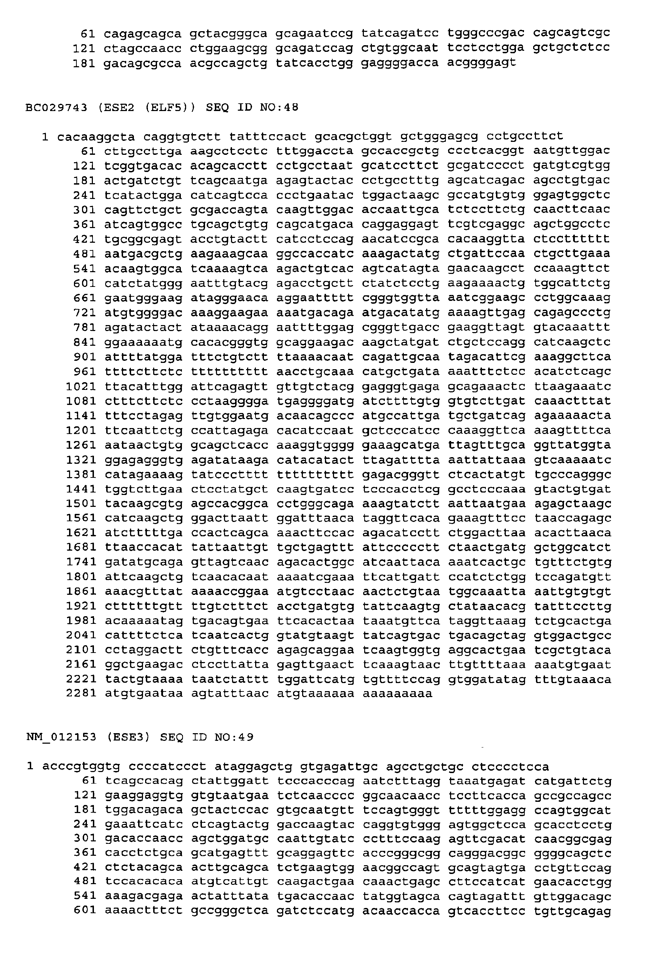

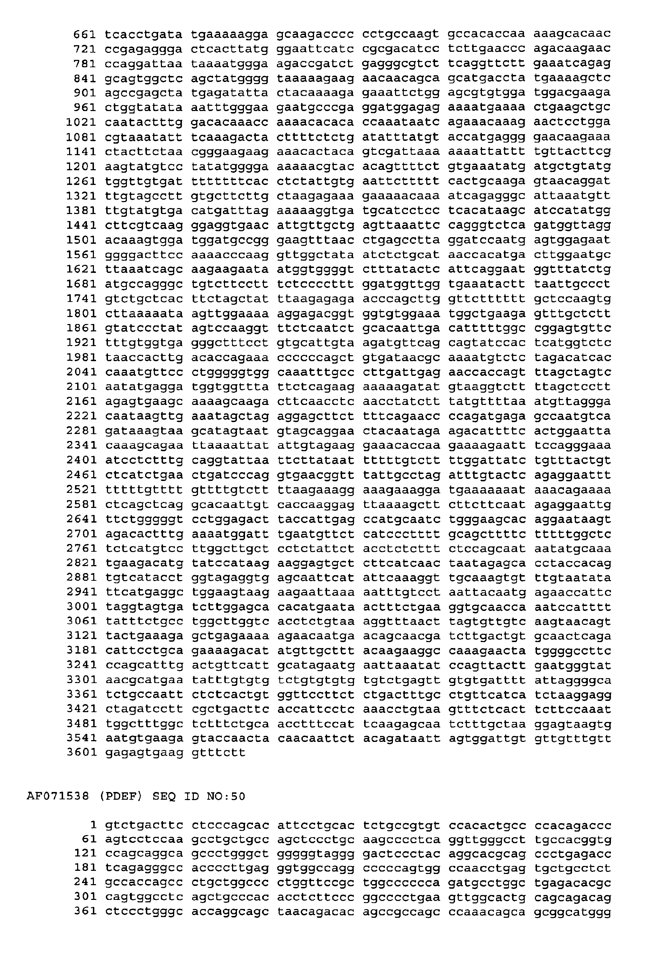

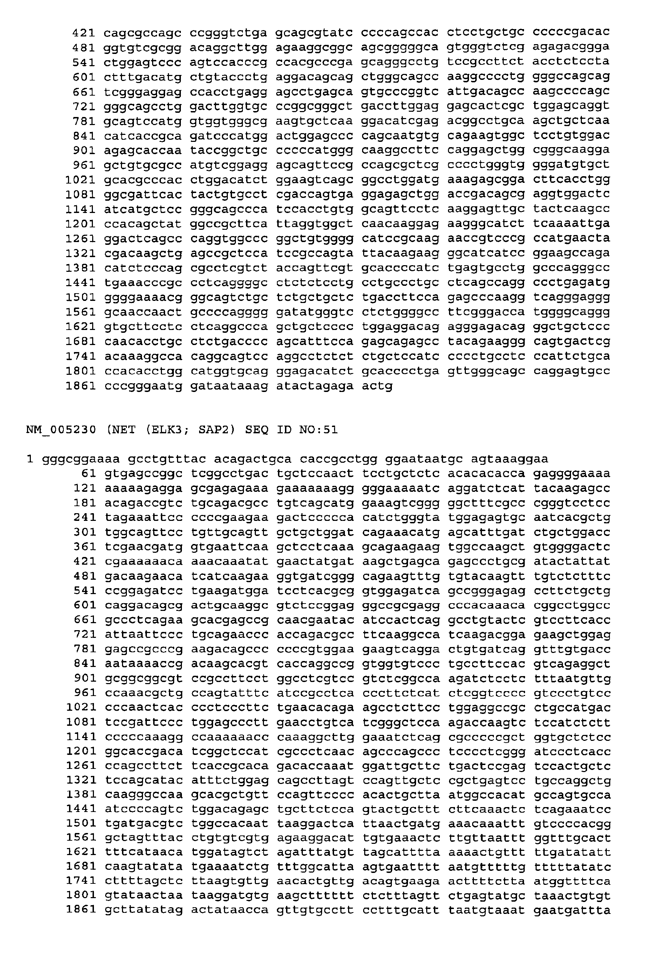

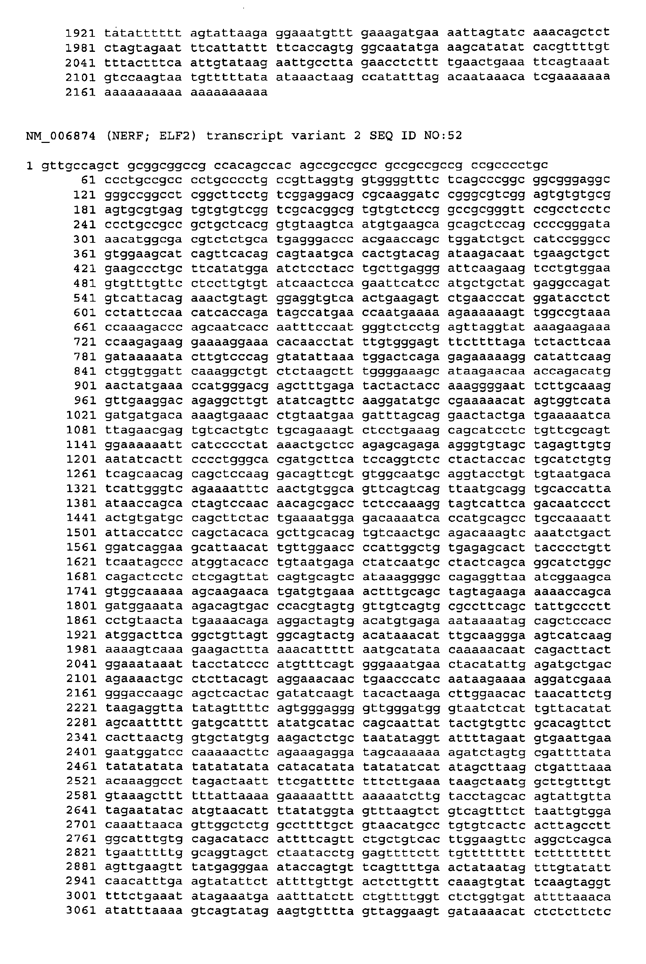

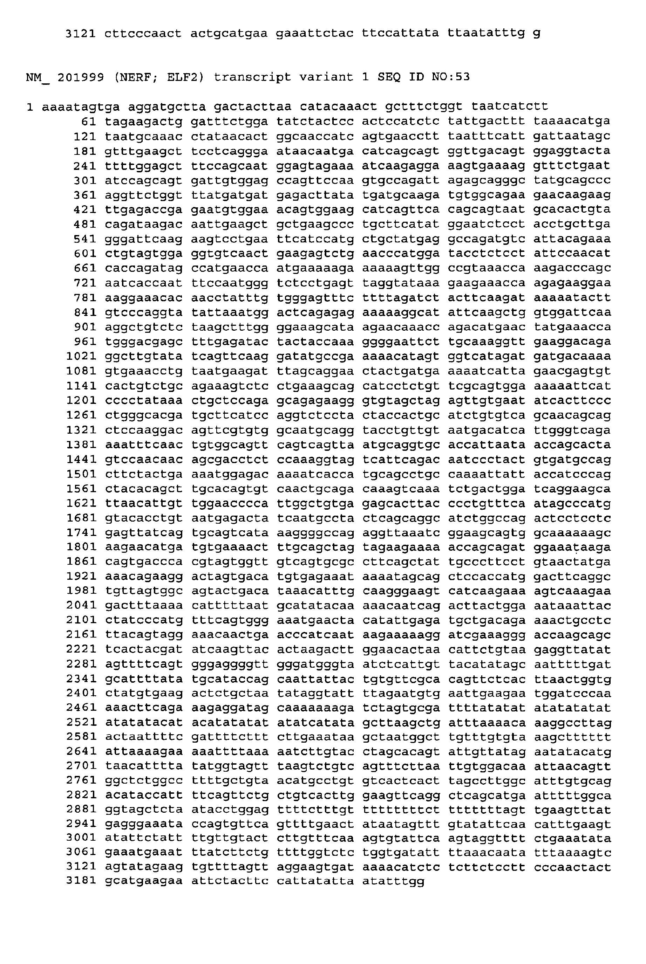

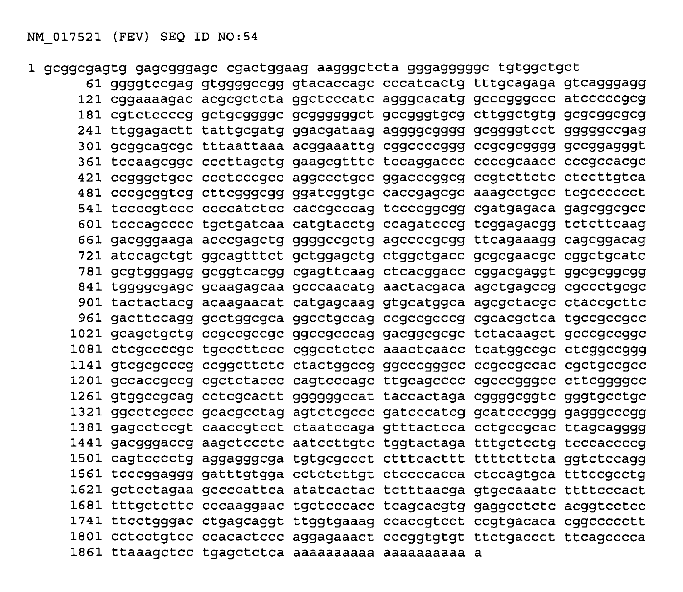

The ETS family of transcription factors regulate the intra-cellular signaling pathways controlling gene expression. As downstream effectors, they activate or repress specific target genes. As upstream effectors, they are responsible for the spacial and temporal expression of numerous growth factor receptors. Almost 30 members of this family have been identified and implicated in a wide range of physiological and pathological processes. These include, but are not limited to: ERG; ETV1 (ER81); FLI1; ETS1; ETS2; ELK1; ETV6 (TEL1); ETV7 (TEL2); GABPα; ELF1; ETV4 (E1AF; PEA3); ETV5 (ERM); ERF; PEA3/E1AF; PU.1; ESE1/ESX; SAP1 (ELK4); ETV3 (METS); EWS/FLI1; ESE1; ESE2 (ELF5); ESE3; PDEF; NET (ELK3; SAP2); NERF (ELF2); and FEV. Exemplary ETS family member sequences are given in Figure 9.

ERG (NM_004449) has been demonstrated to be highly expressed in prostate epithelium relative to other normal human tissues. The ERG gene is located on chromosome 21. The gene is located at 38,675,671- 38,955,488 base pairs from the pter. The ERG gene is 279,817 total bp minus strand orientation. The corresponding ERG cDNA and protein sequences are given at GenBank accesssion nos. M17254 and NP04440 (Swiss Protein acc. no. P11308), respectively.

The ETV1 gene is located on chromosome 7 (GenBank accession nos. NC_000007.11; NC_086703.11; and NT_007819.15). The gene is located at 13,708330 - 13,803,555 base pairs from the pter. The ETV1 gene is 95,225 bp total, minus strand orientation. The corresponding ETV1 cDNA and protein sequences are given at GenBank accession nos. NM_004956 and NP_004947 (Swiss protein acc. no. P50549), respectively.

The human ETV4 gene is located on chromosome 14 (GenBank accession nos. NC_000017.9; NT_010783.14; and NT_086880.1). The gene is at 38,960,740 - 38,979,228 base pairs from the pter. The ETV4 gene is 18,488 bp total, minus strand orientation. The corresponding ETV4 cDNA and protein sequences are given at GenBank accession nos. NM_001986 and NP_01977 (Swiss protein acc. no. P43268), respectively.

The human ETV5 gene is located on chromosome 3 at 3q28 (NC_000003.10 (187309570..187246803). The corresponding ETV5 mRNA and protein sequences are given by GenBank accession nos. NM_004454 and CAG33048, respectivly.

### D. ETS Gene Fusions

Including the initial identification of TMPRSS2:ETS gene fusions, five classes of ETS rearrangements in prostate cancer have been identified (Fig 43). The present invention is not limited to a particular mechanism. Indeed, an understanding of the mechanism is not necessary to practice the present invention. Nonetheless, it is contemplated that upregulated expression of ETS family members via fusion with an ARG or HG or insertion into a locus with increased expression in cancer provides a mechanism for prostate cancers. Knowledge of the class of rearrangement present in a particular individual allows for customized cancer therapy.

### 1. Classes of Gene Rearrangements

TMPRSS2:ETS gene fusions (Class I) represent the predominant class of ETS rearrangements in prostate cancer. Rearrangements involving fusions with untranslated regions from other prostate-specific androgen-induced genes (Class IIa) and endogenous retroviral elements (Class IIb), such as SLC45A3 and HERV-K_22q11.23 respectively, function similarly to TMRPSS2 in ETS rearrangements. Similar to the 5' partners in class I and II rearrangements, C15ORF21 is markedly over-expressed in prostate cancer. However, unlike fusion partners in class I and II rearrangements, C15ORF21 is repressed by androgen, representing a novel class of ETS rearrangements (Class III) involving prostate-specific androgen-repressed 5' fusion partners. By contrast, HNRPA2B1 did not show prostate-specific expression or androgen-responsiveness. Thus, HNRPA2B1:ETV1 represents a novel class of ETS rearrangements (Class IV) where fusions involving non-tissue specific promoter elements drive ETS expression. In Class V rearrangements, the entire ETS gene is rearranged to prostate-specific regions.

Men with advanced prostate cancer are commonly treated with androgen-deprivation therapy, usually resulting in tumor regression. However the cancer almost invariably progresses with a hormone-refractory phenotype. As Class IV rearrangements (such as HNRPA2B1:ETV1) are driven by androgen insensitive promoter elements, the results indicate that these patients may not respond to anti-androgen treatment, as these gene fusions would not be responsive to androgen-deprivation. Anti-androgen treatment of patients with Class III rearrangements may increase ETS fusion expression. For example, C15ORF21:ETV1 was isolated from a patient with hormone-refractory metastatic prostate cancer where anti-androgen treatment increased C15ORF21:ETV1 expression. Supporting this hypothesis, androgen starvation of LNCaP significantly decreased the expression of endogenous PSA and TMPRSS2, had no effect on HNRPA2B1, and increased the expression of C15ORF21 (Fig 49). This allows for customized treatment of men with prostate cancer based on the class of fusion present (e.g., the choice of androgen blocking therapy or other alternative therapies).

Multiple classes of gene rearrangements in prostate cancer indicate a more generalized role for chromosomal rearrangements in common epithelial cancers. For example, tissue specific promoter elements may be fused to oncogenes in other hormone driven cancers, such as estrogen response elements fused to oncogenes in breast cancer. Additionally, while prostate specific fusions (Classes I-III,V) would not provide a growth advantage and be selected for in other epithelial cancers, fusions involving strong promoters of ubiquitously expressed genes, such as HNRPA2B1, result in the aberrant expression of oncogenes across tumor types. In summary, this study supports a role for chromosomal rearrangements in common epithelial tumor development through a variety of mechanisms, similar to hematological malignancies.

### 2. ARG/ETS Gene Fusions

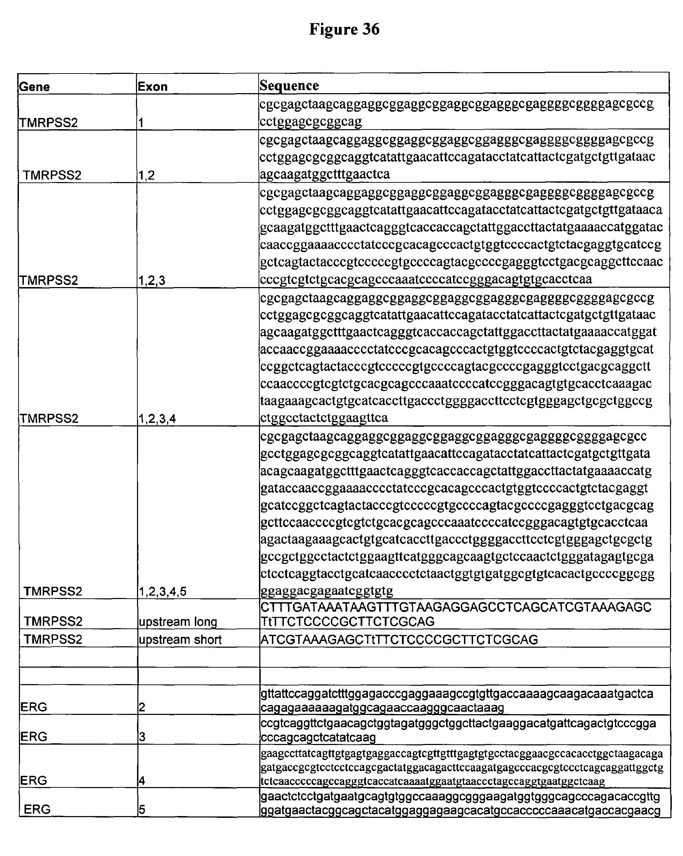

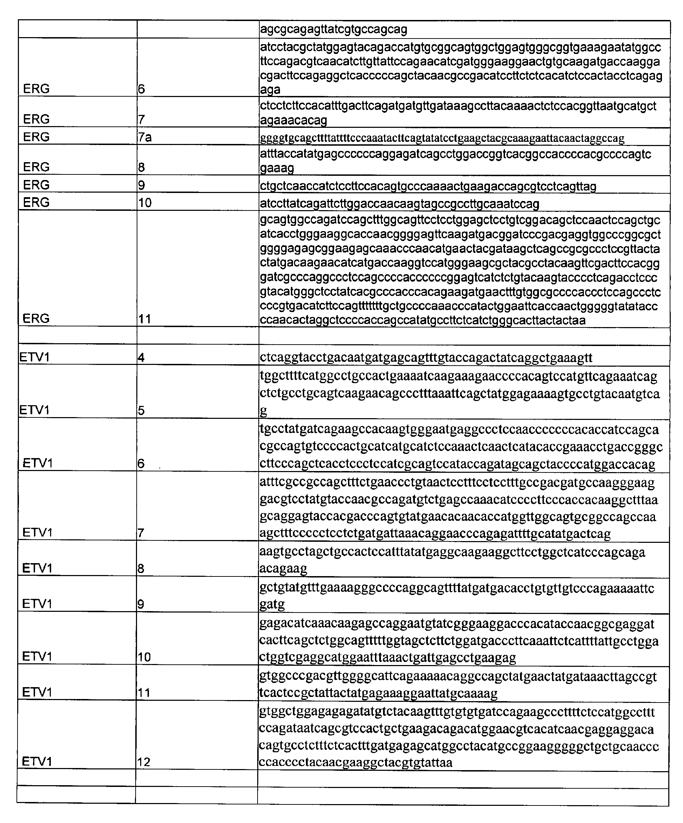

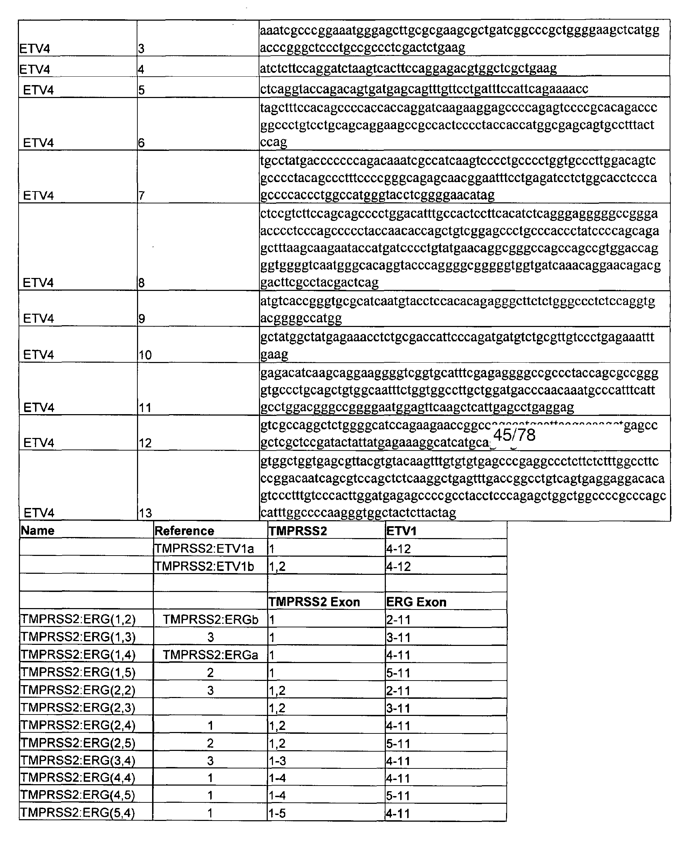

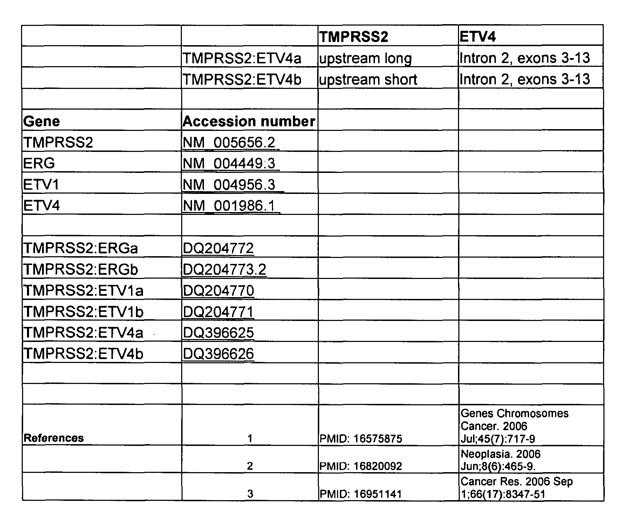

As described above, the present disclosure provides fusions of an ARG to an ETS family member gene. Exemplary gene fusion sequences are given in Figure 36, 51 and 52. For TMPRSS2, ERG, ETV1 and ETV4, the GenBank reference sequence ID's are provided and the exons are aligned using the May 2004 assembly of the UCSC Human Genome. For all identified fusions, Figure 36 provides a complete sequence from the beginning of the TMPRSS2 gene through the fusion and the stop codon of the ETS family member gene. The deposited GenBank sequence for each of the published variants is also provided. Some TMPRSS2:ERG and TMPRSS2:ETV1 fusions are described by the breakpoint exons of TMPRSS2 and the ETS family member gene. For example, TMPRSS2:ERGa, which fuses exon 1 of TMPRSS2 to exons 4 through 11 of ERG, is identified as TMPRSS2:ERG(1,4).

Certain gene fusions are more common than others in prostate cancer. The disclosed method identifies 50-80% of prostate cancers as having recurrent gene fusions of TMPRSS2 with ERG, ETV1, ETV4 or FLI1. Of those, 50-70% are TMPRSS2-ERG, 50%-60% of which result from the deletion of genetic information between the TMPRSS2 and ERG locus on chromosome 21 (described in more detail below), 5-10% are TMPRSS2-ETV1, 1-2% are TMPRSS2-ETV4, and 1-2% are TMPRSS2-FLI1.

Experiments conducted during the course of development of the present invention indicated that certain fusion genes express fusion transcripts, while others do not express a functional transcript (Tomlins et al., Science, 310: 644-648 (2005); Tomlins et al., Cancer Research 66: 3396-3400 (2006)).

### a. ERG Gene Fusions

As described above, gene fusions comprising ERG were found to be the most common gene fusions in prostate cancer. Experiments conducted during the course of development of the present invention identified significant genomic deletions located between *TMPRSS2* and *ERG* on chromosome 21q22.2-3. Deletions were seen in *TMPRSS2:ERG* fusion positive PCA samples. The deletions appear in a consensus area but show variability within this area. In previously published work by Paris et al. (Hum. Mol. Genet. 13:1303-13 (2004)), CGH analysis detected deletions in the CTD-2103O7 BAC that is 6 kb centromeric from TMPRSS2. These deletions were observed in 12.5% (9/72) of clinically localized PCA samples and 33% (5/15) of the metastatic PCA samples. These results support the SNP array data from the current study and indicates that either PCA deletions become more common with progression or that deletions are identified more often in PCA that tend to progress more rapidly. Given the striking intra-tumoral homogeneity of the TMPRSS2:ERG rearrangements, it is more likely that these molecular subtypes are associated with different disease progression characteristics.

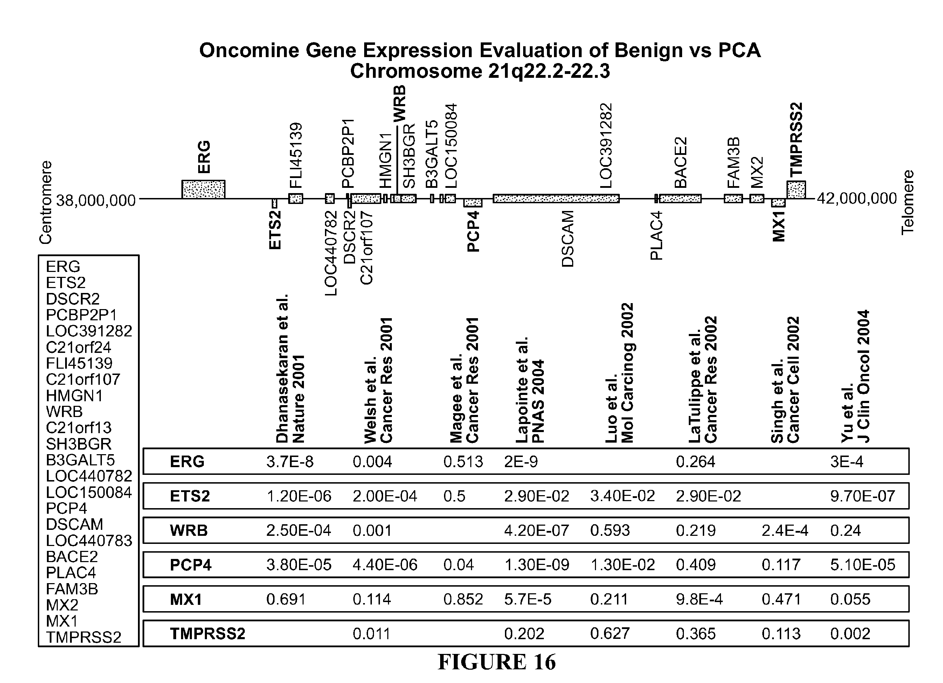

One hundred eighteen clinically localized PCA cases with 49.2% harboring rearrangement of ERG were evaluated. Intronic deletions were observed in 60.3% of these TMPRSS2:ERG fusion positive cases. Almost all PCA samples with marked over expression of ERG have a rearrangement, and the over expression occurs in about the same number of cases as the rearrangement. Using Oncomine, a publicly available compendium of gene expression data, 4 significantly down regulated genes located in the area of the common deletion site were identified (Figure 16).

The present invention is not limited to a particular mechanism. Indeed, an understanding of the mechanism is not necessary to practice the present invention. Nonetheless, the results suggest that nearly half of all PCAs can be defined by the TMPRSS2:ERG rearrangement. The majority of these tumors demonstrate an intronic deletion, which according to the oligonucleotide SNP array genomic analysis is variable in size. However, approximately 30-40% did not demonstrate a deletion and thus might harbor a balanced translocation of TMRPSS2 and ERG. This variability in the extent of the deletion may be associated with disease progression as has been observed with CML. The current study identified significant clinical associations with tumor stage and lymph node status. TMPRSS2:ERG rearranged tumors with deletion also showed a trend towards higher rates of PSA biochemical failure.

Additional experiments conducted during the course of development of the present invention explored the risk of developing metastases or prostate cancer specific death based on the presence of the TMPRSS2:ERG gene fusion in a watchful waiting cohort of early prostate cancers with long term follow-up. The frequency of the TMPRSS2:ERG gene fusion was assessed using 92 cases. The frequency of TMPRSS2:ERG gene fusion in this population-based cohort was 15.2% (14/92), lower than the 50% frequency observed in two hospital-based cohorts. The present invention is not limited to a particular mechanism. Indeed, an understanding of the mechanism is not necessary to practice the present invention. Nonetheless, this difference in TMPRSS2:ERG gene fusion prostate cancers may be due to ethnic and racial genetic differences. These differences may also be explained by the lower percentage of high grade cases in this watchful waiting cohort as compared to the other non-population based studies.

A significant association between TMPRSS2:ERG gene fusion and development of distant metastases and prostate cancer specific death was observed with a cumulative incidence ratio of 3.6 (P = .004, 95% confidence interval=1.5 to 8.9). These data suggest that TMPRSS2:ERG gene fusion prostate cancers have a more aggressive phenotype. Further experiments indicated that genomic deletions in the TMPRSS2:ERG gene fusion were correlated with advanced and/or metastatic prostate cancer (See *e.g.,* Example 5).

The present invention has also demonstrated that androgen can induce the overexpression of ERG, presumably through AREs, in a TMPRSS2-ERG-positive cell line. The present invention is not limited to a particular mechanism. Indeed, an understanding of the mechanism is not necessary to practice the present invention. Nonetheless, collectively, the results suggest that dysregulation of ETS family activity through AREs upstream of TMPRSS2 may drive prostate cancer development.

### b. ETV1 Gene Fusions

Further studies conducted during the course of development of some embodiments of the present invention investigated the discordance between the frequency of ETV1 outlier expression and TMPRSS2:ETV1 positive prostate cancers. The results confirmed previous studies identifying TMPRSS2:ERG gene fusions as the predominant mechanism driving ERG over-expression in prostate cancer. However, in three prostate cancers over-expressing ETV1, novel 5' fusion partners were identified. The present invention is not limited to a particular mechanism. Indeed, an understanding of the mechanism is not necessary to practice the present invention. Nonetheless, the reason for this discrepancy in 5' partners for fusions involving ERG and ETV1 is unclear, however as TMPRSS2 and ERG are located approximately ∼3 MB apart on chromosome 21, their proximity may favor TMPRSS2 as the 5' partner for ERG.

Over-expression of ETV1 under androgen regulation in a transgenic mouse resulted in highly penetrant mPIN (9 of 12, 75%) in the mouse prostate. The development of carcinoma was not obsserved. These results support in vitro studies, where ETV1 over-expression increased invasion in benign prostatic epithelial cells but was not sufficient for transformation, and previous studies indicating that in humans, ETS fusions likely occur during the PIN to carcinoma transition or early in prostate cancer progression (Tomlins et al., Nat Genet 39, 41-51 (2007)). The present invention is not limited to a particular mechanism. Indeed, an understanding of the mechanism is not necessary to practice the present invention. Nonetheless, it is contemplated that, in human prostate cancer development, ETS gene fusions occur in the context of earlier lesions, such as loss of single NKX3-1 and/or PTEN alleles (Tomlins et al., Annual Review of Pathology: Mechanisms of Disease 1, 243-271 (2006)). Furthermore, the development of mPIN in ETV1 transgenic mice without early progression to carcinoma is similar to mouse models of these other early events in human prostate cancer, such as NKX3-1+/- and PTEN+/- mice (Kim et al., Proc Natl Acad Sci U S A 99, 2884-9 (2002); Abdulkadir et al., Mol Cell Biol 22, 1495-503 (2002); Di Cristofano et al., Nat Genet 27, 222-4 (2001)). Thus, it is contemplated that crosses between ARR2Pb-ETV1 mice and these mice will produce oncogene/tumor suppressor models mimicking early events in human prostate cancer development.

In order to identify transcriptional programs regulated by ETV1 in RWPE-ETV 1, expression signatures were loaded into the Molecular Concepts Map, an analytical framework for exploring the network of inter-relationships among a growing collection of molecular concepts, or biologically related gene sets. In addition to being the largest collection of gene sets for association analysis, the MCM is unique in that computes pair-wise associations among all gene sets in the database, allowing the identification and visualization of "enrichment networks" of linked concepts.

This analysis showed that an in vivo signature of genes overexpressed in prostate cancers with ETV1 outlier-expression vs. cancers without outlierexpression of ETS genes was enriched (P = 0.003) in the in vitro signature of genes over-expressed in RWPE-ETV1 cells (Fig 41f), supporting the biological relevance of the in vitro model. More generally, MCM analysis identified a network of molecular concepts related to cell invasion that were enriched in the ETV1 over-expressed signature, consistent with the phenotypic effects described in this report. For example, the signature shared enrichment with concepts representing genes over-expressed in invasive vs. superficial transitional cell bladder cancer (Modlich et al. P = 2.9E-14, Dyrskjot et al. P = 1.2E-8)29, 30 and genes over-expressed in invasive breast ductal carcinoma vs. ductal carcinoma in situ (Schuetz et al. P = 3.8E-13). More directly, the signature shared enrichment with the InterPro concept of proteins containing "Peptidase M10A and M12B, matrixin or adamalysin domains" (P = 8.5E-5), which includes matrix metalloproteinases (MMPs) and a disintegrin and metalloproteinasedomains (ADAMs).

Several MMPs and members of the urokinase plasminogen activator pathway, both of which are known to mediate invasion and are reported to be direct targets of ETS transcription factors, were over-expressed in RWPE-ETV1 cells (Fig 41g). Furthermore, the 'over-expressed in RWPE-ETV1' signature shared overlap with a signature of genes over-expressed in MCM-10A cells over-expressing the STAT3-C oncogene (P = 8.9E-14). In this model, STAT3-C over-expression did not affect proliferation, but increased invasion in an MMP9 dependent manner. The results also parallel studies of EWSR1:ETS gene fusions in Ewing's sarcoma, which demonstrated that knockdown or over-expression of EWS:FLI1 affected invasion, but not proliferation (Smith et al., Cancer Cell 9, 405-16 (2006)). MCM analysis of the under-expressed in RWPE-ETV1 signature revealed enrichment with proliferation related concepts (Fig 50), indicating subtle effects on cellular proliferation not apparent in FACS or proliferation assays.

The present study also demonstrates the utility of using outlier gene expression to guide cytogenetic studies. For example, despite numerous karyotyping, SKY and high resolution array CGH studies, the cryptic ETV1 rearrangement has not been reported in LNCaP (Beheshti et al., Mol Diagn 5, 23-32 (2000); Beheshti et al., Neoplasia 3, 62-9 (2001); Gibas et al., Cancer Genet Cytogenet 11, 399-404 (1984); Watson et al., Hum Genet 120, 795-805 (2007); Pang et al., Prostate 66, 157-72 (2006); Murillo et al., Genes Chromosomes Cancer 45, 702-16 (2006); Shi et al., Prostate 60, 257-71 (2004); Takaha et al., Cancer Res 62, 647-51 (2002); Strefford et al., Cancer Genet Cytogenet 124, 112-21 (2001); Thalmann et al., Cancer Res 54, 2577-81 (1994)), the most commonly used in vitro model of prostate cancer. Similarly, TMPRSS2:ERG fusions are often caused by intrachromosomal deletions between TMPRSS2 and ERG on 21q, which cannot be detected by karyotyping. Likewise, the HNRPA2B1:ETV1 fusion identified here also occurs through an intrachromosomal deletion spanning approximately 15 MB. These results indicate that cryptic rearrangements and intrachromosomal deletions that activate oncogenes may occur in other common epithelial carcinomas.

### c. ETV4 Gene Fusions

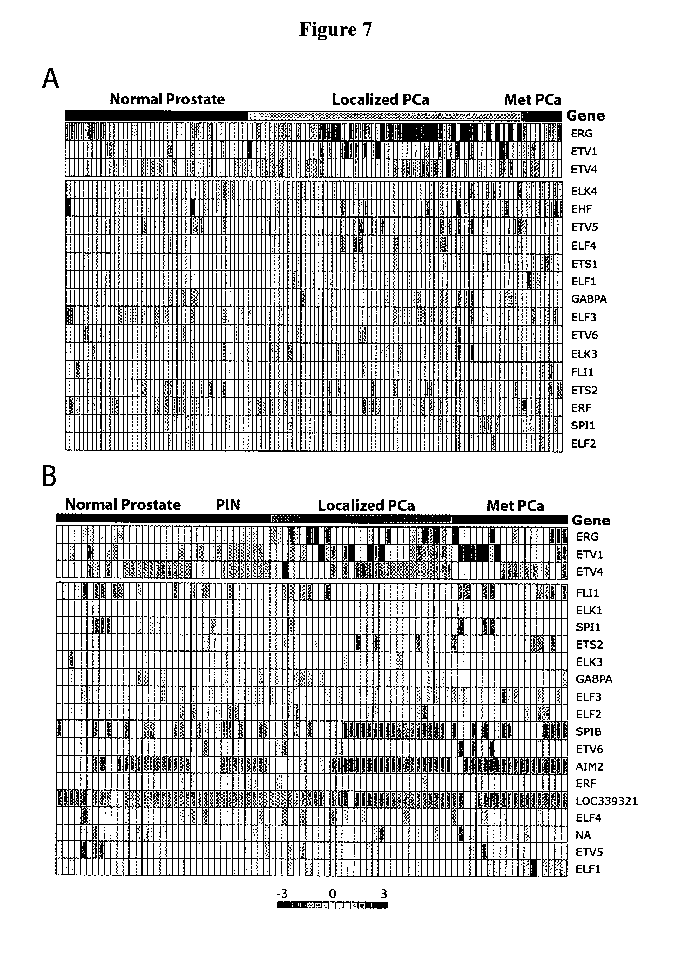

Experiments were conducted to interrogate the expression of all ETS family members monitored in prostate cancer profiling studies from the Oncomine database (Rhodes et al., supra). Marked over-expression of the ETS family member ETV4 was identified in a single prostate cancer case from each of two studies-one profiling grossly dissected tissues (Lapointe et al., supra) (Fig. 7A) and the other profiling laser capture microdissected (LCM) tissues 1 (Fig. 7B). ETV4 was found to be fused to TMPRSS2 in cancerous tissues.

### d. ETV5 Gene Fusions

Additional experiments identified TMPRSS2:ETV5 and SLC45A3:ETV5 gene fusions (Example 20). ETV5 was found to have outlier expression in prostate cancer sample and was subsequently found to be present in gene fusions.

### 3. HG/ETS Gene Fusions

Additional experiments identified HNRPA2B1:ETV1 gene fusions in prostate cancer. HNRPA2B 1 did not show prostate-specific expression or androgen regulation, and is instead expressed strongly across all tumor types. HNRPA2B1 encodes a member of the ubiquitously expressed heteronuclear ribonuclear proteins, and its promoter shares structural similarity to other housekeeping genes (Antoniou et al., Genomics 82, 269-79 (2003)). Furthermore, the HNRPA2B1 promoter has been shown to be unmetheylated and prevents transcriptional silencing of the CMV promoter in transgenes (Williams et al., BMC Biotechnol 5, 17 (2005)). Thus, HNRPA2B1:ETV1 represents a novel class of gene fusions in common epithelial carcinomas, where non-tissue specific promoter elements drive the expression of oncogenes. While TMPRSS2:ETS fusions are functionally analogous to IGH-MYC rearrangements in B cell malignancies, HNRPA2B1:ETV1 is more analogous to inv(3)(q21q26) and t(3;3)(q21;q26) in acute myeloid leukemia, which are thought to result in the placement of EVI under the control of enhancer elements of the constitutively expressed RPN1 gene (Suzukawa et al., Blood 84, 2681-8 (1994); Wieser et al., Leuk Lymphoma 43, 59-65 (2002)).

It is contemplated that other common epithelial cancers are driven by tissue specific promoter elements, such as estrogen response elements fused to oncogenes in breast cancer. Furthermore, while the prostate specific fusions (such as SLC45A3:ETV1) identified here would not provide a growth advantage in other epithelial cancers, it is contemplated that fusions involving strong promoters of ubiquitously expressed genes, such as HNRPA2B1, can result in the aberrant expression of oncogenes across tumor types. The HNRPA2B1:ETV1 fusion identified here occurs through an intrachromosomal deletion spanning approximately 15 MB. These results indicate that cryptic rearrangements and intrachromosomal deletions that activate oncogenes may occur in other common epithelial carcinomas.

### II. Antibodies

The gene fusion proteins of the present invention, including fragments, derivatives and analogs thereof, may be used as immunogens to produce antibodies having use in the diagnostic, research, and therapeutic methods described below. The antibodies may be polyclonal or monoclonal, chimeric, humanized, single chain or Fab fragments. Various procedures known to those of ordinary skill in the art may be used for the production and labeling of such antibodies and fragments. *See, e.g.,* Bums, ed., Immunochemical Protocols, 3rd ed., Humana Press (2005); Harlow and Lane, Antibodies: A Laboratory Manual, Cold Spring Harbor Laboratory (1988); Kozbor et al., Immunology Today 4: 72 (1983); Köhler and Milstein, Nature 256: 495 (1975). Antibodies or fragments exploiting the differences between the truncated ETS family member protein or chimeric protein and their respective native proteins are particularly preferred.

### III. Diagnostic Applications

The fusion of an ARG or HG to an ETS family member gene is detectable as DNA, RNA or protein. Initially, the gene fusion is detectable as a chromosomal rearrangement of genomic DNA having a 5' portion from a transcriptional regulatory region of the ARG or HG and a 3' portion from the ETS family member gene. Once transcribed, the gene fusion is detectable as a chimeric mRNA having a 5' portion from the transcriptional regulatory region of the ARG or HG and a 3' portion from the ETS family member gene. Once translated, the gene fusion is detectable as an amino-terminally truncated ETS family member protein resulting from the fusion of the transcriptional regulatory region of the ARG or HG to the ETS family member gene; a chimeric protein having an amino-terminal portion from the transcriptional regulatory region of the ARG or HG and a carboxy-terminal portion from the ETS family member gene; or, an upregulated, but otherwise indistinguishable, native ETS family member protein. The truncated ETS family member protein and chimeric protein may differ from their respective native proteins in amino acid sequence, post-translational processing and/or secondary, tertiary or quaternary structure. Such differences, if present, can be used to identify the presence of the gene fusion. Specific methods of detection are described in more detail below.

The present invention provides DNA, RNA and protein based diagnostic methods that either directly or indirectly detect the gene fusions. The present invention also provides compositions and kits for diagnostic purposes.

The diagnostic methods of the present invention may be qualitative or quantitative. Quantitative diagnostic methods may be used, for example, to discriminate between indolent and aggressive cancers via a cutoff or threshold level. Where applicable, qualitative or quantitative diagnostic methods may also include amplification of target, signal or intermediary (*e.g.,* a universal primer).

An initial assay may confirm the presence of a gene fusion but not identify the specific fusion. A secondary assay is then performed to determine the identity of the particular fusion, if desired. The second assay may use a different detection technology than the initial assay.

The gene fusions of the present invention may be detected along with other markers in a multiplex or panel format. Markers are selected for their predictive value alone or in combination with the gene fusions. Exemplary prostate cancer markers include, but are not limited to: AMACR/P504S (U.S. Pat. No. 6,262,245); PCA3 (U.S. Pat. No. 7,008,765); PCGEM1 (U.S. Pat. No. 6,828,429); prostein/P501S, P503S, P504S, P509S, P510S, prostase/P703P, P710P (U.S. Publication No. 20030185830); and, those disclosed in U.S. Pat. Nos. 5,854,206 and 6,034,218, and U.S. Publication No. 20030175736, each of which is herein incorporated by reference in its entirety. Markers for other cancers, diseases, infections, and metabolic conditions are also contemplated for inclusion in a multiplex of panel format.

The diagnostic methods of the present invention may also be modified with reference to data correlating particular gene fusions with the stage, aggressiveness or progression of the disease or the presence or risk of metastasis. Ultimately, the information provided by the methods of the present invention will assist a physician in choosing the best course of treatment for a particular patient.

### A. Sample

Any patient sample suspected of containing the gene fusions may be tested according to the methods of the present invention. By way of non-limiting examples, the sample may be tissue (*e.g.,* a prostate biopsy sample or a tissue sample obtained by prostatectomy), blood, urine, semen, prostatic secretions or a fraction thereof (*e.g.,* plasma, serum, urine supernatant, urine cell pellet or prostate cells). A urine sample is preferably collected immediately following an attentive digital rectal examination (DRE), which causes prostate cells from the prostate gland to shed into the urinary tract.

The patient sample typically requires preliminary processing designed to isolate or enrich the sample for the gene fusions or cells that contain the gene fusions. A variety of techniques known to those of ordinary skill in the art may be used for this purpose, including but not limited: centrifugation; immunocapture; cell lysis; and, nucleic acid target capture (*See, e.g.,* EP Pat. No. 1 409 727, herein incorporated by reference in its entirety).

### B. DNA and RNA Detection

The gene fusions of the present invention may be detected as chromosomal rearrangements of genomic DNA or chimeric mRNA using a variety of nucleic acid techniques known to those of ordinary skill in the art, including but not limited to: nucleic acid sequencing; nucleic acid hybridization; and, nucleic acid amplification.

### 1. Sequencing

Illustrative non-limiting examples of nucleic acid sequencing techniques include, but are not limited to, chain terminator (Sanger) sequencing and dye terminator sequencing. Those of ordinary skill in the art will recognize that because RNA is less stable in the cell and more prone to nuclease attack experimentally RNA is usually reverse transcribed to DNA before sequencing.

Chain terminator sequencing uses sequence-specific termination of a DNA synthesis reaction using modified nucleotide substrates. Extension is initiated at a specific site on the template DNA by using a short radioactive, or other labeled, oligonucleotide primer complementary to the template at that region. The oligonucleotide primer is extended using a DNA polymerase, standard four deoxynucleotide bases, and a low concentration of one chain terminating nucleotide, most commonly a di-deoxynucleotide. This reaction is repeated in four separate tubes with each of the bases taking turns as the di-deoxynucleotide. Limited incorporation of the chain terminating nucleotide by the DNA polymerase results in a series of related DNA fragments that are terminated only at positions where that particular di-deoxynucleotide is used. For each reaction tube, the fragments are size-separated by electrophoresis in a slab polyacrylamide gel or a capillary tube filled with a viscous polymer. The sequence is determined by reading which lane produces a visualized mark from the labeled primer as you scan from the top of the gel to the bottom.

Dye terminator sequencing alternatively labels the terminators. Complete sequencing can be performed in a single reaction by labeling each of the di-deoxynucleotide chain-terminators with a separate fluorescent dye, which fluoresces at a different wavelength.

### 2. Hybridization

Illustrative non-limiting examples of nucleic acid hybridization techniques include, but are not limited to, *in situ* hybridization (ISH), microarray, and Southern or Northern blot.

*In situ* hybridization (ISH) is a type of hybridization that uses a labeled complementary DNA or RNA strand as a probe to localize a specific DNA or RNA sequence in a portion or section of tissue (*in situ*)*,* or, if the tissue is small enough, the entire tissue (whole mount ISH). DNA ISH can be used to determine the structure of chromosomes. RNA ISH is used to measure and localize mRNAs and other transcripts within tissue sections or whole mounts. Sample cells and tissues are usually treated to fix the target transcripts in place and to increase access of the probe. The probe hybridizes to the target sequence at elevated temperature, and then the excess probe is washed away. The probe that was labeled with either radio-, fluorescent- or antigen-labeled bases is localized and quantitated in the tissue using either autoradiography, fluorescence microscopy or immunohistochemistry, respectively. ISH can also use two or more probes, labeled with radioactivity or the other non-radioactive labels, to simultaneously detect two or more transcripts.

### a. FISH

In some embodiments, fusion sequences are detected using *fluorescence in situ* hybridization (FISH). The preferred FISH assays for the present invention utilize bacterial artificial chromosomes (BACs). These have been used extensively in the human genome sequencing project (see Nature 409: 953-958 (2001)) and clones containing specific BACs are available through distributors that can be located through many sources, *e.g*., NCBI. Each BAC clone from the human genome has been given a reference name that unambiguously identifies it. These names can be used to find a corresponding GenBank sequence and to order copies of the clone from a distributor.

In some embodiments, the detection assay is a FISH assay utilizing a probe for ETV1 (*e.g.,* bac RP11-692L4), a set of probes for c-ERG:t-ERG break apart (*e.g.,* bac RP11-24A11 and as a probe for t-ERG RP11-372O17 or RP11-137J13). In other embodiments, the FISH assay is performed by testing for ETV1 deletion or amplification with a set of probes, wherein one probe spans the ETV1 locus (*e.g*., bac RP11-692L4) and the other probe hybridizes to chromosome 7 (*e.g.,* a probe on the centromere of the chromosome). In still further embodiments, the method is performed by testing for ERG deletion or amplification with a set of probes, one spanning the ERG locus (*e.g.,* bac RP11-476D17) and one reference probe on chromosome 21 (*e.g*., PR11-32L6; RP11-752M23; RP11-1107H21; RP11-639A7 or RP11-1077M21). In yet other embodiments, the method is performed by testing for TMPRSS2 deletion/amplification with a set of probes, one spanning the TMPRSS2 (*e.g.,* RP11-121A5; RP11-120C17; PR11-814F13; or RR11-535H11) locus and one reference probe on chromosome 21(*e.g*., PR11-32L6; RP11-752M23; RP11-1107H21; RP11-639A7 or RP11-1077M21). In some embodiments, the method further comprises a hybridization using a probe selected from the group including, but not limited to RP11-121A5; RP11-120C17; PR11-814F13; and RR11-535H11.

The present invention further provides a method of performing a FISH assay on human prostate cells, human prostate tissue or on the fluid surrounding said human prostate cells or human prostate tissue. In some embodiments, the assay comprises a hybridization step utilizing a probe selected from the group including, but not limited to, RP11-372O17; RP11-137J13; RP11-692L4; RP11-476D17; PR11-32L6; RP11-752M23; RP11-1107H21; RP11-639A7; RP11-1077M21; RP11-121A5; RP11-120C17; PR11-814F13; and RR11-535H11.

Specific BAC clones that can be used in FISH protocols to detect rearrangements are as follows:
- For testing for an ETV1-TMPRSS2 fusion, one probe spanning the ETV1 and one spanning the TMPRSS2 locus may be used:
   BAC for ETV1: RP 11-692L4
   BAC for TMPRSS2: RP11-121A5, (RP11-120C17, PR11-814F13, RR11-535H11)
- Testing ERG translocation with set of probes for c-ERG:t-ERG break apart:
   BAC for c-ERG: RP11-24A11
   BACs for t-ERG: RP11-372O17, RP11-137J13
- Testing ETV1 deletion/amplification with set of probes, one spanning the ETV1 locus and one reference probe on chromosome 7:
   BAC for ETV1: RP 11-692L4

Testing ERG deletion/amplification with set of probes, one spanning the ERG locus and one reference probe on chromosome 21:
BAC for ERG: RP11-476D17
BACs for reference probe on chromosome 21: *
   - Testing TMPRSS2 deletion/amplification with set of probes, one spanning the TMPRSS2 locus and one reference probe on chromosome 21:
      BACs for TMPRSS2: RP11-121A5, (RP11-120C17, PR11-814F13, RR11-535H11) BACs for reference probe on chromosome 21: *PR11-32L6, RP11-752M23, RP11-1107H21, RP11-639A7, (RP11-1077M21).*

The most preferred probes for detecting a deletion mutation resulting in a fusion between TMPRSS2 and ERG are RP11-24A11 and RP11-137J13. These probes, or those described above, are labeled with appropriate fluorescent or other markers and then used in hybridizations. The Examples section provided herein sets forth one particular protocol that is effective for measuring deletions but one of skill in the art will recognize that many variations of this assay can be used equally well. Specific protocols are well known in the art and can be readily adapted for the present invention. Guidance regarding methodology may be obtained from many references including: In situ Hybridization: Medical Applications (eds. G. R. Coulton and J. de Belleroche), Kluwer Academic Publishers, Boston (1992); In situ Hybridization: In Neurobiology; Advances in Methodology (eds. J. H. Eberwine, K. L. Valentino, and J. D. Barchas), Oxford University Press Inc., England (1994); In situ Hybridization: A Practical Approach (ed. D. G. Wilkinson), Oxford University Press Inc., England (1992)); Kuo, et al., Am. J. Hum. Genet. 49:112-119 (1991); Klinger, et al., Am. J. Hum. Genet. 51:55-65 (1992); and Ward, et al., Am. J. Hum. Genet. 52:854-865 (1993)). There are also kits that are commercially available and that provide protocols for performing FISH assays (available from *e.g*., Oncor, Inc., Gaithersburg, MD). Patents providing guidance on methodology include U.S. 5,225,326; 5,545,524; 6,121,489 and 6,573,043. All of these references may be used along with similar references in the art and with the information provided in the Examples section herein to establish procedural steps convenient for a particular laboratory.

Table 13 below shows additional BAC clones that find use as FISH probes.

**Table 13**

| Gene | **Chromosome** | **RefSeq** | **5' BAC** | **3'BAC** | **Paired** |
|---|---|---|---|---|---|
| EHF | 11p13 | NM_012153 | RP5-1135K18 | RP5-1002E13 | 2 |
| ELF1 | 13q14 | NM_172373 | RP11-88n4 | RP11-53f19 | |
| ELF2 | 4q28 | NM_201999.1 | RP11-22o8 | RP11-375P1 | |
| ELF3 | 1q32 | NM_004433 | RP11-25B7 | RP11-246J15 | |
| ELF4 | Xq25 | NM_001421 | RP5-875H3 | RP4-753P9 | |
| ELF5 | 11p13 | NM_001422.2 | RP5-1002E13 | RP5-1135K18 | 2 |
| ELK1 | Xp11 | NM_005229 | RP1-54B20 | RP1-306D1 | |
| ELK3 | 12q22 | NM_005230 | RP11-69E3 | RP11-510l5 | |
| ELK4 | 1q32 | NM_001973.2 | RP11-131 E5 | RP11-249h15 | |
| ERF | 19q13 | NM_006494.1 | RP11-20813 | RP11-317E13 | |
| ERG | 21q22 | NM_004449.3 | RP11-137J13 | RP11-24A11 | 1 |
| ETS1 | 11q24 | NM_005238.2 | RP11-254C5 | RP11-112m22 | |
| ETS2 | 21q22 | NM_005239.4 | RP11-24A11 | RP11-137J13 | 1 |
| ETV1 | 7p21 | NM_004956.3 | RP11-1149J13 | RP11-34C22 | |
| ETV2 | 19q13 | NM_014209.1 | RP11-32h17 | RP11-92j4 | |
| ETV3 | 1q23 | NM_005240.1 | RP11-91G5 | RP11-1038N13 | 3 |
| ETV4 | 17q21 | NM_001986.1 | RP11-436J4 | RP11-100E5 | |
| ETV5 | 3q27 | NM_004454.1 | RP11-379C23 | RP11-1144N13 | |
| ETV6 | 12p13 | NM_001987.3 | RP11-90N7 | RP11-59h1 | |
| ETV7 | 6p21 | NM_016135.2 | RP3-431A14 | RP1-179N16 | |
| FEV | 2q35 | NM_017521.2 | RP11-316O14 | RP11-129D2 | |
| FL11 | 11q24 | NM_002017.2 | RP11-112M22 | RP11-75P14 | |
| FLJ16478 | 1q23 | NM_001004341 | RP11-91G5 | RP11-1038N13 | 3 |
| SPDEF | 6p21 | NM_012391.1 | RP11-79j23 | RP11-119c22 | |
| SP11 | 11p11 | NM_016135.2 | RP11-56e13 | RP11-29o22 | |
| SPIB | 19q13 | NM_003121.2 | RP11-510l16 | RP11-26P14 | |
| SPIC | 12q23 | NM_152323.1 | RP11-426H24 | RP11-938C1 | |
| TMPRSS2 | 21q22 | NM_005656.2 | RP11-35C4 | RP11-120C17 | |

Additional FISH probes useful in the methods of the present invention are shown in Table 16 (Figure 46).

### b. Microarrays

Different kinds of biological assays are called microarrays including, but not limited to: DNA microarrays (*e.g.,* cDNA microarrays and oligonucleotide microarrays); protein microarrays; tissue microarrays; transfection or cell microarrays; chemical compound microarrays; and, antibody microarrays. A DNA microarray, commonly known as gene chip, DNA chip, or biochip, is a collection of microscopic DNA spots attached to a solid surface (*e.g.,* glass, plastic or silicon chip) forming an array for the purpose of expression profiling or monitoring expression levels for thousands of genes simultaneously. The affixed DNA segments are known as probes, thousands of which can be used in a single DNA microarray. Microarrays can be used to identify disease genes by comparing gene expression in disease and normal cells. Microarrays can be fabricated using a variety of technologies, including but not limiting: printing with fine-pointed pins onto glass slides; photolithography using pre-made masks; photolithography using dynamic micromirror devices; ink-jet printing; or, electrochemistry on microelectrode arrays.

Southern and Northern blotting is used to detect specific DNA or RNA sequences, respectively. DNA or RNA extracted from a sample is fragmented, electrophoretically separated on a matrix gel, and transferred to a membrane filter. The filter bound DNA or RNA is subject to hybridization with a labeled probe complementary to the sequence of interest. Hybridized probe bound to the filter is detected. A variant of the procedure is the reverse Northern blot, in which the substrate nucleic acid that is affixed to the membrane is a collection of isolated DNA fragments and the probe is RNA extracted from a tissue and labeled.

### 3. Amplification

Chromosomal rearrangements of genomic DNA and chimeric mRNA may be amplified prior to or simultaneous with detection. Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (*e.g.,* PCR) require that RNA be reversed transcribed to DNA prior to amplification (*e.g*., RT-PCR), whereas other amplification techniques directly amplify RNA (*e.g*., TMA and NASBA).

The polymerase chain reaction (U.S. Pat. Nos. 4,683,195, 4,683,202, 4,800,159 and 4,965,188), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR *see, e.g.,* U.S. Pat. Nos. 4,683,195, 4,683,202 and 4,800,159; Mullis et al., Meth. Enzymol. 155: 335 (1987); and, Murakawa et al., DNA 7: 287 (1988).

Transcription mediated amplification (U.S. Pat. Nos. 5,480,784 and 5,399,491, ), commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. *See, e.g.,* U.S. Pat. Nos. 5,399,491 and 5,824,518. In a variation described in U.S. Publ. No. 20060046265, TMA optionally incorporates the use of blocking moieties, terminating moieties, and other modifying moieties to improve TMA process sensitivity and accuracy.

The ligase chain reaction (Weiss, R., Science 254: 1292 (1991)), commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded ligated oligonucleotide product.

Strand displacement amplification (Walker, G. et al., Proc. Natl. Acad. Sci. USA 89: 392-396 (1992); U.S. Pat. Nos. 5,270,184 and 5,455,166, commonly referred to as SDA, uses cycles of annealing pairs of primer sequences to opposite strands of a target sequence, primer extension in the presence of a dNTPαS to produce a duplex hemiphosphorothioated primer extension product, endonuclease-mediated nicking of a hemimodified restriction endonuclease recognition site, and polymerase-mediated primer extension from the 3' end of the nick to displace an existing strand and produce a strand for the next round of primer annealing, nicking and strand displacement, resulting in geometric amplification of product. Thermophilic SDA (tSDA) uses thermophilic endonucleases and polymerases at higher temperatures in essentially the same method (EP Pat. No. 0 684 315).

Other amplification methods include, for example: nucleic acid sequence based amplification (U.S. Pat. No. 5,130,238), commonly referred to as NASBA; one that uses an RNA replicase to amplify the probe molecule itself (Lizardi et al., BioTechnol. 6: 1197 (1988)), commonly referred to as Qβ replicase; a transcription based amplification method (Kwoh et al., Proc. Natl. Acad. Sci. USA 86:1173 (1989)); and, self-sustained sequence replication (Guatelli et al., Proc. Natl. Acad. Sci. USA 87: 1874 (1990)). For further discussion of known amplification methods *see* Persing, David H., "In Vitro Nucleic Acid Amplification Techniques" in Diagnostic Medical Microbiology: Principles and Applications (Persing et al., Eds.), pp. 51-87 (American Society for Microbiology, Washington, DC (1993)).

### 4. Detection Methods

Non-amplified or amplified gene fusion nucleic acids can be detected by any conventional means. For example, the gene fusions can be detected by hybridization with a detectably labeled probe and measurement of the resulting hybrids. Illustrative non-limiting examples of detection methods are described below.

One illustrative detection method, the Hybridization Protection Assay (HPA) involves hybridizing a chemiluminescent oligonucleotide probe (*e.g.,* an acridinium ester-labeled (AE) probe) to the target sequence, selectively hydrolyzing the chemiluminescent label present on unhybridized probe, and measuring the chemiluminescence produced from the remaining probe in a luminometer. *See, e.g.,* U.S. Pat. No. 5,283,174 and Norman C. Nelson et al., Nonisotopic Probing, Blotting, and Sequencing, ch. 17 (Larry J. Kricka ed., 2d ed. 1995).

Another illustrative detection method provides for quantitative evaluation of the amplification process in real-time. Evaluation of an amplification process in "real-time" involves determining the amount of amplicon in the reaction mixture either continuously or periodically during the amplification reaction, and using the determined values to calculate the amount of target sequence initially present in the sample. A variety of methods for determining the amount of initial target sequence present in a sample based on real-time amplification are well known in the art. These include methods disclosed in U.S. Pat. Nos. 6,303,305 and 6,541,205. Another method for determining the quantity of target sequence initially present in a sample, but which is not based on a real-time amplification, is disclosed in U.S. Pat. No. 5,710,029.

Amplification products may be detected in real-time through the use of various self-hybridizing probes, most of which have a stem-loop structure. Such self-hybridizing probes are labeled so that they emit differently detectable signals, depending on whether the probes are in a self-hybridized state or an altered state through hybridization to a target sequence. By way of non-limiting example, "molecular torches" are a type of self-hybridizing probe that includes distinct regions of self-complementarity (referred to as "the target binding domain" and "the target closing domain") which are connected by a joining region (*e.g.,* non-nucleotide linker) and which hybridize to each other under predetermined hybridization assay conditions. In a preferred embodiment, molecular torches contain single-stranded base regions in the target binding domain that are from 1 to about 20 bases in length and are accessible for hybridization to a target sequence present in an amplification reaction under strand displacement conditions. Under strand displacement conditions, hybridization of the two complementary regions, which may be fully or partially complementary, of the molecular torch is favored, except in the presence of the target sequence, which will bind to the single-stranded region present in the target binding domain and displace all or a portion of the target closing domain. The target binding domain and the target closing domain of a molecular torch include a detectable label or a pair of interacting labels (*e.g.,* luminescent/quencher) positioned so that a different signal is produced when the molecular torch is self-hybridized than when the molecular torch is hybridized to the target sequence, thereby permitting detection of probe:target duplexes in a test sample in the presence of unhybridized molecular torches. Molecular torches and a variety of types of interacting label pairs are disclosed in U.S. Pat. No. 6,534,274.

Another example of a detection probe having self-complementarity is a "molecular beacon." Molecular beacons include nucleic acid molecules having a target complementary sequence, an affinity pair (or nucleic acid arms) holding the probe in a closed conformation in the absence of a target sequence present in an amplification reaction, and a label pair that interacts when the probe is in a closed conformation. Hybridization of the target sequence and the target complementary sequence separates the members of the affinity pair, thereby shifting the probe to an open conformation. The shift to the open conformation is detectable due to reduced interaction of the label pair, which may be, for example, a fluorophore and a quencher (*e.g.,* DABCYL and EDANS). Molecular beacons are disclosed in U.S. Pat. Nos. 5,925,517 and 6,150,097.

Other self-hybridizing probes are well known to those of ordinary skill in the art. By way of non-limiting example, probe binding pairs having interacting labels, such as those disclosed in U.S. Pat. No. 5,928,862 might be adapted for use in the present invention. Probe systems used to detect single nucleotide polymorphisms (SNPs) might also be utilized in the present invention. Additional detection systems include "molecular switches," as disclosed in U.S. Publ. No. 20050042638, herein incorporated by reference in its entirety. Other probes, such as those comprising intercalating dyes and/or fluorochromes, are also useful for detection of amplification products in the present invention. *See, e.g.,* U.S. Pat. No. 5,814,447 (herein incorporated by reference in its entirety).

### C. Protein Detection

The gene fusions of the present invention may be detected as truncated ETS family member proteins or chimeric proteins using a variety of protein techniques known to those of ordinary skill in the art, including but not limited to: protein sequencing; and, immunoassays.

### 1. Sequencing

Illustrative non-limiting examples of protein sequencing techniques include, but are not limited to, mass spectrometry and Edman degradation.

Mass spectrometry can, in principle, sequence any size protein but becomes computationally more difficult as size increases. A protein is digested by an endoprotease, and the resulting solution is passed through a high pressure liquid chromatography column. At the end of this column, the solution is sprayed out of a narrow nozzle charged to a high positive potential into the mass spectrometer. The charge on the droplets causes them to fragment until only single ions remain. The peptides are then fragmented and the mass-charge ratios of the fragments measured. The mass spectrum is analyzed by computer and often compared against a database of previously sequenced proteins in order to determine the sequences of the fragments. The process is then repeated with a different digestion enzyme, and the overlaps in sequences are used to construct a sequence for the protein.

In the Edman degradation reaction, the peptide to be sequenced is adsorbed onto a solid surface (*e.g.,* a glass fiber coated with polybrene). The Edman reagent, phenylisothiocyanate (PTC), is added to the adsorbed peptide, together with a mildly basic buffer solution of 12% trimethylamine, and reacts with the amine group of the N-terminal amino acid. The terminal amino acid derivative can then be selectively detached by the addition of anhydrous acid. The derivative isomerizes to give a substituted phenylthiohydantoin, which can be washed off and identified by chromatography, and the cycle can be repeated. The efficiency of each step is about 98%, which allows about 50 amino acids to be reliably determined.

### 2. Immunoassays

Illustrative non-limiting examples of immunoassays include, but are not limited to: immunoprecipitation; Western blot; ELISA; immunohistochemistry; immunocytochemistry; flow cytometry; and, immuno-PCR. Polyclonal or monoclonal antibodies detectably labeled using various techniques known to those of ordinary skill in the art (*e.g*., colorimetric, fluorescent, chemiluminescent or radioactive) are suitable for use in the immunoassays.

Immunoprecipitation is the technique of precipitating an antigen out of solution using an antibody specific to that antigen. The process can be used to identify protein complexes present in cell extracts by targeting a protein believed to be in the complex. The complexes are brought out of solution by insoluble antibody-binding proteins isolated initially from bacteria, such as Protein A and Protein G. The antibodies can also be coupled to sepharose beads that can easily be isolated out of solution. After washing, the precipitate can be analyzed using mass spectrometry, Western blotting, or any number of other methods for identifying constituents in the complex.

A Western blot, or immunoblot, is a method to detect protein in a given sample of tissue homogenate or extract. It uses gel electrophoresis to separate denatured proteins by mass. The proteins are then transferred out of the gel and onto a membrane, typically polyvinyldiflroride or nitrocellulose, where they are probed using antibodies specific to the protein of interest. As a result, researchers can examine the amount of protein in a given sample and compare levels between several groups.

An ELISA, short for Enzyme-Linked ImmunoSorbent Assay, is a biochemical technique to detect the presence of an antibody or an antigen in a sample. It utilizes a minimum of two antibodies, one of which is specific to the antigen and the other of which is coupled to an enzyme. The second antibody will cause a chromogenic or fluorogenic substrate to produce a signal. Variations of ELISA include sandwich ELISA, competitive ELISA, and ELISPOT. Because the ELISA can be performed to evaluate either the presence of antigen or the presence of antibody in a sample, it is a useful tool both for determining serum antibody concentrations and also for detecting the presence of antigen.

Immunohistochemistry and immunocytochemistry refer to the process of localizing proteins in a tissue section or cell, respectively, via the principle of antigens in tissue or cells binding to their respective antibodies. Visualization is enabled by tagging the antibody with color producing or fluorescent tags. Typical examples of color tags include, but are not limited to, horseradish peroxidase and alkaline phosphatase. Typical examples of fluorophore tags include, but are not limited to, fluorescein isothiocyanate (FITC) or phycoerythrin (PE).

Flow cytometry is a technique for counting, examining and sorting microscopic particles suspended in a stream of fluid. It allows simultaneous multiparametric analysis of the physical and/or chemical characteristics of single cells flowing through an optical/electronic detection apparatus. A beam of light (*e.g*., a laser) of a single frequency or color is directed onto a hydrodynamically focused stream of fluid. A number of detectors are aimed at the point where the stream passes through the light beam; one in line with the light beam (Forward Scatter or FSC) and several perpendicular to it (Side Scatter (SSC) and one or more fluorescent detectors). Each suspended particle passing through the beam scatters the light in some way, and fluorescent chemicals in the particle may be excited into emitting light at a lower frequency than the light source. The combination of scattered and fluorescent light is picked up by the detectors, and by analyzing fluctuations in brightness at each detector, one for each fluorescent emission peak, it is possible to deduce various facts about the physical and chemical structure of each individual particle. FSC correlates with the cell volume and SSC correlates with the density or inner complexity of the particle (*e.g*., shape of the nucleus, the amount and type of cytoplasmic granules or the membrane roughness).

Immuno-polymerase chain reaction (IPCR) utilizes nucleic acid amplification techniques to increase signal generation in antibody-based immunoassays. Because no protein equivalence of PCR exists, that is, proteins cannot be replicated in the same manner that nucleic acid is replicated during PCR, the only way to increase detection sensitivity is by signal amplification. The target proteins are bound to antibodies which are directly or indirectly conjugated to oligonucleotides. Unbound antibodies are washed away and the remaining bound antibodies have their oligonucleotides amplified. Protein detection occurs via detection of amplified oligonucleotides using standard nucleic acid detection methods, including real-time methods.

### D. Data Analysis

In some embodiments, a computer-based analysis program is used to translate the raw data generated by the detection assay (*e.g*., the presence, absence, or amount of a given gene fusion or other markers) into data of predictive value for a clinician. The clinician can access the predictive data using any suitable means. Thus, in some preferred embodiments, the present invention provides the further benefit that the clinician, who is not likely to be trained in genetics or molecular biology, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. For example, in some embodiments of the present invention, a sample (*e.g*., a biopsy or a serum or urine sample) is obtained from a subject and submitted to a profiling service (*e.g*., clinical lab at a medical facility, genomic profiling business, etc.), located in any part of the world (*e.g*., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g*., a urine sample) and directly send it to a profiling center. Where the sample comprises previously determined biological information, the information may be directly sent to the profiling service by the subject (*e.g*., an information card containing the information may be scanned by a computer and the data transmitted to a computer of the profiling center using an electronic communication systems). Once received by the profiling service, the sample is processed and a profile is produced (*i.e*., expression data), specific for the diagnostic or prognostic information desired for the subject.

The profile data is then prepared in a format suitable for interpretation by a treating clinician. For example, rather than providing raw expression data, the prepared format may represent a diagnosis or risk assessment (*e.g*., likelihood of cancer being present) for the subject, along with recommendations for particular treatment options. The data may be displayed to the clinician by any suitable method. For example, in some embodiments, the profiling service generates a report that can be printed for the clinician (*e.g*., at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counseling based on the results. In some embodiments, the data is used for research use. For example, the data may be used to further optimize the inclusion or elimination of markers as useful indicators of a particular condition or stage of disease.

### E. In vivo Imaging

The gene fusions of the present invention may also be detected using *in vivo* imaging techniques, including but not limited to: radionuclide imaging; positron emission tomography (PET); computerized axial tomography, X-ray or magnetic resonance imaging method, fluorescence detection, and chemiluminescent detection. In some embodiments, *in vivo* imaging techniques are used to visualize the presence of or expression of cancer markers in an animal (*e.g.,* a human or non-human mammal). For example, in some embodiments, cancer marker mRNA or protein is labeled using a labeled antibody specific for the cancer marker. A specifically bound and labeled antibody can be detected in an individual using an *in vivo* imaging method, including, but not limited to, radionuclide imaging, positron emission tomography, computerized axial tomography, X-ray or magnetic resonance imaging method, fluorescence detection, and chemiluminescent detection. Methods for generating antibodies to the cancer markers of the present invention are described below.

The *in vivo* imaging methods of the present invention are useful in the diagnosis of cancers that express the cancer markers of the present invention (*e.g.,* prostate cancer). *In vivo* imaging is used to visualize the presence of a marker indicative of the cancer. Such techniques allow for diagnosis without the use of an unpleasant biopsy. The *in vivo* imaging methods of the present invention are also useful for providing prognoses to cancer patients. For example, the presence of a marker indicative of cancers likely to metastasize can be detected. The *in vivo* imaging methods of the present invention can further be used to detect metastatic cancers in other parts of the body.

In some embodiments, reagents (*e.g.,* antibodies) specific for the cancer markers of the present invention are fluorescently labeled. The labeled antibodies are introduced into a subject (*e.g.,* orally or parenterally). Fluorescently labeled antibodies are detected using any suitable method (*e.g.,* using the apparatus described in U.S. Pat. No. 6,198,107).

In other embodiments, antibodies are radioactively labeled. The use of antibodies *for in vivo* diagnosis is well known in the art. Sumerdon et al., (Nucl. Med. Biol 17:247-254 [1990] have described an optimized antibody-chelator for the radioimmunoscintographic imaging of tumors using Indium-111 as the label. Griffin et al., (J Clin One 9:631-640 [1991]) have described the use of this agent in detecting tumors in patients suspected of having recurrent colorectal cancer. The use of similar agents with paramagnetic ions as labels for magnetic resonance imaging is known in the art (Lauffer, Magnetic Resonance in Medicine 22:339-342 [1991]). The label used will depend on the imaging modality chosen. Radioactive labels such as Indium-111, Technetium-99m, or Iodine-131 can be used for planar scans or single photon emission computed tomography (SPECT). Positron emitting labels such as Fluorine-19 can also be used for positron emission tomography (PET). For MRI, paramagnetic ions such as Gadolinium (III) or Manganese (II) can be used.

Radioactive metals with half-lives ranging from 1 hour to 3.5 days are available for conjugation to antibodies, such as scandium-47 (3.5 days) gallium-67 (2.8 days), gallium-68 (68 minutes), technetiium-99m (6 hours), and indium-111 (3.2 days), of which gallium-67, technetium-99m, and indium-111 are preferable for gamma camera imaging, gallium-68 is preferable for positron emission tomography.

A useful method of labeling antibodies with such radiometals is by means of a bifunctional chelating agent, such as diethylenetriaminepentaacetic acid (DTPA), as described, for example, by Khaw et al. (Science 209:295 [1980]) for In-111 and Tc-99m, and by Scheinberg et al. (Science 215:1511 [1982]). Other chelating agents may also be used, but the 1-(p-carboxymethoxybenzyl)EDTA and the carboxycarbonic anhydride of DTPA are advantageous because their use permits conjugation without affecting the antibody's immunoreactivity substantially.

Another method for coupling DPTA to proteins is by use of the cyclic anhydride of DTPA, as described by Hnatowich et al. (Int. J. Appl. Radiat. Isot. 33:327 [1982]) for labeling of albumin with In-111, but which can be adapted for labeling of antibodies. A suitable method of labeling antibodies with Tc-99m which does not use chelation with DPTA is the pretinning method of Crockford et al., (U.S. Pat. No. 4,323,546, herein incorporated by reference).

A preferred method of labeling immunoglobulins with Tc-99m is that described by Wong et al. (Int. J. Appl. Radiat. Isot., 29:251 [1978]) for plasma protein, and recently applied successfully by Wong et al. (J. Nucl. Med., 23:229 [1981]) for labeling antibodies.

In the case of the radiometals conjugated to the specific antibody, it is likewise desirable to introduce as high a proportion of the radiolabel as possible into the antibody molecule without destroying its immunospecificity. A further improvement may be achieved by effecting radiolabeling in the presence of the specific cancer marker of the present invention, to insure that the antigen binding site on the antibody will be protected. The antigen is separated after labeling.

In still further embodiments, *in vivo* biophotonic imaging (Xenogen, Almeda, CA) is utilized for *in vivo* imaging. This real-time *in vivo* imaging utilizes luciferase. The luciferase gene is incorporated into cells, microorganisms, and animals (*e.g.,* as a fusion protein with a cancer marker of the present invention). When active, it leads to a reaction that emits light. A CCD camera and software is used to capture the image and analyze it.

### F. Compositions & Kits

Compositions for use in the diagnostic methods of the present invention include, but are not limited to, probes, amplification oligonucleotides, and antibodies. Particularly preferred compositions detect a product only when an ARG or HG fuses to an ETS family member gene. These compositions include: a single labeled probe comprising a sequence that hybridizes to the junction at which a 5' portion from a transcriptional regulatory region of an ARG or HG fuses to a 3' portion from an ETS family member gene (i.e., spans the gene fusion junction); a pair of amplification oligonucleotides wherein the first amplification oligonucleotide comprises a sequence that hybridizes to a transcriptional regulatory region of an ARG or HG and the second amplification oligonucleotide comprises a sequence that hybridizes to an ETS family member gene; an antibody to an amino-terminally truncated ETS family member protein resulting from a fusion of a transcriptional regulatory region of an ARG or HG to an ETS family member gene; or, an antibody to a chimeric protein having an amino-terminal portion from a transcriptional regulatory region of an ARG or HG and a carboxy-terminal portion from an ETS family member gene. Other useful compositions, however, include: a pair of labeled probes wherein the first labeled probe comprises a sequence that hybridizes to a transcriptional regulatory region of an ARG or HG and the second labeled probe comprises a sequence that hybridizes to an ETS family member gene.

Any of these compositions, alone or in combination with other compositions of the present invention, may be provided in the form of a kit. For example, the single labeled probe and pair of amplification oligonucleotides may be provided in a kit for the amplification and detection of gene fusions of the present invention. Kits may further comprise appropriate controls and/or detection reagents. The probe and antibody compositions of the present invention may also be provided in the form of an array.

### IV. Prognostic Applications

Experiments conducted during the course of development of the present invention demonstrated a close correlation between gene fusions of the present invention and the prognosis of patients with prostate cancer (see *e.g.,* Example 5 below). Especially in cases where a fusion results from a deletion of the genomic DNA lying between TMPRSS2 and ERG, it has been found that cancer cells assume a more aggressive phenotype. Thus, in some embodiments, assays that are capable of detecting gene fusions between TMPRSS2 and ERG in which there has been a deletion of intervening DNA are used to provide prognoses and help physicians decide on an appropriate therapeutic strategy. For example, in some embodiments, patients with tumors having this particular rearrangement are treated more intensively since their prognosis is significantly worse than patients that lack the rearrangement.

Any assay may be used to determine whether cells are present having a rearrangement of the type discussed above (*e.g.,* those described above).

Although the present invention will most preferably be used in connection with obtaining a prognosis for prostate cancer patients, other epithelial cell tumors may also be examined and the assays and probes described herein may be used in determining whether cancerous cells from these tumors have rearrangements that are likely to make them particularly aggressive, *i.e.,* likely to be invasive and metastatic. Examples of tumors that may be characterized using this procedure include tumors of the breast, lung, colon, ovary, uterus, esophagus, stomach, liver, kidney, brain, skin and muscle. The assays will also be of value to researchers studying these cancers in cell lines and animal models.

Further experiments conducted during the course of development of the present invention demonstrated that chromosomal deletions can be detected by assaying samples to determine whether there is a loss of expression of one or more genes located in the deleted region. For example, approximately 2.8 megabases of genomic DNA is typically deleted in forming a fusion between TMPRSS2 and ERG and at least four genes lying in this area are lost when this occurs. These are the ETS2 gene, the WRB gene, the PCP4 gene and the MX1 gene. A decrease in one or more of these in cancerous prostate cells suggests a poor prognosis.

Accordingly, in some embodiments, the present invention provides a method of assaying epithelial cells for the deletion of chromosomal DNA indicative of a cancer-associated rearrangement, comprising performing a FISH assay using at least a first and a second probe, wherein the first probe is at least 15 nucleotides in length (*e.g*., at least 15, 20, 35, etc.); is bound to a first fluorescent label; and hybridizes under stringent conditions to a first sequence in the human genome wherein the first sequence includes at least a portion of either an androgen responsive gene (*e.g.,* the TMPRSS2 gene) or a ETS family gene (*e.g.,* the ERG gene, the ETV1 gene, or the ETV4 gene); and the second probe: is at least 15 nucleotides in length; is bound to a second fluorescent label that is different from the first fluorescent label; and hybridizes under stringent conditions to a second sequence in the human genome that is different from the first sequence and which includes at least a portion of an androgen responsive gene (*e.g*., the TMPRSS2 gene) or a ETS family gene (*e.g*., the ERG gene, the ETV1 gene, or the ETV4 gene).

In further embodiments, the present invention provides a method for assaying epithelial cells (*e.g*., prostate cells) for a deletion of genomic DNA indicative of a cancer-associated rearrangement, comprising: obtaining a test sample of epithelial cells; assaying the sample of epithelial cells to determine the level of expression of one or more genes selected from the group including, but not limited to, ETS2; WRB; PCP4; and MX1; comparing the expression level determined in step b) with the level in a control sample; and concluding that a deletion has occurred if the level of expression determined for the gene in the test sample is lower than that for a control sample.

### V. Drug Screening Applications

In some embodiments, the present invention provides drug screening assays (*e.g.,* to screen for anticancer drugs). The screening methods of the present invention utilize cancer markers identified using the methods of the present invention (*e.g*., including but not limited to, gene fusions of the present invention). For example, in some embodiments, the present invention provides methods of screening for compounds that alter (*e.g*., decrease) the expression of gene fusions. The compounds or agents may interfere with transcription, by interacting, for example, with the promoter region. The compounds or agents may interfere with mRNA produced from the fusion (*e.g*., by RNA interference, antisense technologies, etc.). The compounds or agents may interfere with pathways that are upstream or downstream of the biological activity of the fusion. In some embodiments, candidate compounds are antisense or interfering RNA agents (*e.g*., oligonucleotides) directed against cancer markers. In other embodiments, candidate compounds are antibodies or small molecules that specifically bind to a cancer marker regulator or expression products of the present invention and inhibit its biological function.

In one screening method, candidate compounds are evaluated for their ability to alter cancer marker expression by contacting a compound with a cell expressing a cancer marker and then assaying for the effect of the candidate compounds on expression. In some embodiments, the effect of candidate compounds on expression of a cancer marker gene is assayed for by detecting the level of cancer marker mRNA expressed by the cell. mRNA expression can be detected by any suitable method. In other embodiments, the effect of candidate compounds on expression of cancer marker genes is assayed by measuring the level of polypeptide encoded by the cancer markers. The level of polypeptide expressed can be measured using any suitable method, including but not limited to, those disclosed herein.

Specifically, the present invention provides screening methods for identifying modulators, *i.e*., candidate or test compounds or agents (*e.g*., proteins, peptides, peptidomimetics, peptoids, small molecules or other drugs) which bind to cancer markers of the present invention, have an inhibitory (or stimulatory) effect on, for example, cancer marker expression or cancer marker activity, or have a stimulatory or inhibitory effect on, for example, the expression or activity of a cancer marker substrate. Compounds thus identified can be used to modulate the activity of target gene products (*e.g*., cancer marker genes) either directly or indirectly in a therapeutic protocol, to elaborate the biological function of the target gene product, or to identify compounds that disrupt normal target gene interactions. Compounds that inhibit the activity or expression of cancer markers are useful in the treatment of proliferative disorders, *e.g*., cancer, particularly prostate cancer.

In one embodiment, the invention provides assays for screening candidate or test compounds that are substrates of a cancer marker protein or polypeptide or a biologically active portion thereof. In another embodiment, the invention provides assays for screening candidate or test compounds that bind to or modulate the activity of a cancer marker protein or polypeptide or a biologically active portion thereof.

The test compounds of the present invention can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g*., Zuckennann et al., J. Med. Chem. 37: 2678-85 [1994]); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckermann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al., J. Med. Chem. 37:1233 [1994].

Libraries of compounds may be presented in solution (*e.g*., Houghten, Biotechniques 13:412-421 [1992]), or on beads (Lam, Nature 354:82-84 [1991]), chips (Fodor, Nature 364:555-556 [1993]), bacteria or spores (U.S. Pat. No. 5,223,409; ), plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. Natl. Acad. Sci. 87:6378-6382 [1990]; Felici, J. Mol. Biol. 222:301 [1991]).

In one embodiment, an assay is a cell-based assay in which a cell that expresses a cancer marker mRNA or protein or biologically active portion thereof is contacted with a test compound, and the ability of the test compound to the modulate cancer marker's activity is determined. Determining the ability of the test compound to modulate cancer marker activity can be accomplished by monitoring, for example, changes in enzymatic activity, destruction or mRNA, or the like.

The ability of the test compound to modulate cancer marker binding to a compound, *e.g*., a cancer marker substrate or modulator, can also be evaluated. This can be accomplished, for example, by coupling the compound, *e.g*., the substrate, with a radioisotope or enzymatic label such that binding of the compound, *e.g*., the substrate, to a cancer marker can be determined by detecting the labeled compound, *e.g*., substrate, in a complex.

Alternatively, the cancer marker is coupled with a radioisotope or enzymatic label to monitor the ability of a test compound to modulate cancer marker binding to a cancer marker substrate in a complex. For example, compounds (*e.g.*, substrates) can be labeled with ¹²⁵I, ³⁵S ¹⁴C or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g*., a cancer marker substrate) to interact with a cancer marker with or without the labeling of any of the interactants can be evaluated. For example, a microphysiometer can be used to detect the interaction of a compound with a cancer marker without the labeling of either the compound or the cancer marker (McConnell et al. Science 257:1906-1912 [1992]). As used herein, a "microphysiometer" (*e.g*., Cytosensor) is an analytical instrument that measures the rate at which a cell acidifies its environment using a light-addressable potentiometric sensor (LAPS). Changes in this acidification rate can be used as an indicator of the interaction between a compound and cancer markers.

In yet another embodiment, a cell-free assay is provided in which a cancer marker protein or biologically active portion thereof is contacted with a test compound and the ability of the test compound to bind to the cancer marker protein, mRNA, or biologically active portion thereof is evaluated. Preferred biologically active portions of the cancer marker proteins or mRNA to be used in assays of the present invention include fragments that participate in interactions with substrates or other proteins, *e.g*., fragments with high surface probability scores.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, *e.g*., using fluorescence energy transfer (FRET) (see, for example, Lakowicz et al., U.S. Pat. No. 5,631,169; Stavrianopoulos et al., U.S. Pat. No. 4,968,103). A fluorophore label is selected such that a first donor molecule's emitted fluorescent energy will be absorbed by a fluorescent label on a second, 'acceptor' molecule, which in turn is able to fluoresce due to the absorbed energy.

Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label should be maximal. A FRET binding event can be conveniently measured through standard fluorometric detection means well known in the art (*e.g*., using a fluorimeter).

In another embodiment, determining the ability of the cancer marker protein or mRNA to bind to a target molecule can be accomplished using real-time Biomolecular Interaction Analysis (BIA) (*see, e.g.,* Sjolander and Urbaniczky, Anal. Chem. 63:2338-2345 [1991] and Szabo et al. Curr. Opin. Struct. Biol. 5:699-705 [1995]). "Surface plasmon resonance" or "BIA" detects biospecific interactions in real time, without labeling any of the interactants (*e.g*., B1Acore). Changes in the mass at the binding surface (indicative of a binding event) result in alterations of the refractive index of light near the surface (the optical phenomenon of surface plasmon resonance (SPR)), resulting in a detectable signal that can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize cancer markers, an anti-cancer marker antibody or its target molecule to facilitate separation of complexed from non-complexed forms of one or both of the proteins, as well as to accommodate automation of the assay. Binding of a test compound to a cancer marker protein, or interaction of a cancer marker protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase-cancer marker fusion proteins or glutathione-S-transferase/target fusion proteins can be adsorbed onto glutathione Sepharose beads (Sigma Chemical, St. Louis, MO) or glutathione-derivatized microtiter plates, which are then combined with the test compound or the test compound and either the non-adsorbed target protein or cancer marker protein, and the mixture incubated under conditions conducive for complex formation (*e.g*., at physiological conditions for salt and pH). Following incubation, the beads or microtiter plate wells are washed to remove any unbound components, the matrix immobilized in the case of beads, complex determined either directly or indirectly, for example, as described above.

Alternatively, the complexes can be dissociated from the matrix, and the level of cancer markers binding or activity determined using standard techniques. Other techniques for immobilizing either cancer markers protein or a target molecule on matrices include using conjugation of biotin and streptavidin. Biotinylated cancer marker protein or target molecules can be prepared from biotin-NHS (N-hydroxy-succinimide) using techniques known in the art (*e.g*., biotinylation kit, Pierce Chemicals, Rockford, EL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g*., using a labeled antibody specific for the immobilized component (the antibody, in turn, can be directly labeled or indirectly labeled with, *e.g*., a labeled anti-IgG antibody).

This assay is performed utilizing antibodies reactive with cancer marker protein or target molecules but which do not interfere with binding of the cancer markers protein to its target molecule. Such antibodies can be derivatized to the wells of the plate, and unbound target or cancer markers protein trapped in the wells by antibody conjugation. Methods for detecting such complexes, in addition to those described above for the GST-immobilized complexes, include immunodetection of complexes using antibodies reactive with the cancer marker protein or target molecule, as well as enzyme-linked assays which rely on detecting an enzymatic activity associated with the cancer marker protein or target molecule.

Alternatively, cell free assays can be conducted in a liquid phase. In such an assay, the reaction products are separated from unreacted components, by any of a number of standard techniques, including, but not limited to: differential centrifugation (see, for example, Rivas and Minton, Trends Biochem Sci 18:284-7 [1993]); chromatography (gel filtration chromatography, ion-exchange chromatography); electrophoresis (*see, e.g.,* Ausubel et al., eds. Current Protocols in Molecular Biology 1999, J. Wiley: New York.); and immunoprecipitation (see, for example, Ausubel et al., eds. Current Protocols in Molecular Biology 1999, J. Wiley: New York). Such resins and chromatographic techniques are known to one skilled in the art (*See e.g.,* Heegaard J. Mol. Recognit 11:141-8 [1998]; Hageand Tweed J. Chromatogr. Biomed. Sci. Appl 699:499-525 [1997]). Further, fluorescence energy transfer may also be conveniently utilized, as described herein, to detect binding without further purification of the complex from solution.

The assay can include contacting the cancer markers protein, mRNA, or biologically active portion thereof with a known compound that binds the cancer marker to form an assay mixture, contacting the assay mixture with a test compound, and determining the ability of the test compound to interact with a cancer marker protein or mRNA, wherein determining the ability of the test compound to interact with a cancer marker protein or mRNA includes determining the ability of the test compound to preferentially bind to cancer markers or biologically active portion thereof, or to modulate the activity of a target molecule, as compared to the known compound.

To the extent that cancer markers can, *in vivo,* interact with one or more cellular or extracellular macromolecules, such as proteins, inhibitors of such an interaction are useful. A homogeneous assay can be used can be used to identify inhibitors.

For example, a preformed complex of the target gene product and the interactive cellular or extracellular binding partner product is prepared such that either the target gene products or their binding partners are labeled, but the signal generated by the label is quenched due to complex formation (see, *e.g.,* U.S. Pat. No. 4,109,496, that utilizes this approach for immunoassays). The addition of a test substance that competes with and displaces one of the species from the preformed complex will result in the generation of a signal above background. In this way, test substances that disrupt target gene product-binding partner interaction can be identified. Alternatively, cancer markers protein can be used as a "bait protein" in a two-hybrid assay or three-hybrid assay (*see, e.g.,* U.S. Pat. No. 5,283,317; Zervos et al., Cell 72:223-232 [1993]; Madura et al., J. Biol. Chem. 268.12046-12054 [1993]; Bartel et al., Biotechniques 14:920-924 [1993]; Iwabuchi et al., Oncogene 8:1693-1696 [1993]; and Brent WO 94/1030), to identify other proteins, that bind to or interact with cancer markers ("cancer marker-binding proteins" or "cancer marker-bp") and are involved in cancer marker activity. Such cancer marker-bps can be activators or inhibitors of signals by the cancer marker proteins or targets as, for example, downstream elements of a cancer markers-mediated signaling pathway.

Modulators of cancer markers expression can also be identified. For example, a cell or cell free mixture is contacted with a candidate compound and the expression of cancer marker mRNA or protein evaluated relative to the level of expression of cancer marker mRNA or protein in the absence of the candidate compound. When expression of cancer marker mRNA or protein is greater in the presence of the candidate compound than in its absence, the candidate compound is identified as a stimulator of cancer marker mRNA or protein expression. Alternatively, when expression of cancer marker mRNA or protein is less (i.e., statistically significantly less) in the presence of the candidate compound than in its absence, the candidate compound is identified as an inhibitor of cancer marker mRNA or protein expression. The level of cancer markers mRNA or protein expression can be determined by methods described herein for detecting cancer markers mRNA or protein.

A modulating agent can be identified using a cell-based or a cell free assay, and the ability of the agent to modulate the activity of a cancer markers protein can be confirmed *in vivo, e.g.,* in an animal such as an animal model for a disease (*e.g.,* an animal with prostate cancer or metastatic prostate cancer; or an animal harboring a xenograft of a prostate cancer from an animal (*e.g*., human) or cells from a cancer resulting from metastasis of a prostate cancer (*e.g.,* to a lymph node, bone, or liver), or cells from a prostate cancer cell line.

This invention further pertains to novel agents identified by the above-described screening assays (*See e.g.,* below description of cancer therapies). Accordingly, it is within the scope of this invention to further use an agent identified as described herein (*e.g*., a cancer marker modulating agent, an antisense cancer marker nucleic acid molecule, a siRNA molecule, a cancer marker specific antibody, or a cancer marker-binding partner) in an appropriate animal model (such as those described herein) to determine the efficacy, toxicity, side effects, or mechanism of action, of treatment with such an agent. Furthermore, novel agents identified by the above-described screening assays can be, *e.g*., used for treatments as described herein.

### VI. Therapeutic Applications

In some embodiments, the present invention provides therapies for cancer (*e.g*., prostate cancer). In some embodiments, therapies directly or indirectly target gene fusions of the present invention.

### A. RNA Interference and Antisense Therapies

In some embodiments, the present invention targets the expression of gene fusions. For example, in some embodiments, the present invention employs compositions comprising oligomeric antisense or RNAi compounds, particularly oligonucleotides (*e*.*g*., those identified in the drug screening methods described above), for use in modulating the function of nucleic acid molecules encoding cancer markers of the present invention, ultimately modulating the amount of cancer marker expressed.

### 1. RNA Interference (RNAi)

In some embodiments, RNAi is utilized to inhibit fusion protein function. RNAi represents an evolutionary conserved cellular defense for controlling the expression of foreign genes in most eukaryotes, including humans. RNAi is typically triggered by double-stranded RNA (dsRNA) and causes sequence-specific mRNA degradation of single-stranded target RNAs homologous in response to dsRNA. The mediators of mRNA degradation are small interfering RNA duplexes (siRNAs), which are normally produced from long dsRNA by enzymatic cleavage in the cell. siRNAs are generally approximately twenty-one nucleotides in length (*e.g*. 21-23 nucleotides in length), and have a base-paired structure characterized by two nucleotide 3'-overhangs. Following the introduction of a small RNA, or RNAi, into the cell, it is believed the sequence is delivered to an enzyme complex called RISC (RNA-induced silencing complex). RISC recognizes the target and cleaves it with an endonuclease. It is noted that if larger RNA sequences are delivered to a cell, RNase III enzyme (Dicer) converts longer dsRNA into 21-23 nt ds siRNA fragments. In some embodiments, RNAi oligonucleotides are designed to target the junction region of fusion proteins.

Chemically synthesized siRNAs have become powerful reagents for genome-wide analysis of mammalian gene function in cultured somatic cells. Beyond their value for validation of gene function, siRNAs also hold great potential as gene-specific therapeutic agents (Tuschl and Borkhardt, Molecular Intervent. 2002; 2(3):158-67).

The transfection of siRNAs into animal cells results in the potent, long-lasting post-transcriptional silencing of specific genes (Caplen et al, Proc Natl Acad Sci U.S.A. 2001; 98: 9742-7; Elbashir et al., Nature. 2001; 411:494-8; Elbashir et al., Genes Dev. 2001;15: 188-200; and Elbashir et al., EMBO J. 2001; 20: 6877-88). Methods and compositions for performing RNAi with siRNAs are described, for example, in U.S. Pat. 6,506,559.

siRNAs are extraordinarily effective at lowering the amounts of targeted RNA, and by extension proteins, frequently to undetectable levels. The silencing effect can last several months, and is extraordinarily specific, because one nucleotide mismatch between the target RNA and the central region of the siRNA is frequently sufficient to prevent silencing (Brummelkamp et al, Science 2002; 296:550-3; and Holen et al, Nucleic Acids Res. 2002; 30:1757-66). An important factor in the design of siRNAs is the presence of accessible sites for siRNA binding. Bahoia et al., (J. Biol. Chem., 2003; 278: 15991-15997) describe the use of a type of DNA array called a scanning array to find accessible sites in mRNAs for designing effective siRNAs. These arrays comprise oligonucleotides ranging in size from monomers to a certain maximum, usually Comers, synthesized using a physical barrier (mask) by stepwise addition of each base in the sequence. Thus the arrays represent a full oligonucleotide complement of a region of the target gene. Hybridization of the target mRNA to these arrays provides an exhaustive accessibility profile of this region of the target mRNA. Such data are useful in the design of antisense oligonucleotides (ranging from 7mers to 25mers), where it is important to achieve a compromise between oligonucleotide length and binding affinity, to retain efficacy and target specificity (Sohail et al, Nucleic Acids Res., 2001; 29(10): 2041- 2045). Additional methods and concerns for selecting siRNAs are described for example, in WO 05054270, WO05038054A1, WO03070966A2, J Mol Biol. 2005 May 13;348(4):883-93, J Mol Biol. 2005 May 13;348(4):871-81, and Nucleic Acids Res. 2003 Aug 1;31(15):4417-24, each of In addition, software (*e.g*., the MWG online siMAX siRNA design tool) is commercially or publicly available for use in the selection of siRNAs.

### 2. Antisense

In other embodiments, fusion protein expression is modulated using antisense compounds that specifically hybridize with one or more nucleic acids encoding cancer markers of the present invention. The specific hybridization of an oligomeric compound with its target nucleic acid interferes with the normal function of the nucleic acid. This modulation of function of a target nucleic acid by compounds that specifically hybridize to it is generally referred to as "antisense." The functions of DNA to be interfered with include replication and transcription. The functions of RNA to be interfered with include all vital functions such as, for example, translocation of the RNA to the site of protein translation, translation of protein from the RNA, splicing of the RNA to yield one or more mRNA species, and catalytic activity that may be engaged in or facilitated by the RNA. The overall effect of such interference with target nucleic acid function is modulation of the expression of cancer markers of the present invention. In the context of the present invention, "modulation" means either an increase (stimulation) or a decrease (inhibition) in the expression of a gene. For example, expression may be inhibited to potentially prevent tumor proliferation.

It is preferred to target specific nucleic acids for antisense. "Targeting" an antisense compound to a particular nucleic acid, in the context of the present invention, is a multistep process. The process usually begins with the identification of a nucleic acid sequence whose function is to be modulated. This may be, for example, a cellular gene (or mRNA transcribed from the gene) whose expression is associated with a particular disorder or disease state, or a nucleic acid molecule from an infectious agent. In the present invention, the target is a nucleic acid molecule encoding a gene fusion of the present invention. The targeting process also includes determination of a site or sites within this gene for the antisense interaction to occur such that the desired effect, *e.g*., detection or modulation of expression of the protein, will result. Within the context of the present invention, a preferred intragenic site is the region encompassing the translation initiation or termination codon of the open reading frame (ORF) of the gene. Since the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function *in vivo.* Thus, the terms "translation initiation codon" and "start codon" can encompass many codon sequences, even though the initiator amino acid in each instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. In the context of the present invention, "start codon" and "translation initiation codon" refer to the codon or codons that are used *in vivo* to initiate translation of an mRNA molecule transcribed from a gene encoding a tumor antigen of the present invention, regardless of the sequence(s) of such codons.

Translation termination codon (or "stop codon") of a gene may have one of three sequences (*i.e.*, 5'-UAA, 5'-UAG and 5'-UGA; the corresponding DNA sequences are 5'-TAA, 5'-TAG and 5'-TGA, respectively). The terms "start codon region" and "translation initiation codon region" refer to a portion of such an mRNA or gene that encompasses from about 25 to about 50 contiguous nucleotides in either direction (*i.e*., 5' or 3') from a translation initiation codon. Similarly, the terms "stop codon region" and "translation termination codon region" refer to a portion of such an mRNA or gene that encompasses from about 25 to about 50 contiguous nucleotides in either direction (*i.e*., 5' or 3') from a translation termination codon.

The open reading frame (ORF) or "coding region," which refers to the region between the translation initiation codon and the translation termination codon, is also a region that may be targeted effectively. Other target regions include the 5' untranslated region (5' UTR), referring to the portion of an mRNA in the 5' direction from the translation initiation codon, and thus including nucleotides between the 5' cap site and the translation initiation codon of an mRNA or corresponding nucleotides on the gene, and the 3' untranslated region (3' UTR), referring to the portion of an mRNA in the 3' direction from the translation termination codon, and thus including nucleotides between the translation termination codon and 3' end of an mRNA or corresponding nucleotides on the gene. The 5' cap of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap. The cap region may also be a preferred target region.

Although some eukaryotic mRNA transcripts are directly translated, many contain one or more regions, known as "introns," that are excised from a transcript before it is translated. The remaining (and therefore translated) regions are known as "exons" and are spliced together to form a continuous mRNA sequence. mRNA splice sites (*i.e*., intron-exon junctions) may also be preferred target regions, and are particularly useful in situations where aberrant splicing is implicated in disease, or where an overproduction of a particular mRNA splice product is implicated in disease. Aberrant fusion junctions due to rearrangements or deletions are also preferred targets. It has also been found that introns can also be effective, and therefore preferred, target regions for antisense compounds targeted, for example, to DNA or pre-mRNA.

In some embodiments, target sites for antisense inhibition are identified using commercially available software programs (*e.g*., Biognostik, Gottingen, Germany; SysArris Software, Bangalore, India; Antisense Research Group, University of Liverpool, Liverpool, England; GeneTrove, Carlsbad, CA). In other embodiments, target sites for antisense inhibition are identified using the accessible site method described in PCT Publ. No. WO0198537A2.

Once one or more target sites have been identified, oligonucleotides are chosen that are sufficiently complementary to the target (*i.e.*, hybridize sufficiently well and with sufficient specificity) to give the desired effect. For example, in preferred embodiments of the present invention, antisense oligonucleotides are targeted to or near the start codon.

In the context of this invention, "hybridization," with respect to antisense compositions and methods, means hydrogen bonding, which may be Watson-Crick, Hoogsteen or reversed Hoogsteen hydrogen bonding, between complementary nucleoside or nucleotide bases. For example, adenine and thymine are complementary nucleobases that pair through the formation of hydrogen bonds. It is understood that the sequence of an antisense compound need not be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired (*i.e*., under physiological conditions in the case of *in vivo* assays or therapeutic treatment, and in the case of *in vitro* assays, under conditions in which the assays are performed).

Antisense compounds are commonly used as research reagents and diagnostics. For example, antisense oligonucleotides, which are able to inhibit gene expression with specificity, can be used to elucidate the function of particular genes. Antisense compounds are also used, for example, to distinguish between functions of various members of a biological pathway.

The specificity and sensitivity of antisense is also applied for therapeutic uses. For example, antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides are useful therapeutic modalities that can be configured to be useful in treatment regimes for treatment of cells, tissues, and animals, especially humans.

While antisense oligonucleotides are a preferred form of antisense compound, the present invention comprehends other oligomeric antisense compounds, including but not limited to oligonucleotide mimetics such as are described below. The antisense compounds in accordance with this invention preferably comprise from about 8 to about 30 nucleobases (*i.e*., from about 8 to about 30 linked bases), although both longer and shorter sequences may find use with the present invention. Particularly preferred antisense compounds are antisense oligonucleotides, even more preferably those comprising from about 12 to about 25 nucleobases.

Specific examples of preferred antisense compounds useful with the present invention include oligonucleotides containing modified backbones or non-natural intemucleoside linkages. As defined in this specification, oligonucleotides having modified backbones include those that retain a phosphorus atom in the backbone and those that do not have a phosphorus atom in the backbone. For the purposes of this specification, modified oligonucleotides that do not have a phosphorus atom in their intemucleoside backbone can also be considered to be oligonucleosides.

Preferred modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Preferred modified oligonucleotide backbones that do not include a phosphorus atom therein have backbones that are formed by short chain alkyl or cycloalkyl intemucleoside linkages, mixed heteroatom and alkyl or cycloalkyl intemucleoside linkages, or one or more short chain heteroatomic or heterocyclic intemucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

In other preferred oligonucleotide mimetics, both the sugar and the intemucleoside linkage (*i.e*., the backbone) of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262.

Further teaching of PNA compounds can be found in Nielsen et al., Science 254:1497 (1991).

Most preferred embodiments of the invention are oligonucleotides with phosphorothioate backbones and oligonucleosides with heteroatom backbones, and in particular --CH₂, --NH--O--CH₂--, --CH₂--N(CH₃)--O--CH₂-- [known as a methylene (methylimino) or MMI backbone], --CH₂--O--N(CH₃)--CH₂--, --CH₂--N(CH₃)--N(CH₃)--CH₂--, and --O--N(CH₃)--CH₂--CH₂-- [wherein the native phosphodiester backbone is represented as --O--P--O--CH₂--] of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. Other preferred oligonucleotides comprise one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O--CH₂CH₂OCH₃, also known as 2'-O-(2-methoxyethyl) or 2'-MOE) (Martin et al., Helv. Chim. Acta 78:486 [1995]) *i.e*., an alkoxyalkoxy group. A further preferred modification includes 2'-dimethylaminooxyethoxy (*i.e*., a O(CH₂)₂ON(CH₃)₂ group), also known as 2'-DMAOE, and 2'-dimethylaminoethoxyethoxy (also known in the art as 2'-O-dimethylaminoethoxyethyl or 2'-DMAEOE), *i.e*., 2*'*-O--CH₂--O--CH₂--N(CH₂)₂.

Other preferred modifications include 2'-methoxy(2'-O--CH₃), 2'-aminopropoxy(2'-OCH₂CH₂CH₂NH₂) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in U.S. Pat. No. 3,687,808. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2. °C and are presently preferred base substitutions, even more particularly when combined with 2'-O-methoxyethyl sugar modifications.

Another modification of the oligonucleotides of the present invention involves chemically linking to the oligonucleotide one or more moieties or conjugates that enhance the activity, cellular distribution or cellular uptake of the oligonucleotide. Such moieties include but are not limited to lipid moieties such as a cholesterol moiety, cholic acid, a thioether, (*e.g*., hexyl-S-tritylthiol), a thiocholesterol, an aliphatic chain, (*e.g*., dodecandiol or undecyl residues), a phospholipid, (*e.g.,* di-hexadecyl-rac-glycerol or triethylammonium 1,2-di-O-hexadecyl-rac-glycero-3-H-phosphonate), a polyamine or a polyethylene glycol chain or adamantane acetic acid, a palmityl moiety, or an octadecylamine or hexylamino-carbonyl-oxycholesterol moiety.

One skilled in the relevant art knows well how to generate oligonucleotides containing the above-described modifications. The present invention is not limited to the antisense oligonucleotides described above. Any suitable modification or substitution may be utilized.

It is not necessary for all positions in a given compound to be uniformly modified, and in fact more than one of the aforementioned modifications may be incorporated in a single compound or even at a single nucleoside within an oligonucleotide. The present invention also includes antisense compounds that are chimeric compounds. "Chimeric" antisense compounds or "chimeras," in the context of the present invention, are antisense compounds, particularly oligonucleotides, which contain two or more chemically distinct regions, each made up of at least one monomer unit, *i.e*., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNaseH is a cellular endonuclease that cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds of the present invention may be formed as composite structures of two or more oligonucleotides, modified oligonucleotides, oligonucleosides and/or oligonucleotide mimetics as described above.

The present invention also includes pharmaceutical compositions and formulations that include the antisense compounds of the present invention as described below.

### B. Gene Therapy

The present invention contemplates the use of any genetic manipulation for use in modulating the expression of gene fusions of the present invention. Examples of genetic manipulation include, but are not limited to, gene knockout (*e.g*., removing the fusion gene from the chromosome using, for example, recombination), expression of antisense constructs with or without inducible promoters, and the like. Delivery of nucleic acid construct to cells *in vitro* or *in vivo* may be conducted using any suitable method. A suitable method is one that introduces the nucleic acid construct into the cell such that the desired event occurs (*e.g*., expression of an antisense construct). Genetic therapy may also be used to deliver siRNA or other interfering molecules that are expressed *in vivo* (*e.g*., upon stimulation by an inducible promoter (*e.g*., an androgen-responsive promoter)).

Introduction of molecules carrying genetic information into cells is achieved by any of various methods including, but not limited to, directed injection of naked DNA constructs, bombardment with gold particles loaded with said constructs, and macromolecule mediated gene transfer using, for example, liposomes, biopolymers, and the like. Preferred methods use gene delivery vehicles derived from viruses, including, but not limited to, adenoviruses, retroviruses, vaccinia viruses, and adeno-associated viruses. Because of the higher efficiency as compared to retroviruses, vectors derived from adenoviruses are the preferred gene delivery vehicles for transferring nucleic acid molecules into host cells *in vivo.* Adenoviral vectors have been shown to provide very efficient *in vivo* gene transfer into a variety of solid tumors in animal models and into human solid tumor xenografts in immune-deficient mice. Examples of adenoviral vectors and methods for gene transfer are described in PCT publications WO 00/12738 and WO 00/09675 and U.S. Pat. Appl. Nos. 6,033,908, 6,019,978, 6,001,557, 5,994,132, 5,994,128, 5,994,106, 5,981,225, 5,885,808, 5,872,154, 5,830,730, and 5,824,544.

Vectors may be administered to subject in a variety of ways. For example, in some embodiments of the present invention, vectors are administered into tumors or tissue associated with tumors using direct injection. In other embodiments, administration is via the blood or lymphatic circulation (*See e.g*., PCT publication 99/02685). Exemplary dose levels of adenoviral vector are preferably 10⁸ to 10¹¹ vector particles added to the perfusate.

### C. Antibody Therapy

In some embodiments, the present disclosure provides antibodies that target prostate tumors that express a gene fusion of the present invention. Any suitable antibody (*e.g*., monoclonal, polyclonal, or synthetic) may be utilized in the therapeutic methods disclosed herein. In preferred embodiments, the antibodies used for cancer therapy are humanized antibodies. Methods for humanizing antibodies are well known in the art (*See e.g*., U.S. Pat. Nos. 6,180,370, 5,585,089, 6,054,297, and 5,565,332).

In some embodiments, the therapeutic antibodies comprise an antibody generated against a gene fusion of the present invention, wherein the antibody is conjugated to a cytotoxic agent. In such embodiments, a tumor specific therapeutic agent is generated that does not target normal cells, thus reducing many of the detrimental side effects of traditional chemotherapy. For certain applications, it is envisioned that the therapeutic agents will be pharmacologic agents that will serve as useful agents for attachment to antibodies, particularly cytotoxic or otherwise anticellular agents having the ability to kill or suppress the growth or cell division of endothelial cells. The present invention contemplates the use of any pharmacologic agent that can be conjugated to an antibody, and delivered in active form. Exemplary anticellular agents include chemotherapeutic agents, radioisotopes, and cytotoxins. The therapeutic antibodies of the present invention may include a variety of cytotoxic moieties, including but not limited to, radioactive isotopes (*e.g*., iodine-131, iodine-123, technicium-99m, indium-111, rhenium-188, rhenium-186, gallium-67, copper-67, yttrium-90, iodine-125 or astatine-211), hormones such as a steroid, antimetabolites such as cytosines (*e.g*., arabinoside, fluorouracil, methotrexate or aminopterin; an anthracycline; mitomycin C), vinca alkaloids (*e.g*., demecolcine; etoposide; mithramycin), and antitumor alkylating agent such as chlorambucil or melphalan. Other embodiments may include agents such as a coagulant, a cytokine, growth factor, bacterial endotoxin or the lipid A moiety of bacterial endotoxin. For example, in some embodiments, therapeutic agents will include plant-, fungus- or bacteria-derived toxin, such as an A chain toxins, a ribosome inactivating protein, α-sarcin, aspergillin, restrictocin, a ribonuclease, diphtheria toxin or pseudomonas exotoxin, to mention just a few examples. In some preferred embodiments, deglycosylated ricin A chain is utilized.

In any event, it is proposed that agents such as these may, if desired, be successfully conjugated to an antibody, in a manner that will allow their targeting, internalization, release or presentation to blood components at the site of the targeted tumor cells as required using known conjugation technology (*See, e.g*., Ghose et al., Methods Enzymol., 93:280 [1983]).

For example, in some embodiments the present invention provides immunotoxins targeted a cancer marker of the present invention (*e.g*., ERG or ETV1 fusions). Immunotoxins are conjugates of a specific targeting agent typically a tumor-directed antibody or fragment, with a cytotoxic agent, such as a toxin moiety. The targeting agent directs the toxin to, and thereby selectively kills, cells carrying the targeted antigen. In some embodiments, therapeutic antibodies employ crosslinkers that provide high *in vivo* stability (Thorpe et al., Cancer Res., 48:6396 [1988]).

In other embodiments, particularly those involving treatment of solid tumors, antibodies are designed to have a cytotoxic or otherwise anticellular effect against the tumor vasculature, by suppressing the growth or cell division of the vascular endothelial cells. This attack is intended to lead to a tumor-localized vascular collapse, depriving the tumor cells, particularly those tumor cells distal of the vasculature, of oxygen and nutrients, ultimately leading to cell death and tumor necrosis.

In preferred embodiments, antibody based therapeutics are formulated as pharmaceutical compositions as described below. In preferred embodiments, administration of an antibody composition of the present invention results in a measurable decrease in cancer (*e.g*., decrease or elimination of tumor).

### D. Pharmaceutical Compositions

The present invention further provides pharmaceutical compositions (*e.g*., comprising pharmaceutical agents that modulate the expression or activity of gene fusions of the present invention). The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary (*e.g*., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, *e.g*., intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

In one embodiment of the present invention the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product.

Agents that enhance uptake of oligonucleotides at the cellular level may also be added to the pharmaceutical and other compositions of the present invention. For example, cationic lipids, such as lipofectin (U.S. Pat. No. 5,705,188), cationic glycerol derivatives, and polycationic molecules, such as polylysine (WO 97/30731), also enhance the cellular uptake of oligonucleotides.

The compositions of the present invention may additionally contain other adjunct components conventionally found in pharmaceutical compositions. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, *e.g*., lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Certain embodiments of the invention provide pharmaceutical compositions containing (a) one or more antisense compounds and (b) one or more other chemotherapeutic agents that function by a non-antisense mechanism. Examples of such chemotherapeutic agents include, but are not limited to, anticancer drugs such as daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil (5-FU), floxuridine (5-FUdR), methotrexate (MTX), colchicine, vincristine, vinblastine, etoposide, teniposide, cisplatin and diethylstilbestrol (DES). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. Other non-antisense chemotherapeutic agents are also within the scope of this invention. Two or more combined compounds may be used together or sequentially.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The administering physician can easily determine optimum dosages, dosing methodologies and repetition rates. Optimum dosages may vary depending on the relative potency of individual oligonucleotides, and can generally be estimated based on EC₅₀s found to be effective in *in vitro* and *in vivo* animal models or based on the examples described herein. In general, dosage is from 0.01 µg to 100 g per kg of body weight, and may be given once or more daily, weekly, monthly or yearly. The treating physician can estimate repetition rates for dosing based on measured residence times and concentrations of the drug in bodily fluids or tissues. Following successful treatment, it may be desirable to have the subject undergo maintenance therapy to prevent the recurrence of the disease state, wherein the oligonucleotide is administered in maintenance doses, ranging from 0.01 µg to 100 g per kg of body weight, once or more daily, to once every 20 years.

### VII. Transgenic Animals

The present invention contemplates the generation of transgenic animals comprising an exogenous cancer marker gene (*e.g*., gene fusion) of the present invention or mutants and variants thereof (*e.g*., truncations or single nucleotide polymorphisms). In preferred embodiments, the transgenic animal displays an altered phenotype (*e.g*., increased or decreased presence of markers) as compared to wild-type animals. Methods for analyzing the presence or absence of such phenotypes include but are not limited to, those disclosed herein. In some preferred embodiments, the transgenic animals further display an increased or decreased growth of tumors or evidence of cancer.

The transgenic animals of the present invention find use in drug (*e.g*., cancer therapy) screens. In some embodiments, test compounds (*e.g*., a drug that is suspected of being useful to treat cancer) and control compounds (*e.g*., a placebo) are administered to the transgenic animals and the control animals and the effects evaluated.

The transgenic animals can be generated via a variety of methods. In some embodiments, embryonal cells at various developmental stages are used to introduce transgenes for the production of transgenic animals. Different methods are used depending on the stage of development of the embryonal cell. The zygote is the best target for micro-injection. In the mouse, the male pronucleus reaches the size of approximately 20 micrometers in diameter that allows reproducible injection of 1-2 picoliters (pl) of DNA solution. The use of zygotes as a target for gene transfer has a major advantage in that in most cases the injected DNA will be incorporated into the host genome before the first cleavage (Brinster et al., Proc. Natl. Acad. Sci. USA 82:4438-4442 [1985]). As a consequence, all cells of the transgenic non-human animal will carry the incorporated transgene. This will in general also be reflected in the efficient transmission of the transgene to offspring of the founder since 50% of the germ cells will harbor the transgene. U.S. Pat. No. 4,873,191 describes a method for the micro-injection of zygotes; the disclosure of this patent is incorporated herein in its entirety.

In other embodiments, retroviral infection is used to introduce transgenes into a non-human animal. In some embodiments, the retroviral vector is utilized to transfect oocytes by injecting the retroviral vector into the perivitelline space of the oocyte (U.S. Pat. No. 6,080,912, incorporated herein by reference). In other embodiments, the developing non-human embryo can be cultured *in vitro* to the blastocyst stage. During this time, the blastomeres can be targets for retroviral infection (Janenich, Proc. Natl. Acad. Sci. USA 73:1260 [1976]). Efficient infection of the blastomeres is obtained by enzymatic treatment to remove the zona pellucida (Hogan et al., in Manipulating the Mouse Embryo, Cold Spring Harbor Laboratory Press, Cold Spring Harbor, N.Y. [1986]). The viral vector system used to introduce the transgene is typically a replication-defective retrovirus carrying the transgene (Jahner et al., Proc. Natl. Acad Sci. USA 82:6927 [1985]). Transfection is easily and efficiently obtained by culturing the blastomeres on a monolayer of virus-producing cells (Stewart, et al., EMBO J., 6:383 [1987]). Alternatively, infection can be performed at a later stage. Virus or virus-producing cells can be injected into the blastocoele (Jahner et al., Nature 298:623 [1982]). Most of the founders will be mosaic for the transgene since incorporation occurs only in a subset of cells that form the transgenic animal. Further, the founder may contain various retroviral insertions of the transgene at different positions in the genome that generally will segregate in the offspring. In addition, it is also possible to introduce transgenes into the germline, albeit with low efficiency, by intrauterine retroviral infection of the midgestation embryo (Jahner *et al., supra* [1982]). Additional means of using retroviruses or retroviral vectors to create transgenic animals known to the art involve the micro-injection of retroviral particles or mitomycin C-treated cells producing retrovirus into the perivitelline space of fertilized eggs or early embryos (PCT International Application WO 90/08832 [1990], and Haskell and Bowen, Mol. Reprod. Dev., 40:386 [1995]).

In other embodiments, the transgene is introduced into embryonic stem cells and the transfected stem cells are utilized to form an embryo. ES cells are obtained by culturing pre-implantation embryos *in vitro* under appropriate conditions (Evans et al., Nature 292:154 [1981]; Bradley et al., Nature 309:255 [1984]; Gossler et al., Proc. Acad. Sci. USA 83:9065 [1986]; and Robertson et al., Nature 322:445 [1986]). Transgenes can be efficiently introduced into the ES cells by DNA transfection by a variety of methods known to the art including calcium phosphate co-precipitation, protoplast or spheroplast fusion, lipofection and DEAE-dextran-mediated transfection. Transgenes may also be introduced into ES cells by retrovirus-mediated transduction or by micro-injection. Such transfected ES cells can thereafter colonize an embryo following their introduction into the blastocoel of a blastocyst-stage embryo and contribute to the germ line of the resulting chimeric animal (for review, *See,* Jaenisch, Science 240:1468 [1988]). Prior to the introduction of transfected ES cells into the blastocoel, the transfected ES cells may be subjected to various selection protocols to enrich for ES cells which have integrated the transgene assuming that the transgene provides a means for such selection. Alternatively, the polymerase chain reaction may be used to screen for ES cells that have integrated the transgene. This technique obviates the need for growth of the transfected ES cells under appropriate selective conditions prior to transfer into the blastocoel.

In still other embodiments, homologous recombination is utilized to knock-out gene function or create deletion mutants (*e.g*., truncation mutants). Methods for homologous recombination are described in U.S. Pat. No. 5,614,396.

### EXPERIMENTAL

The following examples are provided in order to demonstrate and further illustrate certain preferred embodiments and aspects of the present invention and are not to be construed as limiting the scope thereof. Disclosure outside of the claims is for reference.

### Example 1: ERG and ETV1 Gene Fusions

### A. Materials and Methods

### Cancer Outlier Profile Analysis (COPA)

COPA analysis was performed on 132 gene expression data sets in Oncomine 3.0 comprising 10,486 microarray experiments. In addition, data from 99 amplified laser-capture microdissected prostate tissue samples were included in the COPA analysis. COPA has three steps. First, gene expression values are median centered, setting each gene's median expression value to zero. Second, the median absolute deviation (MAD) is calculated and scaled to 1 by dividing each gene expression value by its MAD. Median and MAD were used for transformation as opposed to mean and standard deviation so that outlier expression values do not unduly influence the distribution estimates, and are thus preserved post-normalization. Third, the 75th, 90th, and 95th percentiles of the transformed expression values are tabulated for each gene and then genes are rank-ordered by their percentile scores, providing a prioritized list of outlier profiles.

### Samples

Tissues utilized were from the radical prostatectomy series at the University of Michigan and from the Rapid Autopsy Program (Shah et al., Cancer Res 64, 9209 (Dec 15, 2004)), which are both part of University of Michigan Prostate Cancer Specialized Program of Research Excellence (S.P.O.R.E.) Tissue Core.

Tissues were also obtained from a radical prostatectomy series at the University Hospital Ulm (Ulm, Germany). All samples were collected from consented patients with prior institutional review board approval at each respective institution. Total RNA from all samples was isolated with Trizol (Invitrogen) according to the manufacturer's instructions. Total RNA was also isolated from RWPE, PC3 , PC3+AR (Dai et al., Steroids 61, 531 (1996)), LNCaP, VCaP and DuCaP cell lines. RNA integrity was verified by denaturing formaldehyde gel electrophoresis or the Agilent Bioanalyzer 2100. A commercially available pool of benign prostate tissue total RNA (CPP, Clontech) was also used.

### Quantitative PCR (QPCR)

Quantitative PCR (QPCR) was performed using SYBR Green dye on an Applied Biosystems 7300 Real Time PCR system essentially as described (Chinnaiyan et al., Cancer Res 65, 3328 (2005); Rubin et al., Cancer Res 64, 3814 (2004)). Briefly, 1-5 µg of total RNA was reverse transcribed into cDNA using SuperScript III (Invitrogen) in the presence of random primers or random primers and oligo dT primers. All reactions were performed with SYBR Green Master Mix (Applied Biosystems) and 25 ng of both the forward and reverse primer using the manufacturer's recommended thermocycling conditions. All reactions were subjected to melt curve analysis and products from selected experiments were resolved by electrophoreses on 1.5% agarose gels. For each experiment, threshold levels were set during the exponential phase of the QPCR reaction using Sequence Detection Software version 1.2.2 (Applied Biosystems). The amount of each target gene relative to the housekeeping gene glyceraldehyde-3-phosphate dehydrogenase (GAPDH) for each sample was determined using the comparative threshold cycle (Ct) method (Applied Biosystems User Bulletin #2), with the cDNA sample serving as the calibrator for each experiment described in the figure legend. All oligonucleotide primers were synthesized by Integrated DNA Technologies.
GAPDH primers were as described (Vandesompele et al., Genome Biol 3, RESEARCH0034 (2002)) and all other primers are listed (Table 4).
Approximately equal efficiencies of the primers were confirmed using serial dilutions of prostate cancer cDNA or plasmid templates in order to use the comparative Ct method.

### RNA ligase mediated rapid amplification of cDNA ends (RLM-RACE)

RNA ligase mediated rapid amplification of cDNA ends was performed using the GeneRacer RLM-RACE kit (Invitrogen), according to the manufacturer's instructions. Initially, samples were selected based on expression of ERG or ETV1 by QPCR. Five micrograms of total RNA was treated with calf intestinal phosphatase to remove 5' phosphates from truncated mRNA and non-mRNA and decapped with tobacco acid phyrophosphatase. The GeneRace RNA Oligo was ligated to full length transcripts and reverse transcribed using SuperScript III. To obtain 5' ends, first-strand cDNA was amplified with Platinum Taq High Fidelity (Invitrogen) using the GeneRacer 5' Primer and ETV1 exon 4-5_r for ETV1 or the GeneRacer 5' Primer and ERG exon 4a_r or ERG exon 4b_r for ERG. Primer sequences are given (Table S2). Products were resolved by electrophoresis on 1.5% agarose gels and bands were excised, purified and TOPO TA cloned into pCR 4-TOPO. Purified plasmid DNA from at least 4 colonies was sequenced bi-directionally using M13 Reverse and M13 Forward (-20) primers or T3 and T7 primers on an ABI Model 3730 automated sequencer by the University of Michigan DNA Sequencing Core. RLM-RACEd cDNA was not used for the other assays.

### Reverse-transcription PCR for TMPRSS2:ERG fusion

After identifying TMPRSS2:ERG positive cases using QPCR as described above, the same cDNA samples were PCR amplified with Platinum Taq High Fidelity and TPRSS2:ERG primers. Products were resolved by electrophoresis, cloned into pCR 4-TOPO and sequenced as described above.

### In vitro androgen responsiveness

RWPE, LNCaP, VCap DuCaP, PC3 and PC3 cells stably transfected with the human androgen receptor (PC3+AR) (3) were treated for 24 h with 1% ethanol control or 1 nM of the synthetic androgen R1881. Total RNA was isolated and subjected to reverse transcription and QPCR as described above with ERG exon 5-6_f and _r primers. The relative amount of ERG/GAPDH for each sample was calibrated to the RWPE control sample.

### Fluorescence in situ hybridization (FISH)

Formalin- fixed paraffin-embedded (FFPE) tissue sections from normal peripheral lymphocytes and the metastatic prostate cancer samples MET-26 and MET-28 were used for interphase fluorescence *in situ* hybridization (FISH) analysis. In addition, interphase FISH was performed on a tissue microarray containing cores from FFPE sections of 13 clinically localized prostate cancer and 16 metastatic prostate cancer samples. A two-color, two-signal approach was employed to evaluate the fusion of TMPRSS2 and ETV1, with probes spanning most of the respective gene loci. The biotin-14-dCTP BAC clone RP11-124L22 was used for the ETV1 locus and the digoxin-dUTP lableled BAC clone RPP11-35CD was used for the TMPRSS2 locus. For analyzing gene rearrangements involving ERG, a split-signal probe strategy was used, with two probes spanning the ERG locus (biotin-14-dCTP labeled BAC clone RP11-476D17 and digoxin-dUTP labeled BAC clone RP11-95I21). All BAC clones were obtained from the Children's Hospital of Oakland Research Institute (CHORI). Prior to tissue analysis, the integrity and purity of all probes were verified by hybridization to metaphase spreads of normal peripheral lymphocytes. Tissue hybridization, washing and color detection were performed as described (Rubin et al., Cancer Res 64, 3814 (2004); Garraway et al., Nature 436, 117 (2005)).

### B. Results

### Cancer Outlier Profile Analysis

In recent years, gene expression profiling with DNA microarrays has become a common method to study the cancer transcriptome. Microarray studies have provided great insight into the molecular heterogeneity of cancer, often identifying novel molecular subtypes of disease that correspond to tumor histology, patient outcome, and treatment response (Valk et al., N Engl J Med 350, 1617 (2004)). However, in general, transcriptome analysis has not led to the discovery of novel causal cancer genes. It was hypothesized that rearrangements and highlevel copy number changes that result in marked over-expression of an oncogene should be evident in transcriptome data, but not necessarily by traditional analytical approaches.

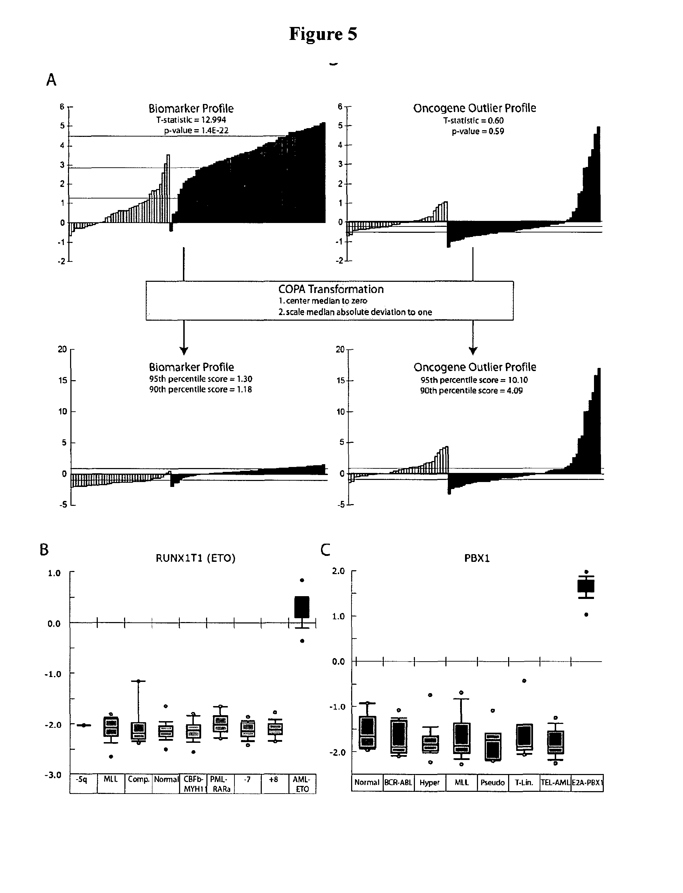

In the majority of cancer types, heterogeneous patterns of oncogene activation have been observed, thus traditional analytical methods that search for common activation of genes across a class of cancer samples (*e.g*., t-test or signal-to-noise ratio) will fail to find such oncogene expression profiles. Instead, a method that searches for marked over-expression in a subset of cases is needed. Experiments conducted during the course of development of the present invention resulted in the development of Cancer Outlier Profile Analysis (COPA). COPA seeks to accentuate and identify outlier profiles by applying a simple numerical transformation based on the median and median absolute deviation of a gene expression profile (Ross et al., Blood 102, 2951 (2003)). This approach is illustrated in Figure 5A. COPA was applied to the Oncomine database (Bittner et al., Nature 406, 536 (2000)), which comprised a compendium of 132 gene expression datasets representing 10,486 microarray experiments. COPA correctly identified several outlier profiles for genes in specific cancer types in which a recurrent rearrangement or high- level amplification is known to occur. The analysis was focused on outlier profiles of known causal cancer genes, as defined by the Cancer Gene Census (Vasselli et al., Proc Natl Acad Sci U S A 100, 6958 (2003)), that ranked in the top 10 outlier profiles in an Oncomine dataset (Table 1 and Table 3). For example, in the Valk et al. acute myeloid leukemia (AML) dataset, RUNX1T1 (ETO) had the strongest outlier profile at the 95th percentile, consistent with this gene's known translocation and oncogenic activity in a subset of AML (Davis et al., Proc Natl Acad Sci U S A 100, 6051 (2003)) (Table 1). The outlier profile precisely associated with cases that had a documented t(8;21) translocation which fuses RUNX1 (AML1) and RUNX1T1 (ETO) (Fig. 5B). Similarly, in the Ross et al. acute lymphoblastic leukemia (ALL) dataset, PBX1 showed the strongest outlier profile at the 90th percentile, consistent with the E2A-PBX1 translocation known to occur in a subset of ALL (Segal et al., J Clin Oncol 21, 1775 (2003)) (Table 1). Again, the outlier expression profile perfectly correlated with the characterized t(1;19) E2A-PBX1 translocation in this panel of ALLs (Fig. S1C).

### Identification of outlier profiles for ETS family members ERG and ETV1 in prostate cancer

Novel COPA predictions were next examined. In several independent datasets, COPA identified strong outlier profiles in prostate cancer for ERG and ETV1, two ETS family transcription factors that are known to be involved in oncogenic translocations in Ewing's sarcoma and myeloid leukemias (Lapointe et al., Proc Natl Acad Sci U S A 101, 811 (2004); Tian et al., N Engl J Med 349, 2483 (2003)). In the Dhanasekaran et al. (Keats et al., Blood 105, 4060 (2005)), Welsh et al. (Dhanasekaran et al., Faseb J 19, 243 (2005)) and Lapointe et al. (Wang et al., Lancet 365, 671 (2005)) prostate cancer gene expression datasets, ERG had the highest scoring outlier profile at the 75th percentile (Table 1), while in the Lapointe et al. and Tomlins et al. (Welsh et al., Cancer Res 61, 5974 (2001)) datasets, ETV1 had the highest scoring outlier profile at the 90th percentile (Table 1). In total, COPA ranked ERG or ETV1 within the top ten outlier genes nine times in seven independent prostate cancer profiling studies. Both ERG and ETV1 are involved in oncogenic translocations in Ewing's sarcoma. Fusion of the 5' activation domain of the EWS gene to the highly conserved 3' DNA binding domain of an ETS family member, such as ERG (t(21;22)(q22;q12)) or ETV1 (t(7;22)(p21;q12)), is characteristic of Ewing's sarcoma (Lapoint et al., supra; Zhan et al., Blood 99, 1745 (2002); Fonseca et al., Cancer Res 64, 1546 (2004)). Because translocations involving ETS family members are functionally redundant in oncogenic transformation, only one type of translocation is typically observed in each case of Ewing's sarcoma.

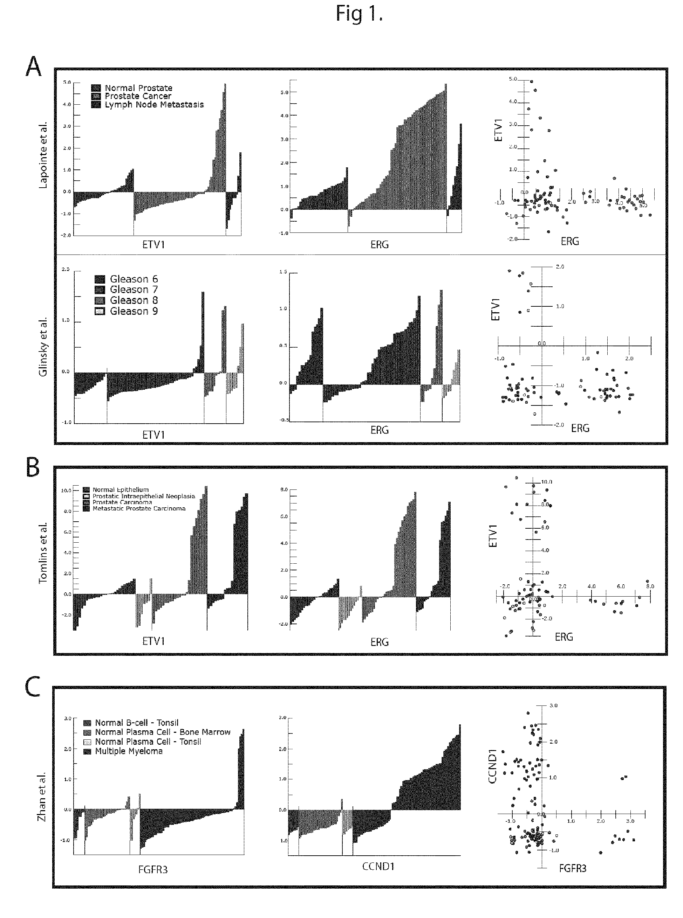

It was contemplated that if ERG and ETV1 are similarly involved in the development of prostate cancer, their outlier profiles should be mutually exclusive, that is, each case should over-express only one of the two genes. Mutations in functionally redundant genes, or genes in the same oncogenic pathway, are unlikely to be co-selected for in neoplastic progression. The joint expression profiles of ERG and ETV1 was examined across several prostate cancer datasets and it was found that they showed mutually exclusive outlier profiles. ERG and ETV1 expression profiles from two large-scale transcriptome studies (Wang et al., supra; Cheok et al., Nat Genet 34, 85 (2003)), which profiled grossly dissected prostate tissues using different microarray platforms, were identified (Fig. 1A, left and middle panels). The study by Lapointe et al. profiled benign prostate tissue, clinically localized prostate cancer, and metastatic prostate cancer, with ERG and ETV1 outlier expression restricted to prostate cancer and metastatic prostate cancer, while the study by Glinsky et al. profiled clinically localized prostate cancer samples only. In both studies, prostate cancers exclusively expressed ERG or ETV1 (Fig. 1A, right panel). Similar results were found in a profiling study of 99 prostate tissue samples obtained by laser capture microdissection (LCM) (Welsh et al., supra). In addition to exclusive outlier expression of either ERG or ETV1 (Fig. 1B, right panel), results from the LCM study demonstrated that ETV1 and ERG are only over-expressed in epithelial cells from prostate cancer or metastatic prostate cancer, but not in the putative precursor lesion prostatic intraepithelial neoplasia (PIN) or adjacent benign epithelia. To directly determine whether the observed exclusive outlier pattern is consistent with other translocations where an activating gene can fuse with multiple partners, the Zhan et al. multiple myeloma dataset (Dhanasekaran et al., Nature 412, 822 (2001)) was examined. Recurrent fusions of the immunoglobulin heavy chain promoter to CCND1 or FGFR3, t(11,14) or t(4,14) respectively, characterize specific subsets of multiple myeloma (Wigle et al., Cancer Res 62, 3005 (2002)). These translocations were reflected in the outlier profile analysis (Fig. 1C), as CCND1 was the highest scoring outlier at the 75th percentile and FGFR3 was the third highest scoring outlier at the 95th percentile (Table 1). Except for two cases, myeloma samples showed exclusive over-expression of CCND1 or FGFR3 (Fig. 1C, right panel). Taken together, the outlier profiles of ERG and ETV1 across multiple prostate cancer data sets are consistent with other causal mutations in various human malignancies. The exclusive over-expression of ERG or ETV1 in individual prostate cancer samples is consistent with other neoplasms in which an activating gene can fuse with biologically redundant partner genes, such as in multiple myeloma.

### Discovery of a recurrent gene fusion of TMPRSS2 to ERG or ETV1 in prostate cancer.

The mechanism of ERG and ETV1 over-expression in individual prostate cancer samples was next determined. Prostate cancer cell lines and clinical specimens that over-expressed ERG or ETV1 were identified by performing quantitative PCR (QPCR) (Fig. 2A). The LNCaP prostate cancer cell line and two specimens obtained from a patient who died of hormone refractory metastatic prostate cancer (MET-26RP, residual primary carcinoma in the prostate and MET-26LN, a lymph node metastasis) markedly over-expressed ETV1 by QPCR (Fig. 2A). Five independent metastatic foci from different anatomical locations as well as the residual carcinoma in the prostate from this patient also over-expressed ETV1 by DNA microarray analysis (Welsh et al., supra), suggesting that ETV1 activation occurred in the primary tumor before widespread metastasis. A lymph node metastasis was also identified from a second patient who died of hormone refractory metastatic prostate cancer (MET-28LN) and two prostate cancer cell lines, VCaP and DuCaP, that over-expressed ERG (Fig. 2A). These cell lines were independently isolated from a vertebral metastasis (VCaP) and a dural metastasis (DuCaP) from a third patient with hormone-refractory prostate cancer (Golub et al., Science 286, 531 (1999); Rosenwald et al., Cancer Cell 3, 185 (2003)). The common over-expression of ERG in these two cell lines again suggests that ERG activation occurred before widespread metastasis. Taken together, these results suggest that specific genetic events may activate ERG or ETV1 in individual samples during prostate tumorigenesis.

In an effort to characterize these genetic events, samples with high ERG or ETV1 expression were tested for chromosomal amplifications at their respective loci (7p21.2 and 21q22.3). By QPCR on genomic DNA, amplification of ERG or ETV1 in samples with respective transcript over-expression (Sotiriou et al., Proc Natl Acad Sci U S A 100, 10393 (2003)) was not found. Next, the occurrence of DNA rearrangements was assayed. Because the primers used for the QPCR described above were located 5' to the known breakpoints for ERG and ETV1 in Ewing's sarcoma, it was unlikely that the same translocations occur in prostate cancer. Accordingly, the expression level of ETV1 exons was measured by exonwalking QPCR in the samples identified above that displayed ETV1 over-expression. Five primer pairs spanning ETV1 exons 2 through 7 were used and LNCaP cells showed essentially uniform over-expression of all measured ETV1 exons, and both MET26 specimens showed > 90% reduction in the expression of ETV1 exons 2 and 3 compared to exons 4-7 (Fig. 2B). Potential explanations for this result include alternative splicing, a novel cancer-specific isoform or an unreported rearrangement.

In order to characterize the full length ETV1 transcript, 5' RNA ligase-mediated rapid amplification of cDNA ends (RLM-RACE) was performed on LNCaP cells and MET26-LN. In addition, RLM-RACE was performed to obtain the full length transcript of ERG in MET28-LN. For PCR amplification of ETV1 from the RLM-RACE cDNA, a forward primer complementary to the RNA-oligonucleotide ligated to the 5' end of complete transcripts and a reverse primer in exon 4, the 5'- most exon that was over-expressed in both LNCaP cells and MET26-LN was used. Utilizing a similar strategy as described above, it was determined that exon 4 of ERG was over-expressed in MET28-LN. A reverse primer in this exon was utilized for PCR amplification of RLM-RACE cDNA. Sequencing of the cloned products revealed fusions of the prostate specific gene TMPRSS2 (28) (21q22.2) with ETV1 in MET26-LN and with ERG in MET28-LN (Fig. 2C). In MET26-LN, two RLM-RACE PCR products were identified.

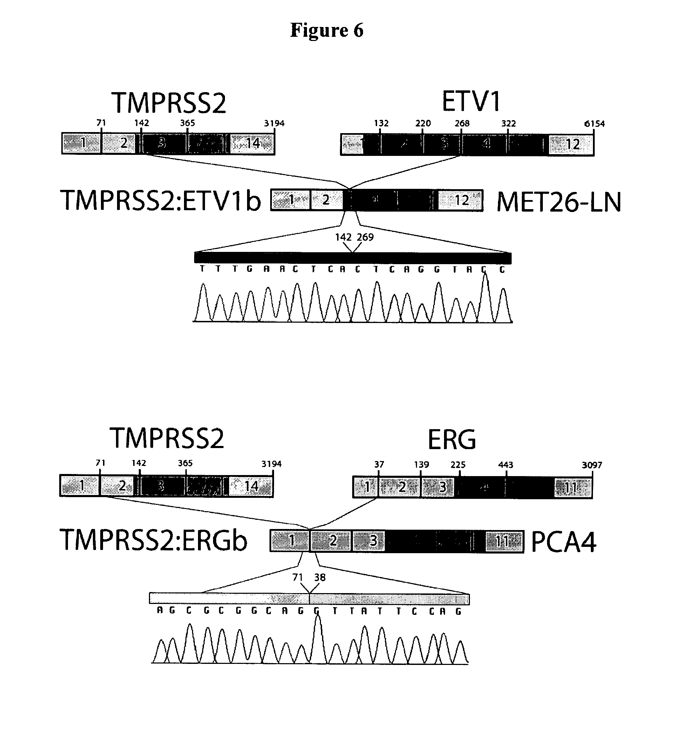

The first product, TMPRSS2:ETV1a, resulted in a fusion of the complete exon 1 of TMPRSS2 with the beginning of exon 4 of ETV1 (Fig. 2C). The second product, TMPRSS2:ETV1b, resulted in a fusion of exons 1 and 2 of TMPRSS2 with the beginning of exon 4 of ETV1 (Fig. 6). Both products are consistent with the exon-walking QPCR described above, where MET26-LN showed loss of over-expression in exons 2 and 3. In MET28-LN, a single RLM-RACE PCR product was identified and sequencing revealed a fusion of the complete exon 1 of TMPRSS2 with the beginning of exon 4 of ERG (TMPRSS2:ERGa) (Fig. 2C).

### Validation of TMPRSS2:ERG and TMPRSS2:ETV1 gene fusions in prostate cancer

Based on these results, QPCR primer pairs were designed with forward primers in TMPRSS2 and reverse primers in exon 4 of ERG and ETV1. SYBR Green QPCR was performed using both primer pairs across a panel of samples from 42 cases of clinically localized prostate cancer and metastatic prostate cancer, with representative results depicted (Fig. 2, D and E). These results demonstrate that only samples with high levels of ETV1 or ERG express the respective fusion product with TMPRSS2. Although QPCR resulted in measurable product after 35 cycles in some negative samples, melt curve analysis revealed distinct products in positive and negative samples, and gel electrophoresis of products after the 40 cycle QPCR analysis revealed only primer dimmers in negative fusion samples (Fig. 2, D and E). The formation of primer dimers may in part be explained by the difficulty in designing primers entirely in exon 1 of TMPRSS2 due to the high GC content (80.3%). However, the specific expression of TMPRSS2:ERGa, TMPRSS2:ETV1a and TMPRSS2:ETV1b fusions was confirmed using Taqman QPCR, with the forward primer spanning the respective fusion, and in each case, products were only detected in the same cases as the SYBR Green QPCR (Sotiriou et al., supra). To further confirm the specificity of the primers used for SYBR Green QPCR and the amplicons, standard reverse-transcription PCR was performed with the same primers as the SYBR Green QPCR on a panel of samples that expressed TMPRSS2:ERGa. Similar sized products were obtained and sequencing of cloned products confirmed the presence of TMPRSS2:ERGa. Two cases, PCA16 and PCA17, which expressed high levels of ETV1 or ERG respectively, but showed no evidence of the translocation by QPCR (Fig. 2, D and E) were identified. RLM-RACE supported these results, as sequencing of the product produced with ETV1 primers in PCA16 revealed no evidence of a fusion transcript and no product could be obtained with ERG primers in PCA17. Similar results were obtained for LNCaP cells, with no evidence of a fusion by RLMRACE or QPCR, consistent with the exon walking QPCR described above.

### Summary of evidence for TMPRSS2 fusion transcripts with ETS family members in prostate cancer samples

Results from three different assays for the TMPRSS2:ERG and TMPRSS2:ETV1 fusion transcripts including sequencing of RLM-RACE products, QPCR and sequencing of RT-PCR products are summarized in Table 2. In addition to QPCR for TMPRSS2 fusions being performed in all samples, the existence of these fusions was confirmed using several techniques on selected samples. For example, in PCA1 (prostate cancer sample 1), TMPRSS2:ERGa was identified using sequencing of RLMRACE products, QPCR and sequencing of RT-PCR products. By QPCR melt curve analysis and gel electrophoresis of QPCR products, PCA4 produced a larger amplicon than expected. Subsequent RLM-RACE analysis confirmed a fusion of the complete exon 1 of TMPRSS2 with the beginning of exon 2 of ERG (TMPRSS2:ERGb) (Fig. 6). Taqman QPCR with the forward primer spanning the TMPRSS2:ERGb junction confirmed the presence of TMPRSS2:ERGb only in PCA4 and Taqman QPCR with the forward primer spanning the TMPRSS2:ERGa junction did not produce a product in this specimen (27). Evidence for the TMPRSS2:ERG and TMPRSS2:ETV1 fusions were only found in cases that over-expressed ERG or ETV1 respectively, by QPCR or DNA microarray. These results are in agreement with the exclusive expression observed in the outlier analysis.

### Fluorescence in situ hybridization (FISH) confirms TMPRSS2:ETV1 translocation and ERG rearrangement

After confirming the existence of the TMPRSS2:ETV1 and TMPRSS2:ERG fusion transcripts, evidence of these rearrangements at the chromosomal level was obtained using interphase fluorescence *in situ* hybridization (FISH) on formalin fixed paraffin embedded (FFPE) specimens. Two different probe strategies were employed: a twocolor, fusion-signal approach to detect TMPRSS2:ETV1 translocations and a two-color, split-signal approach to detect rearrangements of the ERG locus. These probe strategies were validated on the two cases initially used for RLM-RACE, MET26 and MET28 (Fig. 3). Using probes for TMPRSS2 and ETV1, normal peripheral lymphocytes (NPLs) demonstrated a pair of red and a pair of green signals (Fig. 3A). MET26 showed fusion of one pair of signals, indicative of probe overlap (Fig. 3B, yellow arrowhead), consistent with the expression of the TMPRSS2:ETV1 transcript in this sample. In addition, consistent low- level amplification of the ETV1 locus was identified, as indicated by the two remaining signals for ETV1 (Fig. 3B, red arrowheads). Similarly, using probes spanning the 5' and 3' region of the ERG locus, a pair of yellow signals in NPLs was observed (Fig. 3C). In MET28, one pair of probes split into separate green and red signals, indicative of a rearrangement at the ERG locus (Fig. 3D, green and red arrows). This result is consistent with the expression of the TMPRSS2:ERG transcript in this case. Based on these results, the individual FISH analyses described above were performed on serial tissue microarrays containing cores from 13 cases of localized prostate cancer and 16 cases of metastatic prostate cancer (Fig. 3E). As indicated by the matrix, 23 of 29 cases (79.3%) showed evidence of TMPRSS2:ETV1 fusion (7 cases) or ERG rearrangement (16 cases). In addition, 12 of 29 cases (41.4%) showed evidence of low level amplification at the ETV1 locus. Previous reports have identified the genomic location of ETV1, 7p, as one of the most commonly amplified regions in localized and metastatic prostate cancer (Slamon et al., Science 235, 177 (1987)). However it does not appear that 7p amplification drives ETV1 expression, as ETV1 amplification occurred in 6 cases with ERG rearrangements and our transcript data demonstrates that 0 of 19 samples with high ERG expression and the TMPRSS2:ERG fusion also have high ETV1 expression. Furthermore, when both ETV1 amplification and the TMPRSS2:ETV1 fusion were present by FISH, only the individual ETV1 signal was amplified and not the fused signal. Nevertheless, results from this FISH analysis demonstrate the presence of TMPRSS2:ETV1 and ERG rearrangements at the genomic level consistent with the transcript data described above.

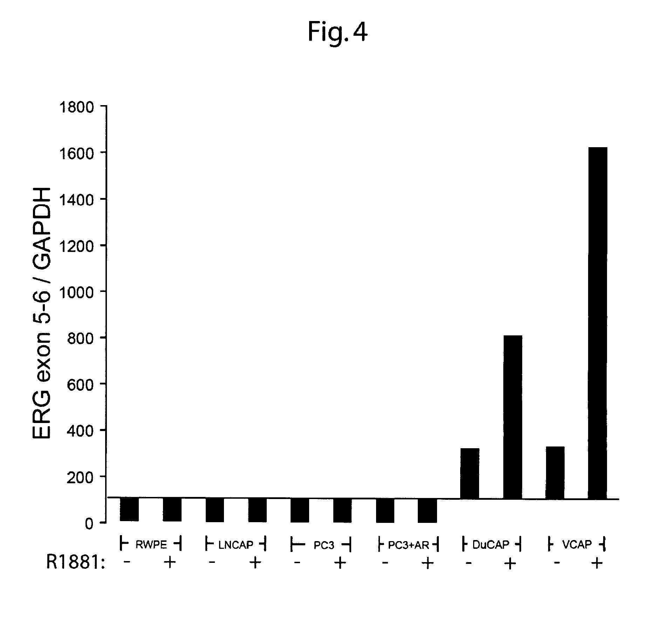

TMPRSS2 is an androgen-regulated gene and fusion with ERG results in androgen regulation of ERG. TMPRSS2 was initially identified as a prostate-specific gene whose expression was increased by androgen in LNCaP cells and also contains androgen responsive elements (AREs) in its promoter (Huang et al., Lancet 361, 1590 (2003); Schwartz et al., Cancer Res 62, 4722 (2002)). Subsequent studies have confirmed high expression in normal and neoplastic prostate tissue and demonstrated that TMPRSS2 is androgen-regulated in androgen-sensitive prostate cell lines (Schwartz et al., Cancer Res 62, 4722 (2002); Ferrando et al., Cancer Cell 1, 75 (2002); Chen et al., Mol Biol Cell 14, 3208 (2003); LaTulippe et al., Cancer Res 62, 4499 (2002)). In addition, while androgen does not increase the expression of TMPRSS2 in the androgen insensitive prostate cancer cell line PC3, stable expression of the androgen receptor in PC3 cells resulted in TMPRSS2 becoming androgen responsive (Schwartz et al., supra; Ferrando et al., supra; Chen et al., supra; LaTulippe et al., supra). In contrast, microarray studies of LNCaP prostate cell lines treated with androgen have not identified ERG or ETV1 as being androgen-responsive (Jain et al., Cancer Res 64, 3907 (2004)) and examination of their promoter sequences did not reveal consensus AREs (Sotiriou et al., supra). It was contemplated that the TMPRSS2:ERGa fusion in DuCaP and VCaP cell lines, which was confirmed by three independent assays in each cell line (Table 2), would result in the androgen regulation of ERG. Using QPCR to assay for ERG expression, it was confirmed that even though ERG was highly expressed in both VCaP and DuCaP cells, treatment with the synthetic androgen R1881 increased the expression of ERG 2.57 fold in DuCaP cells and 5.02 fold in VCaP cells compared to untreated controls (Fig. 4). Expression of ERG was minimal and essentially unchanged after R1881 treatment in RWPE (1.37 fold), LnCaP (0.86 fold), PC3 (1.28 fold) and PC3 cells expressing the androgen receptor (0.73 fold) compared to untreated controls.

Microarray analysis of the same samples confirmed that ERG was only up-regulated in response to androgen in DuCaP and VCaP cells (Sotiriou et al., supra). The present invention is not limited to a particular mechanism. Indeed, an understanding of the mechanism is not necessary to practice the present invention. Nonetheless, it is contemplated that these results suggest a possible mechanism for the aberrant expression of ERG or ETV1 in prostate cancer when respective fusions with TMPRSS2 are present.
Table 1. Cancer Outlier Profile Analysis (COPA). Genes known to undergo causal mutations in cancer that had strong outlier profiles. "X", signifies literature evidence for acquired pathogenomic translocation. "XX" signifies literature evidence for the specific translocation as well as the samples in the specific study that were characterized for that translocation. "Y" signifies consistent with known amplification. "**" signifies ERG and ETV1 outlier profiles in prostate cancer.

**Table 1**

| **Rank** | **%** | **Score** | **Study** | **Cancer** | **Gene** | **Evidence** |
|---|---|---|---|---|---|---|
| 1 | 95 | 20.056 | Valk et al., N Engl J Med 350, 1617 (2004) | Leukemia | *RUNX1T1* | XX |
| 1 | 95 | 15.4462 | Vasselli et al., PNAS USA 100, 6958 (2003) | Renal | *PRO1073* | X |
| 1 | 90 | 12.9581 | Ross et al., Blood 102, 2951 (2003). | Leukemia | *PBX1* | XX |
| 1 | 95 | 10.03795 | Lapointe et al., PNAS USA 101, 811 (Jan 20, 2004) | Prostate | *ETV1* | ** |
| 1 | 90 | 9.1163 | | Prostate | *ETV1* | ** |
| 1 | 90 | 7.4557 | Tian et al., N Engl J Med 349, 2483 (2003) | Myeloma | *WHSC1* | X |
| 1 | 75 | 5.4071 | Dhanasekaran et al., Nature 412, 822 (2001) | Prostate | *ERG* | ** |
| 1 | 75 | 4.3628 | Welsh et al., Cancer Res 61, 5974 (2001) | Prostate | *ERG* | ** |
| 1 | 75 | 4.3425 | Zhan et al., Blood 99, 1745 (2002) | Myeloma | *CCND1* | X |
| 1 | 75 | 3.4414 | Lapointe et al., supra | Prostate | *ERG* | ** |
| 1 | 75 | 3.3875 | Dhanasekaran et al., Faseb J 19, 243 (2005) | Prostate | *ERG* | ** |
| 2 | 90 | 6.7029 | | Prostate | *ERG* | ** |
| 3 | 95 | 13.3478 | Zhan et al., supra | Myeloma | *FGFR3* | X |
| 4 | 75 | 2.5728 | Huang et al., Lancet 361, 1590 (2003) | Breast | *ERBB2* | Y |
| 6 | 90 | 6.6079 | Sotiriou et al., PNAS USA 100, 10393 (2003) | Breast | *ERBB2* | Y |
| 9 | 95 | 17.1698 | Glinsky et al., J Clin Invest 113, 913 (2004) | Prostate | *ETV1* | ** |
| 9 | 90 | 6.60865 | Nielsen et al., Lancet 359, 1301 (2002) | Sarcoma | *SSX1* | X |
| 9 | 75 | 2.2218 | Yu et al., J Clin Oncol 22, 2790 (2004) | Prostate | *ERG* | ** |

Table 2 shows a summary of TMPRSS2 fusion to ETS family member status in prostate cancer samples and cell lines. For all assays, positive results are indicated by "+" and negative results are indicated by "-". Blank cells indicate that the specific assay was not performed for that sample. Over-expression of ERG or ETV1 by quantitative PCR (QPCR) is indicated and samples marked with an asterisk indicate the sample was also assessed by cDNA microarray and over-expression was confirmed. In order to detect TMPRSS2:ERG or TMPRSS2:ETV1 gene fusions, selected samples were subjected to RLM-RACE for the over-expressed ETS family member and samples with the TMPRSS2 fusion after sequencing are indicated. All samples were assayed for TMPRSS2:ETV1 and TMPRSS2:ERG expression by QPCR. Selected cases were also amplified by standard reverse-transcription PCR (RT-PCR) using the same TMPRSS2 fusion primers as for QPCR and amplicons were sequenced. Samples with evidence for TMPRSS2:ETV1 or TMPRSS2:ERG fusion are indicated in the final column.

**Table 2**

| | | | **TMPRSS2:ETS family member gene fusion assays** | | | | **TMPRSS2 :ETS** |
|---|---|---|---|---|---|---|---|
| | | | | | | | |
| | | **QPCR** | **RLM-RACE** | **QPCR** | **QPCR** | **RT-PCR** | **family member fusion** |
| **Case** | **Sample** | **Expression** | **sequencing** | **TMPRSS2: ETV1** | **TMPRSS2: ERG** | **sequencing** | |
| 1 | MET26-LN | ETV1* | + | + | | | + |
| 1 | MET26-RP | ETV1* | | + | | | + |
| 2 | MET28-B | ERG | | - | + | | + |
| 2 | MET28-PTLN | ERG | | - | + | | + |
| 2 | MET28-41 | ERG | | - | + | | + |
| 2 | MET28-LN | ERG | + | - | + | | + |
| 3 | MET 16-44 | ERG | | - | + | | + |
| 3 | MET 16-47 | ERG | | - | + | | + |
| 4 | MET3 | ERG* | | - | + | | + |
| 5 | MET18-25 | ERG* | | - | + | + | + |
| 6 | PCA1 | ERG* | + | - | + | + | + |
| 7 | PCA2 | ERG* | | - | + | + | + |
| 8 | PCA3 | ERG* | | - | + | + | + |
| 9 | PCA4 | ERG* | + | - | + | | + |
| 10 | PCA5 | ERG* | + | - | + | | + |
| 11 | PCA6 | ERG* | | - | + | | + |
| 12 | PCA7 | ERG* | + | - | + | | + |
| 13 | PCA8 | ERG* | | - | + | | + |
| 14 | PCA9 | ERG* | | - | + | | + |
| 15 | PCA10 | ERG* | | - | + | | + |
| 16 | PCA11 | ERG* | | - | + | | + |
| 17 | PCA12 | ERG* | | - | + | | + |
| 18 | PCA13 | ERG* | | - | + | | + |
| 19 | PCA14 | ERG* | | - | + | | + |
| 20 | PCA15 | ERG* | | - | + | | + |
| 21 | PCA16 | ETV1* | - | - | - | | - |
| 22 | PCA17 | ERG* | - | - | - | | - |
| 23 | MET30-LN | - | | - | - | | - |
| 24 | MET17-12 | - | | - | - | | - |
| 25 | MET20-76 | - | | - | - | | - |
| 26 | MET22-61 | - | | - | - | | - |
| 27 | MET5-7 | - | | - | - | | - |
| 28 | PCA18 | - | | - | - | | - |
| 29 | PCA19 | - | | - | - | | - |
| 30 | PCA20 | - | | - | - | | - |
| 31 | PCA21 | - | | - | - | | - |
| 32 | PCA22 | - | | - | - | | - |
| 33 | PCA23 | - | | - | - | | - |
| 34 | PCA24 | - | | - | - | | - |
| 35 | PCA25 | - | | - | - | | - |
| 36 | PCA26 | - | | - | - | | - |
| 37 | PCA27 | - | | - | - | | - |
| 38 | PCA28 | - | | - | - | | - |
| 39 | PCA29 | - | | - | - | | - |
| 40 | PCA30 | - | | - | - | | - |
| 41 | PCA31 | - | | - | - | | - |
| 42 | PCA32 | - | | - | - | | - |
| Cell line | VCap | ERG | + | - | + | | + |
| Cell line | DUCaP | ERG | | - | + | + | + |
| Cell line | LnCaP | ETV1 | - | - | - | | - |
| Cell line | DU145 | - | | - | - | | - |
| Cell line | PC3 | - | | - | - | | - |
| Cell line | RWPE | - | | - | - | | - |

Table 3. Cancer Outlier Profile Analysis (COPA). Genes that are known to undergo causal mutations in cancer that had an outlier profile in the top 10 of a study in Oncomine are shown. "X", signifies literature evidence for acquired pathognomonic translocation. "XX" signifies literature evidence for the specific translocation as well as that the samples in the specific study were characterized for that translocation. "Y" signifies consistent with known amplification. "**" signifies ERG and ETV1 outlier profiles in prostate cancer.

**Table 3**

| **Rank** | **%** | **Score** | **Study** | **Cancer** | **Reference** | **Gene** | **Evidence** |
|---|---|---|---|---|---|---|---|
| 1 | 90 | 21.9346 | Bittner et al. | Melanoma | Nature 406,536 (2000) | CDH1 | |
| 1 | 95 | 20.056 | Valk et al. | Leukemia | Nature 406,536 (2000) | RUNX1T1 | XX |
| 1 | 95 | 15.4462 | Vasselli et al. | Renal | PNAS USA 100, 6958 (2003) | PRO1073 | X (*12*) |
| 1 | 95 | 14.2008 | Segal et al. | Sarcoma | J Clin Oncol 21, 1775 (2003) | MYH11 | |
| 1 | 90 | 12.9581 | Ross et al. | Leukemia | Blood 102,2951 (2003) | PBX1 | XX |
| 1 | 95 | 10.03795 | Lapointe et al. | Prostate | PNAS USA 101, 811 (2004) | ETV1 | ** |
| 1 | 90 | 9.1163 | | Prostate | | ETV1 | ** |
| 1 | 90 | 7.4557 | Tian et al. | Myeloma | N Engl J Med 349, 2483 (2003) | WHSCl | X (16) |
| 1 | 75 | 5.4071 | Dhanasekaran et al. | Prostate | Faseb J 19,243 (2005) | ERG | ** |
| 1 | 75 | 5.2067 | Wang et al. | Breast | Lancet 365,671 (2005) | FOXO3A | |
| 1 | 75 | 4.3628 | Welsh et al. | Prostate | Cancer Res 61, 5974 (2001) | ERG | ** |
| 1 | 75 | 4.3425 | Zhan et al. | Myeloma | Blood 99, 1745 (2002) | CCND1 | X (*21*) |
| 1 | 75 | 3.724 | Cheok et al. | Leukemia | Nat Genet 34, 85 (May, 2003) | PCSK7 | |
| 1 | 75 | 3.4414 | Lapointe et al. | Prostate | PNAS USA 101, 811 (2004) | ERG | ** |
| 1 | 75 | 3.3875 | Dhanasekaran et al. | Prostate | Nature 412, 822 (2001) | ERG | ** |
| 1 | 75 | 2.5913 | Wigle et al. | Lung | Cancer Res 62, 3005 (2002) | IGH@ | |
| 2 | 90 | 12.7953 | Ross et al. | Leukemia | Blood 102,2951 (2003) | HOXA9 | |
| 2 | 95 | 9.2916 | Golub et al. | Leukemia | Science 286,531 (1999) | TRA@ | |
| 2 | 95 | 9.2916 | Golub et al. | Leukemia | Science 286,531 (1999) | TRD@ | |
| 2 | 90 | 8.2292 | Cheok et al. | Leukemia | Nat Genet 34, 85 (May, 2003) | SSX2 | |
| 2 | 90 | 6.7029 | | Prostate | | ERG | ** |
| 3 | 95 | 13.3478 | Zhan et al. | Myeloma | Blood 99, 1745 (2002) | FGFR3 | X (*21*) |
| 3 | 95 | 10.2267 | Cheok et al. | Leukemia | Nat Genet 34, 85 (May, 2003) | ARHGAP26 | |
| 3 | 90 | 5.9174 | | Prostate | | REL | |
| 3 | 75 | 2.6162 | Rosenwald et al. | Lymphoma | Cancer Cell 3, 185 (2003) | TCL1A | |
| 3 | 75 | 2.036 | Sotiriou et al. | Breast | PNAS USA 100, 10393 (2003) | RAD51L1 | |
| 4 | 75 | 8.4985 | Bittner et al. | Melanoma | Nature 406,536 (2000) | TP53 | |
| 4 | 90 | 5.4881 | Golub et al. | Leukemia | Science 286,531 (1999) | LCK | |
| 4 | 75 | 2.5728 | Huang et al. | Breast | Lancet 361, 1590 (2003) | ERBB2 | Y(29) |
| 4 | 75 | 2.0229 | Schwartz et al. | Ovarian | Cancer Res 62, 4722 (2002) | IGL@ | |
| 6 | 90 | 17.3733 | Ferrando et al. | Leukemia | Cancer Cell 1, 75 (2002) | ZBTB16 | |
| 6 | 95 | 9.1267 | Chen et al. | Gastric | Mol Biol Cell 14, 3208 (2003) | FGFR2 | |
| 6 | 90 | 6.6079 | Sotiriou et al. | Breast | PNAS USA 100, 10393 (2003) | ERBB2 | Y(29) |
| 6 | 75 | 5.7213 | LaTulippe et al. | Prostate | Cancer Res 62, 4499 (2002) | NF1 | |
| 6 | 75 | 5.2752 | Jain et al. | Endocrine | Cancer Res 64, 3907 (2004) | PHOX2B | |
| 6 | 90 | 4.8383 | Lapointe et al. | Prostate | PNAS USA 101, 811 (2004) | LAF4 | |
| 6 | 90 | 4.1779 | Alizadeh et al. | Lymphoma | Nature 403,503 (2000) | IRTA1 | |
| 6 | 90 | 3.6325 | Rosenwald et al. | Lymphoma | N Engl J Med 346, 1937 (2002) | IRTA1 | |
| 6 | 75 | 1.85865 | Chen et al. | Liver | Mol Biol Cell 13, 1929 (2002) | HMGA1 | |
| 7 | 95 | 4.7561 | Alon et al. | Colon | Proc Natl Acad Sci USA96, 6745 (1999) | NONO | |
| 7 | 75 | 1.8133 | Chen et al. | Liver | Mol Biol Cell 13, 1929 (2002) | GPC3 | |
| 8 | 90 | 4.7068 | Lacayo et al. | Leukemia | Blood 104,2646 (2004) | EVI1 | |
| 8 | 90 | 4.7068 | Lacayo et al. | Leukemia | Blood 104,2646 (2004) | MDS1 | |
| 9 | 95 | 17.1698 | Glinsky et al. | Prostate | J Clin Invest 113, 913 (2004) | ETV1 | ** |
| 9 | 90 | 15.3889 | Ferrando et al. | Leukemia | Ferrando et al., Cancer Cell 1, 75 (2002) | MN1 | |
| 9 | 90 | 6.60865 | Nielsen et al. | Sarcoma | Lancet 359, 1301 (2002) | SSX1 | X (*42*) |
| 9 | 90 | 4.4875 | Lapointe et al. | Prostate | PNAS USA 101, 811 (2004) | CHEK2 | |
| 9 | 75 | 2.2218 | Yu et al. | Prostate | J Clin Oncol 22, 2790 (2004) | ERG | ** |
| 10 | 95 | 10.6036 | Segal et al. | Sarcoma | Segal et al., J Clin Oncol 21, 1775 (2003) | KIT | |

Table 4. Oligonucleotide primers used in this study. For all primers, the gene, bases and exons (according to alignment of the reference sequences described in the text with the May 2004 assembly of the human genome using the UCSC Genome Browser) are listed. Forward primers are indicated with "f" and reverse primers with "r".

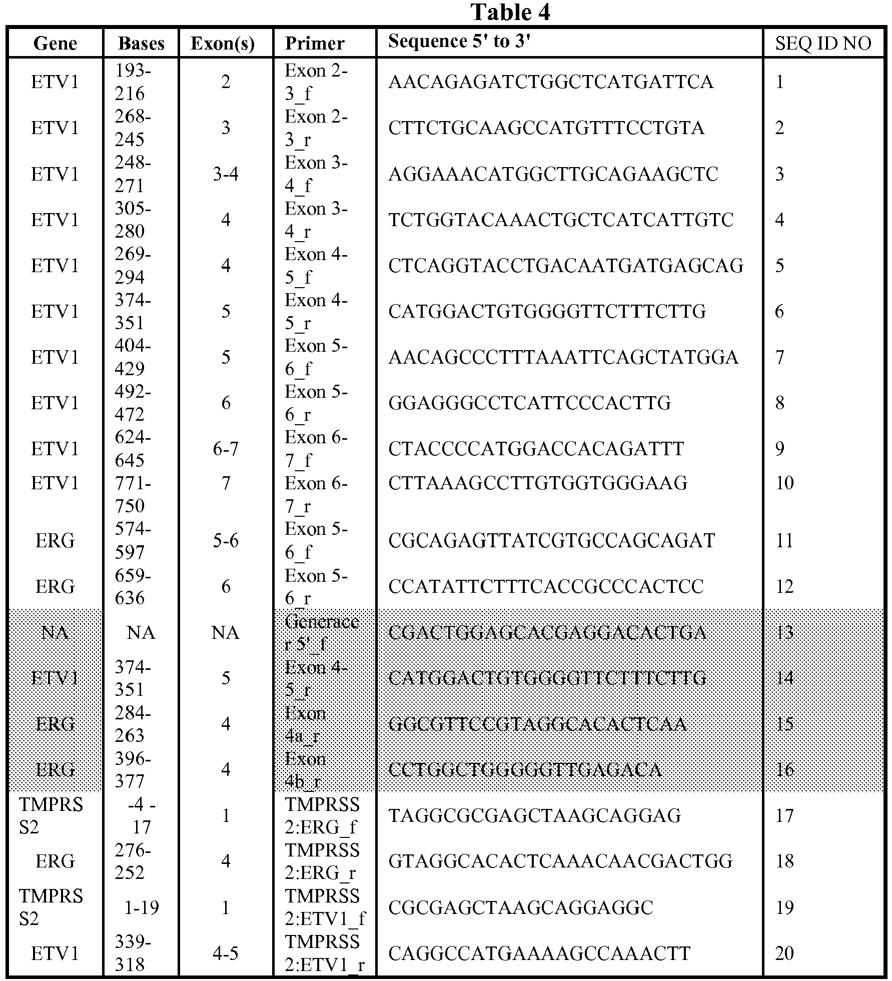

### Example 2: ETV4 Gene Fusions

### A. Materials and Methods

### ETS family expression in profiling studies

To investigate the expression of ETS family members in prostate cancer, two prostate cancer profiling studies were utilized (Lapointe et al., Proc Natl Acad Sci U S A 2004;101:811-6 and Tomlins et al., Science 2005;310:644-8) present in the Oncomine database (Rhodes et al., Neoplasia 2004;6:1-6). Genes with an ETS domain were identified by the Interpro filter 'Ets' (Interpro ID: IPR000418). Heatmap representations were generated in Oncomine using the 'median-center per gene' option, and the color contrast was set to accentuate ERG and ETV1 differential expression.

### Samples

Prostate cancer tissues (PCA1-5) were from the radical prostatectomy series at the University of Michigan, which is part of the University of Michigan Prostate Cancer Specialized Program of Research Excellence (S.P.O.R.E.) Tissue Core. All samples were collected with informed consent of the patients and prior institutional review board approval. Total RNA was isolated with Trizol (Invitrogen, Carlsbad, CA) according to the manufacturer's instructions. A commercially available pool of benign prostate tissue total RNA (CPP, Clontech, Mountain View, CA) was also used.

### Quantitative PCR (QPCR)

QPCR was performed using SYBR Green dye on an Applied Biosystems 7300 Real Time PCR system (Applied Biosystems, Foster City, CA) as described (Tomlins et al., *supra*). The amount of each target gene relative to the housekeeping gene glyceraldehyde-3-phosphate dehydrogenase (GAPDH) for each sample was reported. The relative amount of the target gene was calibrated to the relative amount from the pool of benign prostate tissue (CPP). All oligonucleotide primers were synthesized by Integrated DNA Technologies (Coralville, IA). GAPDH primers were as described (Vandesompele et al., Genome Biol 2002; 3:RESEARCH0034). Primers for exons of ETV4 were as follows (listed 5' to 3'): ETV4_exon2-f: CCGGATGGAGCGGAGGATGA (SEQ ID NO:21), ETV4_exon2-r: CGGGCGATTTGCTGCTGAAG (SEQ ID NO:22), ETV4_exon3-f: GCCGCCCCTCGACTCTGAA (SEQ ID NO:23), ETV4_exon4-r: GAGCCACGTCTCCTGGAAGTGACT (SEQ ID NO:24), ETV4_exon11-f: CTGGCCGGTTCTTCTGGATGC (SEQ ID NO:25), ETV4_exon12-r: CGGGCCGGGGAATGGAGT (SEQ ID NO:26), ETV4_3'UTR-f: CCTGGAGGGTACCGGTTTGTCA (SEQ ID NO:27), ETV4_3'UTR-r: CCGCCTGCCTCTGGGAACAC (SEQ ID NO:28). Exons were numbered by alignment of the RefSeq for ETV4 (NM_001986.1) with the May 2004 freeze of the human genome using the UCSC Genome Browser. For QPCR confirmation of TMPRSS2:ETV4 fusion transcripts, TMPRSS2:ETV4a-f (AAATAAGTTTGTAAGAGGAGCCTCAGCATC (SEQ ID NO:29)) and TMPRSS2:ETV4b-f (ATCGTAAAGAGCTTTTCTCCCCGC (SEQ ID NO:30)), which detects both TMPRSS2:ETV4a and TMPRSS2;ETV4b transcripts, were used with ETV4_exon4-r.

### RNA ligase mediated rapid amplification of cDNA ends (RLM-RACE)

RLM-RACE was performed using the GeneRacer RLM-RACE kit (Invitrogen), according to the manufacturer's instructions as described (Tomlins et al., *supra*). To obtain the 5' end of ETV4, first-strand cDNA from PCA5 was amplified using the GeneRacer 5' Primer and ETV4_exon4-r or ETV4_exon7-r (GAAAGGGCTGTAGGGGCGACTGT (SEQ ID NO:31)). Products were cloned and sequenced as described (Tomlins et al., *supra*). Equivalent 5' ends of the TMPRSS2:ETV4 transcripts were obtained from both primer pairs.

### Fluorescence in situ hybridization (FISH)

Formalin-fixed paraffin-embedded (FFPE) tissue sections were used for interphase FISH. Deparaffinized tissue was treated with 0.2 M HCl for 10 min, 2x SSC for 10 min at 80°C and digested with Proteinase K (Invitrogen) for 10 min. The tissues and BAC probes were co-denatured for 5 min at 94°C and hybridized overnight at 37°C. Post-hybridization washing was with 2x SSC with 0.1% Tween-20 for 5 min and fluorescent detection was performed using anti-digoxigenin conjugated to fluorescein (Roche Applied Science, Indianapolis, IN) and streptavidin conjugated to Alexa Fluor 594 (Invitrogen). Slides were counterstained and mounted in ProLong Gold Antifade Reagent with DAPI (Invitrogen). Slides were examined using a Leica DMRA fluorescence microscope (Leica, Deerfield, IL) and imaged with a CCD camera using the CytoVision software system (Applied Imaging, Santa Clara, CA).

All BACs were obtained from the BACPAC Resource Center (Oakland, CA) and probe locations were verified by hybridization to metaphase spreads of normal peripheral lymphocytes. For detection of TMPRSS2:ETV4 fusion, RP11-35C4 (5' to TMPRSS2) was used with multiple BACs located 3' to ETV4 (distal to ETV4 to proximal: RP11-266I24, RP11-242D8, and RP11-100E5). For detection of ETV4 rearrangements, RP11-436J4 (5' to ETV4) was used with the multiple BACs 3' to ETV4. For each hybridization, areas of cancerous cells were identified by a pathologist and 100 cells were counted per sample. The reported cell count for TMPRSS2:ETV4 fusions used RP11-242D8 and similar results were obtained with all 3' ETV4 BACs. To exclude additional rearrangements in PCA5, FISH was performed with two probes 3' to ETV4 (RP11-266I24 and RP11-242D8), ERG split signal probes (RP11-95I21 and RP11-476D17) and TMPRSS2:ETV1 fusion probes (RP11-35C4 and RP11-124L22). BAC DNA was isolated using a QIAFilter Maxi Prep kit (Qiagen, Valencia, CA) and probes were synthesized using digoxigenin- or biotin-nick translation mixes (Roche Applied Science).

### B. Results

The initial COPA screen led to the characterization of TMPRSS2 fusions with ERG or ETV1 (Example 1). It was further contemplated that prostate cancers negative for these gene fusions harbor rearrangements involving other ETS family members. By interrogating the expression of all ETS family members monitored in prostate cancer profiling studies from the Oncomine database (Rhodes et al., supra), marked over-expression of the ETS family member ETV4 was identified in a single prostate cancer case from each of two studies-one profiling grossly dissected tissues (Lapointe et al., supra) (Fig. 7A) and the other profiling laser capture microdissected (LCM) tissues (Fig. 7B). As these cases did not over-express ERG or ETV1, and no benign prostate tissues showed over-expression, it was contemplated that fusion with TMPRSS2 was responsible for the over-expression of ETV4 in these cases. Although ELF3 was also over-expressed in a fraction of prostate cancer cases, in both studies normal prostate tissue samples also showed marked ELF3 over-expression, indicating that a gene fusion driving expression in both benign and cancerous tissue is unlikely. Thus, the ETV4 over-expressing case (designated here as PCA5) was further analyzed.

Total RNA was isolated from PCA5 and exon-walking quantitative PCR was used (QPCR) to confirm the over-expression of ETV4. QPCR demonstrated that exons 3' to exon 2 of ETV4 were markedly over-expressed in this case compared to pooled benign prostate tissue (CPP) (∼900 fold) and prostate cancers that did not over-express ETV4 and were either TMPRSS2:ERG positive (PCA1-2) or negative (PCA3-4) (Fig. 8A). However, a dramatic decrease (>99%) in the expression of exon 2 of ETV4 relative to distal regions in PCA5 was observed, indicating a possible fusion with TMPRSS2, as observed previously in TMPRSS2:ERG and TMPRSS2:ETV1 positive cases (Tomlins et al., supra).

To identify the 5' end of the ETV4 transcript in PCA5, RNA-ligase mediated rapid amplification of cDNA ends (RLM-RACE) was performed using a reverse primer in exon 7. RLM-RACE revealed two transcripts, each containing 5' ends consisting of sequence located approximately 8 kb upstream of TMPRSS2 fused to sequence from ETV4 (Fig 8B). Specifically, the 5' end of TMPRSS2:ETV4a has 47 base pairs from this region upstream of TMPRSS2, while the 5' end of TMPRSS2:ETV4b has the same terminal 13 base pairs. These 5' ends of both transcripts were fused to the same contiguous stretch consisting of the 9 base pairs of the intron immediately 5' to exon 3 of ETV4 and the reported reference sequence of exons 3 through the reverse primer in exon 7 of ETV4.

The existence of both transcripts in PCA5 and their absence in CPP and PCA1-4 was confirmed using QPCR. To further exclude the presence of fusion transcripts involving known exons from TMPRSS2, QPCR was performed using a forward primer in exon 1 of TMPRSS2 and the ETV4 exon 4 reverse primer, and as expected, no product was detected in CPP or PCA1-5.

Whether other prostate cancers with ETV4 dysregulation might contain TMPRSS2:ETV4 fusion transcripts structurally more similar to TMPRSS2:ERG and TMPRSS2:ETV1 transcripts (which involve known exons from TMPRSS2) is unknown. The TMPRSS2:ETV4 fusions reported here do not contain the well characterized AREs immediately upstream of TMPRSS2. However, evidence exists for androgen responsive enhancers located upstream of the TMPRSS2 sequences present in the TMPRSS2:ETV4 transcripts described here (Rabbitts, Nature 1994;372:143-9). Nevertheless, the marked over-expression of only ETV4 exons involved in the fusion transcript strongly suggests that the gene fusion is responsible for the dysregulation of ETV4. Together, the structure of the TMPRSS2:ETV4 fusion transcripts supports the conclusion that the regulatory elements upstream of TMPRSS2, rather than transcribed TMPRSS2 sequences, drive the dysregulation of ETS family members.

To confirm the fusion of the genomic loci surrounding TMPRSS2 (21q22) and ETV4 (17q21) as demonstrated by RLM-RACE and QPCR, interphase fluorescence *in situ* hybridization (FISH) was used. Using probes 5' to TMPRSS2 and 3' to ETV4, fusion of TMPRSS2 and ETV4 loci was observed in 65% of cancerous cells from PCA5 (Fig. 8D). As further confirmation of the rearrangement of ETV4, using probes 5' and 3' to ETV4, 64% of cancerous cells from PCA5 showed split signals. FISH was also performed on PCA5 using two probes 3' to ETV4, ERG split signal probes and TMPRSS2:ETV1 fusion probes to exclude additional rearrangements, with negative results obtained for each hybridization.

Taken together, the results highlight the use of carefully examining outlier profiles in tumor gene expression data, as most analytical methods discount profiles that do not show consistent deregulation (Eisen et al., Proc Natl Acad Sci U S A 1998;95:14863-8; Golub et al., Science 1999;286:531-7; Tusher et al., Proc Natl Acad Sci U S A 2001;98:5116-21) and would thus fail to identify ETV4 in prostate cancer, which appears rare (2 of 98 cases). Combined with the identification of TMPRSS2:ERG and TMPRSS2:ETV1 fusions, the results presented here show that dysregulation of ETS family members mediated by subversion of AREs or enhancers upstream of TMPRSS2 is a hallmark of prostate tumorigenesis.

### Example 3:Detection of Gene Fusion RNA

This example describes target capture, amplification and qualitative detection of RNA (IVT) containing the sequences of the four gene fusions in four separate qualitative assays: TMPRSS2:ETV1a, TMPRSS2:ETV1b, TMPRSS2:ERGa and TMPRSS2:ERGb with APTIMA formulation reagents and HPA detection each spiked with the appropriate target specific oligonucleotides, primers and probes. Table 5 shows sequences of oligonucleotides used in the assay.

**Table 5**

| Gene Fusion | Sequence (5' to | SEQ ID NO |
|---|---|---|
| TMPRSS2 exon1/Target Capture | AAAAAAAAAAAAAAAAAAAAAAAAAAAAAATTTCUCGAUUCGUCCUCCG | 59 |
| TMPRSS2 exon1/Target Capture | AAAAAAAAAAAAAAAAAAAAAAAAAAAAAATTTAUCCGCGCUCGAUUCGUC | 60 |
| TMPRSS2 exon1/Non-T7 | GAGGGCGAGGGGCGGGGAGCGCC | 61 |
| TMPFSS2 exon2/Non-T7 | CCTATCATTACTCGATGCTGTTGATAACAGC | 62 |
| ETV1a/b exon4/17 | AATTTAATACGACTCACTATAGGGAGAAACTTTCAGCCTGATA | 63 |
| ERGb exon2/T7 | AATTTAATACGACTCACTATAGGGAGACTCTGTGAGTCATTTGTCTTGCTT | 64 |
| ERGe exon4/T7 | AATTTAATACGACTCACTATAGGGAGAGCACACTCAAACAACGACTG | 65 |
| TMPRSS2exon1:ETV1a Junction/AE | GCGCGGCAG-CUCAGGUACCUGAC | 66 |
| TMPRSS2exon2:ETV1b Junction/AE | GCUUUGAACUCA-CUCAGGUACCUCGAC | 67 |
| TMPRSS2exon1:ERGa Junction/AE | GAGCGCGGCAG-GAAGCCUUAUCAGUUG | 68 |
| TMPRSS2exon1:ERGb Junction/AE | GAGCGCGGCAG-GUUAUUCCAGGAUCUUU | 69 |

### A. Materials and Methods

### RNA Target Capture

Lysis buffer contained 15 mM sodium phosphate monobasic monohydrate, 15 mM sodium phosphate dibasic anhydrous, 1.0 mM EDTA disodium dihydrate, 1.0 mM EGTA free acid, and 110 mM lithium lauryl sulfate, pH 6.7. Target capture reagent contained 250 mM HEPES, 310 mM lithium hydroxide, 1.88 M lithium chloride, 100 mM EDTA free acid, at pH 6.4, and 250 µg/ml 1 micron magnetic particles SERA-MAG MG-CM Carboxylate Modified (Seradyn, Inc., Indianapolis, Indiana) having dT)₁₄ oligomers covalently bound thereto. Wash solution contained 10 mM HEPES, 6.5 mM sodium hydroxide, 1 mM EDTA, 0.3% (v/v) ethanol, 0.02% (w/v) methyl paraben, 0.01% (w/v) propyl paraben, 150 mM sodium chloride, 0.1% (w/v) lauryl sulfate, sodium (SDS), at pH 7.5.

### RNA Amplification & Detection

Amplification reagent was a lyophilized form of a 3.6 mL solution containing 26.7 mM rATP, 5.0 mM rCTP, 33.3 mM rGTP and 5.0 mM rUTP, 125 mM HEPES, 8% (w/v) trehalose dihydrate, 1.33 mM dATP, 1.33 mM dCTP, 1.33 mM dGTP and 1.33 mM dTTP, at pH 7.5. The Amplification reagent was reconstituted in 9.7 mL of the amplification reagent reconstitution solution (see below). Before use, 15 pmol each of primer oligomers was added. Amplification reagent reconstitution solution contained 0.4% (v/v) ethanol, 0.10% (w/v) methyl paraben, 0.02% (w/v) propyl paraben, 33 mM KCl, 30.6 mM MgCl₂, 0.003% phenol red. Enzyme reagent was a lyophilized form of a 1.45 mL solution containing 20 mM HEPES, 125 mM N-acetyl-L-cysteine, 0.1 mM EDTA disodium dihydrate, 0.2% (v/v) TRITON7 X-100 detergent, 0.2 M trehalose dihydrate, 0.90 RTU/mL Moloney murine leukemia virus (MMLV) reverse transcriptase, and 0.20 U/mL T7 RNA polymerase, at pH 7.0. One unit (RTU) of activity is defined as the synthesis and release of 5.75 fmol cDNA in 15 minutes at 37°C for MMLV reverse transcriptase, and for T7 RNA polymerase, one unit (U) of activity is defined as the production of 5.0 fmol RNA transcript in 20 minutes at 37°C. Enzyme reagent was reconstituted in 3.6 mL of the enzyme reagent reconstitution solution (see elow). Enzyme reagent reconstitution solution contained 50 mM HEPES, 1 mM EDTA, 10% (v/v) TRITON7 X-100, 120 mM potassium chloride, 20% (v/v) glycerol anhydrous, at pH 7.0. Hybridization reagent contained 100 mM succinic acid free acid, 2% (w/v) lithium lauryl sulfate, 100 mM lithium hydroxide, 15 mM aldrithiol-2, 1.2 M lithium chloride, 20 mM EDTA free acid, 3.0% (v/v) ethanol, at pH 4.7.

Selection reagent contained 600 mM boric acid, 182.5 mM sodium hydroxide, 1% (v/v) TRITON7 X-100, at pH 8.5. The detection reagents comprised detect reagent I, which contained 1 mM nitric acid and 32 mM hydrogen peroxide, and detect reagent II, which contained 1.5 M sodium hydroxide.

### B. Assay Protocol

### Target Capture

1. Prepare samples by making dilutions of IVT stock solution into STM at indicated copy levels for 400 µL sample per reaction tube.
2. Using the repeat pipettor, add 100 µL of the TCR with the TCO to the appropriate reaction tube.
3. Using the micropipettor, add 400 µL of each sample to the properly labeled.
4. Cover the tubes with the sealing card(s) and shake the rack gently by hand. Do not vortex. Incubate the rack at 62°±1°C in a water bath for 30±5 minutes.
5. Remove the rack from the water bath and blot bottoms of tubes dry on absorbent material.
6. Ensure the sealing cards are firmly seated. If necessary, replace with new sealing cards and seal tightly.
7. Without removing sealing cards, incubate the rack at room temperature for 30±5 minutes.
8. Place the rack on the TCS magnetic base for 5 to 10 minutes.
9. Prime the dispense station pump lines by pumping APTIMA Wash Solution through the dispense manifold. Pump enough liquid through the system so that there are no air bubbles in the line and all 10 nozzles are delivering a steady stream of liquid.
10. Turn on the vacuum pump and disconnect the aspiration manifold at the first connector between the aspiration manifold and the trap bottle. Ensure that the vacuum gauge reads greater than 25 in. Hg. It may take 15 seconds to achieve this reading. Reconnect the manifold, and ensure the vacuum gauge is between 7 and 12 in. Hg. Leave the vacuum pump on until all target capture steps are completed.
11. Firmly attach the aspiration manifold to the first set of tips. Aspirate all liquid by lowering the tips into the first TTU until the tips come into brief contact with the bottoms of the tubes. Do not hold the tips in contact with the bottoms of the tubes.
12. After the aspiration is complete, eject the tips into their original tip cassette. Repeat the aspiration steps for the remaining TTUs, using a dedicated tip for each specimen.
13. Place the dispense manifold over each TTU and, using the dispense station pump, deliver 1.0 mL of APTIMA Wash Solution into each tube of the TTU.
14. Cover tubes with a sealing card and remove the rack from the TCS. Vortex once on the multi-tube vortex mixer.
15. Place rack on the TCS magnetic base for 5 to 10 minutes.
16. Aspirate all liquid as in steps 13 and 14.
17. After the final aspiration, remove the rack from the TCS base and visually inspect the tubes to ensure that all liquid has been aspirated. If any liquid is visible, place the rack back onto the TCS base for 2 minutes, and repeat the aspiration for that TTU using the same tips used previously for each specimen.

### Primer Annealing and Amplification

1. Using the repeat pipettor, add 75 µL of the reconstituted Amplification Reagent containing the analyte specific primers to each reaction tube. All reaction mixtures in the rack should now be red in color.
2. Using the repeat pipettor, add 200 µL of Oil Reagent.
3. Cover the tubes with a sealing card and vortex on the multi-tube vortex mixer.
4. Incubate the rack in a water bath at 62°±1°C for 10±5 minutes.
5. Transfer the rack into a water bath at 42°±1°C for 5±2 minutes.
6. With the rack in the water bath, carefully remove the sealing card and, using the repeat pipettor, add 25 µL of the reconstituted Enzyme Reagent to each of the reaction mixtures. All reactions should now be orange in color.
7. Immediately cover the tubes with a fresh sealing card, remove from the water bath, and mix the reactions by gently shaking the rack by hand.
8. Incubate the rack at 42°±1°C for 60±15 minutes.

### Hybridization

1. Remove the rack from the pre-amplification water bath and transfer to the post-amplification area. Add 100 µL of the reconstituted Probe Reagent with analyte specific probe, using the repeat pipettor. All reaction mixtures should now be yellow in color.
2. Cover tubes with a sealing card and vortex for 10 seconds on the multi-tube vortex mixer.
2. Incubate the rack in a 62°±1°C water bath for 20±5 minutes.
3. Remove the rack from the water bath and incubate at room temperature for 5±1 minutes

### Selection

1. Using the repeat pipettor, add 250 µL of Selection Reagent to each tube. All reactions should now be red in color.
2. Cover tubes with a sealing card, vortex for 10 seconds or until the color is uniform, and incubate the rack in a water bath at 62°±1°C for 10±1 minutes.
3. Remove the rack from the water bath. Incubate the rack at room temperature for 15±3 minutes.

### Reading the TTUs

1. Ensure there are sufficient volumes of Auto Detection Regents I and II to complete the tests.
2. Prepare the LEADER Luminometer by placing one empty TTU in cassette position number 1 and perform the WASH protocol.
3. Load the TTUs into the luminometer and run the HC+ Rev B protocol.

### C. Results

The results are shown in Tables 6-9 for 4 assays with each of the TMPRSS2:ERG and TMPRSS2:ETV1 gene fusion IVTs spiked into TCR.

**Table 6**

| **TMPRSS2:ETV1a (copies IVT/reaction)** | **RLU** |
|---|---|
| 0 | 4,945 |
| 0 | 4,599 |
| 10 | 2,185,959 |
| 10 | 2,268,090 |
| 10 | 2,284,908 |
| 100 | 2,270,369 |
| 100 | 2,302,023 |
| 100 | 2,272,735 |
| 1,000 | 2,279,627 |
| 1,000 | 2,285,742 |

**Table 7**

| **TMPRSS2:ETV1b (copies IVT/reaction)** | **RLU** |
|---|---|
| 0 | 7,743 |
| 0 | 6,622 |
| 0 | 7,370 |
| 0 | 6,181 |
| 0 | 7,409 |
| 10 | 7,712 |
| 10 | 7,178 |
| 10 | 7,302 |
| 10 | 8,430 |
| 10 | 8,331 |
| 100 | 774,792 |
| 100 | 285,712 |
| 100 | 3,361,878 |
| 100 | 1,349,368 |
| 100 | 2,757,334 |
| 1,000 | 3,647,502 |
| 1,000 | 3,790,087 |
| 1,000 | 3,813,812 |
| 1,000 | 3,753,743 |
| 1,000 | 3,667,242 |

**Table 8**

| **TMPRSS2:ERGa (copies IVT/reaction)** | **RLU** |
|---|---|
| 0 | 7,938 |
| 0 | 7,505 |
| 10 | 2,043,379 |
| 10 | 387,408 |
| 10 | 978,457 |
| 100 | 2,332,764 |
| 100 | 2,445,544 |
| 100 | 2,530,239 |

**Table 9**

| **TMPRSS2:ERGb (copies IVT/reaction)** | **RLU** |
|---|---|
| 0 | 5,978 |
| 0 | 6,284 |
| 10 | 2,700,069 |
| 10 | 2,768,541 |
| 100 | 2,883,091 |
| 100 | 2,779,233 |
| 1,000 | 2,857,247 |
| 1,000 | 2,957,914 |

### Example 4: FISH Assay for Gene Fusions

This Example describes the use of fluorescence *in situ* hybridization (FISH), to demonstrate that 23 of 29 prostate cancer samples harbor rearrangements in ERG or ETV1. Cell line experiments suggest that the androgen-responsive promoter elements of TMPRSS2 mediate the overexpression of ETS family members in prostate cancer. These results have implications in the development of carcinomas and the molecular diagnosis and treatment of prostate cancer.

Below is a list of the specific BAC probes used in FISH assays.

### Clinical FISH assay for testing aberrations in ETS family members by FISH

- Testing ETV1-TMPRSS2 fusion with one probe spanning the ETV1 and one spanning the TMPRSS2 locus
   BAC for ETV1: RP 11-692L4
   BAC for TMPRSS2: RP11-121A5, (RP11-120C17, PR11-814F13, RR11-535H11)
- Testing ERG translocation with set of probes for c-ERGa-ERG break apart:
   BAC for c-ERG: RP11-24A11
   BACs for t-ERG: RP11-372017, RP11-137J13
- Testing ETV1 deletion/amplification with set of probes, one spanning the ETV1 locus and one reference probe on chromosome 7:
   BAC for ETV1: RP 11-692L4
   BAC for reference robe on chromosome 7: A commercial probe on centromere of chr.
- Testing ERG deletion/amplification with set of probes, one spanning the ERG locus and one reference probe on chromosome 21:
   BAC for ERG: RP11-476D17
   BACs for reference probe on chromosome 21: *
- Testing TMPRSS2 deletion/amplification with set of probes, one spanning the TMPRSS2 locus and one reference probe on chromosome 21:
   BACs for TMPRSS2: RP11-121A5, (RP11-120C17, PR11-814F13, RR11-535H11) BACs for reference probe on chromosome 21: *
      **BACs for reference probe on chromosome 21: PR11-32L6, RP11-752M23, RP11-1107H21, RP11-639A7, (RP11-1077M21)*

### Example 5:TMPRSS2:ERG Fusion Associated Deletions

This example describes the presence of common deletions located between ERG and TMPRSS2 on chromosome 21q22.2-3 associated with the TMPRSS2:ERG fusion. Associations between disease progression and clinical outcome were examined using a wide range of human PCA samples, 6 cell lines, and 13 xenografts.

### A. Materials and Methods

### Clinical Samples

Prostate samples used for this study were collected under an IRB approved protocol. All clinically localized PCA samples were characterized by one pathologist and assigned a Gleason score to eliminate inter-observer differences in pathology reporting. Clinically localized PCA samples were collected as part of an on-going research protocol at the University of Ulm. The hormone refractory samples were taken from the Rapid Autopsy Program of the University of Michigan.

The FISH experiments were conducted on two PCA outcome arrays, which were composed of 897 tissue cores (histospots) from 214 patients. A summary of the patient demographics is presented in Table 10. All patients had undergone radical prostatectomy with pelvic lymphadenectomy at the University of Ulm (Ulm, Germany) between 1989 and 2001. Preoperative PSA ranged between 1 and 314 ng/ml (mean 36 ng/ml). Mean and maximum follow-up was 3.4 and 8.4 yrs, respectively.

### Cell Lines and Xenografts

Androgen independent (PC-3, DU-145, HPV10, and 22Rvl) and androgen sensitive (LNCaP) PCA cell lines were purchased from the American Type Culture Collection (Manassas, VA) and maintained in their defined medium. HPV10 was derived from cells from a high-grade PCA (Gleason score 4+4=8), which were transformed by transfection with HPV 18 DNA(18). 22Rv1 is a human PCA epithelial cell line derived from a xenograft that was serially propagated in mice after castration-induced regression and relapse of the parental, androgen-dependent CWR22 xenograft. The VCAP cell line was from a vertebral metastatic lesion as part of the Rapid Autopsy program at the University of Michigan.

LuCaP 23.1, 35, 73, 77, 81, 86.2, 92.1, and 105 were derived from patients with androgen independent hormone-refractory disease PCA. LuCaP 49 and 115 are from patients with androgen dependent PCA. LuCaP 58 is derived from an untreated patient with clinically advanced metastatic disease and LuCaP 96 was established from a prostate derived tumor growing in a patient with hormone refractory PCA. LuCaP 49 (established from an omental mass) and LuCaP 93 are hormone-insensitive (androgen receptor [AR]-negative) small cell PCAs. These two xenografts demonstrate a neuroendocrine phenotype. LuCaP 23.1 is maintained in SCID mice, and other xenografts are maintained by implanting tumors in male BALB/c nu/nu mice.

### Determining TMPRSS2:ERG Fusion Status using Interphase FISH

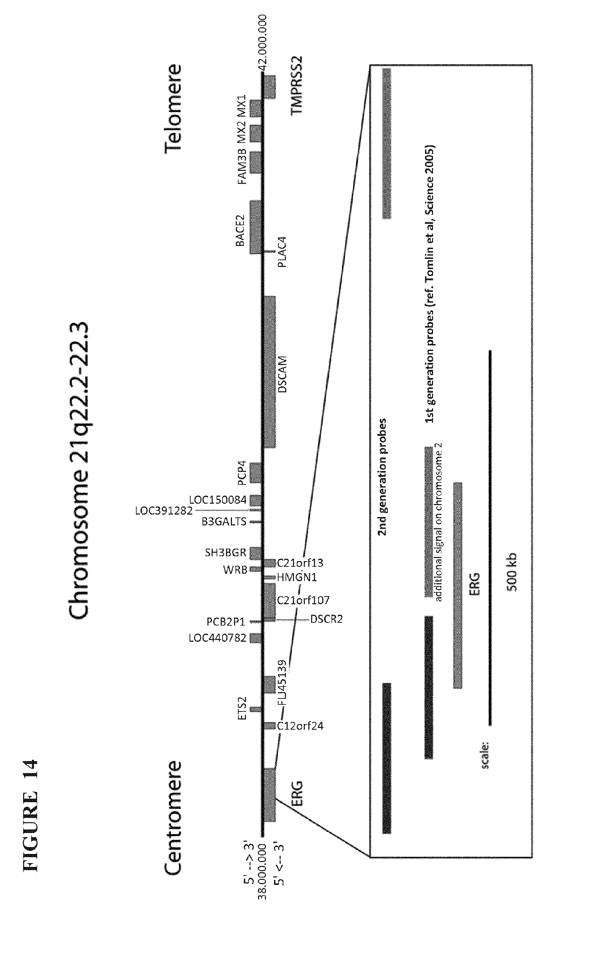

The FISH analysis for the translocation of *TMPRSS2:ERG* is described above and previously (Tomlins, et al., Science 310:644-8 (2005)). This break apart assay is presented in Figures 11 and 14. For analyzing the *ERG* rearrangement on chromosome 21q22.2, a break apart probe system was applied, consisting of the Biotin-14-dCTP labeled BAC clone RP11-24A11 (eventually conjugated to produce a red signal) and the Digoxigenin-dUTP labeled BAC clone RP11-137J13 (eventually conjugated to produce a green signal), spanning the neighboring centromeric and telomeric region of the *ERG* locus, respectively. All BAC clones were obtained from the BACPAC Resource Center, Children's Hospital Oakland Research Institute (CHORI), Oakland, CA.

Using this break apart probe system, a nucleus without *ERG* rearrangement exhibits two pairs of juxtaposed red and green signals. Juxtaposed red-green signals form a yellow fusion signal. A nucleus with an *ERG* rearrangement shows break apart of one juxtaposed red-green signal pair resulting in a single red and green signal for the translocated allele and a combined yellow signal for the non-translocated allele in each cell. Prior to tissue analysis, the integrity and purity of all probes were verified by hybridization to normal peripheral lymphocyte metaphase spreads. Tissue hybridization, washing, and fluorescence detection were performed as described previously (Garraway, et al., Nature 436:117-22 (2005); Rubin, et al., Cancer Res. 64:3814-22 (2004)). At least one TMA core could be evaluated in 59% PCA cases from two TMAs. The technical difficulties with this assay included the absence of diagnostic material to evaluate, weak probe signals, and overlapping cells preventing an accurate diagnosis. The remainder of the analysis focused on the 118 cases of clinically localized PCA that could be evaluated. 15 cases had corresponding hormone naive metastatic lymph node samples that could also be evaluated.

The samples were analyzed under a 100x oil immersion objective using an Olympus BX-51 fluorescence microscope equipped with appropriate filters, a CCD (charge-coupled device) camera and the CytoVision FISH imaging and capturing software (Applied Imaging, San Jose, CA). Evaluation of the tests was independently performed by two pathologists both with experience in analyzing interphase FISH experiments. For each case, it was attempted to score at least 100 nuclei per case. If significant differences between the results of both pathologists were found, the case was refereed by a third pathologist.

### Oligonucleotide SNP Array Analysis

Although SNP arrays were intended for genotyping alleles, the SNP array data can provide information on Loss-of-Heterozygosity (Lieberfarb, et al., Cancer Res 63:4781-5 (2003); Lin, et al., Bioinformatics 20:1233-40 (2004)) and detection of copy number alterations (Zhao, et al., Cancer Cell 3:483-95 (2003)). Using SNP array analysis, it was possible to identify and validate amplified genes in various cancers including melanoma (*MITF*) (Garraway, et al., Nature 436:117-22 (2005)) and PCA (*TPD52*) (Rubin, et al., Cancer Res. 64:3814-22 (2004)).

SNP detection on the 100K array began with a reduction in genome representation. Two aliquots of 250 ng of genomic DNA were digested separately with XbaI HindIII. The digested fragments were independently ligated to an oligonucleotide linker. The resulting products were amplified using a single PCR primer under conditions in which 200-2000 bp PCR fragments were amplified. These fragments represent a sub-fraction of the genome. The SNPs tiled on the arrays have been pre-selected as they lie within these XbaI and HindIII fragments and have been validated as robustly detected on the arrays. The derived amplified pools of DNA were then labeled, fragmented further and hybridized to separate HindIII and XbaI oligonucleotide SNP arrays.

Arrays were scanned with a GeneChip Scanner 3000. Genotyping calls and signal quantification were obtained with GeneChip Operating System 1.1.1 and Affymetrix Genotyping Tools 2.0 software. Only arrays with genotyping call rates exceeding 90% were analyzed further. Raw data files were pre-processed and visualized in dChipSNP Lin, et al., Bioinformatics 20:1233-40 (2004)). In particular, preprocessing included array data normalization to a baseline array using a set of invariant probes and subsequent processing to obtain single intensity values for each SNP on each sample using a model based (PM/MM) method (Li, et al., Proc. Nat'l Acad. Sci. USA 98:31-6 (2001)).

### Quantitative PCR for TMPRSS2:ERG and TMPRSS2:ETV1 Fusion Transcripts

QPCR was performed using SYBR Green dye (Qiagen) on a DNA engine Opticon 2 machine from MJ Research. Total RNA was reverse transcribed into cDNA using TAQMAN reverse transcription reagents (Applied Biosystems) in the presence of random Hexamers. All QPCR reactions were performed with SYBR Green Master Mix (Qiagen). All Oligonucleotide primers were designed at Integrated DNA Technologies. Primers that were described by Tomlin et al. (Science 310:644-8 (2005)) and are specific for the fusion were utilized:
TMPRSS2:ERG_f:TAGGCGCGAGCTAAGCAGGAG (SEQ ID NO:55),
TMPRSS2:ERG_r: GTAGGCACACTCAAACAACGACTGG (SEQ ID NO:56),
TMPRSS2:ETV1_fCGCGAGCTAAGCAGGAGGC, (SEQ ID NO:57)
TMPRSS2:ETV-1_r: CAGGCCATGAAAAGCCAAACTT (SEQ ID NO:58).

GAPDH primers were previously described (Vandesompele, et al., Genome Biol 3: RESEARCH 0034 (2002)). 10 µmol of forward and reverse primer were used and procedures were performed according to the manufacturer's recommended thermocycling conditions. Threshold levels were set during the exponential phase of the QPCR reaction using Opticon Monitor analysis software version 2.02. The amount of each target gene relative to the housekeeping gene glyceraldehyde-3-phosphate dehydrogenase (GAPDH) for each sample was determined using the comparative threshold cycle (Ct) method (Applied Biosystems User Bulletin #2). All reactions were subjected to melt curve analysis and products from selected experiments were resolved by electrophoreses on 2% agarose gel.

### Statistics

The clinical and pathology parameters were explored for associations with rearrangement status and with the presence of the deletion. Chi-squared test and Fisher exact test were used appropriately. Kaplan-Meier analysis was used to generate prostate-specific antigen recurrence free survival curves of the pathology and the genomic alteration parameters. Log-rank test was used to evaluate statistical significance of associations. Patients with prior neo-adjuvant hormone ablation therapy were excluded. All statistics were performed using SPSS 13.0 for Windows (SPSS Inc., Chicago, IL) with a significance level of 0.05.

### B. Results

### Detection ofDeletions on Chromosome 21 Associated with the TMPRSS2:ERG Gene Rearrangement

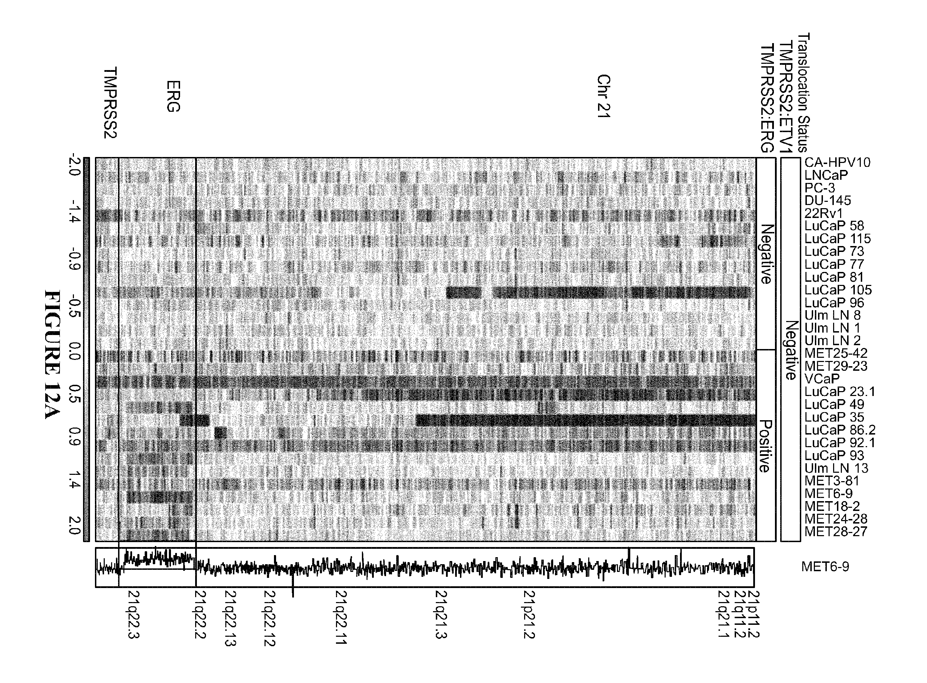

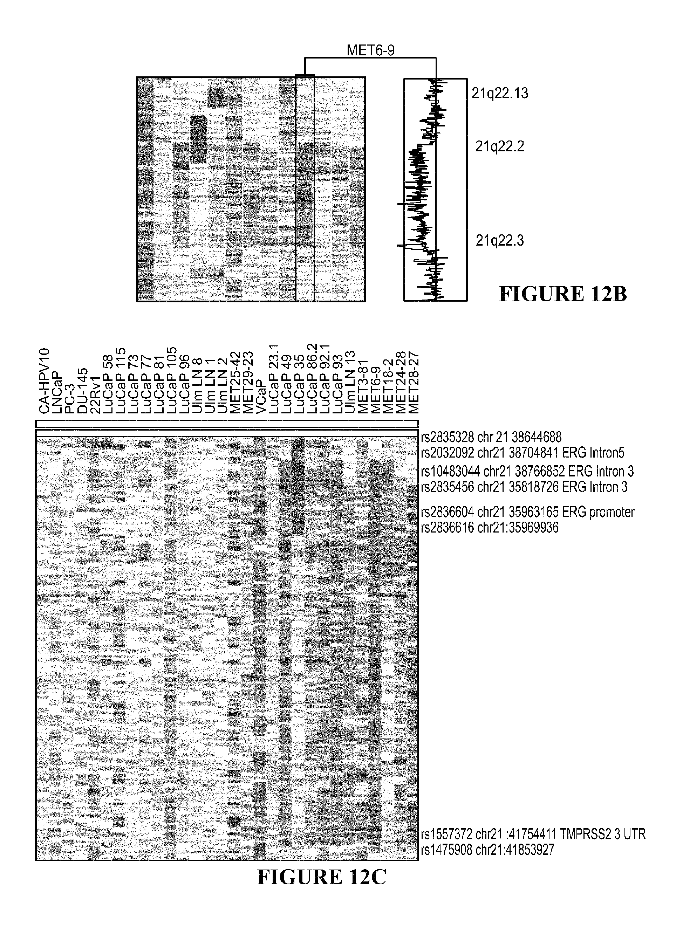

In order to characterize the frequency of the TMPRSS2:ERG rearrangement in PCA, a modified FISH assay from the assay described by Tomlins, et al. (Science 310:644-8 (2005)) was utilzed. The original FISH assay used two probes located on ERG at the centromeric 3' and telomeric 5' ends. The new assay moved the 5' probe in a telomeric direction (Figure 14). Using a PCA screening tissue microarray (TMA), it was observed that approximately 70% of PCA demonstrating TMPRSS2:ERG rearrangement (Figure 11A and 11B) also showed a loss of the green signal corresponding to the telomeric 5' ERG probe (Figure 11C and 11D), suggesting that this chromosomal region was deleted. 100K oligonucleotide SNP arrays were used to characterize the extent of these deletions. By interrogating 30 PCA samples, including cell lines, xenografts and hormone naïve and hormone refractory metastatic PCA samples, genomic loss between ERG and TMPRSS2 on chromosome 21q23 was identified (Figure 12A-C).

The rearrangement status for TMPRSS2:ERG and TMPRSS2:ETV1 was determined for these 30 PCA by FISH and/or qPCR (Figure 12A, gray and light blue bar). Discrete genomic loss was observed in TMPRSS2:ERG rearrangement positive samples involving an area between TMPRSS2 and the ERG loci for LuCaP 49, LuCaP 93, ULM LN 13, MET6-9,MET18-2, MET24-28, and MET28-27. The extent of these discrete deletions was heterogeneous. More extensive genomic loss on chromosome 21 including the area between TMPRSS2 and the ERG loci was observed in LuCaP 35, LuCaP 86.2, LuCaP 92.1, and MET3-81. The VCaP cell line and the xenograft LuCap 23.1 did not demonstrate loss in this region. For a subset of samples 45%(5 out of 11) the deletion occurs in proximity of ERG intron 3. For a majority of samples 64% (7 out of 11) the deletion ends in proximity of the SNP located on TMPRSS2 (the next SNP in the telomeric direction is about 100K bp distant). The VCaP cell line shows copy number gain along the entire chromosome 21.

For TMPRSS2:ERG rearrangement positive tumors, 71% (5 of 7) hormone refractory PCA demonstrate a deletion between TMPRSS2 and the ERG loci whereas deletion was only identified in 25%(1 of 4) hormone naïve metastatic PCA samples (ULM LN 13). There is significant homogeneity for the deletion borders with two distinct sub-classes, distinguished by the start point of the deletion-either at 38.765 Mb or 38.911 Mb. None of the standard PCA cell lines (PC-3, LNCaP, DU-145, or CWR22 (22Rv1)) demonstrated the TMPRSS2:ERG or TMPRSS2:ETV1 fusion. Several of the LuCap xenografts demonstrate TMPRSS2:ERG fusion with deletion including LuCaP 49 (established from an omental mass) and LuCaP 93, both hormone-insensitive (androgen receptor [AR]-negative) small-cell PCAs.

Copy number gain of ERG was observed in a small subset of cases both with and without the TMPRSS2:ERG rearrangement. The VCaP cell line derived from a hormone refractory PCA demonstrated significant copy number gain on chromosome 21 (Figure 12A-C), which was confirmed by FISH.

### TMPRSS2:ERG rearrangement in Primary Prostate Cancer Samples and Hormone Naïve Lymph Node Metastases

To characterize the frequency and potential clinical significance of these observations, 118 clinically localized PCA cases were examined by FISH. The clinical and pathology demographics are presented in Table 10. This cohort of patients is at high risk of disease recurrence as demonstrated by high tumor grades (Gleason grade), pathology stage, and pretreatment PSA levels. Using standard tissue sections from this cohort, where the large areas of the PCA could be studied microscopically, the TMPRSS2:ERG rearrangement was obsesrved to be homogeneous for a given tumor. The TMA experiments confirmed these observations. In PCA cases where 3-6 cores were taken from different areas of the tumor, 100% concordance was observed for TMPRSS2:ERG rearrangement status (i.e. present or absent). It was also observed that in cases with the TMPRSS2:ERG rearrangement with deletion, the deletion was observed in all of the TMA cores from the same patient in 97.9% (94/96) of the cases.

**Table 10: Clinical and Pathological Demographics of 118 Men with Clinically Localized Prostate Cancer Treated by Radial Protatectomy***

| | | **Count** | **Column N %** |
|---|---|---|---|
| Age | < = median | 55 | 50.0% |
| | > median | 55 | 50.0% |
| Preoperative PSA (ng/ml) | <=4 | 6 | 8.2% |
| | >4 and < 10 | 13 | 17.8% |
| | > = 10 | 54 | 74.0% |
| Gleason Score Sum | < 7 | 7 | 6.0% |
| | = 7 | 51 | 43.6% |
| | >7 | 59 | 50.4% |
| Nuclear Grade | 1 | - | - |
| | 2 | 38 | 35.5% |
| | 3 | 69 | 64.5% |
| Pathology Stage (pT) | PT2 | 26 | 22.2% |
| | PT3a | 34 | 29.1% |
| | PT3b | 57 | 48.7% |
| Surgical Margins status | Negative | 30 | 27.8% |
| | Positive | 78 | 72.2% |
| Lymph Node Status (pN) | N₀ | 52 | 44.1% |
| | N₁ | 56 | 47.5% |
| | N₂ | 10 | 8.5% |
| PSA Recurrence | no | 34 | 48.6% |
| | yes | 36 | 51.4% |

| | | | |
|---|---|---|---|
| *Not all data points were available for all 118 cases | | | |

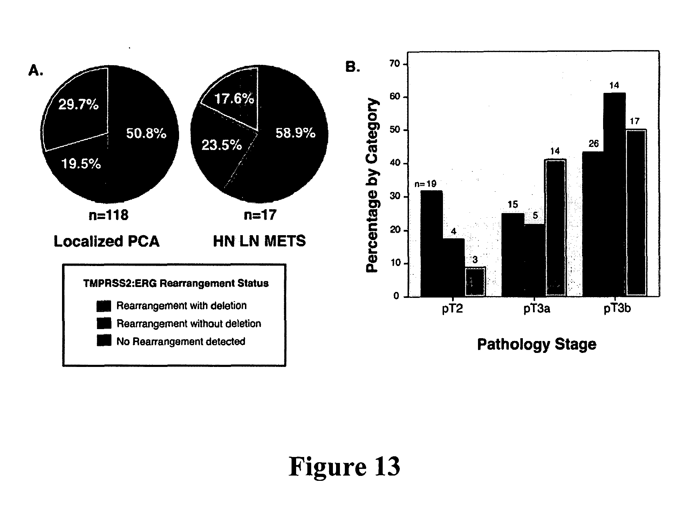

The *TMPRSS2:ERG* rearrangement was identified in 49.2% of the primary PCA samples and 41.2% in the hormone naïve metastatic LN samples (Figure 13A). Deletion of the telomeric probe (green signal) (Figure 1C-D) was observed in 60.3% (35/58) of the primary PCA samples and 42.9% (3/7) of the hormone naive lymph node tumors with TMPRSS2:ERG rearrangement.

In the 15 cases where there was matched primary and hormone naive lymph node tumors, there was 100% concordance for TMPRSS2:ERG rearrangement status with 47% (7 of 15) of the pairs demonstrating the rearrangement. Deletion of the telomeric (green signal) probe was concordantly seen in 42.9%(3 of 7) of the pairs.

### TMPRSS2:ERG rearrangement status and Prostate Cancer Progression

The associations between rearrangement status and clinical and pathological parameters were observed (Figure 13). TMPRSS2:ERG rearrangement with deletion was observed in a higher percentage of PCA cases with advanced tumor stage (pT)(p=0.03) (Figure 13B), and the presence of metastatic disease to regional pelvic lymph nodes (pN₀ versus pN₁₋₂) (p=0.02). Associations between TMPRSS2:ERG rearrangement with and without deletion and clinical outcome as determined by prostate specific antigen (PSA) biochemical failure for 70 patients where follow up data was available were also assesed. Gleason grade, tumor stage, nuclear grade and lymph node status were good predictors of PSA biochemical failure (all p-values < 0.0005). A trend was observed at the univariate level suggesting a PSA recurrence free survival advantage in TMPRSS2:ERG rearranged PCA cases without deletion as determined by the FISH assay.

### Example 6: TMPRSS2:ERG Gene Fusion Associated with Lethal Prostate Cancer

In previous studies, the gene fusions of the 5'-untranslated region of TMPRSS2 (21q22.3) with the ETS transcription factor family members, either ERG (21q22.2), ETV1 (7p21.2) (Tomlins, et al., Science 310:644-8 (2005)), or ETV4 (Tomlins, et al., Cancer Res. 66(7):3396-400 (2006)) provide a mechanism for the over expression of the ETS genes in the majority of prostate cancers. Furthermore, the fusion of an androgen regulated gene, TMPRSS2, and an oncogene suggests that disease progression may vary based on these molecular subtypes. The most common mechanism for gene fusion is loss of about 2.8 megabases of genomic DNA between TMPRSS2 and ERG (Figure 17A and B). This example describes the risk of metastases or prostate cancer specific death based on the presence of the common TMPRSS2:ERG gene fusion.

### A. Methods

The study population comprises men with early prostate cancer (T1a-b, Nx, M0) diagnosed at the Örebro University Hospital, Sweden, between 1977 and 1991 by transurethral resection of the prostate (TURP) or transvesical adenoma enucleation for symptomatic benign prostatic hyperplasia as described by Andrén et al. (J. Urol. 175(4):1337-40 (2006)). Baseline evaluation at diagnosis included physical examination, chest radiography, bone scan and skeletal radiography (if needed). Nodal staging was not carried out. Because this evaluation provided no evidence for distant metastases, patients were followed expectantly and received clinical exams, laboratory tests and bone scans every 6 months during the first 2 years after diagnosis and subsequently at 12-month intervals. Patients, who developed metastases, as determined by bone scan, were treated with androgen deprivation therapy if they exhibited symptoms.

The cause of death in the cohort was determined by review of medical records by the study investigators. A validation study regarding cause of death compared to the Swedish Death Register showed greater than 90% concordance, with no systematic under- or over-reporting of any cause of death (Johansson, et al., Lancet 1(8642):799-803 (1989)). Follow-up of the cohort with respect to mortality was 100% and no patients were lost to follow-up through October 2005. The study endpoint was defined as development of distant metastases or prostate cancer specific death (median follow-up time 9.1 years, maximum 27 years).

All TURP samples were reviewed by one pathologist to confirm a diagnosis of prostate cancer, determine the Gleason score and nuclear grade, and estimate the tumor burden as previously described (J. Urol. 175(4):1337-40 (2006)). A tissue microarray was assembled using a manual arrayer (Rubin, et al., Cancer Epidemiol. Biomarkers Prev. 14(6):1424-32 (2005)). The frequency of the TMPRSS2:ERG rearrangement in prostate cancer was assessed using a modified florescence *in situ* hybridization (FISH) assay from the assay originally described by Tomlins et al (Science 310:644-8 (2005)). The new assay moved the 5' probe approximately 600kb in a telomeric direction. At least one TMA core could be evaluated in 92 of the prostate cancer cases.

### B. Results

In this population-based cohort of men diagnosed with localized cancer, the frequency of TMPRSS2:ERG fusion was 15.2% (14/92) (Figures 17A and B). TMPRSS2:ERG fusion positive tumors were more likely to have a higher Gleason score (two-sided *P* =.014) (Table 11). To assess the relation of fusion status and lethal prostate cancer, cumulative incidence regression was used. A significant association between the presence of the TMPRSS2:ERG gene fusion and metastases or disease specific death (Figure 17C) with a cumulative incidence ratio (CIR) of 3.6 (*P* = .004, 95% confidence interval [CI] = 1.5 to 8.9) was observed. When adjusting for Gleason Score, the CIR was 2.4 (*P* = .07 and 95%CI = 0.9 to 6.1). The present invention is not limited to a particular mechanism. Indeed, an understanding of the mechanism is not necessary to practive the present invention. Nonetheless, it is contemplated that, based on the homogeneity of the TMPRSS2:ERG gene fusion in cells in a given tumor and its presence only in invasive prostate cancers (compared to Prostatic Intraepithelial Neoplasia), it is contemplated that this is an early event, which might, in part, contribute to the biology behind the phenotype of the Gleason patterns.

**Table 11: Prognostic Factors for a Cohort of Men Expectantly Managed for Localized Prostate Cancer Stratified by the TMPRSS2:ERG Gene Fusion Status**

| | **TMPRSS2:ERG Fusion Status** | | |
|---|---|---|---|
| **Variable** | **Negative** | **Positive** | **P value*** |
| No. of patients | 78 | 14 | |
| Age at diagnosis, y | 73 (60 to 103) | 73 (58 to 90) | .683 |
| Gleason Score** | | | |
| Gleason Score <7 | 48 (61.5%) | 3 (21.4%) | .014 |
| Gleason Score =7 | 20 (25.6%) | 6 (42.9%) | |
| Gleason Score >7 | 10 (12.8%) | 5 (35.7%) | |
| Pathologic Stage | | | |
| pT1a | 28 (35.9%) | 2 (14.3%) | .112 |
| pT1b | 50(64.1%) | 12 (85.7%) | |
| Nuclear grade*** | | | |
| 1 | 53 (67.9%) | 7 (53.8%) | .585 |
| 2 | 18 (23.1%) | 4 (30.8%) | |
| 3 | 7 (9.0%) | 2 (15.4%) | |
| Status*** | | | |
| Survived 12 years without metastases or cancer death | 20 (25.6%) | 1 (7.1%) | .016 |
| Death due to other causes within 12 years | 45 (57.7%) | 6 (42.9%) | |
| Distant metastases or death due to prostate Cancer | 13 (16.7%) | 7 (50.0%) | |

| | | | |
|---|---|---|---|
| * Clinical parameters of subjects having the TMPRSS2:ERG fusion and of subjects not having the TMPRSS2:ERG fusion were compared by use of *t* tests or chi-square tests for continuous variable and categorical variables, respectively. ** Gleason Score is obtained by summing the major and minor Gleason patterns. *** For one case nuclear grade was not assessed. **** Individuals who lived at least 12 years and have not developed metastases or died of prostate cancer as of October 2005 are classified as long-term survivors. Individuals who lived less than 12 years and did not develop metastases are classified as short-term survivors. | | | |

### Example 7:Detection of TMPRSS2:ETS fusions in the urine of patients with prostate cancer

### A. Materials and Methods

### Urine Collection, RNA isolation and amplification

Urine samples were obtained from patients following a digital rectal exam before either needle biopsy or radical prostatectomy. Urine was voided into urine collection cups containing DNA/RNA preservative (Sierra Diagnostics). For isolation of RNA, a minimum of 30ml of urine were centrifuged at 400 rpm for 15 min at 4° C. RNAlater (Ambion) was added to the urine sediments and stored at -20° C until RNA isolation. Total RNA was isolated using a Qiagen RNeasy Micro kit according to the manufacturer's instructions. Total RNA was amplified using an OmniPlex Whole Transcriptome Amplification (WTA) kit (Rubicon Genomics) according to the manufacturer's instructions (Tomlins et al., Neoplasia 8:153 [2006]). Twenty five nanograms of total RNA were used for WTA library synthesis and the cDNA library was subjected to one round of WTA PCR amplification. Amplified product was purified using a QIAquick PCR Purification kit (Qiagen). For cell line proof of concept experiments, the indicated number of VCaP or LNCaP cells was spiked into 1 ml of sterile urine and the samples were processed as for voided urine.

### Quantitative PCR

Quantitative PCR (QPCR) was used to detect ERG, ETV1 and TMPRSS2:ERG transcripts from WTA amplified cDNA essentially as described (Tomlins et al., Neoplasia 8:153 [2006], Tomlins et al., Science 310:644 [2005], Example 1 above). For each QPCR reaction, 10 ng of WTA amplified cDNA was used as template. Reactions for ERG, ETV1, PSA and GAPDH used 2x Power SYBR Green Master Mix (Applied Biosystems) and 25ng of both the forward and reverse primers. Reactions for TMPRSS2:ERGa used 2x Taqman Universal PCR Master Mix and a final concentration of 900 nM forward and reverse primers, and 250 nM probe. For the Taqman assay, samples with Ct values greater than 38 cycles were considered to show no amplification. For all samples, the amount of ERG and ETV1 were normalized to the amount of GAPDH. Samples with inadequate amplification of PSA, indicating poor recovery of prostate cells in the urine, were excluded from further analysis. ERG (exon5_6 forward) and ETV1 (exon6_7)², GAPDH³, and PSA⁴ primers were as described. The Taqman primers and probe (MGB labeled) specific for TMPRSS2:ERGa are as follows:
TM-ERGa2_MGB-f;CGCGGCAGGAAGCCTTA (SEQ ID NO:70)
TM-ERGa2_MGB-r;TCCGTAGGCACACTCAAACAAC (SEQ ID NO:71),
TM-ERGa2_MGB-probe;5'-MGB-CAGTTGTGAGTGAGGACC-NFQ-3' (SEQ ID NO:72)

### Fluorescene in situ Hybridization (FISH)

Four µm thick formalin-fixed paraffin-embedded (FFPE) sections from matched needle biopsies were used for interphase fluorescence *in situ* hybridization (FISH), processed and hybridized as described previously (Example 2 and Tomlins et al., Cancer Res 66:3396 [2006]). BAC probes to detect ERG rearrangements, RP11-95121 (5' to ERG) and RP11-476D17 (3' to ERG) were prepared as described previously (Tomlins et al., Cancer Res 66:3396 [2006]; Tomlins et al., Science 310:644 [2005]; Examples 1 and 2 above).

### B. Results

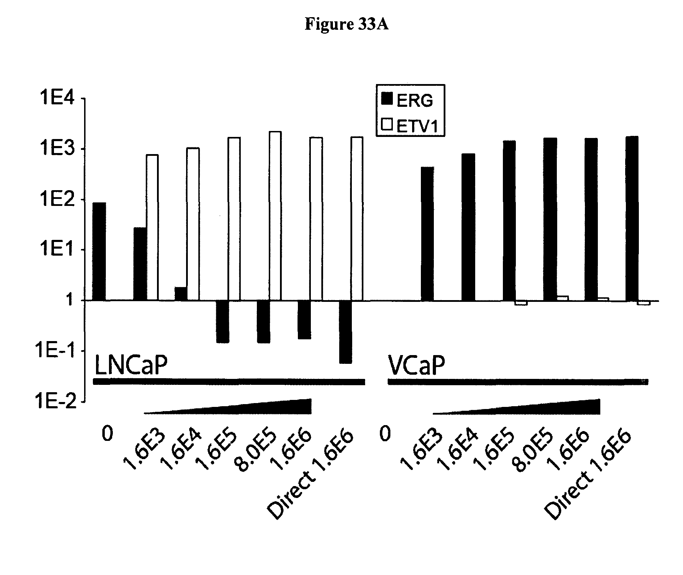

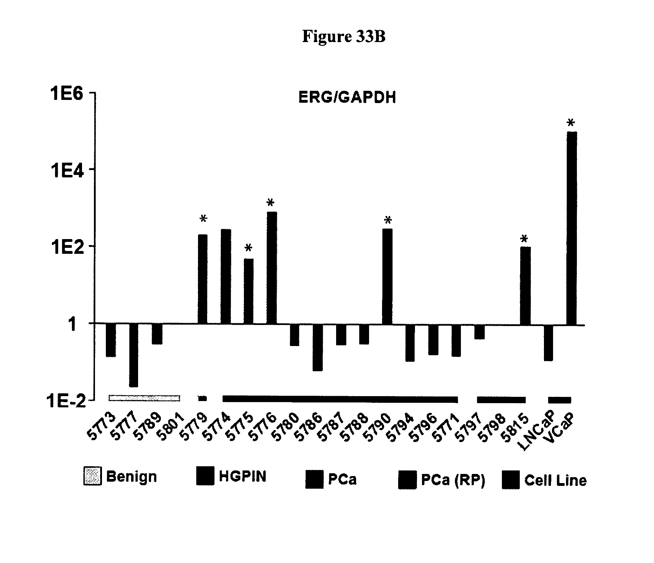

This example describes a non-invasive method to detect prostate cancer by the presence of TMPRSS2:ETS fusion transcripts in prostate cancer cells shed into the urine after a digital rectal exam. Results are shown in Figure 33. As a proof of concept, sterile urine spiked with prostate cancer cell lines expressing high levels of ERG and TMPRSS2:ERG (VCaP) or high levels of ETV1 (LNCaP) was used. As shown in Figure 33A, it was possible to detect ERG over-expression exclusively in VCaP at 1,600 cells and ETV1 over-expression exclusively in LNCaP at 16,000 cells by quantitative PCR (QPCR).

By correlating the number of spiked VCaP and LNCaP cells to *GAPDH* Cₜ (threshold cycle) values, it was observed that, in some cases, urine obtained from patients after a digital rectal exam contained insufficient cell numbers to reliably detect ERG or ETV1 over-expression. Thus, total RNA collected from the urine of patients suspected of having prostate cancer was amplified using OmniPlex Whole Transcriptome Amplification before QPCR analysis. Using this strategy, a cohort of 16 patients where urine was obtained after a digital rectal exam before a needle biopsy to detect prostate cancer was assesed. Subsequent assessment of needle biopsies demonstrated that this cohort contained 4 patients with benign prostates, 1 with high grade prostatic intraepithelial neoplasia (HGPIN) and 11 with prostate cancer. In addition, a cohort of 3 patients with prostate cancer where urine was collected after a digital rectal exam before radical prostatectomy was assesed.

Cohort characteristics are presented in Table 12. Each urine specimen was from a unique patient and was assigned an ID. The source of the sample (pre biopsy or radical prostatectomy (RP) is indicated. The diagnosis following needle biopsy (including benign, high grade prostatic intraepithelial neoplasia (HGPIN), and prostate cancer (PCa)) is indicated. For patients diagnosed as having prostate cancer following needle biopsy, major Gleason, minor Gleason, and Gleason sum score are indicated. For all patients, pre biopsy PSA (ng/ml) and age are reported, if available.

**Table 12**

| **Sample source** | **Diagnosis** | **Biopsy Gleason Major** | **Biopsy Gleason Minor** | **Biopsy Gleason Score** | **Pre-Biopsy PSA (ng/ml)** |
|---|---|---|---|---|---|
| Pre-Biopsy | **Benign** | | | | 4.7 |
| Pre-Biopsy | **Benign** | | | | 8.3 |
| Pre-Biopsy | **Benign** | | | | 6.7 |
| Pre-Biopsy | **Benign** | | | | 4 |
| Pre-Biopsy | **HGPIN** | | | | 9.7 |
| Pre-Biopsy | **Pca** | 3 | 4 | 7 | 3.3 |
| Pre-Biopsy | **Pca** | 3 | 3 | 6 | 5.99 |
| Pre-Biopsy | **Pca** | 3 | 3 | 6 | 2.8 |
| Pre-Biopsy | **Pca** | 3 | 3 | 6 | 5.9 |
| Pre-Biopsy | **Pca** | 4 | 4 | 8 | 10.6 |
| Pre-Biopsy | **Pca** | | | | |
| Pre-Biopsy | **Pca** | 4 | 5 | 9 | 11.8 |
| Pre-Biopsy | **Pca** | 3 | 4 | 7 | 5.5 |
| Pre-Biopsy | **Pca** | 3 | 3 | 6 | 3.8 |
| Pre-Biopsy | **Pca** | 4 | 5 | 9 | 19.3 |
| Pre-Biopsy | **Pca-treated** | 3 | 3 | 6 | |
| Pre-RP | **Pca** | | | | |
| Pre-RP | **Pca** | | | | |
| Pre-RP | **Pca** | | | | |

From the needle biopsy cohort, 5 patients were identified with marked over-expression of ERG, 1 of which was diagnosed by needle biopsy as having HGPIN, while the other 4 were diagnosed as having prostate cancer. From the radical prostatectomy cohort, 1 of 3 patients with prostate cancer were identified as having high ERG expression (Fig 33B). ETV1 over-expression was not detected in any patients from either cohort. To confirm the expression of TMPRSS2:ERG in the samples which over-expressed ERG, a TaqMan primer/probe assay designed to specifically amplify TMPRSS2:ERGa was utilized. This assay robustly amplified product from VCaP cells, which express TMPRSS2:ERGa (Tomlins et al., Science 310:644 [2005]). In addition, 5 of the 6 urine samples from patients with prostate cancer that over-expressed ERG also expressed TMPRSS2:ERGa (Ct values 29.8-38.9), while 0 of the 10 samples from patients without ERG over-expression expressed TMPRSS2:ERGa. As one sample over-expressed ERG without expression of TMPRSS2:ERGa, it is likely that this sample expresses other isoforms of the fusion transcript, such as TMPRSS2:ERGb or more recently identified fusion transcripts (Soller et al., Genes Chromosomes Cancer 45:717 [2006]; Yoshimoto et al., Neoplasia 8:465:2006). To confirm that the presence of TMPRSS2:ERG fusion transcripts indicates the presence of TMPRSS2:ERG positive cancerous tissue, fluorescence in situ hybridization (FISH) was performed using probes designed to detect ERG rearrangements on matched tissue sections from representative cases. Matched tissue was obtained from three patients with detectable TMPRSS2:ERG transcripts in the urine and a diagnosis of cancer, one patient with detectable TMPRSS2:ERG transcripts in the urine and a diagnosis of high grade PIN, and two patients without detectable TMPRSS2:ERG transcripts and a diagnosis of cancer. As shown in Figure 33B, both patients diagnosed with cancer but without detectable TMPRSS2:ERG transcripts in their urine did not harbor ERG rearrangements in cancerous tissue by FISH. All three patients diagnosed with cancer and with detectable TMPRSS2:ERG transcripts in their urine also showed ERG rearrangements in cancerous tissue by FISH. Finally, the patient with a diagnosis of high grade PIN with detectable TMPRSS2:ERG in their urine did not show ERG rearrangements in high grade PIN tissue. This indicates that this patient may have undiagnosed cancer elsewhere in the prostate, resulting in the presence of detectable TMPRSS2:ERG transcripts in their urine.

### Example 8:TMPRSS2 and ETS Family Genes Fusions in Prostate Cancer

This study describes a comprehensive analysis of the frequency for the *TMPRSS2* and *ETS* family genes rearrangements in a screening-based cohort of 111 American men surgically treated for clinically localized prostate cancer.

### A. Materials and Methods

### Study Population, Clinical data and Prostate Sample Collection:

As a source of clinically localized prostate cancers, a tissue microarray (TMA) containing -- cores representing cancer and benign tissue was constructed from 111 men who underwent radical prostatectomy at the University of Michigan as the primary monotherapy (i.e., no adjuvant or neoadjuvant hormonal or radiation therapy). The radical prostatectomy series is part of the University of Michigan Prostate Cancer Specialized Program of Research Excellence (SPORE) Tissue Core. Three cores (0.6 mm in diameter) were taken from each representive tissue block to construct the TMA. The TMA construction protocol has been described (Kononen et al., Nat. Med. 4:844 [1998]; Rubin et al., Am J surg Pathol 26:312 [2002]). Detailed clinical, pathological, and TMA data re maintained on a secure relational database as previously described (Manley et al., Am J. Pathol. 159:837 [2001]).

### Assessment of TMPRSS2-ETS gene fusion using an interphase Fluorescence in situ hybridization assay

Four µm thick tissue micro array sections were used for interphase fluorescence *in situ* hybridization (FISH), processed and hybridized as described previously (Tomlins et al., Science 310:644 [2005]; Tomlins et al., Cancer Res 66:3396 [2006]). Slides were examined using an Axioplan ImagingZ1 microscope (Carl Zeiss) and imaged with a CCD camera using the ISIS software system in Metafer image analysis system (Meta Systems, Altlussheim, Germany). FISH signals were scored manually (100x oil immersion) by pathologists in morphologically intact and non-overlapping nuclei and a minimum of 30 cells or the maximum numbers of cancer cells available in three cores from a case were recorded. Cases without 30 evaluable cells were reported as insufficient hybridization. All BACs were obtained from the BACPAC Resource Center (Oakland, CA), and probe locations were verified by hybridization to metaphase spreads of normal peripheral lymphocytes. For detection of TMPRSS2, ERG and ETV4 rearrangements we used the following probes: RP11-35C4 (5' to TMPRSS2) and RP11-120C17 (3' to TMPRSS2), RP11-95I21 (5' to ERG) and RP11-476DI7 (3' to ERG), and RP11-100E5 (5' to ETV4) and RP11-436J4 (3' to ETV4). For detection of TMPSS2-ETV1 fusion, RP11-35C4 (5' to TMPRSS2) was used with RP11-124L22 (3'to ETV1). BAC DNA was isolated using a QIAFilter Maxi Prep kit (Qiagen, Valencia, CA), and probes were synthesized using digoxigenin- or biotin-nick translation mixes (Roche Applied Science, Indianapolis, IN). The digoxigenin and biotin labeled probes were detected using fluorescein conjugated anti-digoxigenin antibodies (Roche Applied Science) and Alexa 594 conjugated sptreptavidin (Invitrogen, Carlsbad, CA), respectively.

A break apart (TMPRSS2, ERG, ETV4) or fusion (TMPRSS2-ETV1) probe strategy was employed to detect rearrangements at the chromosomal level. Normal signal patterns for TMPRSS2, ERG and ETV4 in DAPI stained nuclei were indicated by two pairs of colocalized green and red signals. For these probes, a rearrangement was confirmed by break apart of one of the two colocalized signals. For TMPRSS2-ETV1 fusion, two pairs of separate red and green were recorded as normal, while one pair of separate and one pair of colocalized signals was recorded as a rearrangement.

### B. Results and Discussion

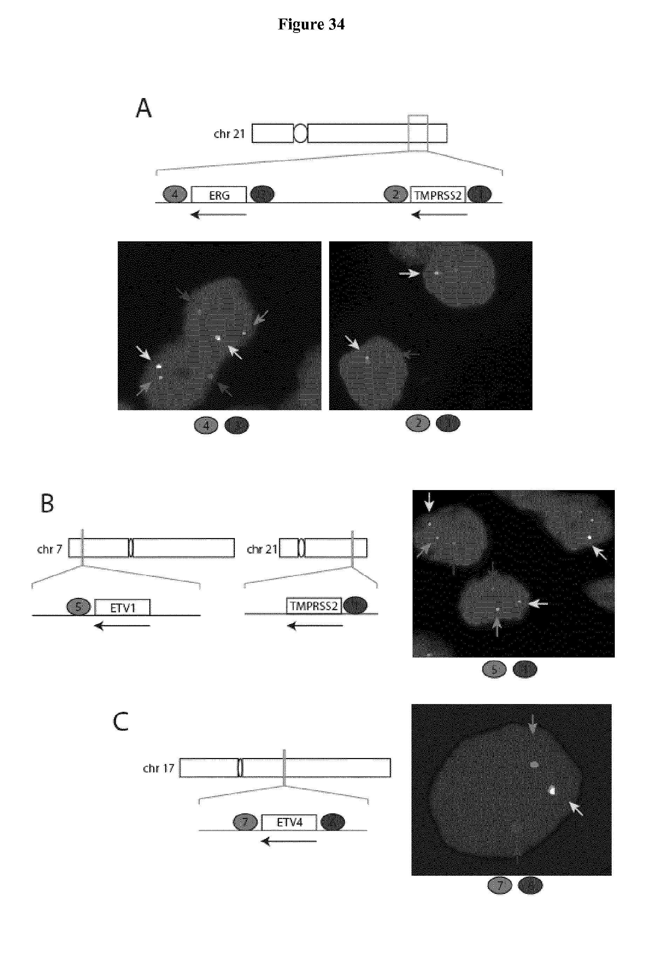

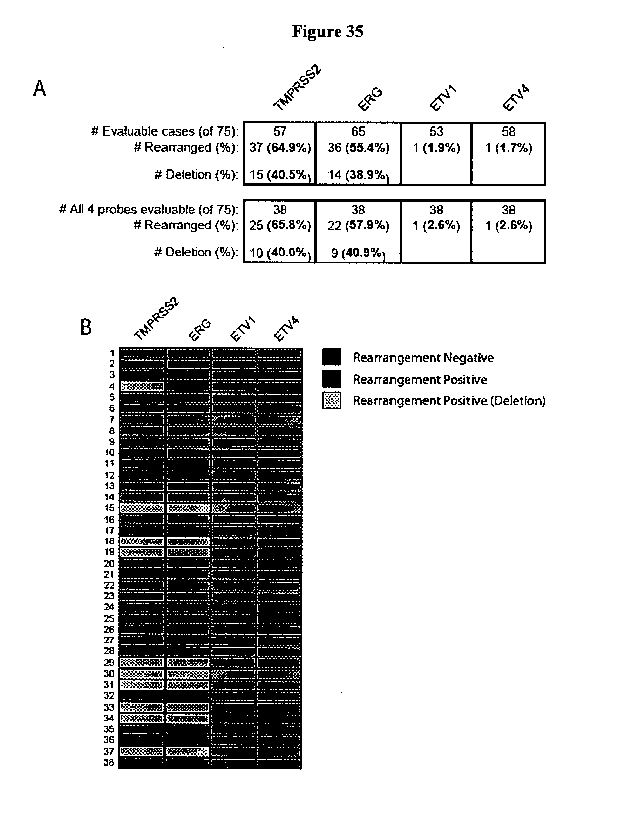

This example describes a comprehensive analysis outlining the signature of TMPRSS2 and ETS transcription factor genes rearrangement in a large screening-based cohort of American men surgically treated for clinically localized prostate cancer. A TMPRSS2 split probe FISH assay approach was used to detect the overall frequency of gene rearrangement in prostate cancer with known ETS family partners ERG, ETV1, ETV4 and other unknown partners, as shown in Figure 34. It was hypothesized that prostate cancers negative for three known ETS partners (ERG, ETV1 and ETV4) may harbor rearrangements involving other ETS family members. The results demonstrate complex molecular signature of TMPRSS and ETS family genes rearrangement in clinically localized prostate cancer (Figure 35A and B). Overall TMPRSS2 was rearranged in 65% of evaluable cases, while ERG, ETV1 and ETV4 were rearranged in 55%, 2% and 2% of evaluable cases (Figure 35A). In 40.5% of cases with TMPRSS2 rearrangement, loss of the 3' probe was observed, consistent with a chromosomal deletion between TMPRSS2 and ERG as a mechanism of gene fusion. These results confirm the high frequency of TMPRSS2:ETS fusions in prostate cancer and confirm previous studies showing that TMPRSS2:ERG are by far the most common type (Tomlins et al., Science 310:644; Pemer et al., Cancer Res 66:3396 [2006]; Yoshimoto et al., Neoplasia 8:4665 [2006]; Soller et al., Genes Chromosomes Cancer 45:717 [2006]; Wang et al., Cancer Res 66:8347 [2006] and above examples).

Similar results were observed when the cohort was limited to just those cases where all four probes were evaluable (Figure 35A and B). This analysis confirmed that TMPRSS2:ETS rearrangements are mutually exclusive, as no cases showed rearrangments of multiple ETS family members. This analysis also demonstrates that a single TMPRSS2 assay can effectively detect almost all ETS rearrangements, as 23 of the 24 cases with ERG, ETV1 or ETV4 rearrangement were detected by the TMPRSS2 assay. In all 9 cases where the 5' ERG probe was deleted, deletion of the 3' TMPRSS2 probe was identified.

Furthermore, two cases were identified with break apart of the TMPRSS2 probes, indicating a rearrangement, without rearrangement of ERG, ETV1 or ETV4 (cases 32 and 36) and cases with TMPRSS2 rearrangement without ERG rearrarngement where ETV1 and/or ETV4 could not be evaluated. These cases suggest that TMPRSS2 may be partnering with novel ETS family members or unrelated oncogenes in prostate cancer. Together, these results suggest that a single TMPRSS2 assay can provide diagnostic and prognostic information in prostate cancer.

### Example 9: PSA Gene Fusions

FISH experiments were used to identify cases that show a split signal by FISH for probes located 5' and 3' to PSA. The 5' and 3' BACs used to detect the PSA split are RP11-510116 and RP11-26P14, respectively. A partner for the PSA gene fusion has not yet been identified. These same probes also pick up a split in the ETS family member SPIB, as it is located very close to PSA.

### Example 10: FLI1 overexpression

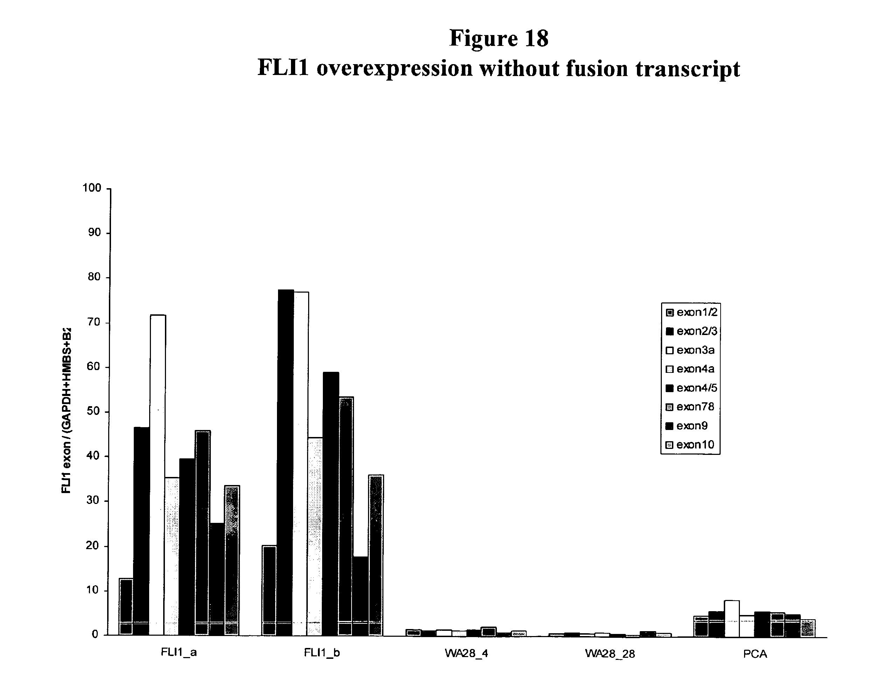

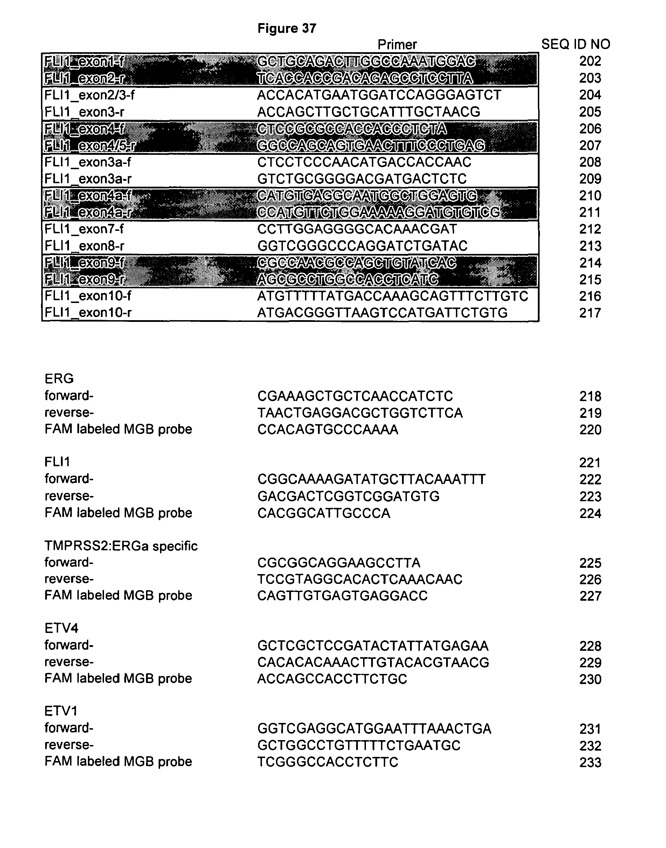

FLI1 expression was assayed in different cell samples not harboring a FLI1 gene fusion. The expression of 5' and 3' exons of FLI1 was measured from a case with high FLI1 expression. Results are shown in Figure 18. No difference in the 5' and 3' transcript abundance was detected. RACE also did not indicate a fusion transcript. FLI1 was overexpressed in prostate cancer relative to control samples. Primers for Fli1 amplification, as well as TaqMan probes, are shown in Figure 37.

FISH was also used to identify samples that have split signals for FLI1, indicating a rearrangement, but these cases do not have TMPRSS2:FLI1 fusion by FISH. BAC probes are shown in Table 13. These cases also have high FLI1 expression.

### Example 11: Tissue Microarrays

Tissue microarrays were used to assay for the presence of gene fusions. TMAs used included prostate cancer progression array, prostate cancer outcome array, warm autopsy array, prostate cancer screening array, Erg negative prostate cancer array, and individual prostate cancer cases. The following gene probes were used on tissue microarrays: TMPRSS2-ETV1 fusion probes, Erg split probes, TMPRSS2 split probes, ETV1 split probes, ETV4 split probes, and FL1 split probes.

In addition, Erg split probes were used on an outcome array. The results are as follows: negative cases: 30, positive case: 29, marginal cases: 1. There was a weak association of Erg positive cases with higher Gleason score (≥7).

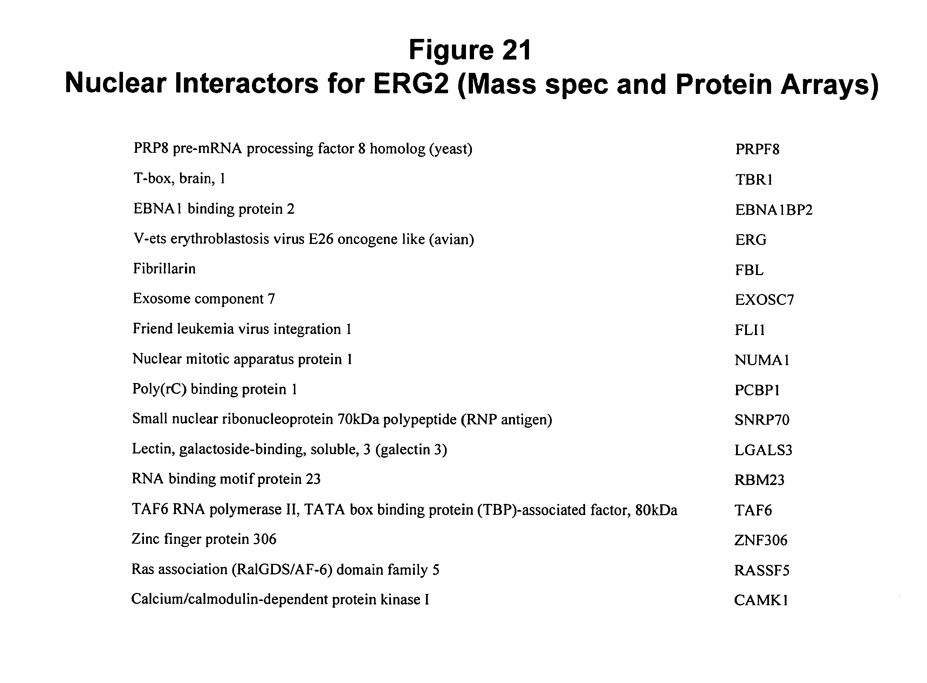

Protein arrays and mass spec were used to identify nuclear interactors for ERG2. The results are shown in Figure 21.

### Example 12: Androgen regulation of Erg expression

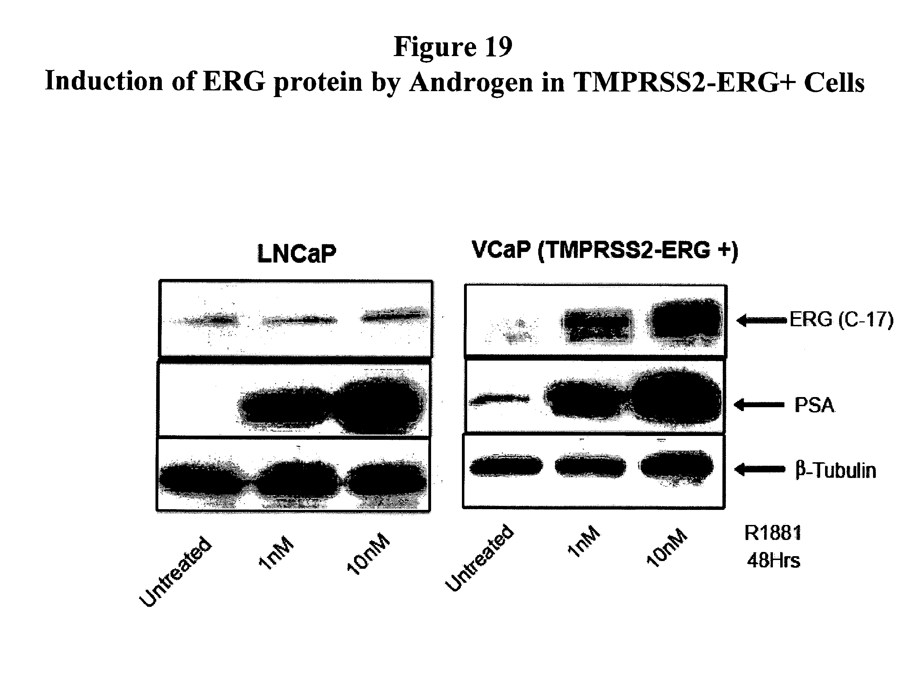

This Example describes the androgen regulation of Erg expression. LNCap (TMPRSS2-ERG-) and VCaP (TMPRSS2-ERG+) cell lines were used. The cells were contacted with varying amounts of R1881 for 48 hrs. Expression of Erg, PSA (+ control) and beta-tubulin (-control) were assayed. The results are shown in Figure 19. ERG expression was found to be androgen dependent in the VCaP, but not the LNCap cells.

### Example 13: Peptide Antibody and Aqua Probe Generation

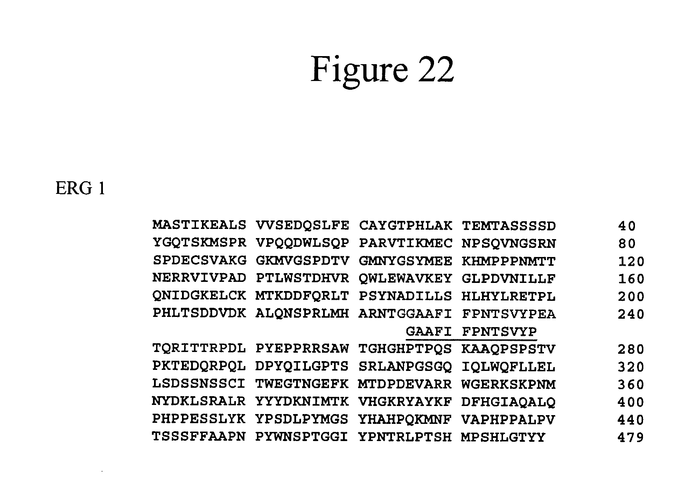

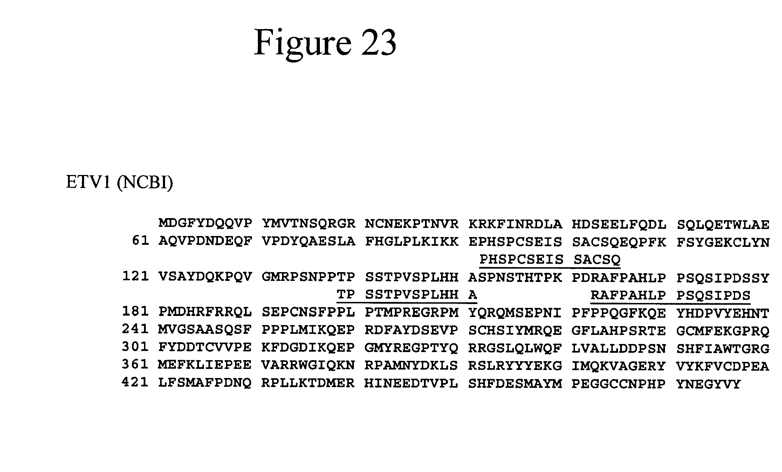

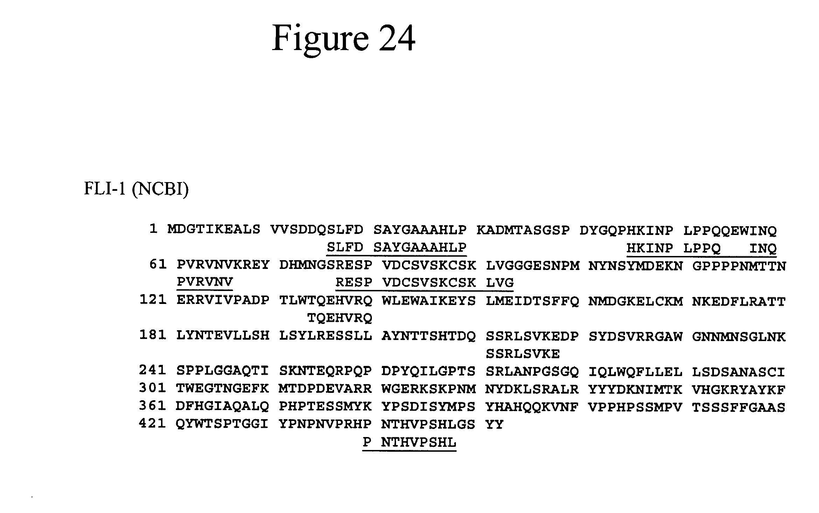

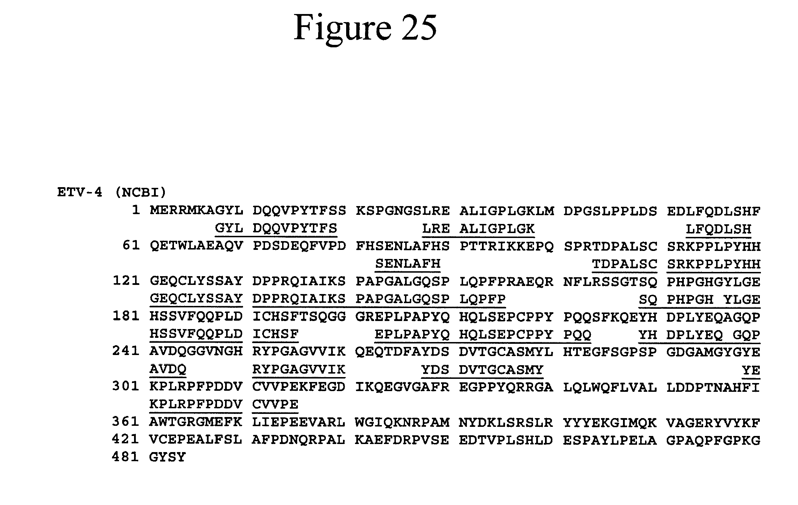

Figures 22-25 shows sequences (underlined) of ERG1, ETV1, FLI-1, and ETV4 for use in peptide antibody generation and for making aqua probes. Primers are designed by Applied Biosystems for all ETS family members. Expression is monitored in prostate cancer cases, with high expression being an indicator of a possible gene fusion and an indicator for FISH.

### Example 14: ETV1 in LnCaP Cells

This Example describes an analysis of the transcriptional response to androgen in VCaP and LNCaP. In addition to detecting a number of transcripts differentially expressed in both cell lines were identified, such as PSA, a number of transcripts uniquely dysregulated in VCaP or LNCaP cells were also identfied. This analysis identified ETV1 as being exclusively responsive to androgen in LNCaP cells. Combined with the over-expression of ETV1 in LNCaP cells, FISH was used to interrogate the ETV1 loci in LNCaP cells.

### A. Materials And Methods

### Cell lines

The prostate cancer cell lines LNCaP (originally derived from a lymph node prostate cancer metastasis) and VCaP (Korenchuk, S. et al., In vivo 15, 163-8 (2001)) (originally derived from a vertebral prostate cancer metastasis) were used for this study. For microarray studies, VCaP and LNCaP cells were grown in charcoal-stripped serum containing media for 24 hours before treatment for 48 hours with 0.1% ethanol or 1 nM of the synthetic androgen methyltrienolone (R1881, NEN Life Science Products, Boston, MA) dissolved in ethanol. For quantitative PCR (QPCR) studies, cells were grown in charcoal-stripped serum containing media for 24 hours, preincubated with 0.1% ethanol, Casodex dissolved in acetone (10 uM, bicalutamide, AstraZeneca Pharmaceuticals, Wilmington, DE) or flutamide dissolved in ethanol (10 uM, Sigma, St. Louis, MO). After 2 hours, 0.1% ethanol or 0.5nM of R1881 was added and the cells were harvested after 48 hours. Total RNA was isolated from all samples with Trizol (Invitrogen, Carlsbad, CA) according to the manufacturer's instructions. RNA integrity was verified by denaturing formaldehyde gel electrophoresis or the Agilent Bioanalyzer 2100 (Agilent Technologies, Palo Alto, CA).

### Microarray analysis

The cDNA microarrays used for this study were constructed essentially as described, except the array contains 32,448 features. Protocols for printing and postprocessing of arrays are available on the Internet. cDNA microarray analysis was done essentially as described. Briefly, total RNA from control and R1881 treated VCaP and LNCaP cell lines were reverse transcribed and labeled with cy5 fluorescent dye. Pooled total RNA from control VCaP or LNCaP samples were reverse transcribed and labeled with cy3 fluorescent dye for all hybridizations from the respective cell lines. The labeled products were then mixed and hybridized to the cDNA arrays. Images were flagged and normalized using the Genepix software package (Axon Instruments Inc., Union City, CA). Data were median-centered by arrays and only genes that had expression values in at least 80% of the samples were used in the analysis.

### Quantitative PCR (QPCR)

QPCR was performed using SYBR Green dye on an Applied Biosystems 7300 Real Time PCR system (Applied Biosystems, Foster City, CA) as described (Tomlins et al., Cancer Res 66, 3396-400 (2006); Tomlins et al., Science 310, 644-8 (2005)). The amount of each target gene relative to the housekeeping gene glyceraldehyde-3-phosphate dehydrogenase (*GAPDH*) for each sample was reported. The relative amount of the target gene in each cell line and/or experiment was calibrated to controls. All oligonucleotide primers were synthesized by Integrated DNA Technologies (Coralville, IA). GAPDH (Vandesompele et al., Genome Biol 3, RESEARCH0034 (2002)), PSA (Specht et al., Am J Pathol 158, 419-29 (2001)), ERG (Exon 5-6_f and Exon 5-6_r) and ETV1 (Exon 6-7_f and Exon 6-7_r) primers (Tomlins et al., Science 310, 644-8 (2005)) were as described.

### Fluorescence in situ hybridization (FISH)

Metaphase spreads were prepared from normal peripheral lymphocytes (NPLs) and LNCaP cells using standard techniques. Slides were treated with 2x SSC for 2 min, 70% ethanol for 2 min and 100% ethanol for 2 min before addition of the probe. Slides were coverslipped and incubated at 75° for 2 min and hybridized overnight at 37° C. Post-hybridization washing was with 2x SSC at 42° C for 5 min, followed by 3 washes in PBST. Fluorescent detection was performed using anti-digoxigenin conjugated to fluorescein (Roche Applied Science, Indianapolis, IN) and streptavidin conjugated to Alexa Fluor 594 (Invitrogen, Carlsbad, CA). Slides were counterstained and mounted in ProLong Gold Antifade Reagent with DAPI (Invitrogen). Slides were examined using a Zeiss Axio Imager Z1 fluorescence microscope (Zeiss, Thornwood, NY) and imaged with a CCD camera using ISIS software (Metasystems, Altlussheim, Germany).

All BACs were obtained from the BACPAC Resource Center (Oakland, CA) and probe locations were verified by hybridization to metaphase spreads of normal peripheral lymphocytes. For hybridization to the ETV1 region on chromosome 7p, four BACs were used (telomeric to centromeric): RP11-124L22, RP11-313C20, RP11-703A4 and RP11-1149J13. For localization to chromosome 14q, the FISH mapped BAC RP11-483K13, which we also confirmed as hybridizing to 14q using NPLs. BAC DNA was isolated using a QIAFilter Maxi Prep kit (Qiagen, Valencia, CA) and probes were synthesized using digoxigenin- or biotin-nick translation mixes (Roche Applied Science).

### B. Results

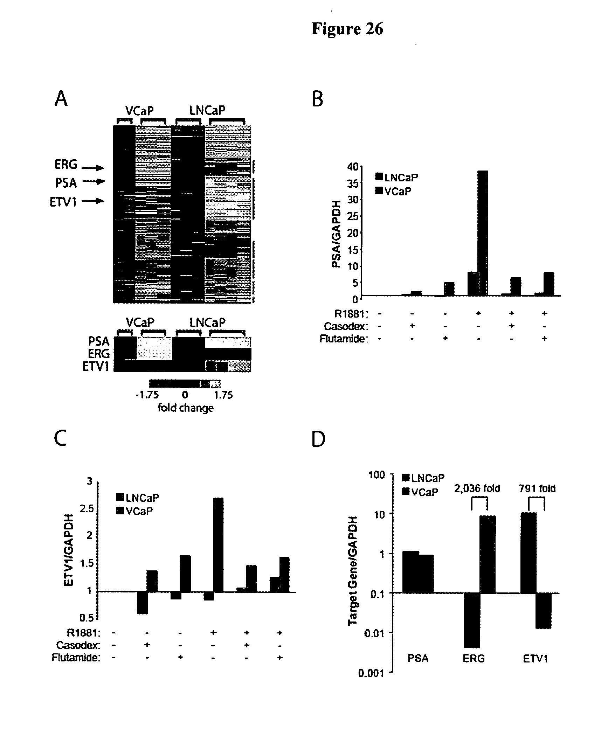

Results are shown in Figures 26-28. Figure 26 shows the over-expression and androgen regulation of ETV1 in the LNCaP prostate cancer cell line. Figure 26A shows expression signature of androgen-regulated genes in VCaP and LNCaP prostate cancer cell lines. Heatmap of genes showing induction or repression in either cell line (3,499 features, p < 0.05 and fold change ratio>=1.5) by 1 nM synthetic androgen R1881 (green) compared to vehicle treatment (gray). Each row represents a gene; each column represents a sample. Yellow and blue cells indicate over- or under-expression, respectively, according to the color scale. Gray cells indicate missing data. Values for each cell line are centered on the corresponding control samples. The locations of PSA, ERG and ETV1 in the heatmap are indicated and their expression is shown in the inset. Figure 26B shows confirmation of PSA induction by androgen in both VCaP and LNCaP cells by quantitative PCR (QPCR). The relative expression of PSA (normalized to GAPDH) in LNCaP (red) and VCaP (blue) cell lines was determined by QPCR. Cells were treated with vehicle or 1 nM R1881 for 48 hours in the presence or absence of the anti-androgens Casodex or Flutamide as indicated. The relative amount of PSA in each sample was calibrated to the amount in the control sample for each cell line. Figure 26C shows ETV1 induction by androgen in LNCaP cells. Using the same samples as B, the relative amount of ETV1 was determined by QPCR. Figure 26D shows that ETV1 is markedly over-expressed in LNCaP cells. The relative expression of PSA, ETV1 and ERG were determined in the 48 hour control samples from each cell line by QPCR. The relative amount of target gene in each sample was calibrated to the average amount of PSA from both cell lines. The fold difference in ERG and ETV1 expression between LNCaP and VCaP is indicated.

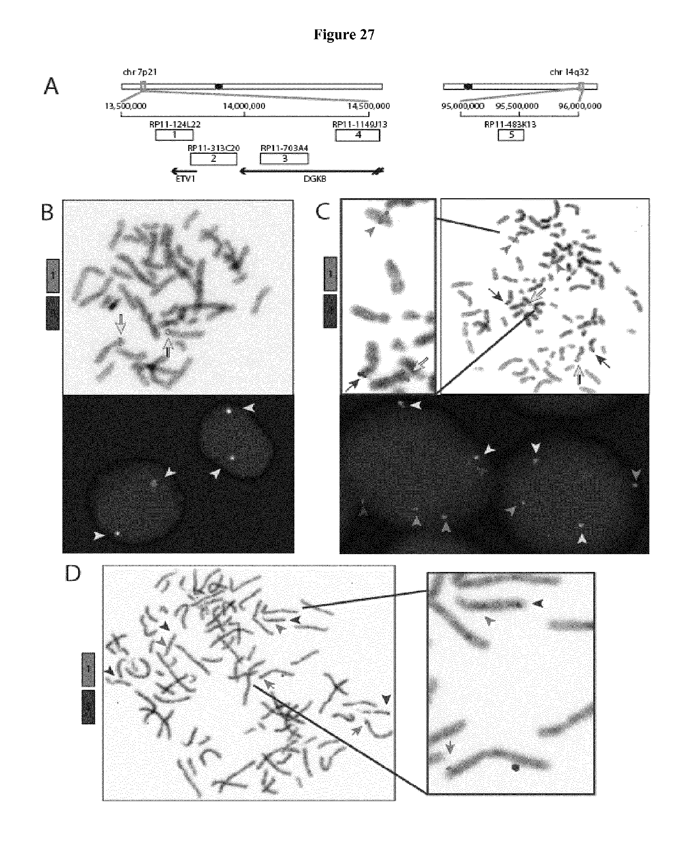

Figure 27 shows rearrangement of ETV1 in LNCaP cells. Figure 27A shows a schematic of BACs used as probes for fluorescence *in situ* hybridization (FISH). The location and coordinates at 7p21 (including the ETV1 locus and surrounding BACs) and 14q32 was determined on the May 2004 freeze of the human genome using the UCSC Genome Browser. BACs used in this study are indicated as numbered rectangles. The location of ETV1 and DGKB are shown with the arrowhead indicating the direction of transcription. Figure 27B shows that RP11-124L22 and RP11-1149J13 co-localize to chromosome 7 in normal peripheral lymphocytes (NPLs). Localization of RP11-124L22 (BAC #1) and RP11-1149J13 (BAC #4) on metaphase spreads (top panel) or interphase cells (bottom panel) was determined by FISH in NPLs. For all metaphase pictures, signals on chromosome 7 are indicated by arrows, while signals on chromosome 14 are indicated by arrowheads of the corresponding probe color. Higher magnification of informative regions of metaphase spreads are shown in boxes. Figure 27C shows localization of BAC #1 and BAC #4 on metaphase spreads (top panel) and interphase cells (bottom panel) was determined in the near tetraploid LNCaP cell line. Two co-localized signals on chromosome 7, two red signals on chromosome 7 and two green signals on a different chromosome were observed. Figure 27D shows signal from RP11-124L22 localizes to chromosome 14 in LNCaP cells. As in C, except RP11-124L22 (BAC #1) was co-hybridized with RP11-483K13 (BAC #5, FISH mapped to chromosome 14q) on LNCaP metaphase spreads. Four red signals from RP11-483K13 localize to chromosome 14q; two green signals localize to chromosome 7p and two green signals localize to chromosome 14q.

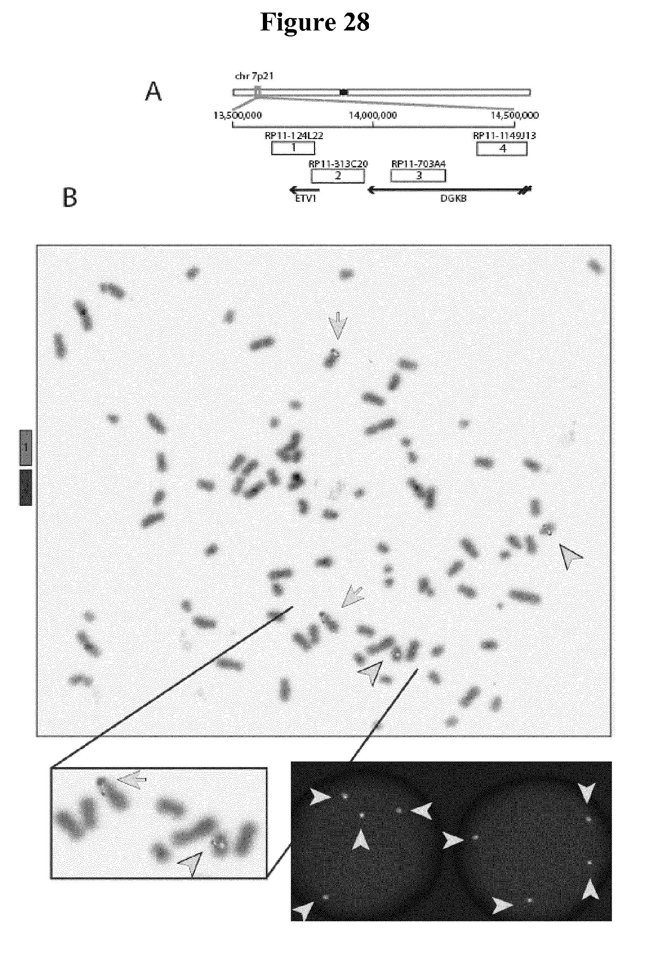

Figure 28 shoes that the entire ETV1 locus is inserted into chromosome 14 in LNCaP cells. Figure 28A shows a schematic of BACs used in this experiment. Figure 28B shows localization of RP11-124L22 (BAC #1) and RP11-313C20 (BAC #2) on metaphase spreads (top panel) and interphase cells (bottom panel) was determined by FISH in LNCaP cells. In metaphase spreads, two pairs of co-localized signals were observed on chromosome 7 (yellow arrows) and chromosome 14 (yellow arrowheads).

These results demonstrate that the entire ETV1 locus is translocated from chromosome 7 to chromosome 14. Although the genomic sequence upstream of the insertion on chromosome 14 is unknown, it is likely that this region contains AREs, which drive the high level of ETV1 observed only in LNCaP cells and the androgen responsiveness. These results suggest that LNCaP cells find use as an in vitro model of ETS gene fusions seen in human prostate cancers.

### Example 15: Knockdown of ETS Family Members in PCA

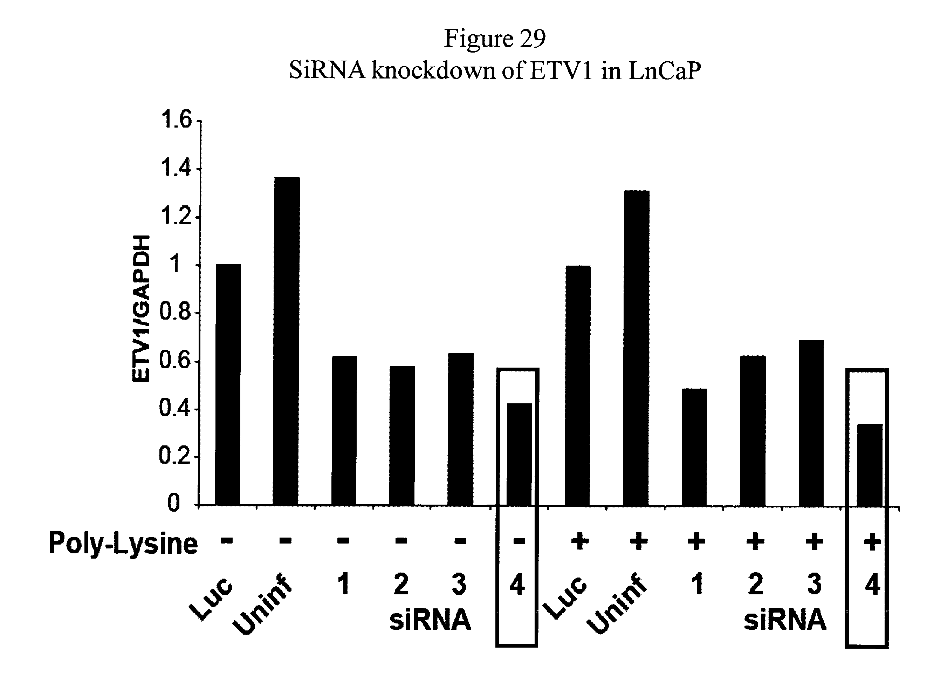

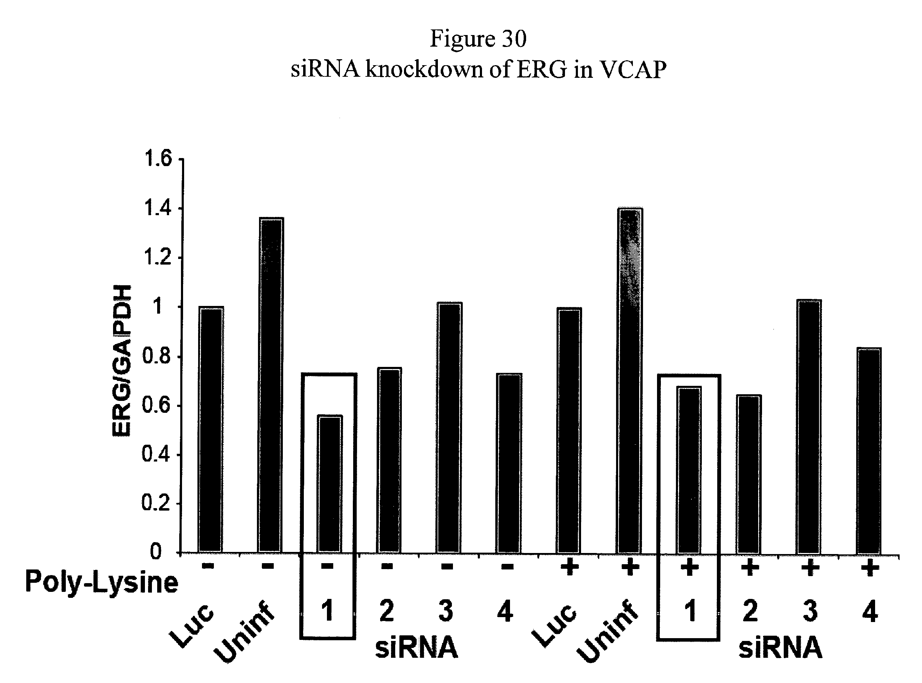

This Example describes the knockdown of ETS family members in prostate cancer. siRNAs were used to knockdown expression of ETV1 and ERG in LnCaP and VCAP. Quantitative PCR was used to confirm the knockdown. Results are shown in Figures 29 and 30. The knockdown did not affect proliferation. Lentivirus expressing shRNA are generated for stable knockdowns.

Microarrays were performed on Agilent 44K Whole Genome arrays to determine which genes were differentially expressed when ERG expression was knocked down in VCaP cells (which have the TMPRSS2:ERG fusion). For this experiment, three conditions were used: knockdown using Dharmacon siRNA for ERG (ERGsi), knockdown of luciferase (control), and untransfected (untrans) VCaP cells. Three hybridizations of ERG/untrans and two of control/untrans were performed. The genes were called as present in all five experiments, had standard deviations less than 0.5 (of the average for both conditions), and showed a fold difference between the ERG and control of < 0.75 or > 1.5. The ERGdif field indicates the fold difference between the ERG and control knockdown experiments, so value less than one means the gene is underexpressed in the ERG knockdown (ERG itself ranks 8 1 st in this analysis).

### Example 16: Transgenic Mice

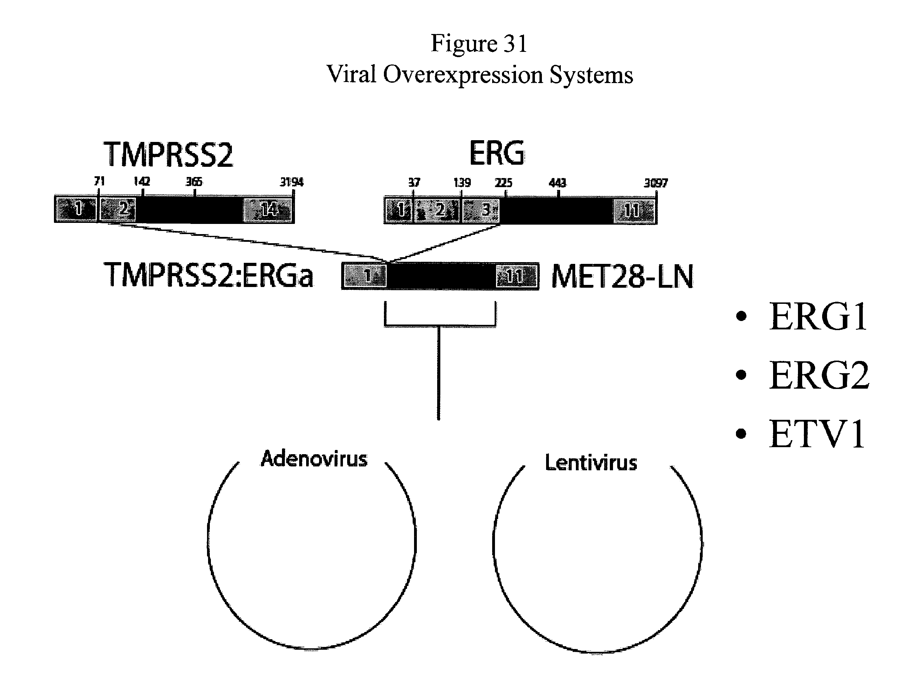

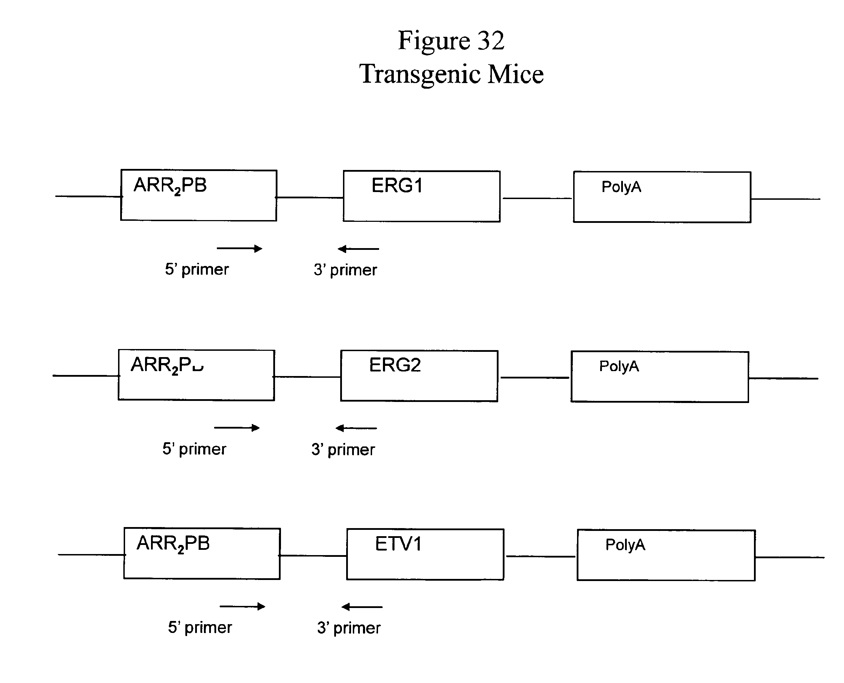

Transgenic mice that over express gene fusions of the present invention, as well as ETS and androgen responsive genes are generated. Figure 31 shows viral overexpression systems for use in generating mice. Figure 32 shows a schematic of genomic insertions in transgenic mice. Such mice find use in research (*e.g*., mechanistic studies) and drug screening applications.

### Example 17: Identification of TMPRSS2:ERGa

As described above (Example 1), fusions of TMPRSS2 to ERG were observed. To determine the expressed protein from the TMPRSS2:ERGa gene fusion, PCR was used to amplify the portion of ERG (NM_004449) from the fusion breakpoint at the beginning of exon 4 to the presumed stop codon in exon 11, inserting a 3x Flag tag immediately upstream of the stop codon, from the VCaP prostate cancer cell line. The product was TA cloned into pCR8/GW/TOPO TA (Invitrogen) and bi directionally sequenced. Sequencing revealed the presence of two distinct isoforms, herein designated as ERG1 (includes exon 6 from ERG isoform 1 (NM_182918, GGGGTGCAGCTTTTATTTTCCCAAATACTTCAGTATATCCTGAAGCTACGCAAAGAA TTACAACTAGGCCAG; SEQ ID NO:73) and ERG2 (does not include this exon). The product was Gateway cloned into the pLenti6/V5-DEST destination vector. This plasmid was transfected directly into PHINX cells for ERG protein production.

### A. Methods

Transfection Assay: Phinx cells were transfected with either ERG2 or the empty vector using Fugene transfection reagent (Roche) as per manufacturer's instructions. A total of ten 150 mm diameter plates were used for each construct. The cells were harvested 48h post-transfection and used for immunoprecipitation assay as described below.

Protein Lysis and Immunoprecipitation: Cells were washed in ice cold PBS containing protease inhibitors and lysed by homogenization in TBS containing 1 % NP40. The supernantant containing proteins were estimated for their protein content using Bradfords Protein Assay (Biorad Laboratories, Hercules, CA) as per manufacturer's instructions. Equal amounts of protein (approximately 30 mg in 15 ml buffer) from all samples were used for immunoprecipitation studies. About 200 microlitres of a 50 % slurry of EZVIEW Red ANTI-FLAG M2 Affinity Gel (Sigma, St Louis, MO) was added to each sample and incubated overnight at 4C. The immunoprecipitate was washed thrice each with TBS containing 0.1 % NP40 and TBS alone. Bound proteins were eluted using FLAG peptide (Sigma, St Louis, MO) as per manufacturer's instruction. The elution was performed three times. Proteins in the eluate were preicipited using 50 % TCA (Sigma, St Louis, MO). The precipitate was washed thrice with ice cold acetone, resuspended in Laemmeli buffer and electrophoresed on 4-20 % BIS-TRIS gel (Invitrogen Corporation, Carlbad, CA). The gels were stained with mass spectrometry compatible silver stain (Silver Quest, Invitrogen Corporation, Carsbad, CA). Bands corresponding to ERG2 and the corresponding region in the vector lane were excised into 6 pieces of 1cm each. Each of the gel pieces were labeled bands 1-6 starting from higher molecular weight region on the gel moving down. Thus Band 1 corresponds to the region containing high molecular weight proteins while band 6 correponds to region of low molecular weight. Based on its native molecular mass of ERG2 (approximately 55 KDa) would migrate in Bands 4 and 5. ERG2 sequence identification was repeated three times and the data was consolidated from all the experiments.

### Protein Identification

The gel bands were collected, destained using the destaining solution provided in the Silver Stain Kit as per manufacturers instruction (Invitrogen Corporation, Carsbad, CA). In gel digestion was performed using Porcine Trypsin (1:50, Promega Corporation, Madison, WI) in 1M Ammonium Bicarbonate, pH 9. The digestion was performed for 16h at 37°C. At the end of 24h the trypsin activity was stopped using 3 % formic acid. The peptides were extracted using 50 % Acetonitrile. The peptides were dried and resuspended in 2 % Acetonitrile containing 0.1 % formic acid and separated by reversed-phase chromatography using a 0.075 mm x 150 mm C18 column attached to a Paradigm HPLC pump (Michrome Bio Resources Inc.). Peptides were eluted using a 45-min gradient from 5 to 95% B (0.1% formic acid/95% acetonitrile), where solvent A was 0.1 % formic acid/2% acetonitrile. A Finnigan LTQ mass spectrometer (Thermo Electron Corp.) was used to acquire spectra, the instrument operating in data-dependent mode with dynamic exclusion enabled. The MS/MS spectra on three most abundant peptide ions in full MS scan were obtained. The spectra are searched using the MASCOT search tool against the composite, non-identical NCBI human reference sequence database. These database search results are validated for peptide assignment accuracy using the PeptideProphet program. This is a mixture model; an expectation maximization evaluation assigning a probability of correct peptide identification based on search result scores and various peptide features including the number of typtic termini. A second program, ProteinProphet, is used to group peptides by protein and combine their probabilities to assign a probability of a correct protein assignment. Discriminatory power increases with the subsequent re-estimation of individual peptide probabilities by way of their NSP value, or number of sibling peptides, which amounts to peptide grouping information and the status of a possible multi-hit protein.

### Results:

**Table 14**

| COVERAGE MAP **(ERG2)** | | | | | |
|---|---|---|---|---|---|
| NOTE: E*BAND*-* represent ERG2 peptides in ERG1 experiments | | | | | |
| **MIQTVPDPAA HI...** (SEQ ID NO:74) | | | | NCBI N-terminal, BAND05- | |
| **20060217** | | | | **SEQ ID NO** | |
| MASTIKEALS VVSEDQSLFE CAYGTPHLAK TEMTASSSSD | | | | 40 | 74 |
| | | | SSSSD | BAND03-20060206 | 75 |
| YGQTSKMSPR | VPQQDWLSQP | PARVTIKMEC | NPSQVNGSRN | 80 | 76 |
| | VPQQDWLSQP | PAR | | BAND01-20060217 | 77 |
| | VPQQDWLSQP | PAR | | BAND02-20060206 | 78 |
| | VPQQDWLSQP | PAR | | BAND02-20060209 | 79 |
| | VPQQDWLSQP | PAR | | BAND02-20060217 | 80 |
| YGQTSKMS | VPQQDWLSQP | PAR | | BAND03-20060206 | 81 |
| | VPQQDWLSQP | PAR | | BAND03-20060209 | 82 |
| | VPQQDWLSQP | PAR | | BAND03-20060217 | 83 |
| | VPQQDWLSQP | PAR | | BAND04-20060206 | 84 |
| | VPQQDWLSQP | PAR MEC | NPSQVNGSR | BAND04-20060209 | 85 |
| | VPQQDWLSQP | PAR | | BAND04-20060217 | 86 |
| | VPQQDWLSQP | PAR | | BAND05-20060217 | 87 |
| SPDECSVAKG GKMVGSPDTV GMNYGSYMEE KHMPPPNMTT | | | | 120 | 88 |
| | | | HMPPPNMTT | BAND01-20060206 | 89 |
| | | | HMPPPNMTT | BAND02-20060206 | 90 |
| | | | HMPPPNMTT | BAND02-20060209 | 91 |
| | | NYGSYMEE | KHMP | BAND02-20060217 | 92 |
| | MVGSPDTV | GMNYGSYMEE | KHMPPPNMTT | BAND03-20060206 | 93 |
| | | | HMPPPNMTT | BAND03-20060209 | 94 |
| | | | HMPPPNMTT | BAND04-20060206 | 95 |
| | MVGSPDTV | GMNYGSYMEE | KHMPPPNMTT | BAND04-20060209 | 96 |
| | MVGSPDTV | GMNYGSYMEE | KHMPPPNMTT | BAND04-20060217 | 97 |
| NERRVIVPAD | PTLWSTDHVR | QWLEWAVKEY | GLPDVNILLF | 160 | 98 |
| NER VIVPAD | PTLWSTDHVR | QWLEWAVKEY | GLPDVNILLF | BAND01-20060206 | 99 |
| NER | | EY | GLPDVNILLF | BAND02-20060206 | 100 |
| NER | | | | BAND02-20060209 | 101 |
| NER VIVPAD | PTLWSTDHVR | QWLEWAVK | | BAND03-20060206 | 102 |
| NERRVIVPAD | PTLWSTDHVR | EY | GLPDVNILLF | BAND03-20060209 | 103 |
| NER VIVPAD | PTLWSTDHVR | QWLEWAVKEY | GLPDVNILLF | BAND04-20060206 | 104 |
| NERRVIVPAD | PTLWSTDHVR | QWLEWAVKEY | GLPDVNILLF | BAND04-20060209 | 105 |
| NERRVIVPAD | PTLWSTDHVR | | | BAND04-20060217 | 106 |
| | | EY | GLPDVNILLF | BAND05-20060206 | 107 |
| QNIDGKELCK | MTKDDFQRLT | PSYNADILLS | HLHYLRETPL | 200 | 108 |
| QNIDGK | LT | PSYNADILLS | HLHYLRETPL | BAND01-20060206 | 109 |
| | | | ETPL | BAND01-20060217 | 110 |
| QNIDGK | | | ETPL | BAND02-20060206 | 111 |
| | | | ETPL | BAND02-20060217 | 112 |
| | | | ETPL | BAND03-20060206 | 113 |
| QNIDGK | LT | PSYNADILLS | HLHYLRETPL | BAND03-20060209 | 114 |
| | | | ETPL | BAND03-20060217 | 115 |
| QNIDGK | LT | PSYNADILLS | HLHYLRETPL | BAND04-20060206 | 116 |
| QNIDGK | LT | PSYNADILLS | HLHYLRETPL | BAND04-20060209 | 117 |
| | LT | PSYNADILLS | HLHYLRETPL | BAND04-20060217 | 118 |
| QNIDGK | | | | BAND05-20060206 | 119 |
| | | PSYNADILLS | HLHYLRETPL | BAND05-20060217 | 120 |
| PHLTSDDVDK | ALQNSPRLMH | ARNTGGAAFI | FPNTSVYPEA | 240 | 121 |
| | PRLMH | ARNT | | BAND01-20060206 | 122 |
| | | | | | |
| PHLTSDDVDK | | | | BAND01-20060206 | 123 |
| PHLTSDDVDK | ALQNSPR | | | BAND01-20060217 | 124 |
| PHLTSDDVDK | ALQNSPR | | | BAND02-20060206 | 125 |
| PHLTSDDVDK | ALQNSPR | | | BAND02-20060217 | 126 |
| PHLTSDDVDK | ALQNSPR | | | BAND03-20060206 | 127 |
| PHLTSDDVDK | | | | BAND03-20060209 | 128 |
| PHLTSDDVDK | ALQNSPR | | | BAND03-20060217 | 129 |
| PHLTSDDVDK | ALQNSPR | | | BAND04-20060206 | 130 |
| PHLTSDDVDK | ALQNSPR | | | BAND04-20060209 | 131 |
| | | RNT | | BAND04-20060209 | 132 |
| | | | | | |
| PHLTSDDVDK | ALQNSPR | | | BAND04-20060217 | 133 |
| PHLTSDDVDK | ALQNSPRL | | | BAND05-20060217 | 134 |
| TQRITTRPDL | PYEPPRRSAW | TGHGHPTPQS | KAAQPSPSTV | 280 | 135 |
| DLPYEPPR | | | | BAND01-20060206 | 136 |
| | | | | | |
| | SAW | TGHGHPTPQS | KAAQPSPSTV | BAND01-20060206 | 137 |
| | PYEPPRR | | | BAND01-20060217 | 138 |
| | SAW | TGHGHPTPQS | KAAQPSPSTV | BAND02-20060206 | 139 |
| | SAW | TGHGHPTPQS | KAAQPSPSTV | BAND02-20060209 | 140 |
| | PYEPPRRSAW | TGHGHPTPQS | KAAQPSPSTV | BAND02-20060217 | 141 |
| | SAW | TGHGHPTPQS | KAAQPSPSTV | BAND03-20060206 | 142 |
| | SAW | TGHGHPTPQS | KAAQPSPSTV | BAND03-20060209 | 143 |
| DL | PYEPPRR | | | BAND03-20060217 | 144 |
| | PYEPPRRSAW | TGHGHPTPQS | KAAQPSPSTV | BAND04-20060206 | 145 |
| DL | PYEPPRRSAW | TGHGHPTPQS | KAAQPSPSTV | BAND04-20060209 | 146 |
| | DL PYEPPRR | | | BAND04-20060209 | 147 |
| | | | | | |
| DL | PYEPPRRSAW | TGHGHPTPQS | KAAQPSPSTV | BAND04-20060217 | 148 |
| | | | AAQPSPSTV | BAND05-20060206 | 149 |
| | SAW | TGHGHPTPQS | KAAQPSPSTV | BAND05-20060209 | 150 |
| DL | PYEPPRRSAW | TGHGHPTPQS | KAAQPSPSTV | BAND05-20060217 | 151 |
| | SAW | TGHGHPTPQS | KAAQPSPSTV | BAND06-20060209 | |
| PKTEDQRPQL | DPYQILGPTS | SRLANPGSGQ | IQLWQFLLEL | 320 | 152 |
| PK | | | | BAND01-20060206 | 153 |
| TEDQRPQL | DPYQILGPTS | SR | | BAND01-20060217 | 154 |
| PKTEDQRPQL | DPYQILGPTS | SR | | BAND02-20060206 | 155 |
| PKTEDQRPQL | DPYQILGPTS | SR | | BAND02-20060209 | 156 |
| PKTEDQRPQL | DPYQILGPTS | SR | | BAND02-20060217 | 157 |
| PKTEDQRPQL | DPYQILGPTS | SR | | BAND03-20060206 | 158 |
| PKTEDQRPQL | DPYQILGPTS | SR | | BAND03-20060209 | 159 |
| TEDQRPQL | DPYQILGPTS | SR | | BAND03-20060217 | 160 |
| PKTEDQRPQL | DPYQILGPTS | SR | | BAND04-20060206 | 161 |
| PKTEDQRPQL | DPYQILGPTS | SR | | BAND04-20060209 | 162 |
| PKTEDQRPQL | DPYQILGPTS | SR | | BAND04-20060217 | 163 |
| PK | | | | BAND05-20060206 | 164 |
| PKTEDQRPQL | DPYQILGPTS | SR | | BAND05-20060209 | 165 |
| PKTEDQRPQL | DPYQILGPTS | SR | | BAND05-20060217 | 166 |
| PK | | | | BAND06-20060209 | 167 |
| LSDSSNSSCI | TWEGTNGEFK | MTDPDEVARR | WGERKSKPNM | 360 | 168 |
| | | MTDPDEVAR | | BAND01-20060206 | 169 |
| | | MTDPDEVAR | | BAND02-20060206 | 170 |
| | | MTDPDEVAR | | BAND03-20060206 | 171 |
| | | MTDPDEVAR | | BAND03-20060209 | 172 |
| | | MTDPDEVAR | | BAND04-20060206 | 173 |
| | | MTDPDEVARR | | BAND04-20060209 | 174 |
| | | TDPDEVARR | KSKPNM | BAND04-20060217 | 175 |
| | | MTDPDEVAR | | BAND05-20060209 | 176 |
| | | | KSKPNM | BAND05-20060217 | 177 |
| | | | | | |
| NYDKLSRALR | YYYDKNIMTK | VHGKRYAYKF | DFHGIAQALQ | 400 | 178 |
| | | F | DFHGIAQALQ | BAND02-20060206 | 179 |
| | | F | DFHGIAQALQ | BAND02-20060209 | 180 |
| | | F | DFHGIAQALQ | BAND03-20060206 | 181 |
| | | F | DFHGIAQALQ | BAND03-20060209 | 182 |
| | YYYDKNIMTK | YAYKF | DFHGIAQALQ | BAND04-20060209 | 183 |
| NYDKLSR | | | | BAND04-20060217 | 184 |
| NYDKLSR | YYYDKNIMTK | | | BAND05-20060217 | 185 |
| PHPPESSLYK | YPSDLPYMGS | YHAHPQKMNF | VAPHPPALPV | 440 | 186 |
| PHPPESSLYK | | | | BAND02-20060206 | 187 |
| PHPPESSLYK | YPSDLPYMGS | YHAH | | BAND02-20060209 | 188 |
| PHPPESSLYK | YPSDLPYMGS | YHAHPQK | | BAND03-20060206 | 189 |
| PHPPESSLYK | YPSDLPYMGS | YHAHPQK | | BAND03-20060209 | 190 |
| | YPSDLPYMGS | YHAHPQK | | BAND04-20060206 | 191 |
| PHPPESSLYK | YPSDLPYMGS | YHAHPQK | | BAND04-20060209 | 192 |
| TSSSFFAAPN | PYWNSPTGGI | YPNTRLPTSH | MPSHLGTYY | 479 | 193 |
| | NSPTG | | | BAND02-20060217 | 194 |
| | SPTGGI | YPNTR | | BAND04-20060209 | 195 |

The table shows the coverage map for ERG2 obtained over 3 different experiments.The underlined aminoacid sequence correponds to the in silico translated sequence of ERG 1 that was cloned from VCAP cells. The aminoacid sequence GGAAFI FPNTSVYPEATQRITTRP (SEQ ID NO:196) corresponds to the exon that is specific to ERG1 and is missing in ERG2. The remaining amino acid sequence correspond to ERG2 sequence identified in each of the three experiments. ERG2 was identified in Bands 1-5 in all the experiments. The peptide sequences for ERG2 obtained in each of these bands is illustrated. A very high coverage of the ERG2 protein was observed over the three experiments. The coverage map showed that the coverage of peptides in the N-terminal region of the cloned protein, corresponding to the first 50 aminoacid residues were rarely observed in the mass spectrometry coverage map. However, the peptide VPQQDWLSQP (SEQ ID NO:197) that starts with aminoacid valine was found to be highly abundant and thus identified in all the experiments. Closer evaluation suggested that aminoacid in the 47^{th} position was an in frame Methionine. The lack of any peptide upstream (Nterminus) of the 47 th methionine in multiple experiments confirms that it is the N-terminal aminoacid of ERG2. Further, the presence of a Arginine residue at the 50^{th} position makes it a potential tryptic cleavage site. Digestion by trypsin at this site would result in a shorter N-terminal peptide MSPR, which is too small for identification by ion trap mass spectrometer and a longer C-terminal peptide VPQQDWLSQP (SEQ ID NO:198), which was identified in all the experiments. Also the peptide sequence MIQTVPDPAA HI (SEQ ID NO:199) was identified in a single experiment at a very low probablility score. This maps to the N-terminus of ERG as reported in NCBI. This sequence was not a part of the ectopically overexpressed construct that was cloned from the VCAP cells. This could have been obtained from the *in vivo* ERG that is expressed in PHINX cells and thus may represent part of the ERG associated with benign cells.

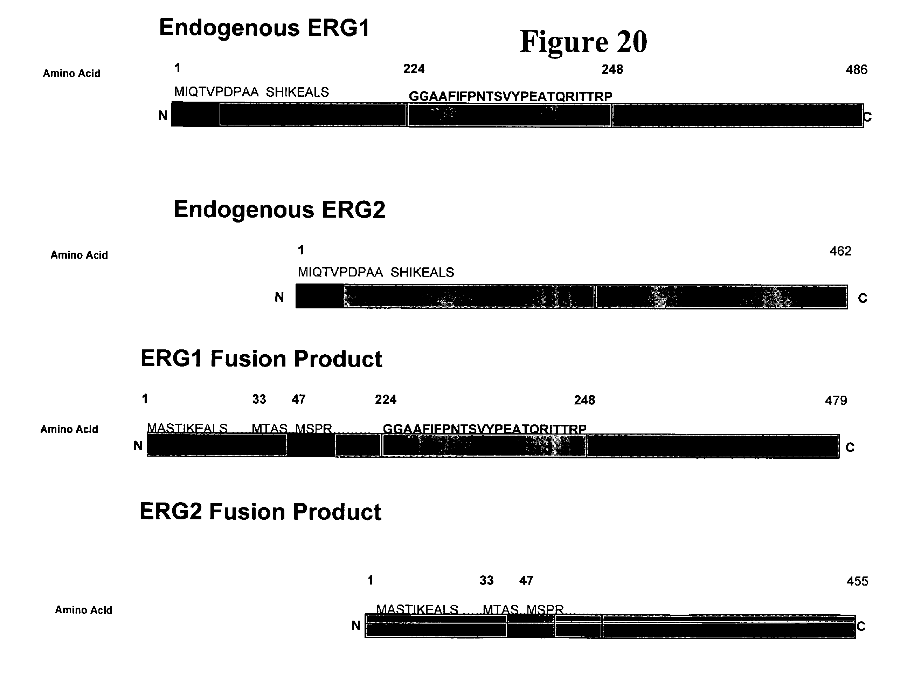

Thus, in summary, the results indicate that the third Methionine is the translational Start site for the TMPRSS2-ERG fusion product. MASTIKEALS VVSEDQSLFE CAYGTPHLAK TEMTA YGQTSK**M**SPR VPQQDWLSQP (SEQ ID NO:200). The First Methionine is the translational START Site for endogenous ERG. **M**IQTVPDPAA HI (SEQ ID NO:201). Figure 20 shows a schematic of the endogenous and fusion polypeptides.

### Example 18: FISH Analysis on Urine Samples

To isolate and prepare prostatic cells from urine, ∼ 30 ml of urine is collected following an attentive digital rectal exam. Imediately, 15 ml of PreservCyt is added, and the sample is centrifuged at 4000 rpm in a 50 ml tube for 10 min at room temperature. The supernatant is discarded, the pellet is resuspended in 15 ml of 0.75 M KCl for 15 min at room temperature, and centrifuged at 4000 rpm in a 50 ml tube for 10 min at room temperature. The supernatant is discarded, and the pellet is resuspended in 10 ml of a 3:1 ratio of methanol: glacial acetic acid. This is then centrifuged at 4000 rpm for 8 min. The supernatant is discarded, except for 200 µl, and the pellet is resuspended. The resuspended pellet is then dropped onto glass slides and allowed to air dry. Hybridization and probe preparation are as in Example 2 abvove, with the ERG 5'/3' and TMPRSS 5'/3' probe pairs.

### Example 19: Additional ETV1 Gene Fusions

### A. Materials and Methods

### Samples and Cell Lines

Prostate tissues were from the radical prostatectomy series at the University of Michigan and from the Rapid Autopsy Program1, which are both part of University of Michigan Prostate Cancer Specialized Program of Research Excellence (S.P.O.R.E.) Tissue Core. All samples were collected with informed consent of the patients and prior institutional review board approval.

The benign immortalized prostate cell line RWPE and the prostate cancer cell lines LNCaP, Du145 NCI-H660 and PC3 were obtained from the ATCC. Primary benign prostatic epithelial cells (PrEC) were obtained from Cambrex Bio Science (Walkersville, MD). The prostate cancer cell lines C4-2B, LAPC4 and MDA-PCa 2B were provided by Evan Keller (University of Michigan). The prostate cancer cell line 22-RV1 was provided by Jill McKoska (University of Michigan). VCaP was derived from a vertebral metastasis from a patient with hormone-refractory metastatic prostate cancer (Korenchuk et al., In Vivo 15, 163-8 (2001)).

For androgen stimulation experiments, LNCaP cells were grown in charcoal-stripped serum containing media for 24 hours before treatment for 24 hours with 1% ethanol or 1 nM of methyltrienolone (R1881, NEN Life Science Products, Boston, MA) dissolved in ethanol. For all samples, total RNA was isolated with Trizol (Invitrogen, Carlsbad, CA) according to the manufacturer's instructions.

### Quantiative PCR (QPCR)

Quantitative PCR (QPCR) was performed using Power SYBR Green Mastermix (Applied Biosystems, Foster City, CA) on an Applied Biosystems 7300 Real Time PCR system as described (Tomlins et al., Cancer Res 66, 3396-400 (2006); Tomlins et al., Recurrent fusion of TMPRSS2 and ETS transcription factor genes in prostate cancer. Science 310, 644-8 (2005)). All oligonucleotide primers were synthesized by Integrated DNA Technologies (Coralville, IA) and are listed in Table 15. HMBS and GAPDH5, and PSA6 primers were as described. Androgen stimulation reactions were performed in quadruplicate, all other reactions were performed in duplicate.

### RNA ligase mediated rapid amplification of cDNA ends (RLM-RACE)

RLM-RACE was performed using the GeneRacer RLM-RACE kit (Invitrogen), according to the manufacturer's instructions as described (Tomlins et al., 2005, supra; Tomlins et al., 2006, supra). To obtain the 5' end of ETV1, first-strand cDNA was amplified with Platinum Taq High Fidelity (Invitrogen) using the GeneRacer 5' primer and ETV1_exon4-5-r. Products were cloned and sequenced bidirectionally as described (Tomlins et al., 2005, supra; Tomlins et al., 2006, supra). RLM-RACEd cDNA was not used for other assays.

### Fluorescence in situ hybridization (FISH)

Interphase FISH on formalin-fixed paraffin-embedded (FFPE) tissue sections was performed as described ((Tomlins et al., 2005, supra). A minimum of 50 nuclei per assay were evaluated. Metaphase spreads of LNCaP and MDA-PCa 2B were prepared using standard cytogenetic techniques. Slides were pre-treated in 2x SSC for 2 min, 70% ethanol for 2 min and 100% ethanol for 2 min, and air dried. Slides and probes were co-denatured at 75 degrees for 2 min, and hybridized overnight at 37°C. Post-hybridization was in 0.5x SSC at 42°C for 5 min, followed by 3 washes in PBST. Fluorescent detection was performed using anti-digoxigenin conjugated to fluorescein (Roche Applied Science, Indianapolis, IN) and streptavidin conjugated to Alexa Fluor 594 (Invitrogen). Slides were counterstained and mounted in ProLong Gold Antifade Reagent with DAPI (Invitrogen). Slides were examined using a Zeiss Axio Imager Z1 fluorescence microscope (Zeiss, Thornwood, NY) and imaged with a CCD camera using ISIS software (Metasystems, Altlussheim, Germany). BACs (listed in Table 16) were obtained from the BACPAC Resource Center (Oakland, CA), and probes were prepared as described (Tomlins et al., 2005, supra). Pre-labeled chromosome 7 centromere and 7p telomeric probes were obtained from Vysis (Des Plaines, IL). The integrity and correct localization of all probes were verified by hybridization to metaphase spreads of normal peripheral lymphocytes.

### Tissue Specific Expression

To determine the tissue specific expression of 5' fusion partners and genes at 14q13-q21, the International Genomics Consortium's expO dataset, consisting of expression profiles from 630 tumors of 29 distinct types, using the Oncomine database. To interrogate the expression of HERV-K_22q11.23, which is not monitored by commercial array platforms, the Lynx Therapeutics normal tissue massively parallel signature sequencing (MPSS) dataset (GSE1747) was queried with the MPSS tag "GATCTTTGTGACCTACT" (SEQ ID NO:308), which unambiguously identifies HERV-K_22q11.23, as described (Stauffer et al., Cancer Immun 4, 2 (2004)). Descriptions of tumor types from the expO dataset and the normal tissue types from the MPSS dataset are provided in Table 17.

### Expression Profiling

Expression profiling of LNCaP, C4-2B, RWPE-ETV1 and RWPE-GUS cells were performed using the Agilent Whole Human Genome Oligo Microarray (Santa Clara, CA). Total RNA isolated using Trizol was purified using the Qiagen RNAeasy Micro kit (Valencia, CA). One µg of total RNA was converted to cRNA and labeled according to the manufacturer's protocol (Agilent). Hybridizations were performed for 16 hrs at 65°C, and arrays were scanned on an Agilent DNA microarray scanner. Images were analyzed and data extracted using Agilent Feature Extraction Software 9.1.3.1, with linear and lowess normalization performed for each array. For the LNCaP and C4-2B hybridizations, the reference for each cell line was pooled benign prostate total RNA (Clontech, Mountain View, CA). A dye flip for each cell line was also performed. Features were ranked by average expression (Log Ratio) in the two LNCaP arrays divided by the average expression in the two C4-2B arrays after correction for the dye flip. For RWPE cells, four hybridizations were performed (duplicate RWPE-ETV1/RWPE-GUS and RWPE-GUS/RWPE-ETV1 hybridizations). Over and under-expressed signatures were generated by filtering to include only features with significant differential expression (PValueLogRatio < 0.01) in all four hybridizations and 2 fold average over- or under-expression (Log Ratio) after correction for the dye flip.

### Southern Hybridization

Genomic DNA (10 µg) from LNCaP, VCaP, pooled normal human male DNA (Promega, Madison, WI) and normal placental DNA (Promega) was digested with EcoRI or PstI (New England Biologicals, Ipswich, MA) overnight. Fragments were resolved on a 0.8% agarose gel at 40 V overnight, transferred to Hybond NX nylon membrane, prehybridized, hybridized with probe and washed according to standard protocols. A series of 22 probes spanning the region of chr 7 implicated by FISH (between RP11-313C20 and RP11-703A4) were generated by PCR amplification with Platinum Taq High Fidelity on pooled normal human male genomic DNA (Table 15). Twenty-five ng of each probe was labeled with dCTP-P32 and used for hybridization.

### Inverse PCR

To identify the ETV1 breakpoint in LNCaP cells an inverse PCR strategy based on the rearrangement identified by Southern blotting was used. Primers A1, A2, A3, which are reverse complemented from the wildtype sequence and are divergent to primers B1, B2, B3, were used for inverse PCR on PstI digested and religated (in order to promoter intramolecular ligation) LNCaP genomic DNA template. Nested PCRs were performed in the following order of primer combinations: A1-B1, A2-B2 and A3-B3. Expand 20 kbplus PCR system (Roche Diagnostics GmbH, Mannheim, Germany) was used for amplifying the fusion product according to the manufactures suggestions. The enriched 3Kb band observed in nested PCRs was cloned into pCR8/GW/TOPO (Invitrogen), miniprep DNA was screened for inserts and positive clones were sequenced (University of Michigan DNA Sequencing Core, Ann Arbor, MI). Fusion was then confirmed by PCR with Platinum Taq High Fidelity using using fusion specific primers (Table 15).

### In vitro over-expression of ETV1

cDNA of ETV1, as present in the TMPRSS2:ETV1 fusion to the reported stop codon of ETV1 (269-1521, NM_004956.3), was amplified by RT-PCR from MET264 and TOPO cloned into the Gateway entry vector pCR8/GW/TOPO (Invitrogen), yielding pCR8-ETV1. To generate adenoviral and lentiviral constructs, pCR8-ETV1 and a control entry clone (pENTR-GUS) were recombined with pAD/CMV/V5 (Invitrogen) and pLenti6/CMV/V5 (Invitrogen), respectively, using LR Clonase II (Invitrogen). Control pAD/CMV/LACZ clones were obtained from Invitrogen. Adenoviruses and Lentiviruses were generated by the University of Michigan Vector Core. The benign immortalized prostate cell line RWPE was infected with lentiviruses expressing ETV1 or GUS, and stable clones were generated by selection with blasticidin (Inivtrogen). Benign PrEC were infected with adenoviruses expressing ETV1 or LACZ, as stable lines could not be generated in primary PrEC cells. Cell counts were estimated by trypsinizing cells and analysis by Coulter counter at the indicated time points in triplicate. For invasion assays, equal numbers of PREC-ETV1 and -LACZ (48 hours after infection) or stable RPWE-ETV1 and -GUS cells were seeded onto the basement membrane matrix (EC matrix, Chemicon, Temecula, CA) present in the insert of a 24 well culture plate, with fetal bovine serum added to the lower chamber as a chemoattractant. After 48 hours, non-invading cells and EC matrix were removed by a cotton swab. Invaded cells were stained with crystal violet and photographed. The inserts were treated with 10% acetic acid and absorbance was measured at 560nm.

### ETV1 knockdown

For siRNA knockdown of ETV1 in LNCaP cells, the individual siRNAs composing the Dharmacon SMARTpool against ETV1 (MU-003801-01, Chicago, IL) were tested for ETV1 knockdown by qPCR, and the most effective single siRNA (D-003801-05) was used for further experiments. siCONTROL Non-Targeting siRNA #1 (D-001210-01) or siRNA against ETV1 was transfected into LNCaP cells using Oligofectamine (Invitrogen). After 24 hours a second identical transfection was carried out and cells were harvested 24 hours later for RNA isolation and invasion assays as described below. For shRNA knockdown of ETV1 in LNCaP cells, the shRNAmir construct against ETV1 from the pMS2 retroviral vector (V2HS_61929, Open Biosystems, Huntsville, AL) was cloned into an empty pGIPZ lentivral vector (RHS4349, Open Biosystems) according to the manufacturer's protocol. pGIPZ lentiviruses with shRNAmirs against ETV1 or a non silencing control (RHS4346) were generated by the University of Michigan Vector Core. LNCaP cells were infected with lentiviruses, and 48 hours later cells were used for invasion assays as described below. Representative results from 6 independent experiments are shown.

### Invasion assays

Equal numbers of the indicated cells were seeded onto the basement membrane matrix (EC matrix, Chemicon, Temecula, CA) present in the insert of a 24 well culture plate, with fetal bovine serum added to the lower chamber as a chemoattractant. After 48 hours, non-invading cells and EC matrix were removed by a cotton swab. Invaded cells were stained with crystal violet and photographed. The inserts were treated with 10% acetic acid and absorbance was measured at 560nm.

### FACS cell cycle analysis

RWPE-ETV1 and RWPE-GUS cells were assessed by FACS for cell cycle characterization. Cells were washed with 2x PBS and approximately 2 x 10⁶ cells were resuspended in PBS before fixation in 70% ethanol. Pelleted cells were washed and treated with RNase (100 µg/ml final concentration) and propidium iodide (10 µg/ml final concentration) at 37°C for 30 min. Stained cells were analyzed on a LSR II flow cytometer (BD Biosciences, San Jose, CA) running FACSDivia, and cell cycle phases were calculated using ModFit LT (Verity Software House, Topsham, ME).

### Soft agar assay

A 0.6% (wt/vol) bottom layer of low melting point agarose in normal medium was prepared in six-well culture plates. On top, a layer of 0.3% agarose containing 1 x 10⁴ RWPE-GUS, RWPE-ETV1 or DU145 (positive control) cells was placed. After 12 days, foci were stained with crystal violet and counted.

### Immunoblot Analyses

Cells were homogenized in NP40 lysis buffer containing 50 mM Tris-HCl (pH 7.4), 1 % NP40 (Sigma, St. Louis, MO), and complete proteinase inhibitor mixture (Roche). Fifteen µg of protein extracts were mixed with SDS sample buffer and electrophoresed onto a 10% SDS-polyacrylamide gel under reducing conditions. The separated proteins were transferred onto nitrocellulose membranes (Amersham Pharmacia Biotech, Piscataway, NJ). The membrane was incubated for 1 h in blocking buffer [Tris-buffered saline with 0.1% Tween (TBS-T) and 5% nonfat dry milk]. Primary antibody was applied at the indicated dilution in blocking buffer overnight at 4°C. After washing three times with TBS-T buffer, the membrane was incubated with horseradish peroxidase-linked donkey anti-mouse IgG antibody (Amersham Pharmacia Biotech) at a 1:5,000 dilution for 1 hour at room temperature. The signals were visualized with the enhanced chemiluminescence detection system (Amersham Pharmacia Biotech) and autoradiography.

Mouse monoclonal anti-MMP-3 (IM36L, Calbiochem, San Diego) was applied at 1:500 dilution and mouse monoclonal anti-uPA (IM13L, Calbiochem) was applied at 1:500 dilution, and mouse anti-GAPDH antibody (Abcam, Cambridge, MA) was applied at 1:30,000 dilution for loading control.

### Transgenic ETV1 mice

For in vivo over-expression of ETV1, a C terminal 3XFLAG-epitope tagged construct was generated by PCR using pCR8-ETV1 as the template with the reverse primer encoding a triple FLAG tag before the stop codon. The product was TOPO cloned into pCR8. To generate a prostate specific ETV1 transgenic construct, 3xFLAG-ETV1 was inserted into pBSII (Stratagene, La Jolla, CA) downstream of a modified small composite probasin promoter (ARR2PB) and upstream of a bovine growth hormone polyA site (PA-BGH). The ARR2PB sequence contains the original probasin sequence PB (-426/+28) plus two additional androgen response elements. The construct was sequenced and tested for promoter inducibility by androgen in LNCaP cells upon transient transfection before microinjection into FVB mouse eggs. The ARR2PB-ETV1 plasmid was linearized with PvuI/KpnI//SacII and microinjected into fertilized FVB mouse eggs and surgically transplanted into a pseudo-pregnant female by the University of Michigan Transgenic Animal Model Core. Transgenic founders were screened by PCR using genomic DNA isolated from tail snips. Transgenic ARR2PB-ETV1 founders were crossed with FVB mice and the transgene-positive male mice offspring were sacrificed at various time points. Prostates from transgenic mice were dissected using a Nikon dissection scope, fixed in 10% buffered formalin and embedded in paraffin. Five µm sections were stained with hematoxylin and eosin, and evaluated by three pathologists according to the criteria provided in The Consensus Report from the Bar Harbor Meeting of the Mouse Models of Human Cancer Consortium Prostate Pathology Committee (Nam et al., Cancer Biol Ther 6 (2007)).

For immunohistochemical detection of ETV1-FLAG, the basal cell markers p63 and cytokeratin 5 (CK5), and smooth muscle actin, deparaffinized slides were subjected to microwave-citrate antigen retrieval and incubated with rabbit anti-FLAG polyclonal antibody ( 1:50 dilution, overnight incubation, Cell Signaling Technology, #2368), mouse monoclonal anti-p63 antibody (1:100 dilution, 45' incubation, LabVision, MS1081P1), mouse monoclonal anti-smooth muscle actin antibody( 1:50 dilution, 30' incubation, DakoAb M0851) and rabbit polyclonal anti-CK5 antibody (1:500 dilution, 30' incubation, AbCam, ab24647), respectively. Visualization of p63 and SMA was performed using a standard biotin-avidin complex technique using M.O.M Immunodetection kit (PK2200, Vector Laboratories). FLAG and CK5 were detected using Envision+System-HRP (DAB) kit (K4011, DakoCytomation).

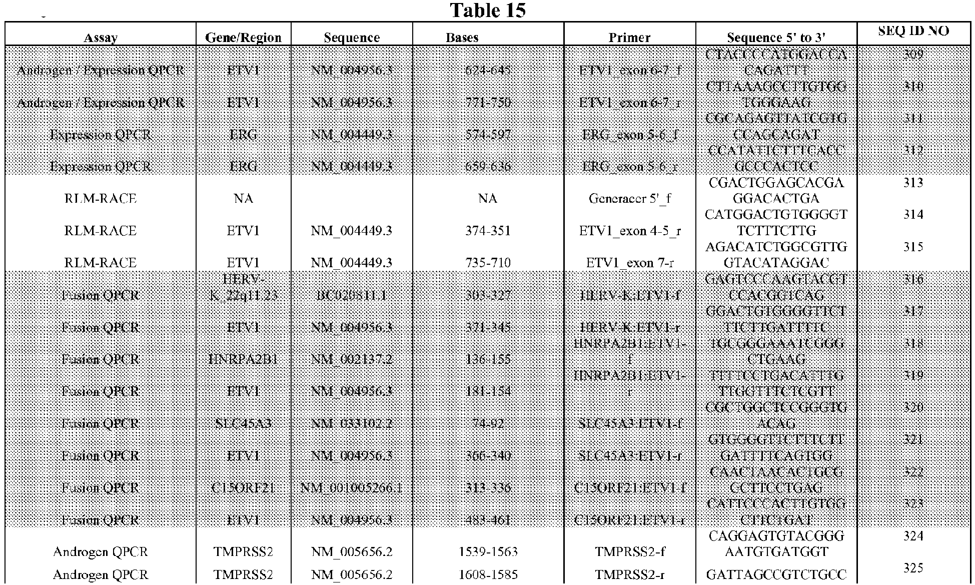

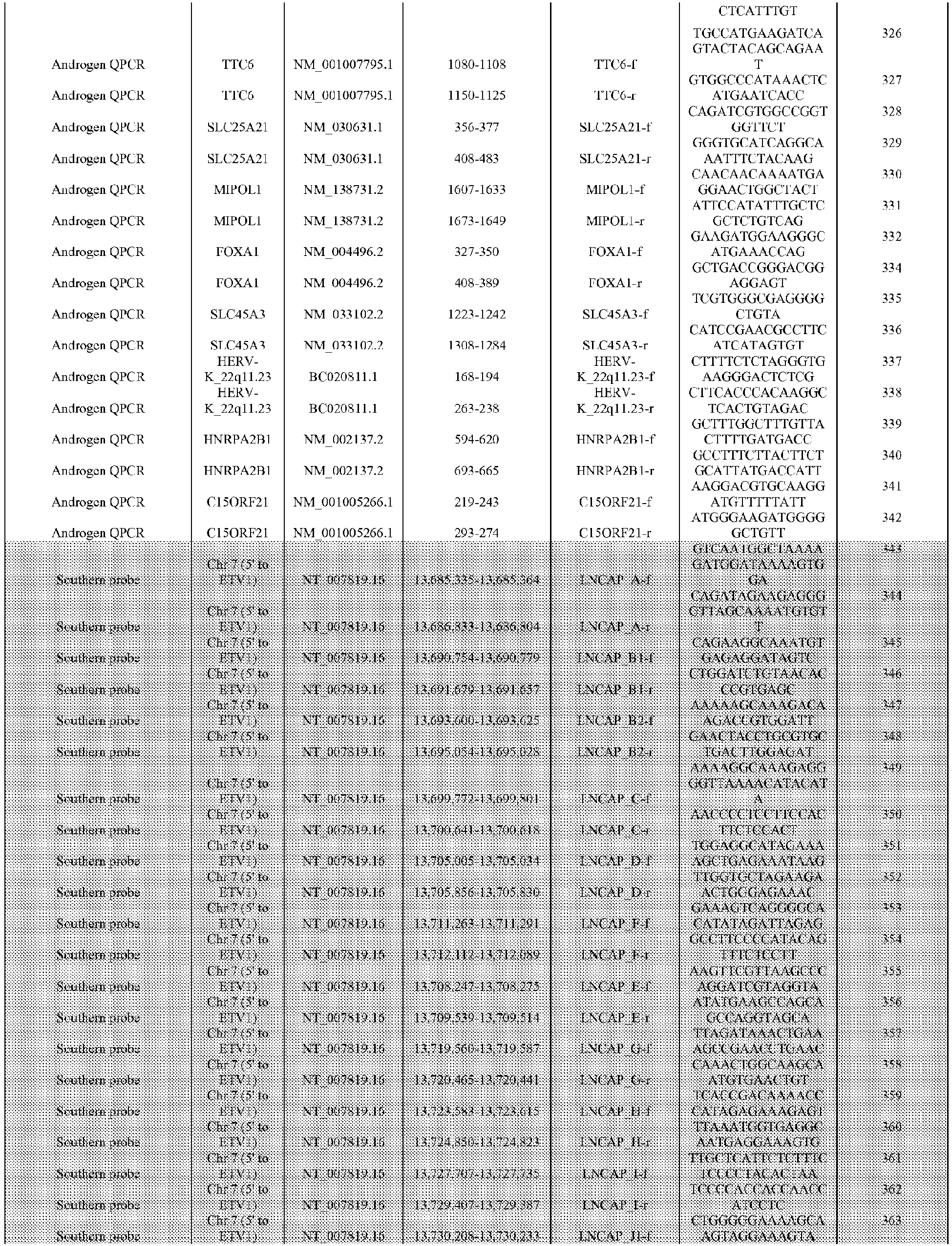

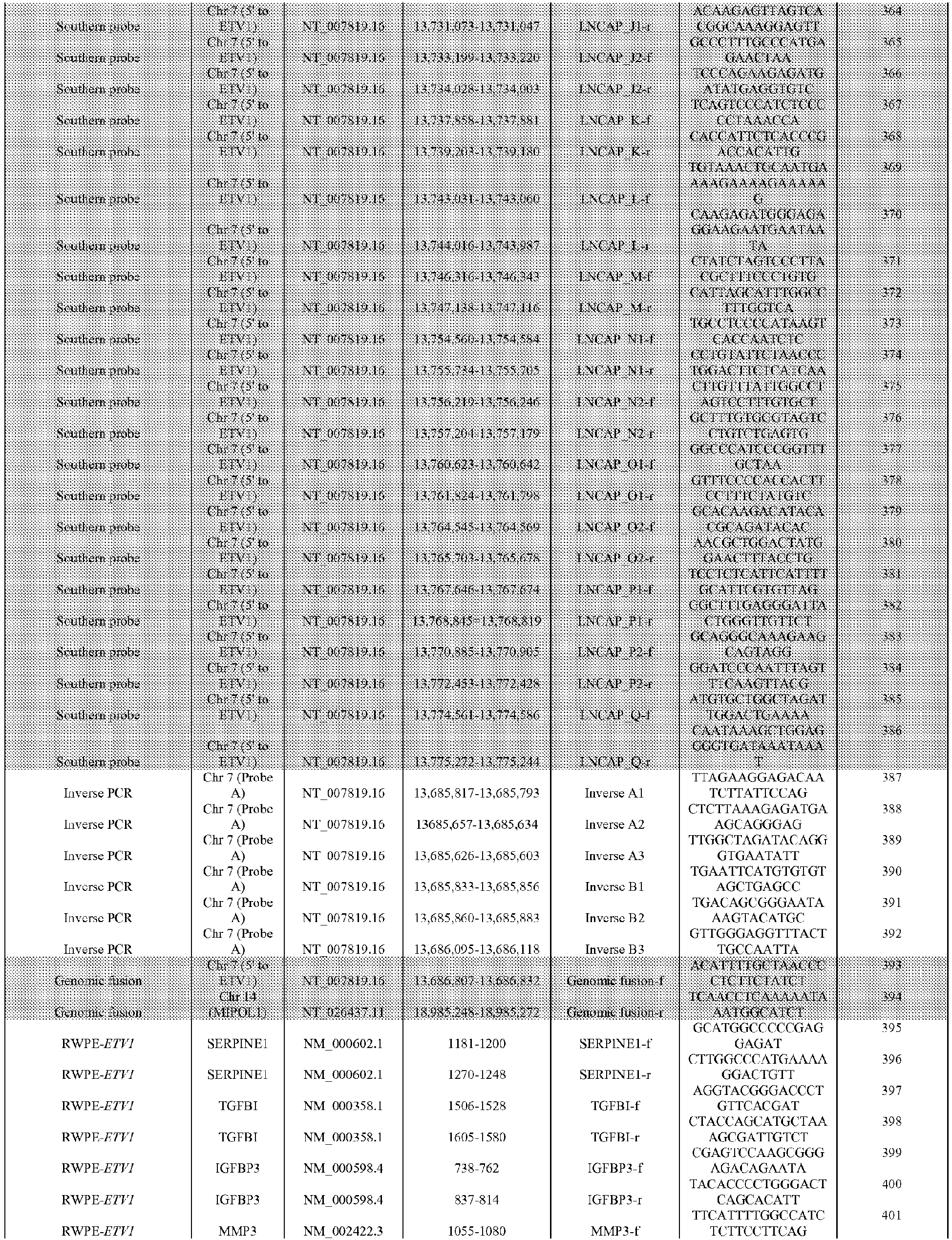

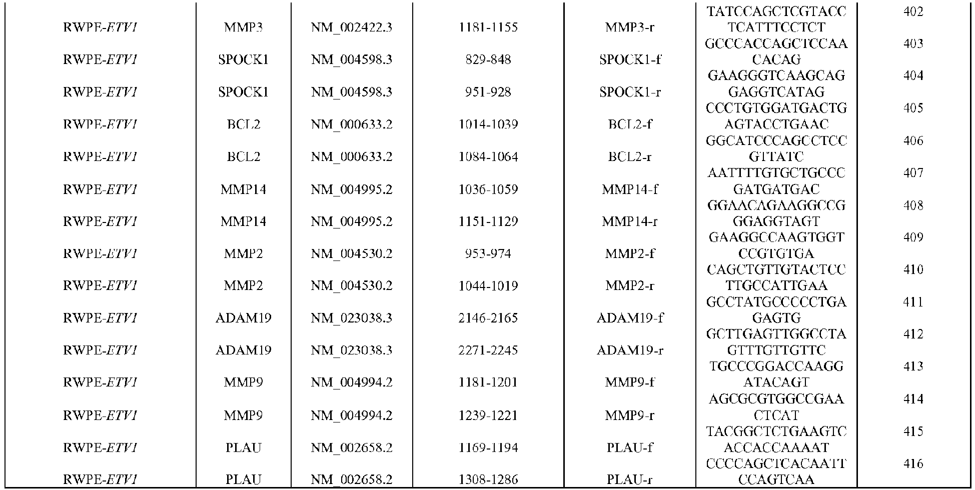

**Table 16**

| **Probe #** | **Gene/Region** | **Localization** | **Probe** |
|---|---|---|---|
| 1 | ETV1 | 3' | RP11-124L22 |
| 2 | ETV1 | 5' | RP11-703A4 |
| 3 | Chr 14q13.3-14q21.1 | C | RP11-945C4 |
| 4 | Chr 14q13.3-14q21.1 | T | RP11-107E23 |
| 5 | HNRPA2B13' | 3' | RP11-11F13 |
| 6 | HNRPA2B15' | 5' | RP11-379M24 |
| 7 | HERV-K_22q11.23 5' | 5' | RP11-61N10 |
| 8 | HERV-K_22q11.23 3' | 3' | RP11-71G19 |
| 9 | SLC45A3 | 3' | RP11-249h15 |
| 10 | SLC45A3 | 5' | RP11-131E5 |
| 11 | C15ORF21 | 5' | RP11-474E1 |
| 12 | C15ORF21 | 3' | RP11-626F7 |
| 13 | 14q32 | 3' | RP11-483K13 |
| 14 | ETV1 | 5' | RP11-313C20 |
| 15 | Chr 7 centromere | C | CEP 7 |
| 16 | Chr 7p telomere | T | TelVysion 7p |
| 17 | TMPRSS2 | 5' | RP11-35C4 |

**Table 17**

| **International Genomics Consortium's expO dataset (Bittner_Multi-cancer at oncomine)** | | | | **Lynx Therapeutics Human Tissue MPSS Dataset (GSE1747)** | |
|---|---|---|---|---|---|
| **#** | **Cancer Type** | **n** | | **#** | **Normal Tissue Type** |
| 1 | Bladder Papillary Carcinoma | 4 | | 1 | adrenal gland |
| 2 | Bladder Transitional cell carcinoma | 10 | | 2 | Bladder |
| 3 | Breast Ductal Carcinoma | 95 | | 3 | bone marrow |
| 4 | Cervix Squamous Cell Carcinoma | 10 | | 4 | brain - amygdala |
| 5 | Colon Adenocarcinoma | 104 | | 5 | brain - caudate nucleus |
| 6 | Metastatic Colon Carcinoma | 16 | | 6 | brain - cerebellum |
| 7 | Mucinous Colon Carcinoma | 12 | | 7 | brain - corpus callosum |
| 8 | Endometrial Adenocarcinoma | 5 | | 8 | fetal brain |
| 9 | Endometrial Endometrioid Carcinoma | 45 | | 9 | brain - hypothalmus |
| 10 | Endometrial Mixed Mullerian tumor | 6 | | 10 | brain - thalamus |
| 11 | Metastatic Endometrial Carcinoma | 7 | | 11 | Monocytes |
| 12 | Soft Tissue Sarcoma | 13 | | 12 | peripheral blood lymphocytes |
| 13 | Clear cell renal carcinoma | 78 | | 13 | Heart |
| 14 | Papillary Renal Cell Carcinoma | 6 | | 14 | Kidney |
| 15 | Lung Adenocarcinoma | 19 | | 15 | Lung |
| 16 | Bronchioloalveolar carcinoma | 7 | | 16 | mammary gland |
| 17 | Squamous Cell Lung Carcinoma | 17 | | 17 | Pancreas |
| 18 | Ovarian Adenocarcinoma | 20 | | 18 | pituitary gland |
| 19 | Ovarian Endometrioid Carcinoma | 13 | | 19 | Placenta |
| 20 | Metastatic Ovarian Carcinoma | 36 | | 20 | Retina |
| 21 | Ovarian Mucinous Carcinoma | 4 | | 21 | salivary gland |
| 22 | Ovarian Papillary Carcinoma | 38 | | 22 | small intestine |
| 23 | Pancreatic Ductal Carcinoma | 3 | | 23 | spinal cord |
| 24 | Rectosigmoid Adenocarcinoma | 15 | | 24 | Spleen |
| 25 | Rectal Adenocarcinoma | 13 | | 25 | Stomach |
| 26 | Renal Pelvis Transitional cell carcinoma | 4 | | 26 | Testis |
| 27 | Metastatic Melanoma | 5 | | 27 | Thymus |
| 28 | Papillary Thyroid Carcinoma | 10 | | 28 | Thyroid |
| | Prostate Adenocarcinoma | 15 | | 29 | Trachea |
| | | | | 30 | colon transversum |
| | | | | 31 | Uterus |
| | | | | | Prostate |

### B. Results

### Identification of novel 5' ETS fusion partners in prostate cancer

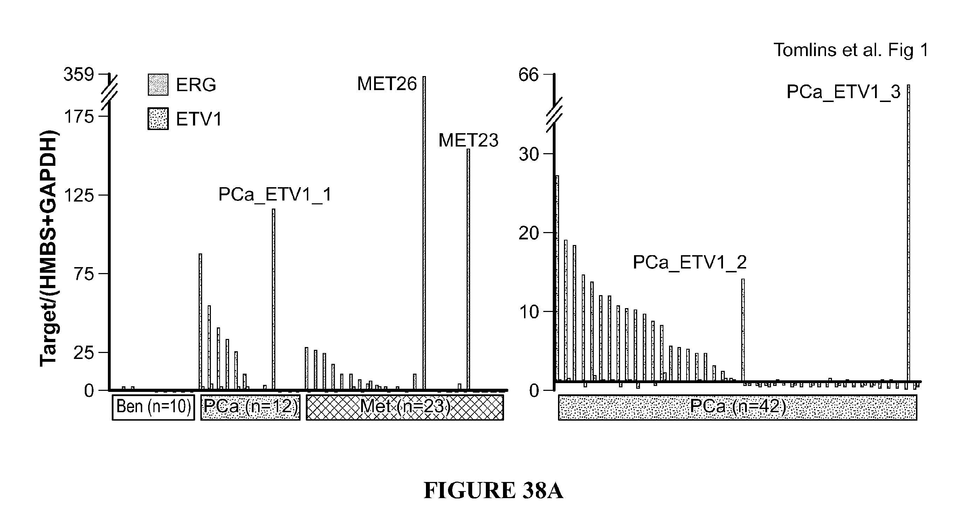

By qPCR, two cohorts of prostate tissue samples were screened for ERG and ETV1 expression to identify cases with outlier-expression of ETV1. As shown in Figure 38a, across the two cohorts, 26 and 3 of 54 localized prostate cancer samples showed ERG (48%) and ETV1 (5.5%) outlier-expression, respectively. Additionally, two hormone-refractory metastatic prostate cancer samples, MET26 and MET23, showed ETV1 outlier-expression. By qPCR, 25 of the 26 localized samples (96%) with outlier-expression of ERG expressed TMPRSS2:ERG fusion transcripts. Besides MET26, no samples, including the 4 ETV1 outliers (PCa_ETV1_1-3 and MET23), expressed TMPRSS2:ETV1 fusion transcripts.

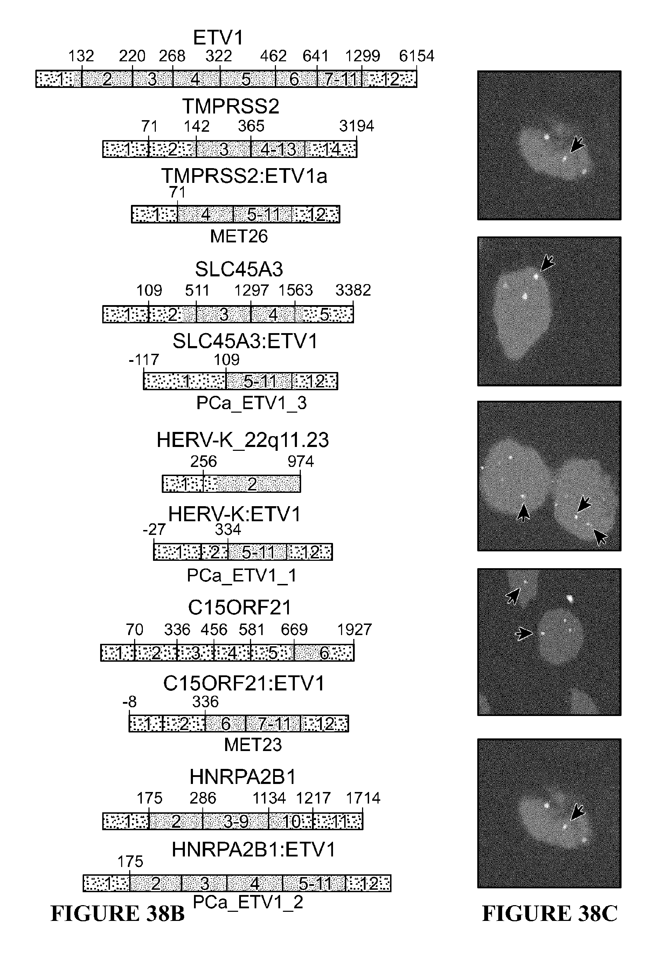

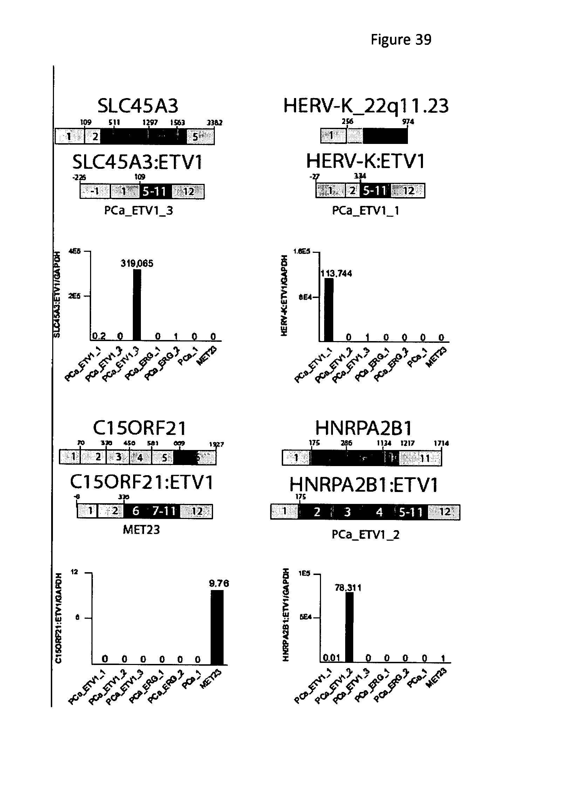

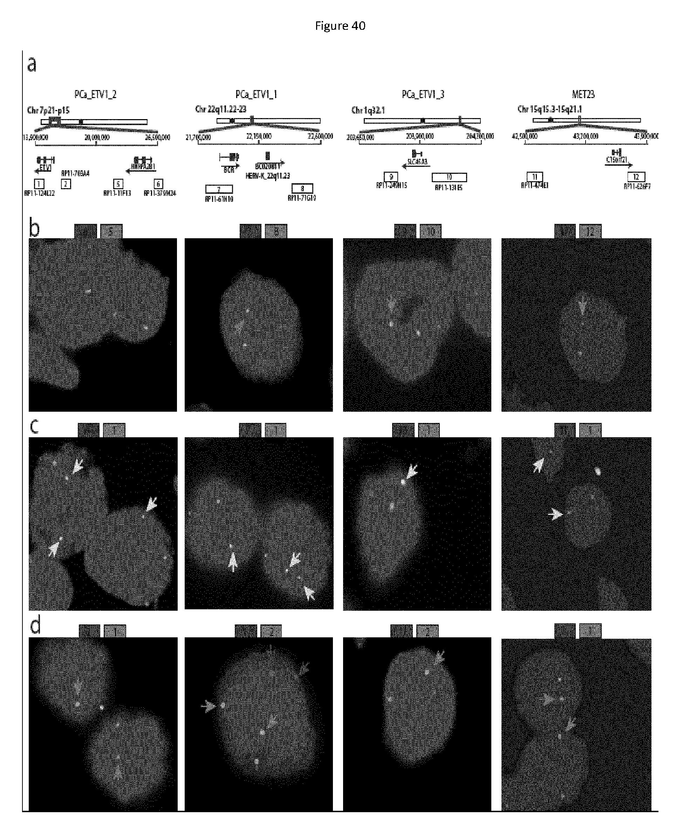

To characterize the ETV1 transcript structure in these cases, 5' RNA ligase mediated rapid amplification of cDNA ends (RLM-RACE) was performed. Rather than 5' exons from TMPRSS2 as in MET26, all four samples contained unique 5' sequences (Fig38b). In PCa_ETV1_1, exons 1-4 of ETV1 were replaced with two exons from 22q11.23 with homology to the 5' long terminal repeat (LTR) and gag sequence of human endogenous retrovirus family K (hereafter referred to as HERV-K_22q11.23). In PCa_ETV1_2, exon 1 of ETV1 was replaced with exon 1 from HNRPA2B1 (7p15), while in PCa_ETV1_3, exons 1-4 of ETV1 were replaced with additional upstream sequence and exon 1 of SLC45A3 (1q32). In MET23, exons 1-5 of ETV1 were replaced with exons 1 and 2 from C15ORF21 (15q21) (Fig 1b). The exclusive expression of these fusion transcripts was confirmed by qPCR and fusion at the genomic level in these cases by FISH (Fig 38c, 39 & 40). In PCa_ETV1_2, FISH demonstrated deletion of the probes 3' to HNRPA2B1 and 5' to ETV1, with corresponding fusion of the probes 5' to HNRPA2B1 and 3' to ETV1, consistent with an intrachromosomal deletion between HNRP2A2B1 and ETV1, which are oriented head to tail ∼ 13 MB apart on 7p (Fig 38c & 40). Sequences of gene fusions are shown in Figure 51.

### Distinct functional classes of 5'fusion partners

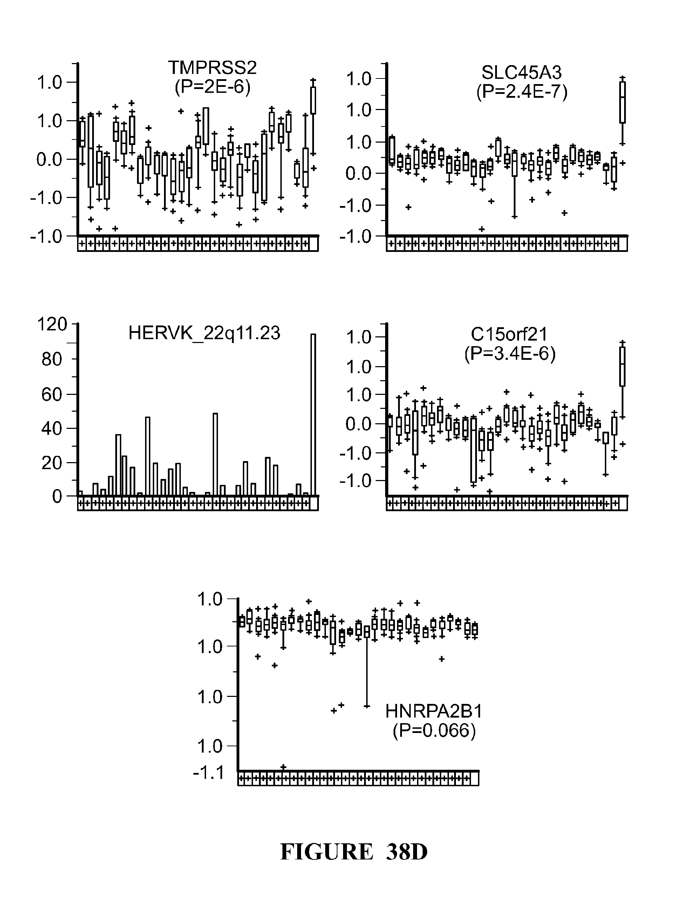

HERV-K_22q11.23:ETV1, SLC45A3:ETV1 and C150RF21:ETV1 fusions contain no predicted translated sequences from the 5' partner, and the HNRPA2B1 sequence in the HNRPA2B1:ETV1 fusion would contribute only two residues to a fusion protein. Thus, as the promoter elements of the 5' partners likely drive the aberrant ETV1 expression in these cases, the tissue specificity and androgen regulation of these genes was characterized. To examine the tissue specificity of SLC45A3, C15ORF21 and HNRPA2B1, the International Genomics Consortium's expO dataset, consisting of expression profiles from 630 tumors of 29 distinct types, was searched using the Oncomine database (Rhodes et al., Neoplasia 9, 166-80 (2007)). Similar to TMPRSS2, SLC45A3 showed marked over-expression in prostate cancer (median = 2.45 standard deviations above the median per array) compared to all other tumor types (median = 0.33, P = 2.4E-7). C15ORF21 showed similar over-expression in prostate cancer (median = 2.06 vs. -0.12, P = 3.4E-6). By contrast, HNRPA2B1 showed high expression in prostate and other tumor types (median = 2.36 vs. 2.41, P > 0.05) (Fig 38d). Although HERV-K_22q11.23 is not monitored by the DNA microarrays used in the expO dataset, it is measured unambiguously by massively parallel signature sequencing (MPSS), as described by Stauffer et al (Cancer Immun 4, 2 (2004)). Thus, the expression of HERV-K_22q11.23 was searched in the Lynx Therapeutics MPSS dataset containing profiles from 32 normal tissue types (Jongeneel et al., Genome Res 15, 1007-14 (2005)), which showed that HERV-K_22q11.23 was expressed at the highest levels in normal prostate tissue (94 transcripts per million) compared to the 31 other normal tissues (median = 9 transcripts per million) (Fig 38d).

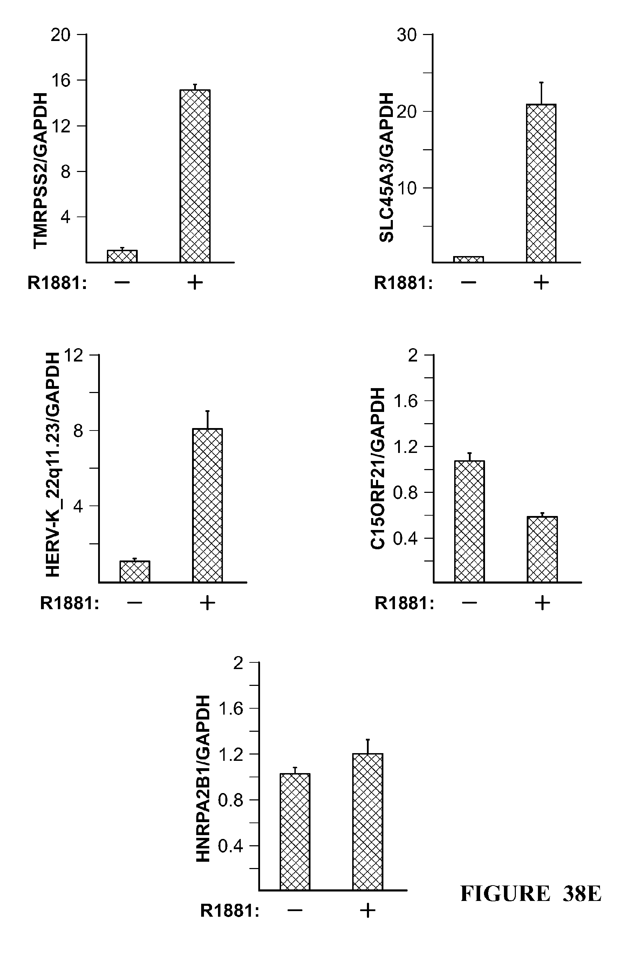

By qPCR, the endogenous expression of SLC45A3 (21.6 fold, P = 6.5E-4) and HERV-K_22q11.23 (7.8 fold, P = 2.4E-4) in the LNCaP prostate cancer cell line are strongly increased by the synthetic androgen R1881, similar to TMPRSS2 (14.8 fold, P = 9.95E-7). Conversely, the expression of C150RF21 is significantly decreased (1.9 fold, P = 0.0012) by R1881 stimulation. Lastly, the expression of HNRPA2B1 is not significantly changed by androgen stimulation (1.17 fold, P = 0.29)) (Fig 38e).

### ETV1 induces invasion in benign prostate cells

As the 5' partners do not contribute coding sequence to the ETV1 transcript, the common result of the different classes of ETV1 rearrangements in clinical samples and prostate cancer cell lines is aberrant over-expression of truncated ETV1. Thus, this event was recapitulated in vitro and in vivo to determine the role of aberrant ETS family member expression in prostate cancer. Adenoviral and lentiviral constructs were designed to over-express ETV1 as expressed in the index TMPRSS2:ETV1 fusion positive case, MET26 (beginning at exon 4 through the reported stop codon of ETV1) (Fig 41a). The benign immortalized prostate epithelial cell line RWPE was infected with lentivirus expressing ETV1, selected for stable RWPE-ETV1 cells, and transiently over-expressed ETV1 in the primary benign prostate epithelial cell line PrEC by infection with adenovirus expressing ETV1. In both RWPE and PrEC cells, over-expression of ETV1 had no detectable effect on proliferation (Fig 44a-b), and cell cycle analysis showed no difference in the percentage of RWPE-ETV1 and RWPE-GUS cells in S phase (Fig 44c). Additionally, soft agar transformation assays showed that ETV1 over-expression was not sufficient to transform RWPE cells (Fig 44d).

ETV1 over-expression markedly increased invasion in both RWPE (3.4 fold, P = 0.0005) and PrEC (6.3 fold, P = 0.0006) (Fig 41b-c). Additionally, ETV1 knockdown in LNCaP using either siRNA or shRNA designed against different sequences significantly inhibited invasion (Fig 41d-e & Fig 45), consistent with previous work (Cai et al., Mol Endocrinol (2007)). These results demonstrate that ETV1 over-expression induces invasion, an important oncogenic phenotype. To investigate the transcriptional program regulated by stable ETV1 over-expression, RWPE-ETV1 cells were profiled and the expression signatures were analyzed against a compendium of biologically related concepts called the Molecular Concepts Map (MCM). The MCM is a resource to look for associations between more than 20,000 biologically related gene sets by disproportionate overlap (Tomlins et al., Nat Genet 39, 41-51 (2007)). As shown in Figure 41f, MCM analysis identified a network of molecular concepts related to cell invasion that were enriched in our ETV1 over-expressed signature, consistent with the phenotypic effects described above. By qPCR and immunoblotting, the over-expression of multiple genes previously implicated in invasion was confirmed (Laufs et al., Cell Cycle 5, 1760-71 (2006); Fingleton, Front Biosci 11, 479-91 (2006)), including matrix metalloproteinases and members of the urokinase plasminogen activator pathway, in RWPE-ETV1 cells (Fig 41 g & 47). These results are consistent with a recent study demonstrating that ETV1 mediates invasion in LNCaP cells through matrix metalloproteinases (Cai et al., supra).

### ETV1 expression in the mouse prostate induces mPIN

The effects of ETV1 over-expression in vivo was next investigated using transgenic mice expressing a FLAG-tagged, truncated version of ETV1 under the control of the modified probasin promoter (ARR2Pb-ETV1) (Fig 41a), which drives strong transgene expression exclusively in the prostate under androgen regulation (Ellwood-Yen et al., Cancer Cell 4, 223-38 (2003)). This transgene is functionally analogous to the androgen-induced gene fusions of ETV1 identified in human prostate cancer (i.e., TMPRSS2:ETV1, SLC45A3:ETV1, and HERV-K_22q11.23:ETV1). Multiple ARR2Pb-ETV1 founders were obtained and expanded for phenotypic analysis. By 12-14 weeks of age, 6 of 8 (75%) ARR2Pb-ETV1 transgenic mice developed mouse prostatic intraepithelial neoplasia (mPIN) (Table 18 and Fig 42). Consistent with the definition of mPIN, we observed focal proliferative lesions contained within normal glands in the prostates of ARR2Pb-ETV1 mice (Fig 42a-f), exhibiting nuclear atypia, including stratification, hyperchromasia and macronucleoli. mPIN was observed in all three prostatic lobes (anterior, ventral and dorsolateral) of ARR2Pb-ETV1 mice, and most commonly in the ventral lobe (7/11, 63.6%) (Table 18). By immunohistochemistry, strong ETV1-FLAG expression was observed exclusively in mPIN foci, and not benign glands, in ARR2Pb-ETV1 mice (Fig 48), and qPCR confirmed that transgene expression was limited to the prostate.

All lesions were confirmed to be in situ by the presence of an intact fibromuscular layer, as demonstrated by contiguous smooth muscle actin staining (Fig 42g-h). However, immunohistochemistry with the basal cell markers cytokeratin 5 and p63 demonstrated loss of the circumferential basal epithelial layer in ARR2Pb-ETV1 mPIN compared to benign glands (Fig 42i-1), indicating disruption of the basal cell layer. These results demonstrate that ETV1 induces a neoplastic phenotype in the mouse prostate and provides support of an oncogenic role for ETS gene fusions in human prostate cancer.

**Table 18**

| ARR2Pb-ETV1 Mouse | Founder | Age | AP | VP | DLP | Diagnosis | Liver |
|---|---|---|---|---|---|---|---|
| 1 | 287 | 12 wk | Normal | Hyperplasia | Hyperplasia | Hyperplasia | Normal |
| 2 | 666 | 12 wk | Normal | Hyperplasia | Normal | Hyperplasia | Normal |
| 3 | 666 | 12 wk | mPIN | No tissue | mPIN | mPIN | NA |
| 4 | 666 | 12 wk | Normal | mPIN | Adipose tissue | mPIN | Normal |
| 5 | 287 | 12-14 wk | Normal | mPIN | Normal | mPIN | Normal |
| 6 | 287 | 12-14 wk | Normal | Normal | mPIN | mPIN | Normal |
| 7 | 339 | 12-14 wk | Normal | mPIN | Hyperplasia | mPIN | Normal |
| 8 | 339 | 12-14 wk | Normal | mPIN | Hyperplasia | mPIN | Normal |
| 9 | 287 | 19 wk | Normal | Hyperplasia | Hyperplasia | Hyperplasia | Normal |
| 10 | 287 | 20 wk | Normal | mPIN | Hyperplasia | mPIN | NA |
| 11 | 287 | 33 wk | Hyperplasia | mPIN | Hyperplasia | mPIN | Normal |
| 12 | 287 | 43 wk | Normal | mPIN | mPIN | mPIN | Normal |
| | | Total | 1/12 (8.3%) | 7/11 (63.6%) | 3/12 (25%) | 9/12 (75%) | |

### Example 20: Further Gene Fusions

This Example describes the identification of TMPRSS2:ETV5 and SLC45A3:ETV5 gene fusions. For detection of ETV5 outlier expression by SYBR Green QPCR, the following primer pair was used:
ETV5_Exon13-f: 5'-CCGAAGGCTTTGCTTACTAAGTTTCTGA-3' (SEQ ID NO:417)
ETV5_Exon13-r: 5'-CACTGCCCTTGTTTGCCTGAATG-3'(SEQ ID NO:418)
For identification of the TMPRSS2:ETV5 fusion from PCa_ETV5_1, the following primer for RLM-RACE was used:
ETV5_Exon6-r: 5'-AGCTCCCGTTTGATCTTGGTTGG-3'(SEQ ID NO:419)
For identification of the SLC45A3:ETV5 fusion from PCa_ETV5_2, the following primer was used:
ETV5_Exon11-r: 5'-CATGGTAGGCTCCTGTTTGACTTTG-3'(SEQ ID NO:420)
Figure 52 shows the sequences of the above noted fusions. For TMPRSS2:ETV5, 3 splice variants were identified. The sequences for all have been given. Figure 53 shows the identification of two prostate cancer (PCa) cases as showing ETV5 outlier expression by QPCR. Panel B shows the structure of the fusion transcripts for both cases, as determined by RACE.

Although the invention has been described in connection with specific embodiments, it should be understood that the invention as claimed should not be unduly limited to such specific embodiments. Indeed, various modifications and variations of the described compositions and methods of the invention will be apparent to those of ordinary skill in the art.

## Claims

1. A method for identifying prostate cancer in a patient, comprising:
detecting a presence or an absence in a patient sample of a SLC45A3 gene fusion having a 5' portion from a transcriptional regulatory region of an androgen regulated gene defined as SLC45A3 and having a 3' portion from an ETS family member gene;
wherein the presence in the sample of the gene fusion is indicative of prostate cancer in the patient.

2. The method of claim 1, wherein the sample is of prostate cells, prostatic secretions or a fraction thereof.

3. The method of claim 1 or claim 2, wherein the transcriptional regulatory region of the androgen regulated gene comprises a promoter region of the androgen regulated gene.

4. The method of claim 1 or claim 2, wherein step (b) comprises detecting chromosomal rearrangements of genomic DNA having a 5' portion from the transcriptional regulatory region of the androgen regulated gene and having a 3' portion from the ETS family member gene.

5. The method of claim 1 or claim 2, wherein step (b) comprises detecting chimeric mRNA transcripts having a 5' portion from the transcriptional regulatory region of the androgen regulated gene and having a 3' portion from the ETS family member gene.

6. The method of claim 1 or claim 2, wherein detection is carried out utilizing a sequencing technique, a hybridization technique, or an amplification technique.

7. The method of claim 1 or claim 2, wherein the detecting step comprises:
(a) use of an oligonucleotide probe comprising a sequence that hybridizes to a junction of a chimeric genomic DNA or a chimeric mRNA in which a 5' portion of the chimeric genomic DNA or the chimeric mRNA is from a transcriptional regulatory region of a SLC45A3 gene and a 3' portion of the chimeric genomic DNA or the chimeric mRNA is from an ETS family member gene;
(b) use of a first labeled oligonucleotide probe comprising a sequence that hybridizes to a 5' portion of a chimeric genomic DNA or a chimeric mRNA from a transcriptional regulatory region of a SLC45A3 gene and a second labeled oligonucleotide probe comprising a sequence that hybridizes to a 3' portion of the chimeric genomic DNA or the chimeric mRNA from an ETS family member gene; or
(c) use of a first amplification oligonucleotide comprising a sequence that hybridizes to a 5' portion of a chimeric genomic DNA or a chimeric mRNA from a transcriptional regulatory region of a SLC45A3 gene and a second amplification oligonucleotide comprising a sequence that hybridizes to a 3' portion of the chimeric genomic DNA or the chimeric mRNA from an ETS family member gene.

8. A method for identifying prostate cancer in a biological sample from a patient, which sample is tissue, blood, plasma, serum, urine, urine supernatant, urine cell pellet, semen, prostatic secretions, or prostate cells; the method comprising:
detecting a presence or an absence of a gene fusion in the biological sample, wherein the gene fusion has a 5' portion comprising a portion of a transcriptional regulatory region of an androgen regulated gene defined as SLC45A3 and a 3' portion comprising a portion of an ETS family member gene,
wherein detecting the presence of the gene fusion in the biological sample indicates the presence of prostate cancer in the sample.

9. The method of claim 8, wherein the transcriptional regulatory region of the androgen regulated gene comprises a promoter region of the androgen regulated gene.

10. The method of claim 8, wherein step (b) comprises detecting chimeric mRNA transcripts having a 5' portion from the transcriptional regulatory region of the androgen regulated gene and a 3' portion from the ETS family member gene.

11. A composition comprising:
(a) an oligonucleotide probe comprising a sequence that hybridizes to a junction of a chimeric genomic DNA or a chimeric mRNA in which a 5' portion of the chimeric genomic DNA or the chimeric mRNA is from a transcriptional regulatory region of a SLC45A3 gene and a 3' portion of the chimeric genomic DNA or the chimeric mRNA is from an ETS family member gene; or
(b) a first labeled oligonucleotide probe comprising a sequence that hybridizes to a 5' portion of a chimeric genomic DNA or a chimeric mRNA from a transcriptional regulatory region of a SLC45A3 gene and a second labeled oligonucleotide probe comprising a sequence that hybridizes to a 3' portion of the chimeric genomic DNA or the chimeric mRNA from an ETS family member gene; or
(c) a first amplification oligonucleotide comprising a sequence that hybridizes to a 5' portion of a chimeric genomic DNA or a chimeric mRNA from a transcriptional regulatory region of a SLC45A3 gene and a second amplification oligonucleotide comprising a sequence that hybridizes to a 3' portion of the chimeric genomic DNA or the chimeric mRNA from an ETS family member gene.

## Patentansprüche

1. Verfahren zur Identifizierung von Prostatakrebs bei einem Patienten, das Folgendes umfasst:
das Nachweisen der Gegenwart oder Abwesenheit einer SLC45A3-Genfusion, die einen 5'-Abschnitt einer Transkriptionsregulierungsregion eines als SLC45A3 definierten androgenregulierten Gens und einen 3'-Abschnitt eines der ETS-Familie angehörigen Gens aufweist, in einer Probe des Patienten;
worin die Gegenwart der Genfusion in der Probe ein Indikator für Prostatakrebs in dem Patienten ist.

2. Verfahren nach Anspruch 1, worin die Probe von Prostatazellen, Prostatasekretionen oder eines Anteils davon stammt.

3. Verfahren nach Anspruch 1 oder Anspruch 2, worin die Transkriptionsregulierungsregion des androgenregulierten Gens eine Promotorregion des androgenregulierten Gens umfasst.

4. Verfahren nach Anspruch 1 oder Anspruch 2, worin Schritt (b) das Detektieren von Chromosomenumordnungen genomischer DNA umfasst, die einen 5'-Abschnitt einer Transkriptionsregulierungsregion des androgenregulierten Gens und einen 3'-Abschnitt eines der ETS-Familie angehörigen Gens aufweist.

5. Verfahren nach Anspruch 1 oder Anspruch 2, worin Schritt (b) das Detektieren chimärer mRNA-Transkripte umfasst, die einen 5'-Abschnitt einer Transkriptionsregulierungsregion des androgenregulierten Gens und einen 3'-Abschnitt eines der ETS-Familie angehörigen Gens aufweist.

6. Verfahren nach Anspruch 1 oder Anspruch 2, worin die Detektion unter Anwendung eines Sequenzierungsverfahrens, eines Hybridisierungsverfahrens oder eines Amplifikationsverfahrens durchgeführt wird.

7. Verfahren nach Anspruch 1 oder Anspruch 2, worin der Detektionsschritt Folgendes umfasst:
(a) die Verwendung einer Oligonucleotidsonde, die eine Sequenz umfasst, die an eine Verbindungsstelle einer chimären genomischen DNA oder einer chimären mRNA hybridisiert, worin ein 5'-Abschnitt der chimären genomischen DNA oder der chimären DNA von einer Transkriptionsregulierungsregion eines SLC45A3-Gens stammt und ein 3'-Abschnitt der chimären genomischen DNA oder der chimären DNA von einem der ETS-Familie angehörigen Gen stammt;
(b) die Verwendung einer ersten markierten Oligonucleotidsonde, die eine Sequenz umfasst, die an einen 5'-Abschnitt einer chimären genomischen DNA oder einer chimären mRNA von einer Transkriptionsregulierungsregion eines SLC45A3-Gens hybridisiert, und einer zweiten markierten Oligonucleotidsonde, die eine Sequenz umfasst, die an einen 3'-Abschnitt der chimären genomischen DNA oder der chimären DNA von einem der ETS-Familie angehörigen Gen hybridisiert; oder
(c) die Verwendung eines ersten Amplifikationsoligonucleotids, das eine Sequenz umfasst, die an einen 5'-Abschnitt einer chimären genomischen DNA oder einer chimären mRNA von einer Transkriptionsregulierungsregion eines SLC45A3-Gens hybridisiert, und eines zweiten Amplifikationsoligonucleotids, das eine Sequenz umfasst, die an einen 3'-Abschnitt der chimären genomischen DNA oder der chimären DNA von einem der ETS-Familie angehörigen Gen hybridisiert.

8. Verfahren zur Identifizierung von Prostatakrebs in einer biologischen Probe aus einem Patienten, wobei die Probe Gewebe, Blut, Plasma, Serum, Urin, Urinüberstand, Urinzellpellet, Samenflüssigkeit, Prostatasekretionen oder Prostatazellen sind; wobei das Verfahren Folgendes umfasst:
das Nachweisen der Gegenwart oder Abwesenheit einer Genfusion in der biologischen Probe, worin die Genfusion einen 5'-Abschnitt, der eine Transkriptionsregulierungsregion eines als SLC45A3 definierten androgenregulierten Gens umfasst, und einen 3'-Abschnitt, der einen Abschnitt eines der ETS-Familie angehörigen Gens umfasst, aufweist;
worin das Nachweisen der Gegenwart der Genfusion in der biologischen Probe ein Indikator für das Vorliegen von Prostatakrebs in der Probe ist.

9. Verfahren nach Anspruch 8, worin die Transkriptionsregulierungsregion des androgenregulierten Gens eine Promotorregion des androgenregulierten Gens umfasst.

10. Verfahren nach Anspruch 8, worin Schritt (b) das Detektieren von Chromosomenumordnungen genomischer DNA umfasst, die einen 5'-Abschnitt der Transkriptionsregulierungsregion des androgenregulierten Gens und einen 3'-Abschnitt von dem der ETS-Familie angehörigen Gen aufweist.

11. Zusammensetzung, die Folgendes umfasst:
(a) eine Oligonucleotidsonde, die eine Sequenz umfasst, die an eine Verbindungsstelle einer chimären genomischen DNA oder einer chimären mRNA hybridisiert, worin ein 5'-Abschnitt der chimären genomischen DNA oder der chimären DNA von einer Transkriptionsregulierungsregion eines SLC45A3-Gens stammt und ein 3'-Abschnitt der chimären genomischen DNA oder der chimären DNA von einem der ETS-Familie angehörigen Gen stammt;
(b) eine erste markierte Oligonucleotidsonde, die eine Sequenz umfasst, die an einen 5'-Abschnitt einer chimären genomischen DNA oder einer chimären mRNA von einer Transkriptionsregulierungsregion eines SLC45A3-Gens hybridisiert, und eine zweite markierte Oligonucleotidsonde, die eine Sequenz umfasst, die an einen 3'-Abschnitt der chimären genomischen DNA oder der chimären DNA von einem der ETS-Familie angehörigen Gen hybridisiert; oder
(c) ein erstes Amplifikationsoligonucleotid, das eine Sequenz umfasst, die an einen 5'-Abschnitt einer chimären genomischen DNA oder einer chimären mRNA von einer Transkriptionsregulierungsregion eines SLC45A3-Gens hybridisiert, und ein zweites Amplifikationsoligonucleotid, das eine Sequenz umfasst, die an einen 3'-Abschnitt der chimären genomischen DNA oder der chimären DNA von einem der ETS-Familie angehörigen Gen hybridisiert.

## Revendications

1. Procédé pour identifier le cancer de la prostate chez un patient, comprenant :
la détection d'une présence ou d'une absence, dans un échantillon du patient, d'une fusion génique de SLC45A3 comportant une partie 5' issue d'une région de régulation de la transcription d'un gène régulé par les androgènes défini en tant que SLC45A3 et comportant une partie 3' issue d'un gène membre de la famille ETS ;
où la présence dans l'échantillon de la fusion génique indique le cancer de la prostate chez le patient.

2. Procédé selon la revendication 1, dans lequel l'échantillon est des cellules de la prostate, des sécrétions prostatiques ou une fraction de celles-ci.

3. Procédé selon la revendication 1 ou la revendication 2, dans lequel la région de régulation de la transcription du gène régulé par les androgènes comprend une région promotrice du gène régulé par les androgènes.

4. Procédé selon la revendication 1 ou la revendication 2, dans lequel l'étape (b) comprend la détection de réarrangements chromosomiques d'ADN génomique comportant une partie 5' issue de la région de régulation de la transcription du gène régulé par les androgènes et comportant une partie 3' issue du gène membre de la famille ETS.

5. Procédé selon la revendication 1 ou la revendication 2, dans lequel l'étape (b) comprend la détection de transcrits d'ARNm chimérique comportant une partie 5' issue de la région de régulation de la transcription du gène régulé par les androgènes et comportant une partie 3' issue du gène membre de la famille ETS.

6. Procédé selon la revendication 1 ou la revendication 2, dans lequel la détection est réalisée en utilisant une technique de séquençage, une technique d'hybridation, ou une technique d'amplification.

7. Procédé selon la revendication 1 ou la revendication 2, dans lequel l'étape de détection comprend :
(a) l'utilisation d'une sonde oligonucléotidique comprenant une séquence qui s'hybride à une jonction d'un ADN génomique chimérique ou d'un ARNm chimérique dans lequel une partie 5' de l'ADN génomique chimérique ou de l'ARNm chimérique est issue d'une région de régulation de la transcription d'un gène SLC45A3 et une partie 3' de l'ADN génomique chimérique ou de l'ARNm chimérique est issue d'un gène membre de la famille ETS ;
(b) l'utilisation d'une première sonde oligonucléotidique marquée comprenant une séquence qui s'hybride à une partie 5' d'un ADN génomique chimérique ou d'un ARNm chimérique issue d'une région de régulation de la transcription d'un gène SLC45A3 et d'une seconde sonde oligonucléotidique marquée comprenant une séquence qui s'hybride à une partie 3' de l'ADN génomique chimérique ou de l'ARNm chimérique issue d'un gène membre de la famille ETS ; ou
(c) l'utilisation d'un premier oligonucléotide d'amplification comprenant une séquence qui s'hybride à une partie 5' d'un ADN génomique chimérique ou d'un ARNm chimérique issue d'une région de régulation de la transcription d'un gène SLC45A3 et d'un second oligonucléotide d'amplification comprenant une séquence qui s'hybride à une partie 3' de l'ADN génomique chimérique ou de l'ARNm chimérique issue d'un gène membre de la famille ETS.

8. Procédé pour identifier le cancer de la prostate dans un échantillon biologique d'un patient, l'échantillon étant un tissu, du sang, du plasma, du sérum, de l'urine, un surnageant d'urine, un culot cellulaire d'urine, du sperme, des sécrétions prostatiques, ou des cellules de la prostate ; le procédé comprenant :
la détection d'une présence ou d'une absence d'une fusion génique dans l'échantillon biologique, où la fusion génique comporte une partie 5' comprenant une partie d'une région de régulation de la transcription d'un gène régulé par les androgènes défini en tant que SLC45A3 et une partie 3' comprenant une partie d'un gène membre de la famille ETS,
où la détection de la présence de la fusion génique dans l'échantillon biologique indique la présence du cancer de la prostate dans l'échantillon.

9. Procédé selon la revendication 8, dans lequel la région de régulation de la transcription du gène régulé par les androgènes comprend une région promotrice du gène régulé par les androgènes.

10. Procédé selon la revendication 8, dans lequel l'étape (b) comprend la détection de transcrits d'ARN chimérique comportant une partie 5' issue de la région de régulation de la transcription du gène régulé par les androgènes et une partie 3' issue du gène membre de la famille ETS.

11. Composition comprenant :
(a) une sonde oligonucléotidique comprenant une séquence qui s'hybride à une jonction d'un ADN génomique chimérique ou d'un ARNm chimérique dans lequel une partie 5' de l'ADN génomique chimérique ou de l'ARNm chimérique est issue d'une région de régulation de la transcription d'un gène SLC45A3 et une partie 3' de l'ADN génomique chimérique ou de l'ARNm chimérique est issue d'un gène membre de la famille ETS ; ou
(b) une première sonde oligonucléotidique marquée comprenant une séquence qui s'hybride à une partie 5' d'un ADN génomique chimérique ou d'un ARNm chimérique issue d'une région de régulation de la transcription d'un gène SLC45A3 et une seconde sonde oligonucléotidique marquée comprenant une séquence qui s'hybride à une partie 3' de l'ADN génomique chimérique ou de l'ARNm chimérique issue d'un gène membre de la famille ETS ; ou
(c) un premier oligonucléotide d'amplification comprenant une séquence qui s'hybride à une partie 5' d'un ADN génomique chimérique ou d'un ARNm chimérique issue d'une région de régulation de la transcription d'un gène SLC45A3 et un second oligonucléotide d'amplification comprenant une séquence qui s'hybride à une partie 3' de l'ADN génomique chimérique ou de l'ARNm chimérique issue d'un gène membre de la famille ETS.


### SEQUENCE LISTING

<110> The Regents of the University of Michigan
<120> Recurrent Gene Fusions in Prostate Cancer
<130> HMK/FP6841837
<140> EP
   <141> 2008-07-03
<150> EP 08772418.3
   <151> 2008-07-03
<150> PCT/US2008/069204
   <151> 2008-07-03
<150> US 11/825,552
   <151> 2007-07-06
<160> 428
<170> PatentIn version 3.5
<210> 1
   <211> 24
   <212> DNA
   <213> Homo sapiens
<400> 1
   aacagagatc tggctcatga ttca 24
<210> 2
   <211> 24
   <212> DNA
   <213> Homo sapiens
<400> 2
   cttctgcaag ccatgtttcc tgta 24
<210> 3
   <211> 24
   <212> DNA
   <213> Homo sapiens
<400> 3
   aggaaacatg gcttgcagaa gctc 24
<210> 4
   <211> 26
   <212> DNA
   <213> Homo sapiens
<400> 4
   tctggtacaa actgctcatc attgtc 26
<210> 5
   <211> 26
   <212> DNA
   <213> Homo sapiens
<400> 5
   ctcaggtacc tgacaatgat gagcag 26
<210> 6
   <211> 24
   <212> DNA
   <213> Homo sapiens
<400> 6
   catggactgt ggggttcttt cttg 24
<210> 7
   <211> 26
   <212> DNA
   <213> Homo sapiens
<400> 7
   aacagccctt taaattcagc tatgga 26
<210> 8
   <211> 21
   <212> DNA
   <213> Homo sapiens
<400> 8
   ggagggcctc attcccactt g 21
<210> 9
   <211> 22
   <212> DNA
   <213> Homo sapiens
<400> 9
   ctaccccatg gaccacagat tt 22
<210> 10
   <211> 22
   <212> DNA
   <213> Homo sapiens
<400> 10
   cttaaagcct tgtggtggga ag 22
<210> 11
   <211> 24
   <212> DNA
   <213> Homo sapiens
<400> 11
   cgcagagtta tcgtgccagc agat 24
<210> 12
   <211> 24
   <212> DNA
   <213> Homo sapiens
<400> 12
   ccatattctt tcaccgccca ctcc 24
<210> 13
   <211> 23
   <212> DNA
   <213> Homo sapiens
<400> 13
   cgactggagc acgaggacac tga 23
<210> 14
   <211> 24
   <212> DNA
   <213> Homo sapiens
<400> 14
   catggactgt ggggttcttt cttg 24
<210> 15
   <211> 22
   <212> DNA
   <213> Homo sapiens
<400> 15
   ggcgttccgt aggcacactc aa 22
<210> 16
   <211> 20
   <212> DNA
   <213> Homo sapiens
<400> 16
   cctggctggg ggttgagaca 20
<210> 17
   <211> 21
   <212> DNA
   <213> Homo sapiens
<400> 17
   taggcgcgag ctaagcagga g 21
<210> 18
   <211> 25
   <212> DNA
   <213> Homo sapiens
<400> 18
   gtaggcacac tcaaacaacg actgg 25
<210> 19
   <211> 19
   <212> DNA
   <213> Homo sapiens
<400> 19
   cgcgagctaa gcaggaggc 19
<210> 20
   <211> 22
   <212> DNA
   <213> Homo sapiens
<400> 20
   caggccatga aaagccaaac tt 22
<210> 21
   <211> 20
   <212> DNA
   <213> Homo sapiens
<400> 21
   ccggatggag cggaggatga 20
<210> 22
   <211> 20
   <212> DNA
   <213> Homo sapiens
<400> 22
   cgggcgattt gctgctgaag 20
<210> 23
   <211> 19
   <212> DNA
   <213> Homo sapiens
<400> 23
   gccgcccctc gactctgaa 19
<210> 24
   <211> 24
   <212> DNA
   <213> Homo sapiens
<400> 24
   gagccacgtc tcctggaagt gact 24
<210> 25
   <211> 21
   <212> DNA
   <213> Homo sapiens
<400> 25
   ctggccggtt cttctggatg c 21
<210> 26
   <211> 18
   <212> DNA
   <213> Homo sapiens
<400> 26
   cgggccgggg aatggagt 18
<210> 27
   <211> 22
   <212> DNA
   <213> Homo sapiens
<400> 27
   cctggagggt accggtttgt ca 22
<210> 28
   <211> 20
   <212> DNA
   <213> Homo sapiens
<400> 28
   ccgcctgcct ctgggaacac 20
<210> 29
   <211> 30
   <212> DNA
   <213> Homo sapiens
<400> 29
   aaataagttt gtaagaggag cctcagcatc 30
<210> 30
   <211> 24
   <212> DNA
   <213> Homo sapiens
<400> 30
   atcgtaaaga gcttttctcc ccgc 24
<210> 31
   <211> 23
   <212> DNA
   <213> Homo sapiens
<400> 31
   gaaagggctg taggggcgac tgt 23
<210> 32
   <211> 327
   <212> DNA
   <213> Homo sapiens
<400> 32
   ccgtcggcgc cgagggagtt agtgcgaccc ggctcggcgc gcacggccaa ggcacgcgcg      60
   ctggcacacg cgggcgcgga cacgcgcgga cacacacgtg cgggacacgc cctcccccga     120
   cggcggcgct aacctctcgg ttattccagg atctttggag acccgaggaa agccgtgttg     180
   accaaaagca agacaaatga ctcacagaga aaaaagatgg cagaaccaag ggcaactaaa     240
   gccgtcaggt tctgaacagc tggtagatgg gctggcttac tgaaggacat gattcagact     300
   gtcccggacc cagcagctca tatcaag                                         327
<210> 33
   <211> 6158
   <212> DNA
   <213> Homo sapiens
<400> 33
   gttgatagaa gtccagatcc tgaggaaatc tccagctaaa tgctcaaaat ataaaatact 60
   gagctgagat ttgcgaagag cagcagcatg gatggatttt atgaccagca agtgccttac 120
   atggtcacca atagtcagcg tgggagaaat tgtaacgaga aaccaacaaa tgtcaggaaa 180
   agaaaattca ttaacagaga tctggctcat gattcagaag aactctttca agatctaagt 240
   caattacagg aaacatggct tgcagaagct caggtacctg acaatgatga gcagtttgta 300
   ccagactatc aggctgaaag tttggctttt catggcctgc cactgaaaat caagaaagaa 360
   ccccacagtc catgttcaga aatcagctct gcctgcagtc aagaacagcc ctttaaattc 420
   agctatggag aaaagtgcct gtacaatgtc agtgcctatg atcagaagcc acaagtggga 480
   atgaggccct ccaacccccc cacaccatcc agcacgccag tgtccccact gcatcatgca 540
   tctccaaact caactcatac accgaaacct gaccgggcct tcccagctca cctccctcca 600
   tcgcagtcca taccagatag cagctacccc atggaccaca gatttcgccg ccagctttct 660
   gaaccctgta actcctttcc tcctttgccg acgatgccaa gggaaggacg tcctatgtac 720
   caacgccaga tgtctgagcc aaacatcccc ttcccaccac aaggctttaa gcaggagtac 780
   cacgacccag tgtatgaaca caacaccatg gttggcagtg cggccagcca aagctttccc 840
   cctcctctga tgattaaaca ggaacccaga gattttgcat atgactcaga agtgcctagc 900
   tgccactcca tttatatgag gcaagaaggc ttcctggctc atcccagcag aacagaaggc 960
   tgtatgtttg aaaagggccc caggcagttt tatgatgaca cctgtgttgt cccagaaaaa 1020
   ttcgatggag acatcaaaca agagccagga atgtatcggg aaggacccac ataccaacgg 1080
   cgaggatcac ttcagctctg gcagtttttg gtagctcttc tggatgaccc ttcaaattct 1140
   cattttattg cctggactgg tcgaggcatg gaatttaaac tgattgagcc tgaagaggtg 1200
   gcccgacgtt ggggcattca gaaaaacagg ccagctatga actatgataa acttagccgt 1260
   tcactccgct attactatga gaaaggaatt atgcaaaagg tggctggaga gagatatgtc 1320
   tacaagtttg tgtgtgatcc agaagccctt ttctccatgg cctttccaga taatcagcgt 1380
   ccactgctga agacagacat ggaacgtcac atcaacgagg aggacacagt gcctctttct 1440
   cactttgatg agagcatggc ctacatgccg gaagggggct gctgcaaccc ccacccctac 1500
   aacgaaggct acgtgtatta acacaagtga cagtcaagca gggcgttttt gcgcttttcc 1560
   ttttttctgc aagatacaga gaattgctga atctttgttt tatttctgtt gtttgtattt 1620
   tatttttaaa taataataca caaaaagggg cttttcctgt tgcattattc tatggtctgc 1680
   catggactgt gcactttatt tgagggtggg tgggagtaat ctaaacattt attctgtgta 1740
   acaggaagct aatgggtgaa tgggcagagg gatttgggga ttacttttta cttaggcttg 1800
   ggatggggtc ctacaagttt tgagtatgat gaaactatat catgtctgtt tgatttcata 1860
   acaacataag ataatgttta ttttatcggg gtatctatgg tacagttaat ttcacgttgt 1920
   gtaaatatcc acttggagac tatttgcctt gggcattttc ccctgtcatt tatgagtctc 1980
   tgcaggtgta caaaaaaacc ccaatctact gtaaatggca gtttaattgt tagaaatgac 2040
   tgtttttgca ccacttgtaa aaaggtattt agcgattgca tttgctgttt gttgttttat 2100
   tttgctttat atatgacttg cagaggataa ccataaaatg ggtaattctc tctgaagttg 2160
   aataatcacc atgactgtaa atgaggggca caattttgga ctctggcgcc aaactgagtc 2220
   ataggccagt agcattacgt gtatctggtg ccaccttgct gtttagatac aaatcatacc 2280
   gtcttttaaa tattttgaag cccatttcag ttaaataatg acatgtcatg gtcctttgga 2340
   atcttcattt aaatgttaaa tctggaatca aaatgaagca aaaaatatct gtctcctttt 2400
   cactttcttc agtacataaa tacattattt aatcaataag aattaactgt actaaatcat 2460
   gtattatgct gttctagtta cagcaaacac tctttaagaa aaatatccaa tacactaaat 2520
   aggtactata gtaattttta gacatggtac ccattgatat gcatttaaac cttttactgc 2580
   tgtgttatgt tgataacata tataaatatt agataatgct aatgcttctg ctgctgtctt 2640
   ttctgtaata ttctctttca tgctgaattt actatgacca tttataagca gtgcagttaa 2700
   ctacagatag catttcagga caaaatagat gactcaaacc atttattgct taaaaaatag 2760
   cttacgccat gctatgctat aagcagcttt tatgcacatt gacaaatgaa gagtaagctt 2820
   cagcttgcta aaggaaactg tggaaccttt tgtaactttt ggtgatatgg aaaattattt 2880
   acaaaccgtc aaagaatatg aggaagttgc tgtatgacat agtgctggca ctgatattat 2940
   ccatcatctc tttttggaca cttctgtaaa tgtgattgga ttgtttgaaa gaagatttaa 3000
   agtttcaaag ttttttgttc tgtttttgct ttgcatttgg agaaaatatt gaaagcaggg 3060
   tatgttgttt cattcacctt gaaaaaacca tgagtaaatg gggatataga atctctgaat 3120
   agctcgctaa aagattcaag caagggacat gaattttgtt ccatctatca ataatatcca 3180
   gaagaacaac ttttttaaag agtctatagc aaaaagcaaa aaaaaaaaaa aattctaaac 3240
   acaaagtcaa aataaaccta ttgtaaaagc atttcgtgat gagcatgaaa aagattgttt 3300
   aaagatgatc cccccagcta cccattttcc aaaactacac agatcacagc tcatttctct 3360
   aagtggagca gttatcaaga aacccaaaca ccaaaattgc tactcttcac atttaatcct 3420
   acaaaaagta ctccaatttc aaaatatgta tgtaacctgc gatttcaatg attgttgttc 3480
   atatacatca tgtattattt tggcccattt tgggcctaaa aaagaaaact atgccttaaa 3540
   aatcagaacc ttttctcccc actatgctta tgtggccatc tacagcactt agaataaaaa 3600
   cagatgttaa aatattcagt gaaagtttta ttggaaaaag gaattgagat atataattga 3660
   gatttggtga aattgaagga gaaaatttaa gtgagtcttt aaaatatatt ctgaatgaaa 3720
   actgtattga ggattcattt ttgttccttt tttttctttt tctcttttct cctttttctt 3780
   ctttttaata gtctagtttt agtcagtcag tgaggaagaa ttgggccatg ctaacgttat 3840
   cacaagagaa caatggcaga aatggtatta gttatataat atttaaggac aaactatatg 3900
   ttttgctgtt ttaacgtagt gactcactga actaaataca taattgacca acattaagtg 3960
   tatttccaat acagaagggt tgaaaatatt acattataaa ctcttttgaa aaatgtatct 4020
   aaaatttttt aagttctgtt ttgattccac tttttggttg agtttttatg tttttgtttt 4080
   caggtagatt aataaatctg gcagctgatt tctgcaagat tcttgtgttt tgaatttctc 4140
   attgaattgg ctactcaaac atagaaatca tttgttaatg atgtaatgtc ttctctcagc 4200
   ttttatcttc actgctgttt gctgtctctt gatgatgaca tgttaatacc caatagatta 4260
   attgcaacaa acacttatac tcaaataact aagtaaaaat aatttttctt gttatgtcca 4320
   tgaaaagtgc ttcagaataa aaatccacaa gactgacagt gcagaacatt tttctcaaat 4380
   catgggcgga tcttggaggt ctagtttccc gtagatgctg taaccaatta ccacaacttc 4440
   agtaatttac acaaatttat cttatagttc tggaggcaga agttcaaaag aagccttaag 4500
   agactaaaac caagatgtcc ttaggtctgg ttccttctgg aggctccagg ggagattctt 4560
   ccagctttca cttctagagt ctgctgacat tccttggctc ctggctacat cacttcaatc 4620
   tctgcttcca tggtcacata ctcttctact atagtcaaat ttccttcctg cctcttataa 4680
   ggatgcttgt gattacattt aggggatgct cagataatcc aggacaatct ctccatctca 4740
   agatccttaa cttaatgacg tgtgccaagt ccctttggct agataattat tcataggtcc 4800
   cagggattag gacatggatg taaggggtga gggcagggct gttattcaga acaccgcacg 4860
   gaggaggaag actgtgtagc aaagactcta attgatttac tcaggaacag tggagttctg 4920
   ctgagggatc taggatttga aagtactaga gtttgctttt atttaccact gagatatttt 4980
   ccccttattc tgcataaata attttgaaaa ctttctatat taaatttcaa ctattccact 5040
   aaaatgtctg gtaatcacat caagccttta gattattcaa atccttcccc agcccccagg 5100
   aaaacactaa gtcatgaaac agaaaaacag aaggtatgat aataatagta ataacagtta 5160
   aatcagtggt ctaatccaga ttttattttt taatacattt cttttggtgt taatatgggt 5220
   tactatgtga tcttatcatt tgctagtgat tattacttat taggtaagaa caatgtgtaa 5280
   aatatgtcta ttactcaaaa gaacaattgc aaaatgagtc aacttatctt tatataacca 5340
   ggaaagaaat atattgccag aagctacaga attttgccag atgataggga tttctaaaat 5400
   gagccacttt gtctatcatg cagccttttc agagcttgta atgagaaaac attacagagg 5460
   agaaggtcat ttggatgttt gttacttgga atcctagaaa acaaaaacta aaatttaaaa 5520
   ataagaagtg agtaagctat tttccatttg cgatttggta tggagaagag aggaaataga 5580
   attattaaaa aaatacaaat tgggtaaaag tgatggtgga aaaaatataa agaaggcaaa 5640
   tgtacatatt aagcaattct actaagaatt ggaaaaatca agtttcaaaa agatggtaat 5700
   agttgggcat gatactagaa aatttcaccc agtttattca gagctcaact agtactttta 5760
   ggacttcttt ttttatatac atgagactca ctttgacata cttaaaaaaa aaacagttta     5820
   tggaaagtac agtttaagag gagaatttga ttagactaag tggatatctt tatagaaata     5880
   ttaatgattt cagaattttc agttacaagt gtatataccg tggctattgt ttatggattc     5940
   atatgtaagg tagggtcttt tttgcatata gactccagta ttagttactt tcattctaaa     6000
   attatattta tgcttctatg gggaagaaaa tttttaattc acttggttgt attaaaatta     6060
   tacttacggt ttgagaaaac atgctatgaa aatcatgatt atagcaaatt aaatatgctc     6120
   aaaatttaaa tctaaaataa aagcccagaa actgaaaa                             6158
<210> 34
   <211> 5228
   <212> DNA
   <213> Homo sapiens
<400> 34
   cgggcgaggg ccgggcagga ggagcgggcg cggcgcgggc gaggctggga cccgagcgcg 60
   ctcacttcgc cgcaaagtgc caacttcccc tggagtgccg ggcgcgcacc gtccgggcgc 120
   gggggaaaga aaggcagcgg gaatttgaga tttttgggaa gaaagtcgga tttcccccgt 180
   ccccttcccc ctgttactaa tcctcattaa aaagaaaaac aacagtaact gcaaacttgc 240
   taccatcccg tacgtccccc actcctggca ccatgaaggc ggccgtcgat ctcaagccga 300
   ctctcaccat catcaagacg gaaaaagtcg atctggagct tttcccctcc ccggatatgg 360
   aatgtgcaga tgtcccacta ttaactccaa gcagcaaaga aatgatgtct caagcattaa 420
   aagctacttt cagtggtttc actaaagaac agcaacgact ggggatccca aaagaccccc 480
   ggcagtggac agaaacccat gttcgggact gggtgatgtg ggctgtgaat gaattcagcc 540
   tgaaaggtgt agacttccag aagttctgta tgaatggagc agccctctgc gccctgggta 600
   aagactgctt tctcgagctg gccccagact ttgttgggga catcttatgg gaacatctag 660
   agatcctgca gaaagaggat gtgaaaccat atcaagttaa tggagtcaac ccagcctatc 720
   cagaatcccg ctatacctcg gattacttca ttagctatgg tattgagcat gcccagtgtg 780
   ttccaccatc ggagttctca gagcccagct tcatcacaga gtcctatcag acgctccatc 840
   ccatcagctc ggaagagctc ctctccctca agtatgagaa tgactacccc tcggtcattc 900
   tccgagaccc tctccagaca gacaccttgc agaatgacta ctttgctatc aaacaagaag 960
   tcgtcacccc agacaacatg tgcatgggga ggaccagtcg tggtaaactc gggggccagg 1020
   actcttttga aagcatagag agctacgata gttgtgatcg cctcacccag tcctggagca 1080
   gccagtcatc tttcaacagc ctgcagcgtg ttccctccta tgacagcttc gactcagagg 1140
   actatccggc tgccctgccc aaccacaagc ccaagggcac cttcaaggac tatgtgcggg 1200
   accgtgctga cctcaataag gacaagcctg tcattcctgc tgctgcccta gctggctaca 1260
   caggcagtgg accaatccag ctatggcagt ttcttctgga attactcact gataaatcct 1320
   gtcagtcttt tatcagctgg acaggagatg gctgggaatt caaactttct gacccagatg 1380
   aggtggccag gagatgggga aagaggaaaa acaaacctaa gatgaattat gagaaactga 1440
   gccgtggcct acgctactat tacgacaaaa acatcatcca caagacagcg gggaaacgct 1500
   acgtgtaccg ctttgtgtgt gacctgcaga gcctgctggg gtacacccct gaggagctgc 1560
   acgccatgct ggacgtcaag ccagatgccg acgagtgatg gcactgaagg ggctggggaa 1620
   accctgctga gaccttccaa ggacagccgt gttggttgga ctctgaattt tgaattgtta 1680
   ttctattttt tattttccag aactcatttt ttaccttcag gggtgggagc taagtcagtt 1740
   gcagctgtaa tcaattgtgc gcagttggga aaggaaagcc aggacttgtg gggtgggtgg 1800
   gaccagaaat tcttgagcaa attttcagga gagggagaag ggccttctca gaagcttgaa 1860
   ggctctggct taacagagaa agagactaat gtgtccaatc atttttaaaa atcatccatg 1920
   aaaaagtgtc ttgagttgtg gacccattag caagtgacat tgtcacatca gaactcatga 1980
   aactgatgta aggcaattaa tttgcttctg tttttaggtc tgggagggca aaaaagaggt 2040
   gggtgggatg aaacatgttt tgggggggga tgcactgaaa atctgagaac tatttaccta 2100
   tcactctagt tttgaagcaa agatggactt cagtggggag gggccaaaac cgttgttgtg 2160
   ttaaaattta ttttattaaa ttttgtgcca gtattttttt tcttaaaaat cgtcttaagc 2220
   tctaaggtgg tctcagtatt gcaatatcat gtaagtttgt ttttatttgc cggctgagga 2280
   ttctgtcaca atgaaagaaa actgtttata tagaccccat tggaaaagca aaacgctctc 2340
   actgagatca gggatcccaa attcatggga cttatataag aaggacaatt aatgctgatt 2400
   tgggtacagg ggaattatgt gtgtgaatgt catctacaat taaaaaaaat tagcacatcc 2460
   ctttacttac ttgttatcag tggattctcg gggtttggac ttaatgttga gctaagaagc 2520
   attaagtctt tgaactgaat gtattttgca tccctggttt tggacgacag taaacgtagg 2580
   agcactgttg aagtcctgga agggagatcg aaggaggaag attgacttgg ttctttctta 2640
   gtcctatatc tgtagcatag atgacttgga ataaaagctg tatgcatggg cattacccct 2700
   caggtcctaa gaaataagtc ctgaatgcat gtcgttccaa actaacactc tgtaattttt 2760
   cttttatgtc ttattttcca agagtcctcc attttttgca ccccctcacc gccaactctg 2820
   ttattcagta gagagaagtg tacggctttc tgattggtga gtgaaaaagt aacttgagac 2880
   acgacctaag ttgaagagtt tagacttgct gagttttaga agtgatggaa attaagagag 2940
   catttcaata aaatgtgact tggctgtctt tggaagagaa gtgcaaggct ttcctttgaa 3000
   gaatttaaat tagtccggta ggatgtcagg tgagactgtg tatgcaaaat gaatggcaca 3060
   ggtgatgcca gggcctcttg cttgggtctg atgtcttggc acagggtaag tgaaggttaa 3120
   ttccagaaga gaggaatgac ttgaaggcaa aggaaactaa ggaaggaggt tcagtgagga 3180
   aaataaggtt gtccatgaga tttgaataga tttttagttc cccaaggttt taaatacaaa 3240
   catagtcaag caaggtagtc atctttctgc tggttgtgag ggggaatctg aaaatggagt 3300
   tttagaggaa aagtcaacat ctaactagtg aggaaaagtg cctaatacaa ttagaatctc 3360
   cctcactcta tagttgccca gttgaaagga taaggaggag gggtggcttt tatggacttc 3420
   catgagagaa ggaaagaaat atttcaggta agcttctcag ggctggccct ttttgggatt 3480
   tggatgagaa attggaagta ctaactactt tctagcatat ctttaagaaa attgattgtt 3540
   atttactccc agatcctctt gcagacccag aattatcagg aacatagctc tgtgattcat 3600
   gagtgtcccc atactgatga attggagcat ccatatggaa agcaaaggca gaattatccc 3660
   agctgtatta ttttgatctt ttggatgcag gtgccttaat gaagctctca aaatatttta 3720
   ggagctgctc agggagtgtt gggtggaact gtttggacta cattgttttc tcttagatta 3780
   tgtgattttt gttgggcact ggcaaaaggt gtgtgtgtga atgtgtgcat gtgtgtgaat 3840
   gttgtgtgtg tgtgtgtgtg tgtgtgtgtg tgtgtgtgtg tttgcagaca tgcaaaactg 3900
   cagctgaaat aataccttag atttctaggt aagtctttcc acatttcaat aatgggtaag 3960
   agtagaacca gggccgggta tcaattattg cttgctgttt gcaaccaggc ataaaatcac 4020
   tttctcaaat catccaccgt tcctattaaa tttatgccgg aaactctcct tctgtgagta 4080
   taactcctgc agttcctata gcagataaga tataagaaag tgcctcctag tgctcctccg 4140
   cccgcttgtt tgctaaaatt ccctttctct ctaagtccac cattttcaag atttgtagat 4200
   agtgtattag ttaagacagc tttgtcgatc tggccagatg ttttttctcc tttgtccaaa 4260
   ggccagagac catcccagga agagtggtgg gtggtttata cactggaaat gttgcgttta 4320
   tgctttttaa aaacacacgt taacttcaga ggaaggatgg gcaaatctgg tctagctggg 4380
   tgaaacccct attttcccag agatgcctta acctttgttg gttttggctt tagggttcag 4440
   agtcactttt gttcccttct ccattctgga gagggacttc ccctacatag agccctgatt 4500
   tttgtggctg tggggattgg aggtagcatt caaagatcag atgtgctttt cctcactttg 4560
   gagatgaaca ctctgggttt tacagcatta acctgcctaa ccttcatggt gagaaataca 4620
   ccatctctct tctagtcatg ctgtgcatgc cgcttactct gttggggtct atataaattt 4680
   gttgaactct tacctacatt ccaaagaagt ttcaaggaac cataaatata tgtatacata 4740
   tacatatata aaatatatat attaaaataa aattatcagg aatactgcct cagttattga 4800
   actttttttt ttaagaatac ttttttttta agctgagaag tatagggatg aaaaagatgt 4860
   tatattgtgt ttgactattt tccaacttgt attttcatat aatttatatt ttttaaaagc 4920
   tgaaaattta gaagcaagat gaaaaaaagg aaaagcaggt gctttttaaa aatcagaact 4980
   gaggtagctt agagatgtag cgatgtaagt gtcgatgttt ttttaaaaaa aaatgcaaaa 5040
   aaattcttat ggcggagttt tttgtttgtt tattttagta gctgatgctg gcacatcatt 5100
   ttgctggaga gttttttata tactgtagcc tgatttcata ttgtatttta aactgtgtga 5160
   aattaaaaac aaagaatttc attcataaaa aaaaaaaaaa aaaaaaaaaa aaaaaaaaaa 5220
   aaaaaaaa 5228
<210> 35
   <211> 3672
   <212> DNA
   <213> Homo sapiens
<400> 35
   gcccggttac ttcctccaga gactgacgag tgcggtgtcg ctccagctca gagctcccgg      60
   agccgcccgg ccagcgtccg gcctccctga tcgtctctgg ccggcgccct cgccctcgcc     120
   cggcgcgcac cgagcagccg cgggcgccga gcagccaccg tcccgaccaa gcgccggccc     180
   tgcccgcagc ggcaggatga atgatttcgg aatcaagaat atggaccagg tagcccctgt     240
   ggctaacagt tacagaggga cactcaagcg ccagccagcc tttgacacct ttgatgggtc     300
   cctgtttgct gtttttcctt ctctaaatga agagcaaaca ctgcaagaag tgccaacagg     360
   cttggattcc atttctcatg actccgccaa ctgtgaattg cctttgttaa ccccgtgcag     420
   caaggctgtg atgagtcaag ccttaaaagc taccttcagt ggcttcaaaa aggaacagcg     480
   gcgcctgggc attccaaaga acccctggct gtggagtgag caacaggtat gccagtggct     540
   tctctgggcc accaatgagt tcagtctggt gaacgtgaat ctgcagaggt tcggcatgaa     600
   tggccagatg ctgtgtaacc ttggcaagga acgctttctg gagctggcac ctgactttgt     660
   gggtgacatt ctctgggaac atctggagca aatgatcaaa gaaaaccaag aaaagacaga     720
   agatcaatat gaagaaaatt cacacctcac ctccgttcct cattggatta acagcaatac     780
   attaggtttt ggcacagagc aggcgcccta tggaatgcag acacagaatt accccaaagg     840
   cggcctcctg gacagcatgt gtccggcctc cacacccagc gtactcagct ctgagcagga     900
   gtttcagatg ttccccaagt ctcggctcag ctccgtcagc gtcacctact gctctgtcag     960
   tcaggacttc ccaggcagca acttgaattt gctcaccaac aattctggga ctcccaaaga    1020
   ccacgactcc cctgagaacg gtgcggacag cttcgagagc tcagactccc tcctccagtc    1080
   ctggaacagc cagtcgtcct tgctggatgt gcaacgggtt ccttccttcg agagcttcga    1140
   agatgactgc agccagtctc tctgcctcaa taagccaacc atgtctttca aggattacat    1200
   ccaagagagg agtgacccag tggagcaagg caaaccagtt atacctgcag ctgtgctggc    1260
   cggcttcaca ggaagtggac ctattcagct gtggcagttt ctcctggagc tgctatcaga    1320
   caaatcctgc cagtcattca tcagctggac tggagacgga tgggagttta agctcgccga    1380
   ccccgatgag gtggcccgcc ggtggggaaa gaggaaaaat aagcccaaga tgaactacga    1440
   gaagctgagc cggggcttac gctactatta cgacaagaac atcatccaca agacgtcggg    1500
   gaagcgctac gtgtaccgct tcgtgtgcga cctccagaac ttgctggggt tcacgcccga    1560
   ggaactgcac gccatcctgg gcgtccagcc cgacacggag gactgaggtc gccgggacca    1620
   ccctgagccg gccccaggct cgtggactga gtgggaagcc catcctgacc agctgctccg    1680
   aggacccagg aaaggcagga ttgaaaatgt ccaggaaagt ggccaagaag cagtggcctt 1740
   attgcatccc aaaccacgcc tcttgaccag gctgcctccc ttgtggcagc aacggcacag 1800
   ctaattctac tcacagtgct tttaagtgaa aatggtcgag aaagaggcac caggaagccg 1860
   tcctggcgcc tggcagtccg tgggacggga tggttctggc tgtttgagat tctcaaagga 1920
   gcgagcatgt cgtggacaca cacagactat ttttagattt tcttttgcct tttgcaacca 1980
   ggaacagcaa atgcaaaaac tctttgagag ggtaggaggg tgggaaggaa acaaccatgt 2040
   catttcagaa gttagtttgt atatattatt ataatcttat aattgttctc agaatccctt 2100
   aacagttgta tttaacagaa attgtatatt gtaatttaaa ataattatat aactgtattt 2160
   gaaataagaa ttcagacatc tgaggtttta tttcattttt caatagcaca tatggaattt 2220
   tgcaaagatt taatctgcca agggccgact aagagaagtt gtaaagtatg tattatttac 2280
   atttaataga cttacaggga taaggcctgt ggggggtaat ccctgctttt tgtgtttttt 2340
   tgtttgtttg tttgtttgtt tttggggggt tttcttgcct tggttgtctg gcaaggactt 2400
   tgtacatttg ggagttttta tgagaaactt aaatgttatt atctgggctt atatctggcc 2460
   tctgctttct cctttaattg taaagtaaaa gctataaagc agtatttttc ttgacaaatg 2520
   gcatatgttt tccacttctt tgcatgcgtt taagtcagtt tatacacaaa atggatttta 2580
   ttttttagtt taactgtgtt tctccgacag ctcacctctc tctgaccacc cagccatttc 2640
   cttcctgtgc tccacgttct tctgtgtgat taaaataaga atattatttt tggaaatatg 2700
   caactccttt tcagagatca ggagggattt atgtagcagc tatttttact gcaaaagtaa 2760
   ttcactggaa aaaaaatgta atttgtaaga aagctttatt tttatctcag ctctatgtaa 2820
   agttaaagtt actgtacaga gctgaaggac ggggggcggt aggggtcttg atgaaacctc 2880
   ttgaacgaag cacagtttgt cccatctttg ttcactcgtg tgtctcaacc atcttaatag 2940
   catgctgctc ctttttgctc agtgtccaca gcaagatgac gtgattctta ttttcttgga 3000
   cacagactat tctgaggcac agagcgggga cttaagatgg gaaagagaaa gcatcggagc 3060
   cattcattcg gagaaaacgt tttgatcaaa atggagactt ttgtagtcgt ttcaaaagag 3120
   cacctgagtc atgtgtattc ccggccttta taaatgaccc ggtcaagttg gtttcaaagt 3180
   ccgacaggct tgtctgttta ctagctgcgt ggccttggac gggtggctga catctgtaaa 3240
   gaatcctcct gtgatgaaac tgaggaatcg ggtggccggg caagctggga agagcaaagc 3300
   cagagctgcg ctgcctcaat acccacaaaa gaccattccc agtatacata agcacaggat 3360
   gtttttctca agagggatgt atttatcact tggacatctg tttataatat aaacagacat 3420
   gtgactggga acatcttgct gccaaaagaa tcctaggcag tggctcattg tatgtgaggt 3480
   tgaaccacgt gaaattgcca atattaggct ggcttttatc tacaaagaag gagtttcatg 3540
   gggttcagcc taacagttat ggaaactaca gtccttataa accattggca tggtaataaa 3600
   cagatcttaa gtataaaaat tttgtaattg ggcctttact ctctcaataa taaagtattt 3660
   tgtttatata aa 3672
<210> 36
   <211> 2668
   <212> DNA
   <213> Homo sapiens
<400> 36
   cgctacacac aggtacccct gggatggcgt gagcactccc ccagcgatgg acccatctgt 60
   gacgctgtgg cagtttctgc tgcagctgct gagagagcaa ggcaatggcc acatcatctc 120
   ctggacttca cgggatggtg gtgaattcaa gctggtggat gcagaggagg tggcccggct 180
   gtgggggcta cgcaagaaca agaccaacat gaattacgac aagctcagcc gggccttgcg 240
   gtactactat gacaagaaca tcatccgcaa ggtgagcggc cagaagttcg tctacaagtt 300
   tgtgtcctac cctgaggtcg cagggtgctc cactgaggac tgcccgcccc agccagaggt     360
   gtctgttacc tccaccatgc caaatgtggc ccctgctgct atacatgccg ccccagggga     420
   cactgtctct ggaaagccag gcacacccaa gggtgcagga atggcaggcc caggcggttt 480
   ggcacgcagc agccggaacg agtacatgcg ctcgggcctc tattccacct tcaccatcca 540
   gtctctgcag ccgcagccac cccctcatcc tcggcctgct gtggtgctcc ccagtgcagc 600
   tcctgcaggg gcagcagcgc ccccctcggg gagcaggagc accagtccaa gccccttgga 660
   ggcctgtctg gaggctgaag aggccggctt gcctctgcag gtcatcctga ccccgcccga 720
   ggccccaaac ctgaaatcgg aagagcttaa tgtggagccg ggtttgggcc gggctttgcc 780
   cccagaagtg aaagtagaag ggcccaagga agagttggaa gttgcggggg agagagggtt 840
   tgtgccagaa accaccaagg ccgagccaga agtccctcca caggagggcg tgccagcccg 900
   gctgcccgcg gttgttatgg acaccgcagg gcaggcgggc ggccatgcgg cttccagccc 960
   tgagatctcc cagccgcaga agggccggaa gccccgggac ctagagcttc cactcagccc 1020
   gagcctgcta ggtgggccgg gacccgaacg gaccccagga tcgggaagtg gctccggcct 1080
   ccaggctccg gggccggcgc tgaccccatc cctgcttcct acgcatacat tgaccccggt 1140
   gctgctgaca cccagctcgc tgcctcctag cattcacttc tggagcaccc tgagtcccat 1200
   tgcgccccgt agcccggcca agctctcctt ccagtttcca tccagtggca gcgcccaggt 1260
   gcacatccct tctatcagcg tggatggcct ctcgaccccc gtggtgctct ccccagggcc 1320
   ccagaagcca tgactactac caccaccacc accacccctt ctggggtcac tccatccatg 1380
   ctctctccag ccagccatct caaggagaaa catagttcaa ctgaaagact catgctctga 1440
   ttgtggtggg gtggggatcc ttgggaagaa ttactcccaa gagtaactct cattatctcc 1500
   tccacagaaa acacacagct tccacaactt ctctgttttc tgtcagtccc ccagtggccg 1560
   cccttacacg tctcctactt caatggtagg ggcggtttat ttatttattt tttgaaggcc 1620
   actgggagga gcctgaccta accttttagg gtggttagga catctccccc acctccccac 1680
   ttttttcccc aagacaagac aatcgaggtc tggcttgaga acgacctttc tttctttatt 1740
   tctcagcctg cccttgggga gatgagggag ccctgtctgc gtttttggat gtgagtagaa 1800
   gagttagttt gttttgtttt attattcctg gccatactca ggggtccagg aagaatttgt 1860
   accatttaat gggttgggag tcttggccaa ggaagaatca cacccttgga atagaaattt 1920
   ccacctcccc aacctttctc tcagacagct tatccttttc aaccaacttt ttggccaggg 1980
   aggaatgtcc cttttgttct tccccctgag aagccattcc tttgtctgcc aacctccctg 2040
   gggtcctgcc tgtttcctcc caatggaggg tttttttggg gggtggtccc cgtctggggg 2100
   gcccctccag ccagtactcc aggtctccct gtctctcccc cgctgccatt ttgatagtat 2160
   aatctatttt taaatggggc ttttcaatag gggagaggga gtcatctctt cctatatttg 2220
   gtggggtggg tgggaaggaa gggatttggg ggggaatctt cctgccgcct cccccactcc 2280
   aagtgtttat ttttgatacc aaacatgaat tttcagttcc ctccctccca gccccccaat 2340
   ttcctgcggg cgggtacaaa ggaccctttc aatgtccctg gagttgggag ggaggaatgg 2400
   gggacataaa gcctgtcctg tctctattct aggcaagaga gagtgggttc aaaagactcc 2460
   tgggctcacc tgttagcgct ggcccagccc aggccttggg acctgggggt tggtgatttg 2520
   ggggacagtg ctacactcgt ctccactgtt tgttttactt ccccaaaatg gacctttttt 2580
   ttttctaaag agtcccagag aatggggaat tgttcctgta aatatatatt tttcaaagtg 2640
   aaaaaaaaaa aaaaaaaaaa aaaaaaaa 2668
<210> 37
   <211> 5992
   <212> DNA
   <213> Homo sapiens
<400> 37
   gcgtcccggg tccccgcgcc gcgccgcgac ctgcagaccc cgccgccgcg ctcgggcccg 60
   tctcccacgc ccccgccgcc ccgcgcgccc aactccgccg gccgccccgc cccgccccgc 120
   gcgctccaga cccccggggc ggctgccggg agagatgctg gaagaaactt cttaaatgac 180
   cgcgtctggc tggccgtgga gcctttctgg gttggggaga ggaaaggaaa gtggaaaaaa 240
   cctgagaact tcctgatctc tctcgctgtg agacatgtct gagactcctg ctcagtgtag 300
   cattaagcag gaacgaattt catatacacc tccagagagc ccagtgccga gttacgcttc 360
   ctcgacgcca cttcatgttc cagtgcctcg agcgctcagg atggaggaag actcgatccg 420
   cctgcctgcg cacctgcgct tgcagccaat ttactggagc agggatgacg tagcccagtg 480
   gctcaagtgg gctgaaaatg agttttcttt aaggccaatt gacagcaaca cgtttgaaat 540
   gaatggcaaa gctctcctgc tgctgaccaa agaggacttt cgctatcgat ctcctcattc 600
   aggtgatgtg ctctatgaac tccttcagca tattctgaag cagaggaaac ctcggattct 660
   tttttcacca ttcttccacc ctggaaactc tatacacaca cagccggagg tcatactgca      720
   tcagaaccat gaagaagata actgtgtcca gaggaccccc aggccatccg tggataatgt      780
   gcaccataac cctcccacca ttgaactgtt gcaccgctcc aggtcaccta tcacgacaaa      840
   tcaccggcct tctcctgacc ccgagcagcg gcccctccgg tcccccctgg acaacatgat      900
   ccgccgcctc tccccggctg agagagctca gggacccagg ccgcaccagg agaacaacca      960
   ccaggagtcc taccctctgt cagtgtctcc catggagaat aatcactgcc cagcgtcctc     1020
   cgagtcccac ccgaagccat ccagcccccg gcaggagagc acacgcgtga tccagctgat     1080
   gcccagcccc atcatgcacc ctctgatcct gaacccccgg cactccgtgg atttcaaaca     1140
   gtccaggctc tccgaggacg ggctgcatag ggaagggaag cccatcaacc tctctcatcg     1200
   ggaagacctg gcttacatga accacatcat ggtctctgtc tccccgcctg aagagcacgc     1260
   catgcccatt gggagaatag cagactgtag actgctttgg gattacgtct atcagttgct     1320
   ttctgacagc cggtacgaaa acttcatccg atgggaggac aaagaatcca aaatattccg     1380
   gatagtggat cccaacggac tggctcgact gtggggaaac cataagaaca gaacaaacat     1440
   gacctatgag aaaatgtcca gagccctgcg ccactactac aaactaaaca ttatcaggaa     1500
   ggagccagga caaaggcttt tgttcaggtt tatgaaaacc ccagatgaaa tcatgagtgg     1560
   ccgaacagac cgtctggagc acctagagtc ccaggagctg gatgaacaaa tataccaaga     1620
   agatgaatgc tgaaggaacc aacagtccac ctcagcgggc cagcagccca gggaacccct     1680
   gcccaccagg attgctggaa gtgtgacgga gcaggcgggc tgaggagagt ggaaaaggaa     1740
   gcgacccaga aatggcaggg acacttctct tgcagaccaa gagggaccct ggagcacctt     1800
   agacaaacta cccagcacag gcggggctgg aattctggcg gagggcatga gcctgggact     1860
   ccatgtcacg tttccttctg atttggaatc tctccatctg taattcctca ccctcaccct     1920
   tccaccgttg ttagtatcat ggtgtttttg tttttgtttt tgttttaaga acctgcagtt     1980
   tgactcttca tcgttcatct aggggaagac atctgatgtt gttttcctat ggaaatatat     2040
   atctattata tatatatttt ttgcaaatct cacaaagtgc ggcaagccca gctggtcagg     2100
   aaagagaata cttgcagagg ggttcaggtt cctctttttc ctgccacgtg gatcaggtct     2160
   gttcctgtta ctgttgggtc ttggctgaaa aaaaaaaatg cttttaaaaa agataaaatg     2220
   aaaaggagag ctctcttttt ctctctcttg ctctgttctt cccttggtcc cctctgtcct     2280
   cccgccctgc ctgcagttga gattcagatg ccttctgaca gagttcagcc tcttggagag     2340
   tcttggggat tgttggcacc taaacagaat cagtgacccg ggtgctttgt ggccagcagc     2400
   acagaatcaa acccgcatcc cagcattggg ccacccatct gagggaggcc aaaatcatca     2460
   cagatgctgc tgtgctgcag acagatacat gctagtccag agagccgccc ctgagatggc     2520
   tgtgagaacc atgtgtctaa ggcgtaagat aaggatggaa ggctgtccaa gttatttgga     2580
   aggcctcggc agcttgggat tagcttggga gcgcagcgct gcaaagtgga aaatatgaaa     2640
   agaccacaca ggcccagcag tccagaaact gggcaaaaat attctgcagt ggggatttat 2700
   ttttccaaag caggtaacag aggctagtga gaaagaaaag ctcctctctg ctccattcca 2760
   aaggccatct tgtggtcagt ttcatgccct cacctgattt tttttttttt tttttttttt 2820
   caattcctaa ccttttttaa agtttcctgg tctccactgg acacagagct ttggagacgg 2880
   aggatcccag agggcagtct cagttgcaat cagtgtgtgc ccagcctggg cagacaggaa 2940
   attcctcgga tacattattt tttctttctt tcatagctgt gtctcagaaa ggacccattt 3000
   gtggctcttt ttcacctcaa aataagatcg atggtatctt gtaaaatgag ggtagtgcca 3060
   cttcttagta tttttgaaag ctgttttaga tttttttttt ttttcctttt ctagccatct 3120
   aaattgactc ttccaatata ggtctcagaa atccaatatt tggagtacaa tttcttttaa 3180
   tccagattac acctgcctta caaagcaccc cctccttgtt cccctctgtt tcctctactc 3240
   agttggggga gaaactcaca gctcctccgg gatacatatg tgccctcagc agcagctccc 3300
   aggtgaagtt accagacccc tgggcttctc cccagctttt tctgagttga gtcagacatg 3360
   tagagtttgg gtcacacagg caagaggaat tttccctcgg ccttactgac aaggacacca 3420
   acctagggtg caaacagatg gactatggtt caaggacact ggaattgagg agctgatcaa 3480
   ggctctcttc agccttgctc tgtccctgcc tcttatcaga gcacaggtag acacacgggc 3540
   atagccagcc cactcctact gtcacaggcg ccccaccatt caaccttccg ggaggtcagg 3600
   gaccttctat atgaggcgag tgggtctcag tctgcttgaa tggtgatgag attctgctgg 3660
   atctcagcac gctgcaggtg tcttttgaga gcattcagta ggacatggtg atccctattt 3720
   cagcctctaa gatgactggt attctatctg aaatgcagag attaagccaa atacctgatg 3780
   tattgtgaaa gccactgatt ttaagaatgg agagaaaggg attttttact gcatccctct 3840
   gtatgaatat gaaatcagag accagggcat gatgttgcta ggattagagc ctctcagtct 3900
   ggcctcttca cccaagtgca agaactcagt ctcttactgt tcaaagaatc ttaacagttg 3960
   aattatggag ggaaattccc ttttgcccca agcatttcta tatttaaagc aatatcccag 4020
   gagaatatgt tagacttagg atgatacctt cagccacttg aagaagaaat agaaggcgct 4080
   cattccaata tagtctttat ttcccattca gatacaggtt gagcatccct aatctgaaca 4140
   gttaaaaccc ccaaatgccc caaaatccaa accttcctga acgctatgac accatgagtg 4200
   gaaaattcca cacctaacaa acacatttgc tttcttatgg ttcaatgtac acaaactgtt 4260
   ttatatagaa aatgatttca aatatcataa aattaccttc aggctatgtg tataaagtat 4320
   atatgagcca taaatgaatt ttgtgtttag actttgtgtc catccccaag atctctcatt 4380
   ttatatatat atatatatat atatatatat atatatatat atatatatac acacacacac 4440
   acatacacaa atattccagg atacaaaaaa aaacatttaa aaatccgaga cccagaacac 4500
   ttctggtccc aagcatttca gataagggat atcaatctgt actaccaata aggatttcgt 4560
   aattccccta actgcaaatg tcctcttcat ttgttcttta tgagaaaacc cgggtagtgc 4620
   cagcacctgg atacagtatt tacaccctgc agaccctaaa gatttcagat tcagttagca 4680
   aaccttgatg aagcacctgc tggacactga gggacccaaa gctcaatcag ccataatccc 4740
   tgctttcaga gtttatattg tacctgccta atccacccgg cgtgactcat ttcaacacta 4800
   agtactaggg gtgttgtcag gagacaaatc tgaagtcagg agaggaaaat gcaaaggagc 4860
   cctgccgtgt gatggatgtg cattctcact tgggtcttga agttctcatt cctacatctc 4920
   aagctagcca ggcagtctcc tctctatcag aagaaagcac tggtaattgg ctagactggc 4980
   tatgttgaag gtaacatgaa ctctaagatc ttgacccagg gcgacttggt tttgcttaag 5040
   gtggcatcac caatgttcca aatcctttag ggagatgagg gtatccccac agaaaaagag 5100
   gaataataga ccaatggatt ttctcctttc accagtatgt ttggaaccct ctgatccaat 5160
   gtcctttgat actgatctct tgtccaaatg agaatgtcgc tttagctgaa attcaaatgg 5220
   ctgtgacaat ttaccgaaat gatgaagtaa ccaccattcc cacctttcac tgcctaggct 5280
   ccaagtctga atacattttt gaaataggaa ctcccttttg caaaaaagaa acctgggtgt 5340
   cagggaggtg aagtgacttg ccctaggagc agacagcatg ccaagaatgg aattaggctc 5400
   aggatccagc ctgggctcac cctgtgtggc tcattcccac ccaggaaact gaagataaaa 5460
   gatttgggaa aacacaccaa gaaaaagggg cagttttctt tgcccaagca tttggtgcta 5520
   gttagaggct gttcactctc tcctgctcct cttcggagta gaaataaagg ctgtgacaca 5580
   aggaagccag tggggtggga gggaggcacc ataatccctc cctaaaaccc acagaagact 5640
   aacctgatac tcttttgacc caactgcatc aacactaaac agctgcagac cccctgaatc 5700
   tttcacacat gccaagtgaa cattcttgat gatttctctt tgtgaccgca accacctgca 5760
   aaccagaacg actctagaat ttccttcccc gccccccttt ttgtttagtt tctaatctct 5820
   tgtttatgag gtgtggggtt tataagggac tgaatcaaat gaatgtaaca aaaaagaaaa 5880
   aaaaaacaaa aaaaaatgcc ttttctcagg gccagtgagt tgcaaataat ttttaaagaa 5940
   aagcctataa ttacatcatc tcaataaatt ttttataaaa aaaaaaaaaa aa 5992
<210> 38
   <211> 1670
   <212> DNA
   <213> Homo sapiens
<400> 38
   gatttcctcc cacgcgacct tccagttctc ggagccaggt taggggtttg gcggaggagg 60
   actgcggggc gcgggcctag ggccccagca gccacggcca ggggagcgct caagacagaa 120
   agccggtggc ttcctcacct ccacctgtaa tgcaggaggg agaattggct atttctccta 180
   taagccctgt ggcagccatg cctcccctag gcacccacgt gcaagccaga tgtgaagctc 240
   aaattaacct gctgggtgaa ggggggatct gcaagctgcc aggaagactc cgcatccagc 300
   ccgcactgtg gagcagggag gacgtgctgc actggctgcg ctgggcagag caggagtact 360
   ctctgccatg caccgcggag cacgggttcg agatgaacgg acgcgccctc tgcatcctca 420
   ccaaggacga cttccggcac cgtgcgccca gctcaggtga cgtcctgtat gagctgctcc 480
   agtacatcaa gacccagcgg cgagccctgg tgtgtggacc cttttttgga gggatcttca 540
   ggctgaagac gcccacccag cactctccag tccccccgga agaggtgact ggcccctctc 600
   agatggacac ccgaaggggc cacctgctgc agccaccaga cccagggctt accagcaact 660
   tcggccacct ggatgaccct ggcctggcaa ggtggacccc tggcaaggag gagtccctca 720
   acttatgtca ctgtgcagag ctcggctgca ggacccaggg ggtctgttcc ttccccgcga 780
   tgccgcaggc ccccattgac ggcaggatcg ctgactgccg cctgctgtgg gattacgtgt 840
   atcagctgct ccttgatacc cgatatgagc cctacatcaa gtgggaagac aaggacgcca 900
   agatcttccg agttgtggat ccaaatgggc tcgccagact ctggggaaat cacaagaacc 960
   gggtgaacat gacctacgag aagatgtctc gtgccctgcg ccactattat aagcttaata 1020
   tcattaagaa ggaaccgggg cagaaactcc tgttcagatt tctaaagact ccgggaaaga 1080
   tggtccagga caagcacagc cacctggagc cgctggagag ccaggagcag gacagaatag 1140
   agttcaagga caagaggcca gaaatctctc cgtgaggggc aggtggactc caggcacccg 1200
   gtaccgatgg ggcagggacc gagtctccca tgaaggcaga ctcctcctcc cagcagagca 1260
   gcaggatccc cagccagact ctgtacccac aggattacag ccattgcttg ggaaggctgg 1320
   gaggcctccc atccaggaca ctgggggcag gagtgtcatc ttttgggcag ggcaatcctg 1380
   gggctaaatg aggtacaggg gaatggactc tcccctactg cacccctggg agaggaagcc 1440
   aggcaccgat agagcaccca gccccacccc tgtaaatgga atttaccaga tgaagggaat 1500
   gaagtccctc actgagcctc agatttcctc acctgtgaaa tgggctgagg caggaaatgg 1560
   gaaaaagtgt tagtgcttcc aggcggcact gacagcctca gtaacaataa aaacaatggt 1620
   agctgaaaaa aaaaaaaaaa aaaaaaaaaa aaaaaaaaaa aaaaaaaaaa 1670
<210> 39
   <211> 4071
   <212> DNA
   <213> Homo sapiens
<400> 39
   gagtccagcc gctggtgcgc ggagcggttc accgtcttcg gagcggttcg gcccagcctt      60
   tcgcccaggc gcccaggccc gctgcgcgcg tgcgtgagcg cgcctgcgcc gccagggccg     120
   ctgcaagggg aggagagcgg ccgcctcagg aggatccctt ttcccccaga aattactcaa     180
   tgctgaaacc tctcaaagtg gtattagaga cgctgaaagc accatggacg ggttttatga     240
   tcagcaagtc ccttttatgg tcccagggaa atctcgatct gaggaatgca gagggcggcc     300
   tgtgattgac agaaagagga agtttttgga cacagatctg gctcacgatt ctgaagagct     360
   atttcaggat ctcagtcaac ttcaagaggc ttggttagct gaagcacaag ttcctgatga      420
   tgaacagttt gtcccagatt ttcagtctga taacctggtg cttcatgccc cacctccaac      480
   caagatcaaa cgggagctgc acagcccctc ctctgagctg tcgtcttgta gccatgagca      540
   ggctcttggt gctaactatg gagaaaagtg cctctacaac tattgtgcct atgataggaa      600
   gcctccctct gggttcaagc cattaacccc tcctacaacc cccctctcac ccacccatca      660
   gaatccccta tttcccccac ctcaggcaac tctgcccacc tcagggcatg cccctgcagc      720
   tggcccagtt caaggtgtgg gccccgcccc cgccccccat tcgcttccag agcctggacc      780
   acagcagcaa acatttgcgg tcccccgacc accacatcag cccctgcaga tgccaaagat      840
   gatgcctgaa aaccagtatc catcagaaca gagatttcag agacaactgt ctgaaccctg      900
   ccaccccttc cctcctcagc caggagttcc tggagataat cgccccagtt accatcggca      960
   aatgtcagaa cctattgtcc ctgcagctcc cccgccccct cagggattca aacaagaata    1020
   ccatgaccca ctctatgaac atggggtccc gggcatgcca gggcccccag cacacgggtt     1080
   ccagtcacca atgggaatca agcaggagcc tcgggattac tgcgtcgatt cagaagtgcc     1140
   taactgccag tcatcctaca tgagaggggg ttatttctcc agcagccatg aaggtttttc     1200
   atatgaaaaa gatccccgat tatactttga cgacacttgt gttgtgcctg agagactgga     1260
   aggcaaagtc aaacaggagc ctaccatgta tcgagagggg cccccttacc agaggcgagg    1320
   ttcccttcag ctgtggcagt tcctggtcac ccttcttgat gacccagcca atgcccactt     1380
   cattgcctgg acaggtcgag gcatggagtt caagctgata gaaccggaag aggttgctcg     1440
   gcgctggggc atccagaaga accggccagc catgaactat gacaagctga gccgctctct     1500
   ccgctattac tatgaaaagg gcatcatgca gaaggtggct ggagagcgat acgtctacaa     1560
   atttgtctgt gacccagatg ccctcttctc catggctttc ccggataacc agcgtccgtt     1620
   cctgaaggca gagtccgagt gccacctcag cgaggaggac accctgccgc tgacccactt     1680
   tgaagacagc cccgcttacc tcctggacat ggaccgctgc agcagcctcc cctatgccga     1740
   aggctttgct tactaagttt ctgagtggcg gagtggccaa accctagagc tagcagttcc     1800
   cattcaggca aacaagggca gtggttttgt ttgtgttttt ggttgttcct aaagcttgcc     1860
   ctttgagtat tatctggaga acccaagctg tctctggatt ggcaccctta aagacagata     1920
   cattggctgg ggagtgggaa cagggagggg cagaaaacca ccaaaaggcc agtgcctcaa     1980
   ctcttgattc tgatgaggtt tctgggaaga gatcaaaatg gagtctcctt accatggaca     2040
   atacatgcaa agcaatatct tgttcaggtt agtacccgca aaacgggaca tgatgtgaca     2100
   atctcgatcg atcatggact actaaatggc ctttacatag aagggctctg atttgcacaa     2160
   tttgttgaaa aatcacaaac ccatagaaaa gtgagtaggc taagttgggg aggctcaaac     2220
   cattaagggt taaaaataca tcttaaacat tggaaagctc ttctagctga atctgaaata     2280
   ttaccccttg tctagaaaaa ggggggcagt cagaacagct gttccccact ccgtgttctc      2340
   aaaatcataa accatggcta ctcttgggaa ccacccggcc atgtggtcgc caagtagagc 2400
   aagccccctt tctcttccca atcacgtggc tgagtgtgga tgacttttat tttaggagaa 2460
   gggcgattaa cacttttgac agtattttgt tttgccctga tttgggggat tgttttgttt 2520
   tggtggttgt tttggaaaaa cagtttataa actgattttt gtagttttgg tatttaaagc 2580
   aaaaaaacga aaaacaaaaa acaaaaacaa accttttggt aatgtgcact gtgtctttag 2640
   ccagggccgt gcaacttatg aagacactgc agcttgagag gggctttgct gaggcttccc 2700
   cttggccatg tgaaagcccg ccttgttgcc tgctttgtgc tttctgcacc agacaacctg 2760
   atggaacatt tgcacctgag ttgtacattt ttgaagtgtg cagggcagcc tggacacaag 2820
   cttagattct ctatgtatag ttccccgtgt tcactaacat gccctctctg gaaagcatat 2880
   gtatataaca tgtgtcatgt cctttggaaa cctggtcacc tggtgaaaac ccttgggatt 2940
   cttccctggg catgactgat gacaatttcc atttcatcag tttgttttgt tttccttttt 3000
   ctttaaatct tggactttaa accctacctg tgtgattcag tagggtttga gacttagctg 3060
   tgatactgac aggtaagcaa cagtgctagc attctagatt cctgcctttt tttaaaaaga 3120
   aattattctc attgctgtat tatattggaa aagttttaaa caaccaagct aaagctatgt 3180
   gaaagttgag ctcaaagtag aggaaaagtt actggtggta ccttgctgcc tgctctgctg 3240
   gtagaattct gtgctccccg tgacacttag tacattaaga atgactacac tgttcctcgt 3300
   atgtgaagga ggcagtgctg actccgtgag tgtgagacac gtgctttgaa ctgcttttct 3360
   attcatggag cactccatag tctcaaactg tcccccttat gaccaacagc acatttgtga 3420
   agaggttcgc agggataagg ggtgcacttt atagctatgg aaacatgaga ttctcctcta 3480
   ttggaagcta attagcccac aaaggtggta aacctgtaga ttgggcctta attagcattg 3540
   tactctaatc aaaggactct ttctaaacca tatttatagc tttcttaacc tacacatagt 3600
   ctatacatag atgcatattt tacccccagc tggctagaga tttatttgtt gtaaatgctg 3660
   tatagatttg gttttccttt ctttacttac cctggtttgg attttttttt tttttttttt 3720
   tgaatggatt tatgctgtct tagcaatatg acaataatcc tctgtagctt gagctacccc 3780
   tcccctgctg taacttacgt gacctgtgct gtcactgggc ataggacagc ggcatcacgg 3840
   ttgcattccc attggactca tgcacctccc ggatggtttt tgtttttttc gggggttctt 3900
   tggggtttgt ttgtttgctt cttttccaga gtgtggaaag tctacagtgc agaaaggctt 3960
   gaacctgcca gctgatttga aatactttca ccctgcgcag ggccgtatgc atcctgccaa 4020
   gctgcgttat attctgtact gtgtacaata aagaagtttg cttttcgttt a 4071
<210> 40
   <211> 3499
   <212> DNA
   <213> Homo sapiens
<400> 40
   gccaagaagc ttgagagaag aaaaatttca gaaaaattgt ctcaatttga ctagaatatc      60
   aatgaaccag gaaaactgaa gcaccttccc taaagaaaac ttgggtatac aattactcca     120
   cagacagagc tgagggtttt ttacccaaat cagtcactgg attttgctgc ctgatacgtg     180
   aatcttcttg gaatttttct catgtggatc taaggggaat gctttattat ggctgctgtt     240
   gtccaacaga acgacctagt atttgaattt gctagtaacg tcatggagga tgaacgacag     300
   cttggtgatc cagctatttt tcctgccgta attgtggaac atgttcctgg tgctgatatt     360
   ctcaatagtt atgccggtct agcctgtgtg gaagagccca atgacatgat tactgagagt     420
   tcactggatg ttgctgaaga agaaatcata gacgatgatg atgatgacat cacccttaca     480
   gttgaagctt cttgtcatga cggggatgaa acaattgaaa ctattgaggc tgctgaggca     540
   ctcctcaata tggattcccc tggccctatg ctggatgaaa aacgaataaa taataatata     600
   tttagttcac ctgaagatga catggttgtt gccccagtca cccatgtgtc cgtcacatta     660
   gatgggattc ctgaagtgat ggaaacacag caggtgcaag aaaaatatgc agactcaccg     720
   ggagcctcat caccagaaca gcctaagagg aaaaaaggaa gaaaaactaa accaccacga     780
   ccagattccc cagccactac gccaaatata tctgtgaaga agaaaaacaa agatggaaag     840
   ggaaacacaa tttatctttg ggagttttta ctggcactgc tccaggacaa ggctacttgt     900
   cctaaataca tcaagtggac ccagcgagag aaaggcattt ttaaattggt ggattctaaa     960
   gcagtgtcca ggttgtgggg gaagcacaaa aacaaacctg atatgaatta tgagaccatg    1020
   ggaagagcac tcaggtacta ttaccaaagg ggtattctgg caaaagtgga aggtcagcgc    1080
   ttggtgtatc agtttaaaga aatgccaaaa gatcttatat atataaatga tgaggatcca    1140
   agttccagca tagagtcttc agatccatca ctatcttcat cagccacttc aaataggaat    1200
   caaaccagcc ggtcgagagt atcttcaagt ccaggggtaa aaggaggagc cactacagtt    1260
   ctaaaaccag ggaattctaa agctgcaaaa cccaaagatc ctgtggaagt tgcacaacca    1320
   tcagaagttt tgaggacagt gcagcccacg cagtctccat atcctaccca gctcttccgg    1380
   actgttcatg tagtacagcc agtacaggct gtcccagagg gagaagcagc tagaaccagt    1440
   accatgcagg atgaaacatt aaattcttcc gttcagagta ttaggactat acaggctcca    1500
   acccaagttc cagtggttgt gtctcctagg aatcagcagt tgcatacagt aacactccaa    1560
   acagtgccac tcacaacagt tatagccagc acagatccat cagcaggtac tggatctcag    1620
   aagtttattt tacaagccat tccatcatca cagcccatga cagtactgaa agaaaatgtc    1680
   atgctgcagt cacaaaaggc gggctctcct ccttcaattg tcttgggccc tgcccaggtt    1740
   cagcaggtcc ttactagcaa tgttcagacc atttgcaatg gaaccgtcag tgtggcttcc    1800
   tctccatcct tcagtgctac tgcacctgtg gtgacctttt ctcctcgcag ttcacagctg    1860
   gttgctcacc cacctggcac tgtaatcact tcagttatca aaactcaaga aacaaaaact    1920
   cttacacagg aagtagagaa aaaggaatct gaagatcatt tgaaagagaa cactgagaaa 1980
   acggagcagc agccacagcc ttatgtgatg gtagtgtcca gttccaatgg atttacttct 2040
   caggtagcta tgaaacaaaa cgaactgctg gaacccaact ctttttagtt aatataccaa 2100
   agcttatgaa taattgtttg ttaattgaac attttcaatt atatgcagac tgactgattc 2160
   taagataaat tctaaggagg tttctaattt tgtaattgtt aaaaatagag ttaattttga 2220
   ctttgttaga tgagggagga aaactcaact gtttctcttt gttatctaaa tgtttcagaa 2280
   ttcaatcgtg aaggaacagg cattttacac tatgaagaca ttcttttgag atttttattt 2340
   cagttgctat atcataagca tttttaaagt ttcttttcta attttacatt gtattagatt 2400
   ttctgattct tttgtaaata cagaacttaa atagaaggca acaggaaatt tatataggaa 2460
   ctattttcat tccacttgtg taagttaagt cttgactctt tcaaatgcaa aaaacctatt 2520
   ttatgctttg ttaaaattat ggtgtcactt agattgactt tagttgactg cactatataa 2580
   tatagaacta tgaatatgta gaataacatg aaaaattgga ggtgctggtg gtatggctga 2640
   ccctgtttca gaagcaggat agtataaaag catcagccta agaatggcac tcccactaac 2700
   tagctatgta atcttgacct ctttgggctt tagttcctct cataaaagga agagatgtat 2760
   tggattagac tagattatca ccactttctc ttctagttct aattttttta attctaatac 2820
   ctatattttc aagttatgtc aattaaatca ttatcaggtt atttcctaat gtaagaatag 2880
   ctaaaatgtt gcagagaaat aagtgaccca acaaaattta ttcatctgtt atgggtaaga 2940
   tctgccataa attcttccta aataatttgt ttactaactc tttaggccac tgtgctttgc 3000
   ggtccattag taaacttgtg ttgctaagtg ctaaacagaa tactgctatt ttgagagagt 3060
   caagactctt tcttaagggc caagaaagca acttgagcct tgggctaatc tggctgagta 3120
   gtcagttata aaagcataat tgctttatat tttggatcat tttttactgg gggcggactt 3180
   ggggggggtt gcatacaaag ataacatata tatccaactt tctgaaatga aatgttttta 3240
   gattactttt tcaactgtaa ataatgtaca tttaatgtca caagaaaaaa atgtcttctg 3300
   caaattttct agtataacag aaatttttgt agatgaaaaa aatcattatg tttagaggtc 3360
   taatgctatg ttttcatatt acagagtgaa tttgtattta aacaaaaatt taaattttgg 3420
   aatcctctaa acatttttgt atctttaatt ggtttattat taaataaatc atataaaaat 3480
   tctcaaaaaa aaaaaaaaa 3499
<210> 41
   <211> 2212
   <212> DNA
   <213> Homo sapiens
<400> 41
   gcccggctcc tgggagcagg tctcggcccc cgcttggggc cccggccgtg cggccggagg 60
   gagcggccgg atggagcgga ggatgaaagc cggatacttg gaccagcaag tgccctacac 120
   cttcagcagc aaatcgcccg gaaatgggag cttgcgcgaa gcgctgatcg gcccgctggg 180
   gaagctcatg gacccgggct ccctgccgcc cctcgactct gaagatctct tccaggatct 240
   aagtcacttc caggagacgt ggctcgctga agctcaggta ccagacagtg atgagcagtt 300
   tgttcctgat ttccattcag aaaacctagc tttccacagc cccaccacca ggatcaagaa 360
   ggagccccag agtccccgca cagacccggc cctgtcctgc agcaggaagc cgccactccc 420
   ctaccaccat ggcgagcagt gcctttactc cagtgcctat gaccccccca gacaaatcgc 480
   catcaagtcc cctgcccctg gtgcccttgg acagtcgccc ctacagccct ttccccgggc 540
   agagcaacgg aatttcctga gatcctctgg cacctcccag ccccaccctg gccatgggta 600
   cctcggggaa catagctccg tcttccagca gcccctggac atttgccact ccttcacatc 660
   tcagggaggg ggccgggaac cctcccagcc cccctaccaa caccagctgt cggagccctg 720
   cccaccctat ccccagcaga gctttaagca agaataccat gatcccctgt atgaacaggc 780
   gggccagcca gccgtggacc agggtggggt caatgggcac aggtacccag gggcgggggt 840
   ggtgatcaaa caggaacaga cggacttcgc ctacgactca gatgtcaccg ggtgcgcatc 900
   aatgtacctc cacacagagg gcttctctgg gccctctcca ggtgacgggg ccatgggcta 960
   tggctatgag aaacctctgc gaccattccc agatgatgtc tgcgttgtcc ctgagaaatt 1020
   tgaaggagac atcaagcagg aaggggtcgg tgcatttcga gaggggccgc cctaccagcg 1080
   ccggggtgcc ctgcagctgt ggcaatttct ggtggccttg ctggatgacc caacaaatgc 1140
   ccatttcatt gcctggacgg gccggggaat ggagttcaag ctcattgagc ctgaggaggt 1200
   cgccaggctc tggggcatcc agaagaaccg gccagccatg aattacgaca agctgagccg 1260
   ctcgctccga tactattatg agaaaggcat catgcagaag gtggctggtg agcgttacgt 1320
   gtacaagttt gtgtgtgagc ccgaggccct cttctctttg gccttcccgg acaatcagcg 1380
   tccagctctc aaggctgagt ttgaccggcc tgtcagtgag gaggacacag tccctttgtc 1440
   ccacttggat gagagccccg cctacctccc agagctggct ggccccgccc agccatttgg 1500
   ccccaagggt ggctactctt actagccccc agcggctgtt ccccctgccg caggtgggtg 1560
   ctgccctgtg tacatataaa tgaatctggt gttggggaaa ccttcatctg aaacccacag 1620
   atgtctctgg ggcagatccc cactgtccta ccagttgccc tagcccagac tctgagctgc 1680
   tcaccggagt cattgggaag gaaaagtgga gaaatggcaa gtctagagtc tcagaaactc 1740
   ccctgggggt ttcacctggg ccctggagga attcagctca gcttcttcct aggtccaagc 1800
   cccccacacc ttttccccaa ccacagagaa caagagtttg ttctgttctg ggggacagag 1860
   aaggcgcttc ccaacttcat actggcagga gggtgaggag gttcactgag ctccccagat 1920
   ctcccactgc ggggagacag aagcctggac tctgccccac gctgtggccc tggagggtac 1980
   cggtttgtca gttcttggtg ctctgtgttc ccagaggcag gcggaggttg aagaaaggaa 2040
   cctgggatga ggggtgctgg gtataagcag agagggatgg gttcctgctc caagggaccc 2100
   tttgcctttc ttctgccctt tcctaggccc aggcctgggt ttgtacttcc acctccacca     2160
   catctgccag accttaataa aggcccccac ttctcccaaa aaaaaaaaaa aa             2212
<210> 42
   <211> 2667
   <212> DNA
   <213> Homo sapiens
<400> 42
   tctgagaggc gaggccgggt gaggcggcga gggcggcccg acgggcgcgg gacgggacgg      60
   ggcagcgagg gcgccgggag ccgcggcccg gaatcggggc gcttcgcccc gggcccccca     120
   gcatgaagac cccggcggac acagggtttg ccttcccgga ttgggcctac aagccagagt     180
   cgtcccctgg ctcaaggcag atccagctgt ggcactttat cctggagctg ctgcggaagg     240
   aggagtacca gggcgtcatt gcctggcagg gggactacgg ggaattcgtc atcaaagacc     300
   ctgatgaggt ggcccggctg tggggcgttc gcaagtgcaa gccccagatg aattacgaca     360
   agctgagccg ggccctgcgc tattactata acaagcgcat tctgcacaag accaagggga     420
   aacggttcac ctacaagttc aatttcaaca aactggtgct ggtcaattac ccattcattg     480
   atgtggggtt ggctgggggt gcagtgcccc agagtgcccc gccagtgccg tcgggtggta     540
   gccacttccg cttccctccc tcaacgccct ccgaggtgct gtcccccacc gaggaccccc     600
   gctcaccacc agcctgctct tcatcttcat cttccctctt ctcggctgtg gtggcccgcc     660
   gcctgggccg aggctcagtc agtgactgta gtgatggcac gtcagagctg gaggaaccgc     720
   tgggagagga tccccgcgcc cgaccacccg gccctccgga tctgggtgcc ttccgagggc     780
   ccccgctggc ccgcctgccc catgaccctg gtgtcttccg agtctatccc cggcctcggg     840
   gtggccctga acccctcagc cccttccctg tgtcgcctct ggccggtcct ggatccctgc     900
   tgccccctca gctctccccg gctctgccca tgacgcccac ccacctggcc tacactccct     960
   cgcccacgct gagcccgatg taccccagtg gtggcggggg gcccagcggc tcagggggag    1020
   gctcccactt ctccttcagc cctgaggaca tgaaacggta cctgcaggcc cacacccaaa    1080
   gcgtctacaa ctaccacctc agcccccgcg ccttcctgca ctaccctggg ctggtggtgc    1140
   cccagcccca gcgccctgac aagtgcccgc tgccgcccat ggcacccgag accccaccgg    1200
   tcccctcctc ggcctcgtca tcctcttctt cttcttcctc cccattcaag tttaagctcc    1260
   agcggccccc actcggacgc cggcagcggg cagctgggga gaaggccgta gccgctgctg    1320
   acaagagcgg tggcagtgca ggcgggctgg ctgagggggc aggggcgcta gccccaccgc    1380
   ccccgccacc acagatcaag gtggagccca tctcggaagg cgagtcggag gaggtagagg    1440
   tgactgacat cagtgatgag gatgaggaag acggggaggt gttcaagacg ccccgtgccc    1500
   cacctgcacc ccctaagcct gagcccggcg aggcacccgg ggcatcccag tgcatgcccc    1560
   tcaagctacg ctttaagcgg cgctggagtg aagactgtcg cctcgaaggg ggtgggggcc    1620
   ccgctggggg ctttgaggat gagggtgagg acaagaaggt gcgtggggag gggcctgggg 1680
   aggctggggg gcccctcacc ccaaggcggg tgagctctga cctccagcat gccacggccc 1740
   agctctccct ggagcaccga gactcctgag ggctgtgggc aggggacctg tgtgccccgc 1800
   accccccatg cttcttttgc tgccttaagc cccctatgcc ctggaggtga gggcagctct 1860
   cttgtctctt ccctgcctcc tcccttttcc ctccccacat tttgtataaa actttaattt 1920
   ctttttttta aaaatggtgg gggtgggtgg gtgcccaggg ctaggggcta ttccctgtct 1980
   ctgtgggttt ctaagctctg ggcaaattgg tggtaggggg agggaggggg aagttaaggg 2040
   ggtcacctcc attctgggga atttatattt gaattgaggc tttggcctta acacccagga 2100
   acttttctat tacaatcgct taggaagtaa agccttgtct ccctccctgt tctctgcctc 2160
   ttgtacccct ctgacccacc cgctctgccc cactcccagc cctcctcagc cccagccctg 2220
   cctgccctgc ccctccaggg ggccatgagt gcctaggttt ctcatacccc acaaggtcac 2280
   agcaggggag ggagggacaa ttttataatg aaccaaaaat tccatgtgtt ggggggtggg 2340
   gggcggagga gggtgagggg tgccgcccat gggccacaaa tctctacaag tgcctgctat 2400
   ccctctccca ctccccaccc cagcaccggt ccaacccctt catccccagc tgctcctagg 2460
   actggcccat gggcaggcgg gtggggggat gggaaggggg tgccctgaaa ccaaactgga 2520
   agccccctct gcctcccagc tggggcctct ggggtggggt ggggggctgt ggtcaagcct 2580
   tattctgtat tggggactga gggtgggggg agtagagggg ccgctggaga atgtattcaa 2640
   aacaataaac tttggacctt tggaaaa 2667
<210> 43
   <211> 1364
   <212> DNA
   <213> Homo sapiens
<400> 43
   aaaatcagga acttgtgctg gccctgcaat gtcaagggag ggggctcacc cagggctcct 60
   gtagctcagg gggcaggcct gagccctgca cccgccccac gaccgtccag cccctgacgg 120
   gcaccccatc ctgaggggct ctgcattggc ccccaccgag gcaggggatc tgaccgactc 180
   ggagcccggc tggatgttac aggcgtgcaa aatggaaggg tttcccctcg tcccccctcc 240
   atcagaagac ctggtgccct atgacacgga tctataccaa cgccaaacgc acgagtatta 300
   cccctatctc agcagtgatg gggagagcca tagcgaccat tactgggact tccaccccca 360
   ccacgtgcac agcgagttcg agagcttcgc cgagaacaac ttcacggagc tccagagcgt 420
   gcagcccccg cagctgcagc agctctaccg ccacatggag ctggagcaga tgcacgtcct 480
   cgataccccc atggtgccac cccatcccag tcttggccac caggtctcct acctgccccg 540
   gatgtgcctc cagtacccat ccctgtcccc agcccagccc agctcagatg aggaggaggg 600
   cgagcggcag agccccccac tggaggtgtc tgacggcgag gcggatggcc tggagcccgg 660
   gcctgggctc ctgcctgggg agacaggcag caagaagaag atccgcctgt accagttcct 720
   gttggacctg ctccgcagcg gcgacatgaa ggacagcatc tggtgggtgg acaaggacaa 780
   gggcaccttc cagttctcgt ccaagcacaa ggaggcgctg gcgcaccgct ggggcatcca 840
   gaagggcaac cgcaagaaga tgacctacca gaagatggcg cgcgcgctgc gcaactacgg 900
   caagacgggc gaggtcaaga aggtgaagaa gaagctcacc taccagttca gcggcgaagt 960
   gctgggccgc gggggcctgg ccgagcggcg ccacccgccc cactgagccc gcagcccccg 1020
   ccggccccgc caggcctccc cgctggccat agcattaagc cctcgcccgg cccggacaca 1080
   gggaggacgc tcccggggcc cagaggcagg actgtggcgg gccgggctcc gtcacccgcc 1140
   cctcccccca ctccaggccc cctccacatc ccgcttcgcc tccctccagg actccacccc 1200
   ggctcccgac gccagctggg cgtcagaccc accggcaacc ttgcagagga cgacccgggg 1260
   tactgccttg ggagtctcaa gtccgtatgt aaatcagatc tcccctctca cccctcccac 1320
   ccattaacct cctcccaaaa aacaagtaaa gttattctca atcc 1364
<210> 44
   <211> 3034
   <212> DNA
   <213> Homo sapiens
<400> 44
   tttcttgtta aacaaacacc taatttattt cttggaggtt ttgttcagct gtcctaattt      60
   atgactttac attccttctg gtgctaaact gctcaagtag cctcttgtat caagtgtgac     120
   ctgattcctt aagaatttta cttaatgaga acctctaagc tagaaactct tgctaggtgt     180
   ttcatgcacc ttattttctt taatcattac aacaactcta agattgggtt ctctccacct     240
   tataaatgat gactgtttta gagaggttaa ggttgcttaa aattggtgag ttagtgaggg     300
   gtagagccac gaatggattt ctggtcgctg cctccatcgt cagggcaagc ttttcccacg     360
   actccagcgc ttccatttgt cagtccccag gctagaaagc cacagtgcta atttagtatt     420
   tatcaagcgt ttgtagtgtc ctgggatctg gcacttcgat gagaaagctg tgacggcccc     480
   aacttctaac agcgagtggt aaggaggacg agggacacag gagggaggag actctcccca     540
   aagcttagca ccaacagaag tggtcccccg caggttgctc tgcgagcgcc acctcttccc     600
   tccaaccgag gagaaagtgg cgcgcctttg aggagtccga ggtcccggcc caggcggcag     660
   cttgggtcct ggcgggttcc ggacgggcgc ctcagggacc tggaagcaac cgcaccgaac     720
   gcgacggaga gcggcgagac gactccagga ggcgcccgag ctacatcccc cggccacacc     780
   aaacccgggt ttgctggcag acgcggctca cgacacccct tagggtcgca gcccctcccc     840
   cggaagtgac gtgtagcgac tacggcgtct gggagggacc caggagcagt cggggggttt     900
   gagagtggcg gcggccgcgg agggcctggc aggccccgcc gctgcaagga acgccccgaa     960
   cgcgcgcgcc cggcgtgtag cggccccaag acccgcgccg ccgctgccgc gtgcgggggc    1020
   ggggagggcg gggcgccagg agccgcggcg gcgggagatg cgggcggctg cgggcacccg 1080
   gcgggctcgg cttggccgcc gccgccttct acggctccgc cgcgggggtc gcagcggctg 1140
   ccgcgccgtc ctcgagtttc cagcgtgagg aggaggctga gggcggagag gcgcatcgtg 1200
   ttcgaggcgg agaccgaggg ggagccccgc gcgcggcgtc gctcattgct atggacagtg 1260
   ctatcaccct gtggcagttc cttcttcagc tcctgcagaa gcctcagaac aagcacatga 1320
   tctgttggac ctctaatgat gggcagttta agcttttgca ggcagaagag gtggctcgtc 1380
   tctgggggat tcgcaagaac aagcctaaca tgaattatga caaactcagc cgagccctca 1440
   gatactatta tgtaaagaat atcatcaaaa aagtgaatgg tcagaagttt gtgtacaagt 1500
   ttgtctctta tccagagatt ttgaacatgg atccaatgac agtgggcagg attgagggtg 1560
   actgtgaaag tttaaacttc agtgaagtca gcagcagttc caaagatgtg gagaatggag 1620
   ggaaagataa accacctcag cctggtgcca agacctctag ccgcaatgac tacatacact 1680
   ctggcttata ttcttcattt actctcaact ctttgaactc ctccaatgta aagcttttca 1740
   aattgataaa gactgagaat ccagccgaga aactggcaga gaaaaaatct cctcaggagc 1800
   ccacaccatc tgtcatcaaa tttgtcacga caccttccaa aaagccaccg gttgaacctg 1860
   ttgctgccac catttcaatt ggcccaagta tttctccatc ttcagaagaa actatccaag 1920
   ctttggagac attggtttcc ccaaaactgc cttccctgga agccccaacc tctgcctcta 1980
   acgtaatgac tgcttttgcc accacaccac ccatttcgtc cataccccct ttgcaggaac 2040
   ctcccagaac accttcacca ccactgagtt ctcacccaga catcgacaca gacattgatt 2100
   cagtggcttc tcagccaatg gaacttccag agaatttgtc actggagcct aaagaccagg 2160
   attcagtctt gctagaaaag gacaaagtaa ataattcatc aagatccaag aaacccaaag 2220
   ggttagaact ggcacccacc cttgtgatca cgagcagtga tccaagccca ctgggaatac 2280
   tgagcccatc tctccctaca gcttctctta caccagcatt tttttcacag acacccatca 2340
   tactgactcc aagccccttg ctctccagta tccacttctg gagtactctc agtcctgttg 2400
   ctcccctaag tccagccaga ctgcaaggtg ctaacacact tttccagttt ccttctgtac 2460
   tgaacagtca tgggccattc actctgtctg ggctggatgg accttccacc cctggcccat 2520
   tttccccaga cctacagaag acataaccta tgcacttgtg gaatgagaga accgaggaac 2580
   gaagaaacag acattcaaca tgattgcatt tgaagtgagc aattgatagt tctacaatgc 2640
   tgataataga ctattgtgat ttttgccatt ccccattgaa aacatctttt taggattctc 2700
   tttgaatagg actcaagttg gactatatgt ataaaaatgc cttaattgga gtctaaactc 2760
   cacctccctc tgtcttttcc ttttcttttt ctttccttcc ttccttttct tttctccttt 2820
   aaaaatattt tgagctttgt gctgaagaag tttttggtgg gctttagtga ctgtgctttg 2880
   caaaagcaat taagaacaaa gttactcctt ctggctattg ggaccctttg gccaggaaaa 2940
   attatgctta gaatctatta tttaaagaaa tatttgtgaa atgaaaaaaa aaaaaaaaaa 3000
   aaaaaaaaaa aaaaaaaaaa aaaaaaaaaa aaaa 3034
<210> 45
   <211> 3077
   <212> DNA
   <213> Homo sapiens
<400> 45
   tttcttgtta aacaaacacc taatttattt cttggaggtt ttgttcagct gtcctaattt      60
   atgactttac attccttctg gtgctaaact gctcaagtag cctcttgtat caagtgtgac     120
   ctgattcctt aagaatttta cttaatgaga acctctaagc tagaaactct tgctaggtgt     180
   ttcatgcacc ttattttctt taatcattac aacaactcta agattgggtt ctctccacct     240
   tataaatgat gactgtttta gagaggttaa ggttgcttaa aattggtgag ttagtgaggg     300
   gtagagccac gaatggattt ctggtcgctg cctccatcgt cagggcaagc ttttcccacg     360
   actccagcgc ttccatttgt cagtccccag gctagaaagc cacagtgcta atttagtatt     420
   tatcaagcgt ttgtagtgtc ctgggatctg gcacttcgat gagaaagctg tgacggcccc     480
   aacttctaac agcgagtggt aaggaggacg agggacacag gagggaggag actctcccca     540
   aagcttagca ccaacagaag tggtcccccg caggttgctc tgcgagcgcc acctcttccc     600
   tccaaccgag gagaaagtgg cgcgcctttg aggagtccga ggtcccggcc caggcggcag     660
   cttgggtcct ggcgggttcc ggacgggcgc ctcagggacc tggaagcaac cgcaccgaac     720
   gcgacggaga gcggcgagac gactccagga ggcgcccgag ctacatcccc cggccacacc     780
   aaacccgggt ttgctggcag acgcggctca cgacacccct tagggtcgca gcccctcccc     840
   cggaagtgac gtgtagcgac tacggcgtct gggagggacc caggagcagt cggggggttt     900
   gagagtggcg gcggccgcgg agggcctggc aggccccgcc gctgcaagga acgccccgaa     960
   cgcgcgcgcc cggcgtgtag cggccccaag acccgcgccg ccgctgccgc gtgcgggggc    1020
   ggggagggcg gggcgccagg agccgcggcg gcgggagatg cgggcggctg cgggcacccg    1080
   gcgggctcgg cttggccgcc gccgccttct acggctccgc cgcgggggtc gcagcggctg    1140
   ccgcgccgtc ctcgagtttc cagcgtgagg aggaggctga gggcggagag gcgcatcgtg    1200
   ttcgaggcgg agaccgaggg ggagccccgc gcgcggcgtc gctcattgct atggacagtg    1260
   ctatcaccct gtggcagttc cttcttcagc tcctgcagaa gcctcagaac aagcacatga    1320
   tctgttggac ctctaatgat gggcagttta agcttttgca ggcagaagag gtggctcgtc    1380
   tctgggggat tcgcaagaac aagcctaaca tgaattatga caaactcagc cgagccctca    1440
   gatactatta tgtaaagaat atcatcaaaa aagtgaatgg tcagaagttt gtgtacaagt    1500
   ttgtctctta tccagagatt ttgaacatgg atccaatgac agtgggcagg attgagggtg    1560
   actgtgaaag tttaaacttc agtgaagtca gcagcagttc caaagatgtg gagaatggag    1620
   ggaaagataa accacctcag cctggtgcca agacctctag ccgcaatgac tacatacact     1680
   ctggcttata ttcttcattt actctcaact ctttgaactc ctccaatgta aagcttttca     1740
   aattgataaa gactgagaat ccagccgaga aactggcaga gaaaaaatct cctcaggagc     1800
   ccacaccatc tgtcatcaaa tttgtcacga caccttccaa aaagccaccg gttgaacctg     1860
   ttgctgccac catttcaatt ggcccaagta tttctccatc ttcagaagaa actatccaag     1920
   ctttggagac attggtttcc ccaaaactgc cttccctgga agccccaacc tctgcctcta     1980
   acgtaatgac tgcttttgcc accacaccac ccatttcgtc cataccccct ttgcaggaac     2040
   ctcccagaac accttcacca ccactgagtt ctcacccaga catcgacaca gacattgatt     2100
   cagtggcttc tcagccaatg gaacttccag agaatttgtc actggagcct aaagaccagg     2160
   attcagtctt gctagaaaag gacaaagtaa ataattcatc aagatccaag aaacccaaag     2220
   ggttagaact ggcacccacc cttgtgatca cgagcagtga tccaagccca ctgggaatac     2280
   tgagcccatc tctccctaca gcttctctta caccagcatt tttttcacag gtagcttgct     2340
   cgctctttat ggtgtcacca ttgctttcat ttatttgccc ttttaagcaa atccagaatt     2400
   tatacactca agtttgcttt ctgttactta ggtttgtctt agaaaggtta tgtgtgactg     2460
   tcatgtgaaa gttaccccat ttctcatctt aattaggatt gctaaaatag aaagtttgga     2520
   gtattttctt aaaaaattca ttgttctaca agtaaataaa tattttgatt tttctatttc     2580
   ctcctaaaga aagtacacac actctctcgc tctctctcgg tcttataaaa ctcgttggtg     2640
   tcttataaaa caaacagtga taatctcaag ttagaaaaca gtaggtcctg agaaccataa     2700
   gaaaaatgac tggtgtgatg ttgagtaaca agttggtaca gttactttag ctatttatta     2760
   acttgctcat ctcatagaac attttagtag atttttcaca cacctcatta ttaaaaaaaa     2820
   acaaacatgc tggtgtcttg gttacccatt attcctctgt acctgaattc aggttggttt     2880
   ttctatttgg aaaagacttt ataaatgttg gcttaaaaag aggttgagca ccagaatctc     2940
   agaatttacc accaaagaac tcatccatgt aaccaaaaac cacttgtacc cccaaaaact     3000
   attgaaataa aaatttaaaa aattttaaaa aaaaaaaaaa aaaaaaaaaa aaaaaaaaaa     3060
   aaaaaaaaaa aaaaaaa                                                  3077
<210> 46
   <211> 1443
   <212> DNA
   <213> Homo sapiens
<400> 46
   ggggggggtg gatgaggagg agccggagac gccgcggagg agaccggacc gaagacggac      60
   cgtgccggga agagcaggcg ggtgaaaatg aaagccggct gtagcatcgt ggaaaagcca     120
   gaaggaggtg gagggtatca gtttcctgac tgggcctaca aaacagagtc atccccaggc     180
   tcccggcaga tccagctgtg gcacttcatc ctggagctgc tgcagaagga agagttccgc     240
   catgtcatcg cctggcagca gggagagtac ggggaatttg tcatcaagga tccagatgag     300
   gtggcccgcc tctggggccg caggaaatgc aaaccacaga tgaattatga caagctgagc     360
   cgggccctca gatactatta caacaagagg atccttcata aaacaaaagg gaaaagattt     420
   acctataaat ttaacttcaa caagctggtg atgcccaact acccattcat caacattcgg     480
   tcaagtggta agatacaaac tcttttggta gggaattaat tttgaattga aaagaatttt     540
   taaaaatcca aatctaagac atggcatgtt taggaagatt ttagaaacac taaaataatg     600
   tgatcctttg gattgcctca atgttcttac tcaagtcatc tcacttataa ggagagttat     660
   aggctattca gtatcaagat agatttcttt ggtttatttg gttggttccc ttttctgcat     720
   attgtttgta atctcctaga tactattacg ctatcttgtt tgggaatgat gtttcatagg     780
   tttgtgatga tctttacgtt caggactcag ttttaacacc cagcccagtg gttctttcat     840
   agatgggaac ctgtttctac aaacacttcc gattttctgt gaaactacca agctctccct     900
   tatcaagtga atatcatcaa aaccacagca tccttgatca gagaaggggg aggttcacat     960
   gtttgcagtg aaaagcagtg tctttgatct gcaacagcaa atcctcagag aaaaagattc    1020
   tggggttact tgaccttctc tcctgttaag tgcagtaggg cttcccctct tgactttcct    1080
   ggttatagct ttccatcaca gctccccaca ttctctcttg atgttgaaag cagtctctca    1140
   aaagactttg ttgttgtgtg gttttttgtt tgtgattttt ttccttatgc aaatcatact    1200
   cctgcccaag aaaatacagt agttcccctt atctgagcag tatatgttct aagaccccta     1260
   gtagattcgc aaaccacaga tagtaccaaa ctccattcat atatatgatg ttttttcttc    1320
   ccttaacccc actcatatgt atctgtgata acgtttaatt tataaattag gcacagtaag    1380
   agattaatga caataataaa atagaaaaat tataaaaaaa aaaaaaaaaa aaaaaaaaaa    1440
   aaa                                                                  1443
<210> 47
   <211> 229
   <212> DNA
   <213> Homo sapiens
<400> 47
   cccactagtt acccacccca aactggatcc tacagccaag ctccaagtca atatagccaa 60
   cagagcagca gctacgggca gcagaatccg tatcagatcc tgggcccgac cagcagtcgc 120
   ctagccaacc ctggaagcgg gcagatccag ctgtggcaat tcctcctgga gctgctctcc 180
   gacagcgcca acgccagctg tatcacctgg gaggggacca acggggagt 229
<210> 48
   <211> 2319
   <212> DNA
   <213> Homo sapiens
<400> 48
   cacaaggcta caggtgtctt tatttccact gcacgctggt gctgggagcg cctgccttct 60
   cttgccttga aagcctcctc tttggaccta gccaccgctg ccctcacggt aatgttggac 120
   tcggtgacac acagcacctt cctgcctaat gcatccttct gcgatcccct gatgtcgtgg 180
   actgatctgt tcagcaatga agagtactac cctgcctttg agcatcagac agcctgtgac 240
   tcatactgga catcagtcca ccctgaatac tggactaagc gccatgtgtg ggagtggctc 300
   cagttctgct gcgaccagta caagttggac accaattgca tctccttctg caacttcaac 360
   atcagtggcc tgcagctgtg cagcatgaca caggaggagt tcgtcgaggc agctggcctc 420
   tgcggcgagt acctgtactt catcctccag aacatccgca cacaaggtta ctcctttttt 480
   aatgacgctg aagaaagcaa ggccaccatc aaagactatg ctgattccaa ctgcttgaaa 540
   acaagtggca tcaaaagtca agactgtcac agtcatagta gaacaagcct ccaaagttct 600
   catctatggg aatttgtacg agacctgctt ctatctcctg aagaaaactg tggcattctg 660
   gaatgggaag atagggaaca aggaattttt cgggtggtta aatcggaagc cctggcaaag 720
   atgtggggac aaaggaagaa aaatgacaga atgacatatg aaaagttgag cagagccctg 780
   agatactact ataaaacagg aattttggag cgggttgacc gaaggttagt gtacaaattt 840
   ggaaaaaatg cacacgggtg gcaggaagac aagctatgat ctgctccagg catcaagctc 900
   attttatgga tttctgtctt ttaaaacaat cagattgcaa tagacattcg aaaggcttca 960
   ttttcttctc tttttttttt aacctgcaaa catgctgata aaatttctcc acatctcagc 1020
   ttacatttgg attcagagtt gttgtctacg gagggtgaga gcagaaactc ttaagaaatc 1080
   ctttcttctc cctaagggga tgaggggatg atcttttgtg gtgtcttgat caaactttat 1140
   tttcctagag ttgtggaatg acaacagccc atgccattga tgctgatcag agaaaaacta 1200
   ttcaattctg ccattagaga cacatccaat gctcccatcc caaaggttca aaagttttca 1260
   aataactgtg gcagctcacc aaaggtgggg gaaagcatga ttagtttgca ggttatggta 1320
   ggagagggtg agatataaga catacatact ttagatttta aattattaaa gtcaaaaatc 1380
   catagaaaag tatccctttt tttttttttt gagacgggtt ctcactatgt tgcccagggc 1440
   tggtcttgaa ctcctatgct caagtgatcc tcccacctcg gcctcccaaa gtactgtgat 1500
   tacaagcgtg agccacggca cctgggcaga aaagtatctt aattaatgaa agagctaagc 1560
   catcaagctg ggacttaatt ggatttaaca taggttcaca gaaagtttcc taaccagagc 1620
   atctttttga ccactcagca aaacttccac agacatcctt ctggacttaa acacttaaca 1680
   ttaaccacat tattaattgt tgctgagttt attccccctt ctaactgatg gctggcatct 1740
   gatatgcaga gttagtcaac agacactggc atcaattaca aaatcactgc tgtttctgtg 1800
   attcaagctg tcaacacaat aaaatcgaaa ttcattgatt ccatctctgg tccagatgtt 1860
   aaacgtttat aaaaccggaa atgtcctaac aactctgtaa tggcaaatta aattgtgtgt 1920
   cttttttgtt ttgtctttct acctgatgtg tattcaagtg ctataacacg tatttccttg 1980
   acaaaaatag tgacagtgaa ttcacactaa taaatgttca taggttaaag tctgcactga     2040
   cattttctca tcaatcactg gtatgtaagt tatcagtgac tgacagctag gtggactgcc     2100
   cctaggactt ctgtttcacc agagcaggaa tcaagtggtg aggcactgaa tcgctgtaca     2160
   ggctgaagac ctccttatta gagttgaact tcaaagtaac ttgttttaaa aaatgtgaat     2220
   tactgtaaaa taatctattt tggattcatg tgttttccag gtggatatag tttgtaaaca     2280
   atgtgaataa agtatttaac atgtaaaaaa aaaaaaaaa                            2319
<210> 49
   <211> 3617
   <212> DNA
   <213> Homo sapiens
<400> 49
   acccgtggtg ccccatccct ataggagctg gtgagattgc agcctgctgc ctcccctcca 60
   tcagccacag ctattggatt tcccacccag aatctttagg taaatgagat catgattctg 120
   gaaggaggtg gtgtaatgaa tctcaacccc ggcaacaacc tccttcacca gccgccagcc 180
   tggacagaca gctactccac gtgcaatgtt tccagtgggt tttttggagg ccagtggcat 240
   gaaattcatc ctcagtactg gaccaagtac caggtgtggg agtggctcca gcacctcctg 300
   gacaccaacc agctggatgc caattgtatc cctttccaag agttcgacat caacggcgag 360
   cacctctgca gcatgagttt gcaggagttc acccgggcgg cagggacggc ggggcagctc 420
   ctctacagca acttgcagca tctgaagtgg aacggccagt gcagtagtga cctgttccag 480
   tccacacaca atgtcattgt caagactgaa caaactgagc cttccatcat gaacacctgg 540
   aaagacgaga actatttata tgacaccaac tatggtagca cagtagattt gttggacagc 600
   aaaactttct gccgggctca gatctccatg acaaccacca gtcaccttcc tgttgcagag 660
   tcacctgata tgaaaaagga gcaagacccc cctgccaagt gccacaccaa aaagcacaac 720
   ccgagaggga ctcacttatg ggaattcatc cgcgacatcc tcttgaaccc agacaagaac 780
   ccaggattaa taaaatggga agaccgatct gagggcgtct tcaggttctt gaaatcagag 840
   gcagtggctc agctatgggg taaaaagaag aacaacagca gcatgaccta tgaaaagctc 900
   agccgagcta tgagatatta ctacaaaaga gaaattctgg agcgtgtgga tggacgaaga 960
   ctggtatata aatttgggaa gaatgcccga ggatggagag aaaatgaaaa ctgaagctgc 1020
   caatactttg gacacaaacc aaaacacaca ccaaataatc agaaacaaag aactcctgga 1080
   cgtaaatatt tcaaagacta cttttctctg atatttatgt accatgaggg gaacaagaaa 1140
   ctacttctaa cgggaagaag aaacactaca gtcgattaaa aaaattattt tgttacttcg 1200
   aagtatgtcc tatatgggga aaaaacgtac acagttttct gtgaaatatg atgctgtatg 1260
   tggttgtgat tttttttcac ctctattgtg aattcttttt cactgcaaga gtaacaggat 1320
   ttgtagcctt gtgcttcttg ctaagagaaa gaaaaacaaa atcagagggc attaaatgtt 1380
   ttgtatgtga catgatttag aaaaaggtga tgcatcctcc tcacataagc atccatatgg 1440
   cttcgtcaag ggaggtgaac attgttgctg agttaaattc cagggtctca gatggttagg 1500
   acaaagtgga tggatgccgg gaagtttaac ctgagcctta ggatccaatg agtggagaat 1560
   ggggacttcc aaaacccaag gttggctata atctctgcat aaccacatga cttggaatgc 1620
   ttaaatcagc aagaagaata atggtggggt ctttatactc attcaggaat ggtttatctg 1680
   atgccagggc tgtcttcctt tctccccttt ggatggttgg tgaaatactt taattgccct 1740
   gtctgctcac ttctagctat ttaagagaga acccagcttg gttctttttt gctccaagtg 1800
   cttaaaaata agttggaaaa aggagacggt ggtgtggaaa tggctgaaga gtttgctctt 1860
   gtatccctat agtccaaggt ttctcaatct gcacaattga catttttggc cggagtgttc 1920
   tttgtggtga gggctttcct gtgcattgta agatgttcag cagtatccac tcatggtctc 1980
   taaccacttg acaccagaaa ccccccagct gtgataacgc aaaatgtctc tagacatcac 2040
   caaatgttcc ctgggggtgg caaatttgcc cttgattgag aaccaccagt ttagctagtc 2100
   aatatgagga tggtggttta ttctcagaag aaaaagatat gtaaggtctt ttagctcctt 2160
   agagtgaagc aaaagcaaga cttcaacctc aacctatctt tatgttttaa atgttaggga 2220
   caataagttg aaatagctag aggagcttct tttcagaacc ccagatgaga gccaatgtca 2280
   gataaagtaa gcatagtaat gtagcaggaa ctacaataga agacattttc actggaatta 2340
   caaagcagaa ttaaaattat attgtagaag gaaacaccaa gaaaagaatt tccagggaaa 2400
   atcctctttg caggtattaa ttcttataat tttttgtctt ttggattatc tgtttactgt 2460
   ctcatctgaa ctgatcccag gtgaacggtt tattgcctag atttgtactc agaggaattt 2520
   tttttgtttt gttttgtctt ttaagaaagg aaagaaagga tgaaaaaaat aaacagaaaa 2580
   ctcagctcag gcacaattgt caccaaggag ttaaaagctt cttcttcaat agaggaattg 2640
   ttctgggggt cctggagact taccattgag ccatgcaatc tgggaagcac aggaataagt 2700
   agacactttg aaaatggatt tgaatgttct catccctttt gcagcttttc tttttggctc 2760
   tctcatgtcc ttggcttgct cctctattct acctctcttt ctccagcaat aatatgcaaa 2820
   tgaagacatg tatccataag aaggagtgct cttcatcaac taatagagca cctaccacag 2880
   tgtcatacct ggtagaggtg agcaattcat attcaaaggt tgcaaagtgt ttgtaatata 2940
   ttcatgaggc tggaagtaag aagaattaaa aatttgtcct aattacaatg agaaccattc 3000
   taggtagtga tcttggagca cacatgaata actttctgaa ggtgcaacca aatccatttt 3060
   tatttctgcc tggcttggtc acctctgtaa aggtttaact tagtgttgtc aagtaacagt 3120
   tactgaaaga gctgagaaaa agaacaatga acagcaacga tcttgactgt gcaactcaga 3180
   cattcctgca gaaaagacat atgttgcttt acaagaaggc caaagaacta tggggccttc 3240
   ccagcatttg actgttcatt gcatagaatg aattaaatat ccagttactt gaatgggtat 3300
   aacgcatgaa tatttgtgtg tctgtgtgtg tgtctgagtt gtgtgatttt attaggggca 3360
   tctgccaatt ctctcactgt ggttccttct ctgactttgc ctgttcatca tctaaggagg     3420
   ctagatcctt cgctgacttc accattcctc aaacctgtaa gtttctcact tcttccaaat     3480
   tggctttggc tctttctgca acctttccat tcaagagcaa tctttgctaa ggagtaagtg     3540
   aatgtgaaga gtaccaacta caacaattct acagataatt agtggattgt gttgtttgtt     3600
   gagagtgaag gtttctt                                                    3617
<210> 50
   <211> 1894
   <212> DNA
   <213> Homo sapiens
<400> 50
   gtctgacttc ctcccagcac attcctgcac tctgccgtgt ccacactgcc ccacagaccc      60
   agtcctccaa gcctgctgcc agctccctgc aagcccctca ggttgggcct tgccacggtg     120
   ccagcaggca gccctgggct gggggtaggg gactccctac aggcacgcag ccctgagacc     180
   tcagagggcc accccttgag ggtggccagg cccccagtgg ccaacctgag tgctgcctct     240
   gccaccagcc ctgctggccc ctggttccgc tggcccccca gatgcctggc tgagacacgc     300
   cagtggcctc agctgcccac acctcttccc ggcccctgaa gttggcactg cagcagacag     360
   ctccctgggc accaggcagc taacagacac agccgccagc ccaaacagca gcggcatggg     420
   cagcgccagc ccgggtctga gcagcgtatc ccccagccac ctcctgctgc cccccgacac     480
   ggtgtcgcgg acaggcttgg agaaggcggc agcgggggca gtgggtctcg agagacggga     540
   ctggagtccc agtccacccg ccacgcccga gcagggcctg tccgccttct acctctccta     600
   ctttgacatg ctgtaccctg aggacagcag ctgggcagcc aaggcccctg gggccagcag     660
   tcgggaggag ccacctgagg agcctgagca gtgcccggtc attgacagcc aagccccagc     720
   gggcagcctg gacttggtgc ccggcgggct gaccttggag gagcactcgc tggagcaggt     780
   gcagtccatg gtggtgggcg aagtgctcaa ggacatcgag acggcctgca agctgctcaa     840
   catcaccgca gatcccatgg actggagccc cagcaatgtg cagaagtggc tcctgtggac     900
   agagcaccaa taccggctgc cccccatggg caaggccttc caggagctgg cgggcaagga     960
   gctgtgcgcc atgtcggagg agcagttccg ccagcgctcg cccctgggtg gggatgtgct    1020
   gcacgcccac ctggacatct ggaagtcagc ggcctggatg aaagagcgga cttcacctgg    1080
   ggcgattcac tactgtgcct cgaccagtga ggagagctgg accgacagcg aggtggactc    1140
   atcatgctcc gggcagccca tccacctgtg gcagttcctc aaggagttgc tactcaagcc    1200
   ccacagctat ggccgcttca ttaggtggct caacaaggag aagggcatct tcaaaattga    1260
   ggactcagcc caggtggccc ggctgtgggg catccgcaag aaccgtcccg ccatgaacta    1320
   cgacaagctg agccgctcca tccgccagta ttacaagaag ggcatcatcc ggaagccaga    1380
   catctcccag cgcctcgtct accagttcgt gcaccccatc tgagtgcctg gcccagggcc    1440
   tgaaacccgc cctcaggggc ctctctcctg cctgccctgc ctcagccagg ccctgagatg      1500
   ggggaaaacg ggcagtctgc tctgctgctc tgaccttcca gagcccaagg tcagggaggg      1560
   gcaaccaact gccccagggg gatatgggtc ctctggggcc ttcgggacca tggggcaggg      1620
   gtgcttcctc ctcaggccca gctgctcccc tggaggacag agggagacag ggctgctccc      1680
   caacacctgc ctctgacccc agcatttcca gagcagagcc tacagaaggg cagtgactcg      1740
   acaaaggcca caggcagtcc aggcctctct ctgctccatc cccctgcctc ccattctgca      1800
   ccacacctgg catggtgcag ggagacatct gcacccctga gttgggcagc caggagtgcc      1860
   cccgggaatg gataataaag atactagaga actg                                   1894
<210> 51
   <211> 2180
   <212> DNA
   <213> Homo sapiens
<400> 51
   gggcggaaaa gcctgtttac acagactgca caccgcctgg ggaataatgc agtaaaggaa 60
   gtgagccggc tcggcctgac tgctccaact tcctgctctc acacacacca gaggggaaaa 120
   aaaaagagga gcgagagaaa gaaaaaaagg gggaaaaatc aggatctcat tacaagagcc 180
   acagaccgtc tgcagacgcc tgtcagcatg gaaagtcggg ggctttcgcc cgggtcctcc 240
   tagaaattcc ccccgaagaa gactccccca catctgggta tggagagtgc aatcacgctg 300
   tggcagttcc tgttgcagtt gctgctggat cagaaacatg agcatttgat ctgctggacc 360
   tcgaacgatg gtgaattcaa gctcctcaaa gcagaagaag tggccaagct gtggggactc 420
   cgaaaaaaca aaacaaatat gaactatgat aagctgagca gagccctgcg atactattat 480
   gacaagaaca tcatcaagaa ggtgatcggg cagaagtttg tgtacaagtt tgtctctttc 540
   ccggagatcc tgaagatgga tcctcacgcg gtggagatca gccgggagag ccttctgctg 600
   caggacagcg actgcaaggc gtctccggag ggccgcgagg cccacaaaca cggcctggcc 660
   gccctcagaa gcacgagccg caacgaatac atccactcag gcctgtactc gtccttcacc 720
   attaattccc tgcagaaccc accagacgcc ttcaaggcca tcaagacgga gaagctggag 780
   gagccgcccg aagacagccc ccccgtggaa gaagtcagga ctgtgatcag gtttgtgacc 840
   aataaaaccg acaagcacgt caccaggccg gtggtgtccc tgccttccac gtcagaggct 900
   gcggcggcgt ccgccttcct ggcctcgtcc gtctcggcca agatctcctc tttaatgttg 960
   ccaaacgctg ccagtatttc atccgcctca cccttctcat ctcggtcccc gtccctgtcc 1020
   cccaactcac ccctcccttc tgaacacaga agcctcttcc tggaggccgc ctgccatgac 1080
   tccgattccc tggagccctt gaacctgtca tcgggctcca agaccaagtc tccatctctt 1140
   cccccaaagg ccaaaaaacc caaaggcttg gaaatctcag cgcccccgct ggtgctctcc 1200
   ggcaccgaca tcggctccat cgccctcaac agcccagccc tcccctcggg atccctcacc 1260
   ccagccttct tcaccgcaca gacaccaaat ggattgcttc tgactccgag tccactgctc 1320
   tccagcatac atttctggag cagccttagt ccagttgctc cgctgagtcc tgccaggctg 1380
   caagggccaa gcacgctgtt ccagttcccc acactgctta atggccacat gccagtgcca 1440
   atccccagtc tggacagagc tgcttctcca gtactgcttt cttcaaactc tcagaaatcc 1500
   tgatgacgtc tggccacaat taaggactca ttaactgatg aaacaaattt gtccccacgg 1560
   gctagtttac ctgtgtcgtg agaaggacat tgtgaaactc ttgttaattt ggtttgcact 1620
   tttcataaca tggatagtct agatttatgt tagcatttta aaaactgttt ttgatatatt 1680
   caagtatata tgaaaatctg tttggcatta agtgaatttt aatgtttttg tttttatatc 1740
   cttttagctc ttaagtgttg aacactgttg acagtgaaga acttttctta atggttttca 1800
   gtataactaa taaggatgtg aagctttttt ctctttagtt ctgagtatgc taaactgtgt 1860
   gcttatatag actataacca gttgtgcctt cctttgcatt taatgtaaat gaatgattta 1920
   tatatttttt agtattaaga ggaaatgttt gaaagatgaa aattagtatc aaacagctct 1980
   ctagtagaat ttcattattt ttcaccagtg ggcaatatga aagcatatat cacgttttgt 2040
   tttactttca attgtataag aattgcctta gaacctcttt tgaactgaaa ttcagtaaat 2100
   gtccaagtaa tgtttttata ataaactaag ccatatttag acaataaaca tcgaaaaaaa 2160
   aaaaaaaaaa aaaaaaaaaa 2180
<210> 52
   <211> 3171
   <212> DNA
   <213> Homo sapiens
<400> 52
   gttgccagct gcggcggccg ccacagccac agccgccgcc gccgccgccg ccgcccctgc 60
   ccctgccgcc cctgcccctg ccgttaggtg gtggggtttc tcagcccggc ggcgggaggc 120
   gggccggcct cggcttcctg tcggaggacg cgcaaggatc cgggcgtcgg agtgtgtgcg 180
   agtgcgtgag tgtgtgtcgg tcgcacggcg tgtgtctccg gccgcgggtt ccgcctcctc 240
   ccctgccgcc gctgctcacg gtgtaagtca atgtgaagca gcagctccag ccccgggata 300
   aacatggcga cgtctctgca tgagggaccc acgaaccagc tggatctgct catccgggcc 360
   gtggaagcat cagttcacag cagtaatgca cactgtacag ataagacaat tgaagctgct 420
   gaagccctgc ttcatatgga atctcctacc tgcttgaggg attcaagaag tcctgtggaa 480
   gtgtttgttc ctccttgtgt atcaactcca gaattcatcc atgctgctat gaggccagat 540
   gtcattacag aaactgtagt ggaggtgtca actgaagagt ctgaacccat ggatacctct 600
   cctattccaa catcaccaga tagccatgaa ccaatgaaaa agaaaaaagt tggccgtaaa 660
   ccaaagaccc agcaatcacc aatttccaat gggtctcctg agttaggtat aaagaagaaa 720
   ccaagagaag gaaaaggaaa cacaacctat ttgtgggagt ttcttttaga tctacttcaa 780
   gataaaaata cttgtcccag gtatattaaa tggactcaga gagaaaaagg catattcaag 840
   ctggtggatt caaaggctgt ctctaagctt tggggaaagc ataagaacaa accagacatg 900
   aactatgaaa ccatgggacg agctttgaga tactactacc aaaggggaat tcttgcaaag 960
   gttgaaggac agaggcttgt atatcagttc aaggatatgc cgaaaaacat agtggtcata 1020
   gatgatgaca aaagtgaaac ctgtaatgaa gatttagcag gaactactga tgaaaaatca 1080
   ttagaacgag tgtcactgtc tgcagaaagt ctcctgaaag cagcatcctc tgttcgcagt 1140
   ggaaaaaatt catcccctat aaactgctcc agagcagaga agggtgtagc tagagttgtg 1200
   aatatcactt cccctgggca cgatgcttca tccaggtctc ctactaccac tgcatctgtg 1260
   tcagcaacag cagctccaag gacagttcgt gtggcaatgc aggtacctgt tgtaatgaca 1320
   tcattgggtc agaaaatttc aactgtggca gttcagtcag ttaatgcagg tgcaccatta 1380
   ataaccagca ctagtccaac aacagcgacc tctccaaagg tagtcattca gacaatccct 1440
   actgtgatgc cagcttctac tgaaaatgga gacaaaatca ccatgcagcc tgccaaaatt 1500
   attaccatcc cagctacaca gcttgcacag tgtcaactgc agacaaagtc aaatctgact     1560
   ggatcaggaa gcattaacat tgttggaacc ccattggctg tgagagcact tacccctgtt     1620
   tcaatagccc atggtacacc tgtaatgaga ctatcaatgc ctactcagca ggcatctggc 1680
   cagactcctc ctcgagttat cagtgcagtc ataaaggggc cagaggttaa atcggaagca 1740
   gtggcaaaaa agcaagaaca tgatgtgaaa actttgcagc tagtagaaga aaaaccagca 1800
   gatggaaata agacagtgac ccacgtagtg gttgtcagtg cgccttcagc tattgccctt 1860
   cctgtaacta tgaaaacaga aggactagtg acatgtgaga aataaaatag cagctccacc 1920
   atggacttca ggctgttagt ggcagtactg acataaacat ttgcaaggga agtcatcaag 1980
   aaaagtcaaa gaagacttta aaacattttt aatgcatata caaaaacaat cagacttact 2040
   ggaaataaat tacctatccc atgtttcagt gggaaatgaa ctacatattg agatgctgac 2100
   agaaaactgc ctcttacagt aggaaacaac tgaacccatc aataagaaaa aggatcgaaa 2160
   gggaccaagc agctcactac gatatcaagt tacactaaga cttggaacac taacattctg 2220
   taagaggtta tatagttttc agtgggaggg gttgggatgg gtaatctcat tgttacatat 2280
   agcaattttt gatgcatttt atatgcatac cagcaattat tactgtgttc gcacagttct 2340
   cacttaactg gtgctatgtg aagactctgc taatataggt attttagaat gtgaattgaa 2400
   gaatggatcc caaaaacttc agaaagagga tagcaaaaaa agatctagtg cgattttata 2460
   tatatatata tatatatata catacatata tatatatcat atagcttaag ctgatttaaa 2520
   acaaaggcct tagactaatt ttcgattttc tttcttgaaa taagctaatg gcttgtttgt 2580
   gtaaagcttt tttattaaaa gaaaaatttt aaaaatcttg tacctagcac agtattgtta 2640
   tagaatatac atgtaacatt ttatatggta gtttaagtct gtcagtttct taattgtgga 2700
   caaattaaca gttggctctg gccttttgct gtaacatgcc tgtgtcactc acttagcctt 2760
   ggcatttgtg cagacatacc attttcagtt ctgctgtcac ttggaagttc aggctcagca 2820
   tgaatttttg gcaggtagct ctaatacctg gagttttctt tgtttttttt tctttttttt 2880
   agttgaagtt tatgagggaa ataccagtgt tcagttttga actataatag tttgtatatt 2940
   caacatttga agtatattct attttgttgt actcttgttt caaagtgtat tcaagtaggt 3000
   tttctgaaat atagaaatga aatttatctt ctgttttggt ctctggtgat attttaaaca 3060
   atatttaaaa gtcagtatag aagtgtttta gttaggaagt gataaaacat ctctcttctc 3120
   cttcccaact actgcatgaa gaaattctac ttccattata ttaatatttg g 3171
<210> 53
   <211> 3218
   <212> DNA
   <213> Homo sapiens
<400> 53
   aaaatagtga aggatgctta gactacttaa catacaaact gctttctggt taatcatctt      60
   tagaagactg gatttctgga tatctactcc actccatctc tattgacttt taaaacatga     120
   taatgcaaac ctataacact ggcaaccatc agtgaacctt taatttcatt gattaatagc     180
   gtttgaagct tcctcaggga ataacaatga catcagcagt ggttgacagt ggaggtacta     240
   ttttggagct ttccagcaat ggagtagaaa atcaagagga aagtgaaaag gtttctgaat     300
   atccagcagt gattgtggag ccagttccaa gtgccagatt agagcagggc tatgcagccc     360
   aggttctggt ttatgatgat gagacttata tgatgcaaga tgtggcagaa gaacaagaag     420
   ttgagaccga gaatgtggaa acagtggaag catcagttca cagcagtaat gcacactgta     480
   cagataagac aattgaagct gctgaagccc tgcttcatat ggaatctcct acctgcttga     540
   gggattcaag aagtcctgaa ttcatccatg ctgctatgag gccagatgtc attacagaaa     600
   ctgtagtgga ggtgtcaact gaagagtctg aacccatgga tacctctcct attccaacat     660
   caccagatag ccatgaacca atgaaaaaga aaaaagttgg ccgtaaacca aagacccagc     720
   aatcaccaat ttccaatggg tctcctgagt taggtataaa gaagaaacca agagaaggaa     780
   aaggaaacac aacctatttg tgggagtttc ttttagatct acttcaagat aaaaatactt     840
   gtcccaggta tattaaatgg actcagagag aaaaaggcat attcaagctg gtggattcaa     900
   aggctgtctc taagctttgg ggaaagcata agaacaaacc agacatgaac tatgaaacca     960
   tgggacgagc tttgagatac tactaccaaa ggggaattct tgcaaaggtt gaaggacaga    1020
   ggcttgtata tcagttcaag gatatgccga aaaacatagt ggtcatagat gatgacaaaa    1080
   gtgaaacctg taatgaagat ttagcaggaa ctactgatga aaaatcatta gaacgagtgt    1140
   cactgtctgc agaaagtctc ctgaaagcag catcctctgt tcgcagtgga aaaaattcat    1200
   cccctataaa ctgctccaga gcagagaagg gtgtagctag agttgtgaat atcacttccc    1260
   ctgggcacga tgcttcatcc aggtctccta ctaccactgc atctgtgtca gcaacagcag 1320
   ctccaaggac agttcgtgtg gcaatgcagg tacctgttgt aatgacatca ttgggtcaga 1380
   aaatttcaac tgtggcagtt cagtcagtta atgcaggtgc accattaata accagcacta 1440
   gtccaacaac agcgacctct ccaaaggtag tcattcagac aatccctact gtgatgccag 1500
   cttctactga aaatggagac aaaatcacca tgcagcctgc caaaattatt accatcccag 1560
   ctacacagct tgcacagtgt caactgcaga caaagtcaaa tctgactgga tcaggaagca 1620
   ttaacattgt tggaacccca ttggctgtga gagcacttac ccctgtttca atagcccatg 1680
   gtacacctgt aatgagacta tcaatgccta ctcagcaggc atctggccag actcctcctc 1740
   gagttatcag tgcagtcata aaggggccag aggttaaatc ggaagcagtg gcaaaaaagc 1800
   aagaacatga tgtgaaaact ttgcagctag tagaagaaaa accagcagat ggaaataaga 1860
   cagtgaccca cgtagtggtt gtcagtgcgc cttcagctat tgcccttcct gtaactatga 1920
   aaacagaagg actagtgaca tgtgagaaat aaaatagcag ctccaccatg gacttcaggc 1980
   tgttagtggc agtactgaca taaacatttg caagggaagt catcaagaaa agtcaaagaa 2040
   gactttaaaa catttttaat gcatatacaa aaacaatcag acttactgga aataaattac 2100
   ctatcccatg tttcagtggg aaatgaacta catattgaga tgctgacaga aaactgcctc 2160
   ttacagtagg aaacaactga acccatcaat aagaaaaagg atcgaaaggg accaagcagc 2220
   tcactacgat atcaagttac actaagactt ggaacactaa cattctgtaa gaggttatat 2280
   agttttcagt gggaggggtt gggatgggta atctcattgt tacatatagc aatttttgat 2340
   gcattttata tgcataccag caattattac tgtgttcgca cagttctcac ttaactggtg 2400
   ctatgtgaag actctgctaa tataggtatt ttagaatgtg aattgaagaa tggatcccaa 2460
   aaacttcaga aagaggatag caaaaaaaga tctagtgcga ttttatatat atatatatat 2520
   atatatacat acatatatat atatcatata gcttaagctg atttaaaaca aaggccttag 2580
   actaattttc gattttcttt cttgaaataa gctaatggct tgtttgtgta aagctttttt 2640
   attaaaagaa aaattttaaa aatcttgtac ctagcacagt attgttatag aatatacatg 2700
   taacatttta tatggtagtt taagtctgtc agtttcttaa ttgtggacaa attaacagtt 2760
   ggctctggcc ttttgctgta acatgcctgt gtcactcact tagccttggc atttgtgcag 2820
   acataccatt ttcagttctg ctgtcacttg gaagttcagg ctcagcatga atttttggca 2880
   ggtagctcta atacctggag ttttctttgt ttttttttct tttttttagt tgaagtttat 2940
   gagggaaata ccagtgttca gttttgaact ataatagttt gtatattcaa catttgaagt 3000
   atattctatt ttgttgtact cttgtttcaa agtgtattca agtaggtttt ctgaaatata 3060
   gaaatgaaat ttatcttctg ttttggtctc tggtgatatt ttaaacaata tttaaaagtc 3120
   agtatagaag tgttttagtt aggaagtgat aaaacatctc tcttctcctt cccaactact 3180
   gcatgaagaa attctacttc cattatatta atatttgg 3218
<210> 54
   <211> 1901
   <212> DNA
   <213> Homo sapiens
<400> 54
   gcggcgagtg gagcgggagc cgactggaag aagggctcta gggagggggc tgtggctgct 60
   ggggtccgag gtggggccgg gtacaccagc cccatcactg tttgcagaga gtcagggagg 120
   cggaaaagac acgcgctcta ggctcccatc agggcacatg gcccgggccc atcccccgcg 180
   cgtctccccg gctgcggggc gcggggggct gccgggtgcg cttggctgtg gcgcggcgcg 240
   ttggagactt tattgcgatg ggacgataag aggggcgggg gcggggtcct gggggccgag 300
   gcggcagcgc tttaattaaa acggaaattg cggccccggg ccgcgcgggg gccggagggt 360
   tccaagcggc cccttagctg gaagcgtttc tccaggaccc ccccgcaacc cccgccacgc 420
   ccgggctgcc ccctcccgcc aggccctgcc ggacccggcg ccgtcttctc ctccttgtca 480
   cccgcggtcg cttcgggcgg ggatcggtgc caccgagcgc aaagcctgcc tcgcccccct 540
   tccccgtccc ccccatctcc caccgcccag tccccggcgg cgatgagaca gagcggcgcc 600
   tcccagcccc tgctgatcaa catgtacctg ccagatcccg tcggagacgg tctcttcaag 660
   gacgggaaga acccgagctg ggggccgctg agccccgcgg ttcagaaagg cagcggacag 720
   atccagctgt ggcagtttct gctggagctg ctggctgacc gcgcgaacgc cggctgcatc 780
   gcgtgggagg gcggtcacgg cgagttcaag ctcacggacc cggacgaggt ggcgcggcgg 840
   tggggcgagc gcaagagcaa gcccaacatg aactacgaca agctgagccg cgccctgcgc 900
   tactactacg acaagaacat catgagcaag gtgcatggca agcgctacgc ctaccgcttc 960
   gacttccagg gcctggcgca ggcctgccag ccgccgcccg cgcacgctca tgccgccgcc 1020
   gcagctgctg ccgccgccgc ggccgcccag gacggcgcgc tctacaagct gcccgccggc 1080
   ctcgccccgc tgcccttccc cggcctctcc aaactcaacc tcatggccgc ctcggccggg 1140
   gtcgcgcccg ccggcttctc ctactggccg ggcccgggcc ccgccgccac cgctgccgcc 1200
   gccaccgccg cgctctaccc cagtcccagc ttgcagcccc cgcccgggcc cttcggggcc 1260
   gtggccgcag cctcgcactt ggggggccat taccactaga cggggcggtc gggtgcctgc 1320
   ggcctcgccc gcacgcctag agtctcgccc gatcccatcg gcatcccggg gagggcccgg 1380
   gagcctccgt caaccgtcct ctaatccaga gtttactcca cctgccgcac ttagcagggg 1440
   gacgggaccg aagctccctc aatccttgtc tggtactaga tttgctcctg tcccaccccg 1500
   cagtcccctg aggagggcga tgtgcgccct ctttcacttt ttttcttcta ggtctccagg 1560
   tcccggaggg gatttgtgga cctctcttgt ctccccacca ctccagtgca tttccgcctg 1620
   gctcctagaa gccccattca atatcactac tctttaacga gtgccaaatc ttttcccact 1680
   tttgctcttc cccaaggaac tgctcccacc tcagcacgtg gaggcctctc acggtcctcc 1740
   ttcctgggac ctgagcaggt ttggtgaaag ccaccgtcct ccgtgacaca cggccccctt 1800
   cctcctgtcc ccacactccc aggagaaact cccggtgtgt ttctgaccct ttcagcccca 1860
   ttaaagctcc tgagctctca aaaaaaaaaa aaaaaaaaaa a 1901
<210> 55
   <211> 21
   <212> DNA
   <213> Homo sapiens
<400> 55
   taggcgcgag ctaagcagga g 21
<210> 56
   <211> 25
   <212> DNA
   <213> Homo sapiens
<400> 56
   gtaggcacac tcaaacaacg actgg 25
<210> 57
   <211> 19
   <212> DNA
   <213> Homo sapiens
<400> 57
   cgcgagctaa gcaggaggc 19
<210> 58
   <211> 22
   <212> DNA
   <213> Homo sapiens
<400> 58
   caggccatga aaagccaaac tt 22
<210> 59
   <211> 49
   <212> DNA
   <213> Homo sapiens
<400> 59
   aaaaaaaaaa aaaaaaaaaa aaaaaaaaaa tttcucgauu cguccuccg 49
<210> 60
   <211> 51
   <212> DNA
   <213> Homo sapiens
<400> 60
   aaaaaaaaaa aaaaaaaaaa aaaaaaaaaa tttauccgcg cucgauucgu c 51
<210> 61
   <211> 23
   <212> DNA
   <213> Homo sapiens
<400> 61
   gagggcgagg ggcggggagc gcc 23
<210> 62
   <211> 31
   <212> DNA
   <213> Homo sapiens
<400> 62
   cctatcatta ctcgatgctg ttgataacag c 31
<210> 63
   <211> 43
   <212> DNA
   <213> Homo sapiens
<400> 63
   aatttaatac gactcactat agggagaaac tttcagcctg ata 43
<210> 64
   <211> 51
   <212> DNA
   <213> Homo sapiens
<400> 64
   aatttaatac gactcactat agggagactc tgtgagtcat ttgtcttgct t 51
<210> 65
   <211> 47
   <212> DNA
   <213> Homo sapiens
<400> 65
   aatttaatac gactcactat agggagagca cactcaaaca acgactg 47
<210> 66
   <211> 23
   <212> DNA
   <213> Homo sapiens
<400> 66
   gcgcggcagc ucagguaccu gac 23
<210> 67
   <211> 26
   <212> DNA
   <213> Homo sapiens
<400> 67
   gcuuugaacu cacucaggua ccugac 26
<210> 68
   <211> 27
   <212> DNA
   <213> Homo sapiens
<400> 68
   gagcgcggca ggaagccuua ucaguug 27
<210> 69
   <211> 28
   <212> DNA
   <213> Homo sapiens
<400> 69
   gagcgcggca gguuauucca ggaucuuu 28
<210> 70
   <211> 17
   <212> DNA
   <213> Homo sapiens
<400> 70
   cgcggcagga agcctta 17
<210> 71
   <211> 22
   <212> DNA
   <213> Homo sapiens
<400> 71
   tccgtaggca cactcaaaca ac 22
<210> 72
   <211> 18
   <212> DNA
   <213> Homo sapiens
<400> 72
   cagttgtgag tgaggacc 18
<210> 73
   <211> 72
   <212> DNA
   <213> Homo sapiens
<400> 73
   ggggtgcagc ttttattttc ccaaatactt cagtatatcc tgaagctacg caaagaatta 60
   caactaggcc ag 72
<210> 74
   <211> 40
   <212> PRT
   <213> Homo sapiens
<400> 74
   Met Ala Ser Thr Ile Lys Glu Ala Leu Ser Val Val Ser Glu Asp Gln
   1 5 10 15
   Ser Leu Phe Glu Cys Ala Tyr Gly Thr Pro His Leu Ala Lys Thr Glu
   20 25 30
   Met Thr Ala Ser Ser Ser Ser Asp
   35 40
<210> 75
   <211> 5
   <212> PRT
   <213> Homo sapiens
<400> 75
   Ser Ser Ser Ser Asp
   1 5
<210> 76
   <211> 40
   <212> PRT
   <213> Homo sapiens
<400> 76
   Tyr Gly Gln Thr Ser Lys Met Ser Pro Arg Val Pro Gln Gln Asp Trp
   1 5 10 15
   Leu Ser Gln Pro Pro Ala Arg Val Thr Ile Lys Met Glu Cys Asn Pro
   20 25 30
   Ser Gln Val Asn Gly Ser Arg Asn
   35 40
<210> 77
   <211> 13
   <212> PRT
   <213> Homo sapiens
<400> 77
   Val Pro Gln Gln Asp Trp Leu Ser Gln Pro Pro Ala Arg
   1 5 10
<210> 78
   <211> 13
   <212> PRT
   <213> Homo sapiens
<400> 78
   Val Pro Gln Gln Asp Trp Leu Ser Gln Pro Pro Ala Arg
   1 5 10
<210> 79
   <211> 13
   <212> PRT
   <213> Homo sapiens
<400> 79
   Val Pro Gln Gln Asp Trp Leu Ser Gln Pro Pro Ala Arg
   1 5 10
<210> 80
   <211> 13
   <212> PRT
   <213> Homo sapiens
<400> 80
   Val Pro Gln Gln Asp Trp Leu Ser Gln Pro Pro Ala Arg
   1 5 10
<210> 81
   <211> 21
   <212> PRT
   <213> Homo sapiens
<400> 81
   Tyr Gly Gln Thr Ser Lys Met Ser Val Pro Gln Gln Asp Trp Leu Ser
   1 5 10 15
   Gln Pro Pro Ala Arg
   20
<210> 82
   <211> 13
   <212> PRT
   <213> Homo sapiens
<400> 82
   Val Pro Gln Gln Asp Trp Leu Ser Gln Pro Pro Ala Arg
   1 5 10
<210> 83
   <211> 13
   <212> PRT
   <213> Homo sapiens
<400> 83
   Val Pro Gln Gln Asp Trp Leu Ser Gln Pro Pro Ala Arg
   1 5 10
<210> 84
   <211> 13
   <212> PRT
   <213> Homo sapiens
<400> 84
   Val Pro Gln Gln Asp Trp Leu Ser Gln Pro Pro Ala Arg
   1               5                   10
<210> 85
   <211> 25
   <212> PRT
   <213> Homo sapiens
<400> 85
   Val Pro Gln Gln Asp Trp Leu Ser Gln Pro Pro Ala Arg Met Glu Cys
   1               5                   10                  15
   Asn Pro Ser Gln Val Asn Gly Ser Arg
               20                  25
<210> 86
   <211> 13
   <212> PRT
   <213> Homo sapiens
<400> 86
   Val Pro Gln Gln Asp Trp Leu Ser Gln Pro Pro Ala Arg
   1               5                   10
<210> 87
   <211> 13
   <212> PRT
   <213> Homo sapiens
<400> 87
   Val Pro Gln Gln Asp Trp Leu Ser Gln Pro Pro Ala Arg
   1               5                   10
<210> 88
   <211> 40
   <212> PRT
   <213> Homo sapiens
<400> 88
   Ser Pro Asp Glu Cys Ser Val Ala Lys Gly Gly Lys Met Val Gly Ser
   1               5                   10                  15
   Pro Asp Thr Val Gly Met Asn Tyr Gly Ser Tyr Met Glu Glu Lys His
               20                  25                  30
   Met Pro Pro Pro Asn Met Thr Thr
           35                  40
<210> 89
   <211> 9
   <212> PRT
   <213> Homo sapiens
<400> 89
   His Met Pro Pro Pro Asn Met Thr Thr
   1 5
<210> 90
   <211> 9
   <212> PRT
   <213> Homo sapiens
<400> 90
   His Met Pro Pro Pro Asn Met Thr Thr
   1 5
<210> 91
   <211> 9
   <212> PRT
   <213> Homo sapiens
<400> 91
   His Met Pro Pro Pro Asn Met Thr Thr
   1 5
<210> 92
   <211> 12
   <212> PRT
   <213> Homo sapiens
<400> 92
   Asn Tyr Gly Ser Tyr Met Glu Glu Lys His Met Pro
   1 5 10
<210> 93
   <211> 28
   <212> PRT
   <213> Homo sapiens
<400> 93
   Met Val Gly Ser Pro Asp Thr Val Gly Met Asn Tyr Gly Ser Tyr Met
   1 5 10 15
   Glu Glu Lys His Met Pro Pro Pro Asn Met Thr Thr
   20 25
<210> 94
   <211> 9
   <212> PRT
   <213> Homo sapiens
<400> 94
   His Met Pro Pro Pro Asn Met Thr Thr
   1 5
<210> 95
   <211> 9
   <212> PRT
   <213> Homo sapiens
<400> 95
   His Met Pro Pro Pro Asn Met Thr Thr
   1 5
<210> 96
   <211> 28
   <212> PRT
   <213> Homo sapiens
<400> 96
   Met Val Gly Ser Pro Asp Thr Val Gly Met Asn Tyr Gly Ser Tyr Met
   1 5 10 15
   Glu Glu Lys His Met Pro Pro Pro Asn Met Thr Thr
   20 25
<210> 97
   <211> 28
   <212> PRT
   <213> Homo sapiens
<400> 97
   Met Val Gly Ser Pro Asp Thr Val Gly Met Asn Tyr Gly Ser Tyr Met
   1 5 10 15
   Glu Glu Lys His Met Pro Pro Pro Asn Met Thr Thr
   20 25
<210> 98
   <211> 40
   <212> PRT
   <213> Homo sapiens
<400> 98
   Asn Glu Arg Arg Val Ile Val Pro Ala Asp Pro Thr Leu Trp Ser Thr
   1 5 10 15
   Asp His Val Arg Gln Trp Leu Glu Trp Ala Val Lys Glu Tyr Gly Leu
   20 25 30
   Pro Asp Val Asn Ile Leu Leu Phe
   35 40
<210> 99
   <211> 39
   <212> PRT
   <213> Homo sapiens
<400> 99
   Asn Glu Arg Val Ile Val Pro Ala Asp Pro Thr Leu Trp Ser Thr Asp
   1               5                   10                  15
   His Val Arg Gln Trp Leu Glu Trp Ala Val Lys Glu Tyr Gly Leu Pro
                   20                  25                  30
   Asp Val Asn Ile Leu Leu Phe
           35
<210> 100
   <211> 15
   <212> PRT
   <213> Homo sapiens
<400> 100
   Asn Glu Arg Glu Tyr Gly Leu Pro Asp Val Asn Ile Leu Leu Phe
   1               5                   10                  15
<210> 101
   <211> 3
   <212> PRT
   <213> Homo sapiens
<400> 101
   Asn Glu Arg
   1
<210> 102
   <211> 27
   <212> PRT
   <213> Homo sapiens
<400> 102
   Asn Glu Arg Val Ile Val Pro Ala Asp Pro Thr Leu Trp Ser Thr Asp
   1               5                   10                  15
   His Val Arg Gln Trp Leu Glu Trp Ala Val Lys
               20                  25
<210> 103
   <211> 32
   <212> PRT
   <213> Homo sapiens
<400> 103
   Asn Glu Arg Arg Val Ile Val Pro Ala Asp Pro Thr Leu Trp Ser Thr
   1               5                   10                  15
   Asp His Val Arg Glu Tyr Gly Leu Pro Asp Val Asn Ile Leu Leu Phe
               20                  25                  30
<210> 104
   <211> 39
   <212> PRT
   <213> Homo sapiens
<400> 104
   Asn Glu Arg Val Ile Val Pro Ala Asp Pro Thr Leu Trp Ser Thr Asp
   1               5                   10                  15
   His Val Arg Gln Trp Leu Glu Trp Ala Val Lys Glu Tyr Gly Leu Pro
               20                  25                  30
   Asp Val Asn Ile Leu Leu Phe
           35
<210> 105
   <211> 40
   <212> PRT
   <213> Homo sapiens
<400> 105
   Asn Glu Arg Arg Val Ile Val Pro Ala Asp Pro Thr Leu Trp Ser Thr
   1               5                   10                  15
   Asp His Val Arg Gln Trp Leu Glu Trp Ala Val Lys Glu Tyr Gly Leu
               20                  25                  30
   Pro Asp Val Asn Ile Leu Leu Phe
           35                  40
<210> 106
   <211> 20
   <212> PRT
   <213> Homo sapiens
<400> 106
   Asn Glu Arg Arg Val Ile Val Pro Ala Asp Pro Thr Leu Trp Ser Thr
   1               5                   10                  15
   Asp His Val Arg
               20
<210> 107
   <211> 12
   <212> PRT
   <213> Homo sapiens
<400> 107
   Glu Tyr Gly Leu Pro Asp Val Asn Ile Leu Leu Phe
   1               5                   10
<210> 108
   <211> 40
   <212> PRT
   <213> Homo sapiens
<400> 108
   Gln Asn Ile Asp Gly Lys Glu Leu Cys Lys Met Thr Lys Asp Asp Phe
   1               5                   10                  15
   Gln Arg Leu Thr Pro Ser Tyr Asn Ala Asp Ile Leu Leu Ser His Leu
               20                  25                  30
   His Tyr Leu Arg Glu Thr Pro Leu
           35                  40
<210> 109
   <211> 28
   <212> PRT
   <213> Homo sapiens
<400> 109
   Gln Asn Ile Asp Gly Lys Leu Thr Pro Ser Tyr Asn Ala Asp Ile Leu
   1               5                   10                  15
   Leu Ser His Leu His Tyr Leu Arg Glu Thr Pro Leu
               20                  25
<210> 110
   <211> 4
   <212> PRT
   <213> Homo sapiens
<400> 110
   Glu Thr Pro Leu
   1
<210> 111
   <211> 10
   <212> PRT
   <213> Homo sapiens
<400> 111
   Gln Asn Ile Asp Gly Lys Glu Thr Pro Leu
   1 5 10
<210> 112
   <211> 4
   <212> PRT
   <213> Homo sapiens
<400> 112
   Glu Thr Pro Leu
   1
<210> 113
   <211> 4
   <212> PRT
   <213> Homo sapiens
<400> 113
   Glu Thr Pro Leu
   1
<210> 114
   <211> 28
   <212> PRT
   <213> Homo sapiens
<400> 114
   Gln Asn Ile Asp Gly Lys Leu Thr Pro Ser Tyr Asn Ala Asp Ile Leu
   1 5 10 15
   Leu Ser His Leu His Tyr Leu Arg Glu Thr Pro Leu
   20 25
<210> 115
   <211> 4
   <212> PRT
   <213> Homo sapiens
<400> 115
   Glu Thr Pro Leu
   1
<210> 116
   <211> 28
   <212> PRT
   <213> Homo sapiens
<400> 116
   Gln Asn Ile Asp Gly Lys Leu Thr Pro Ser Tyr Asn Ala Asp Ile Leu
   1               5                   10                  15
   Leu Ser His Leu His Tyr Leu Arg Glu Thr Pro Leu
               20                  25
<210> 117
   <211> 28
   <212> PRT
   <213> Homo sapiens
<400> 117
   Gln Asn Ile Asp Gly Lys Leu Thr Pro Ser Tyr Asn Ala Asp Ile Leu
   1               5                   10                  15
   Leu Ser His Leu His Tyr Leu Arg Glu Thr Pro Leu
               20                  25
<210> 118
   <211> 22
   <212> PRT
   <213> Homo sapiens
<400> 118
   Leu Thr Pro Ser Tyr Asn Ala Asp Ile Leu Leu Ser His Leu His Tyr
   1               5                   10                  15
   Leu Arg Glu Thr Pro Leu
               20
<210> 119
   <211> 6
   <212> PRT
   <213> Homo sapiens
<400> 119
   Gln Asn Ile Asp Gly Lys
   1               5
<210> 120
   <211> 20
   <212> PRT
   <213> Homo sapiens
<400> 120
   Pro Ser Tyr Asn Ala Asp Ile Leu Leu Ser His Leu His Tyr Leu Arg
   1 5 10 15
   Glu Thr Pro Leu
   20
<210> 121
   <211> 40
   <212> PRT
   <213> Homo sapiens
<400> 121
   Pro His Leu Thr Ser Asp Asp Val Asp Lys Ala Leu Gln Asn Ser Pro
   1 5 10 15
   Arg Leu Met His Ala Arg Asn Thr Gly Gly Ala Ala Phe Ile Phe Pro
   20 25 30
   Asn Thr Ser Val Tyr Pro Glu Ala
   35 40
<210> 122
   <211> 9
   <212> PRT
   <213> Homo sapiens
<400> 122
   Pro Arg Leu Met His Ala Arg Asn Thr
   1 5
<210> 123
   <211> 10
   <212> PRT
   <213> Homo sapiens
<400> 123
   Pro His Leu Thr Ser Asp Asp Val Asp Lys
   1 5 10
<210> 124
   <211> 17
   <212> PRT
   <213> Homo sapiens
<400> 124
   Pro His Leu Thr Ser Asp Asp Val Asp Lys Ala Leu Gln Asn Ser Pro
   1 5 10 15
   Arg
<210> 125
   <211> 17
   <212> PRT
   <213> Homo sapiens
<400> 125
   Pro His Leu Thr Ser Asp Asp Val Asp Lys Ala Leu Gln Asn Ser Pro
   1 5 10 15
   Arg
<210> 126
   <211> 17
   <212> PRT
   <213> Homo sapiens
<400> 126
   Pro His Leu Thr Ser Asp Asp Val Asp Lys Ala Leu Gln Asn Ser Pro
   1 5 10 15
   Arg
<210> 127
   <211> 17
   <212> PRT
   <213> Homo sapiens
<400> 127
   Pro His Leu Thr Ser Asp Asp Val Asp Lys Ala Leu Gln Asn Ser Pro
   1 5 10 15
   Arg
<210> 128
   <211> 10
   <212> PRT
   <213> Homo sapiens
<400> 128
   Pro His Leu Thr Ser Asp Asp Val Asp Lys
   1 5 10
<210> 129
   <211> 17
   <212> PRT
   <213> Homo sapiens
<400> 129
   Pro His Leu Thr Ser Asp Asp Val Asp Lys Ala Leu Gln Asn Ser Pro
   1 5 10 15
   Arg
<210> 130
   <211> 17
   <212> PRT
   <213> Homo sapiens
<400> 130
   Pro His Leu Thr Ser Asp Asp Val Asp Lys Ala Leu Gln Asn Ser Pro
   1 5 10 15
   Arg
<210> 131
   <211> 17
   <212> PRT
   <213> Homo sapiens
<400> 131
   Pro His Leu Thr Ser Asp Asp Val Asp Lys Ala Leu Gln Asn Ser Pro
   1 5 10 15
   Arg
<210> 132
   <211> 3
   <212> PRT
   <213> Homo sapiens
<400> 132
   Arg Asn Thr
   1
<210> 133
   <211> 17
   <212> PRT
   <213> Homo sapiens
<400> 133
   Pro His Leu Thr Ser Asp Asp Val Asp Lys Ala Leu Gln Asn Ser Pro
   1 5 10 15
   Arg
<210> 134
   <211> 18
   <212> PRT
   <213> Homo sapiens
<400> 134
   Pro His Leu Thr Ser Asp Asp Val Asp Lys Ala Leu Gln Asn Ser Pro
   1 5 10 15
   Arg Leu
<210> 135
   <211> 40
   <212> PRT
   <213> Homo sapiens
<400> 135
   Thr Gln Arg Ile Thr Thr Arg Pro Asp Leu Pro Tyr Glu Pro Pro Arg
   1 5 10 15
   Arg Ser Ala Trp Thr Gly His Gly His Pro Thr Pro Gln Ser Lys Ala
   20 25 30
   Ala Gln Pro Ser Pro Ser Thr Val
   35 40
<210> 136
   <211> 8
   <212> PRT
   <213> Homo sapiens
<400> 136
   Asp Leu Pro Tyr Glu Pro Pro Arg
   1 5
<210> 137
   <211> 23
   <212> PRT
   <213> Homo sapiens
<400> 137
   Ser Ala Trp Thr Gly His Gly His Pro Thr Pro Gln Ser Lys Ala Ala
   1               5                   10                  15
   Gln Pro Ser Pro Ser Thr Val
               20
<210> 138
   <211> 7
   <212> PRT
   <213> Homo sapiens
<400> 138
   Pro Tyr Glu Pro Pro Arg Arg
   1               5
<210> 139
   <211> 23
   <212> PRT
   <213> Homo sapiens
<400> 139
   Ser Ala Trp Thr Gly His Gly His Pro Thr Pro Gln Ser Lys Ala Ala
   1               5                   10                  15
   Gln Pro Ser Pro Ser Thr Val
               20
<210> 140
   <211> 23
   <212> PRT
   <213> Homo sapiens
<400> 140
   Ser Ala Trp Thr Gly His Gly His Pro Thr Pro Gln Ser Lys Ala Ala
   1               5                   10                  15
   Gln Pro Ser Pro Ser Thr Val
               20
<210> 141
   <211> 30
   <212> PRT
   <213> Homo sapiens
<400> 141
   Pro Tyr Glu Pro Pro Arg Arg Ser Ala Trp Thr Gly His Gly His Pro
   1               5                   10                  15
   Thr Pro Gln Ser Lys Ala Ala Gln Pro Ser Pro Ser Thr Val
               20                  25                  30
<210> 142
   <211> 23
   <212> PRT
   <213> Homo sapiens
<400> 142
   Ser Ala Trp Thr Gly His Gly His Pro Thr Pro Gln Ser Lys Ala Ala
   1               5                   10                  15
   Gln Pro Ser Pro Ser Thr Val
               20
<210> 143
   <211> 23
   <212> PRT
   <213> Homo sapiens
<400> 143
   Ser Ala Trp Thr Gly His Gly His Pro Thr Pro Gln Ser Lys Ala Ala
   1               5                   10                  15
   Gln Pro Ser Pro Ser Thr Val
               20
<210> 144
   <211> 9
   <212> PRT
   <213> Homo sapiens
<400> 144
   Asp Leu Pro Tyr Glu Pro Pro Arg Arg
   1               5
<210> 145
   <211> 30
   <212> PRT
   <213> Homo sapiens
<400> 145
   Pro Tyr Glu Pro Pro Arg Arg Ser Ala Trp Thr Gly His Gly His Pro
   1               5                   10                  15
   Thr Pro Gln Ser Lys Ala Ala Gln Pro Ser Pro Ser Thr Val
             20                  25                  30
<210> 146
   <211> 32
   <212> PRT
   <213> Homo sapiens
<400> 146
   Asp Leu Pro Tyr Glu Pro Pro Arg Arg Ser Ala Trp Thr Gly His Gly
   1               5                   10                  15
   His Pro Thr Pro Gln Ser Lys Ala Ala Gln Pro Ser Pro Ser Thr Val
               20                  25                  30
<210> 147
   <211> 9
   <212> PRT
   <213> Homo sapiens
<400> 147
   Asp Leu Pro Tyr Glu Pro Pro Arg Arg
   1               5
<210> 148
   <211> 32
   <212> PRT
   <213> Homo sapiens
<400> 148
   Asp Leu Pro Tyr Glu Pro Pro Arg Arg Ser Ala Trp Thr Gly His Gly
   1               5                   10                  15
   His Pro Thr Pro Gln Ser Lys Ala Ala Gln Pro Ser Pro Ser Thr Val
               20                  25                  30
<210> 149
   <211> 9
   <212> PRT
   <213> Homo sapiens
<400> 149
   Ala Ala Gln Pro Ser Pro Ser Thr Val
   1               5
<210> 150
   <211> 23
   <212> PRT
   <213> Homo sapiens
<400> 150
   Ser Ala Trp Thr Gly His Gly His Pro Thr Pro Gln Ser Lys Ala Ala
   1               5                   10                  15
   Gln Pro Ser Pro Ser Thr Val
               20
<210> 151
   <211> 32
   <212> PRT
   <213> Homo sapiens
<400> 151
   Asp Leu Pro Tyr Glu Pro Pro Arg Arg Ser Ala Trp Thr Gly His Gly
   1               5                   10                  15
   His Pro Thr Pro Gln Ser Lys Ala Ala Gln Pro Ser Pro Ser Thr Val
               20                  25                  30
<210> 152
   <211> 40
   <212> PRT
   <213> Homo sapiens
<400> 152
   Pro Lys Thr Glu Asp Gln Arg Pro Gln Leu Asp Pro Tyr Gln Ile Leu
   1               5                   10                  15
   Gly Pro Thr Ser Ser Arg Leu Ala Asn Pro Gly Ser Gly Gln Ile Gln
               20                  25                  30
   Leu Trp Gln Phe Leu Leu Glu Leu
           35                  40
<210> 153
   <211> 2
   <212> PRT
   <213> Homo sapiens
<400> 153
   Pro Lys
   1
<210> 154
   <211> 20
   <212> PRT
   <213> Homo sapiens
<400> 154
   Thr Glu Asp Gln Arg Pro Gln Leu Asp Pro Tyr Gln Ile Leu Gly Pro
   1               5                   10                  15
   Thr Ser Ser Arg
               20
<210> 155
   <211> 22
   <212> PRT
   <213> Homo sapiens
<400> 155
   Pro Lys Thr Glu Asp Gln Arg Pro Gln Leu Asp Pro Tyr Gln Ile Leu
   1               5                   10                  15
   Gly Pro Thr Ser Ser Arg
               20
<210> 156
   <211> 22
   <212> PRT
   <213> Homo sapiens
<400> 156
   Pro Lys Thr Glu Asp Gln Arg Pro Gln Leu Asp Pro Tyr Gln Ile Leu
   1               5                   10                  15
   Gly Pro Thr Ser Ser Arg
               20
<210> 157
   <211> 22
   <212> PRT
   <213> Homo sapiens
<400> 157
   Pro Lys Thr Glu Asp Gln Arg Pro Gln Leu Asp Pro Tyr Gln Ile Leu
   1               5                   10                  15
   Gly Pro Thr Ser Ser Arg
               20
<210> 158
   <211> 22
   <212> PRT
   <213> Homo sapiens
<400> 158
   Pro Lys Thr Glu Asp Gln Arg Pro Gln Leu Asp Pro Tyr Gln Ile Leu
   1               5                   10                  15
   Gly Pro Thr Ser Ser Arg
               20
<210> 159
   <211> 22
   <212> PRT
   <213> Homo sapiens
<400> 159
   Pro Lys Thr Glu Asp Gln Arg Pro Gln Leu Asp Pro Tyr Gln Ile Leu
   1 5 10 15
   Gly Pro Thr Ser Ser Arg
   20
<210> 160
   <211> 20
   <212> PRT
   <213> Homo sapiens
<400> 160
   Thr Glu Asp Gln Arg Pro Gln Leu Asp Pro Tyr Gln Ile Leu Gly Pro
   1 5 10 15
   Thr Ser Ser Arg
   20
<210> 161
   <211> 22
   <212> PRT
   <213> Homo sapiens
<400> 161
   Pro Lys Thr Glu Asp Gln Arg Pro Gln Leu Asp Pro Tyr Gln Ile Leu
   1 5 10 15
   Gly Pro Thr Ser Ser Arg
   20
<210> 162
   <211> 22
   <212> PRT
   <213> Homo sapiens
<400> 162
   Pro Lys Thr Glu Asp Gln Arg Pro Gln Leu Asp Pro Tyr Gln Ile Leu
   1 5 10 15
   Gly Pro Thr Ser Ser Arg
   20
<210> 163
   <211> 22
   <212> PRT
   <213> Homo sapiens
<400> 163
   Pro Lys Thr Glu Asp Gln Arg Pro Gln Leu Asp Pro Tyr Gln Ile Leu
   1 5 10 15
   Gly Pro Thr Ser Ser Arg
   20
<210> 164
   <211> 2
   <212> PRT
   <213> Homo sapiens
<400> 164
   Pro Lys
   1
<210> 165
   <211> 22
   <212> PRT
   <213> Homo sapiens
<400> 165
   Pro Lys Thr Glu Asp Gln Arg Pro Gln Leu Asp Pro Tyr Gln Ile Leu
   1 5 10 15
   Gly Pro Thr Ser Ser Arg
   20
<210> 166
   <211> 22
   <212> PRT
   <213> Homo sapiens
<400> 166
   Pro Lys Thr Glu Asp Gln Arg Pro Gln Leu Asp Pro Tyr Gln Ile Leu
   1 5 10 15
   Gly Pro Thr Ser Ser Arg
   20
<210> 167
   <211> 2
   <212> PRT
   <213> Homo sapiens
<400> 167
   Pro Lys
   1
<210> 168
   <211> 40
   <212> PRT
   <213> Homo sapiens
<400> 168
   Leu Ser Asp Ser Ser Asn Ser Ser Cys Ile Thr Trp Glu Gly Thr Asn
   1               5                   10                  15
   Gly Glu Phe Lys Met Thr Asp Pro Asp Glu Val Ala Arg Arg Trp Gly
               20                  25                  30
   Glu Arg Lys Ser Lys Pro Asn Met
           35                  40
<210> 169
   <211> 9
   <212> PRT
   <213> Homo sapiens
<400> 169
   Met Thr Asp Pro Asp Glu Val Ala Arg
   1               5
<210> 170
   <211> 9
   <212> PRT
   <213> Homo sapiens
<400> 170
   Met Thr Asp Pro Asp Glu Val Ala Arg
   1               5
<210> 171
   <211> 9
   <212> PRT
   <213> Homo sapiens
<400> 171
   Met Thr Asp Pro Asp Glu Val Ala Arg
   1               5
<210> 172
   <211> 9
   <212> PRT
   <213> Homo sapiens
<400> 172
   Met Thr Asp Pro Asp Glu Val Ala Arg
   1 5
<210> 173
   <211> 9
   <212> PRT
   <213> Homo sapiens
<400> 173
   Met Thr Asp Pro Asp Glu Val Ala Arg
   1 5
<210> 174
   <211> 10
   <212> PRT
   <213> Homo sapiens
<400> 174
   Met Thr Asp Pro Asp Glu Val Ala Arg Arg
   1 5 10
<210> 175
   <211> 15
   <212> PRT
   <213> Homo sapiens
<400> 175
   Thr Asp Pro Asp Glu Val Ala Arg Arg Lys Ser Lys Pro Asn Met
   1 5 10 15
<210> 176
   <211> 9
   <212> PRT
   <213> Homo sapiens
<400> 176
   Met Thr Asp Pro Asp Glu Val Ala Arg
   1 5
<210> 177
   <211> 6
   <212> PRT
   <213> Homo sapiens
<400> 177
   Lys Ser Lys Pro Asn Met
   1 5
<210> 178
   <211> 40
   <212> PRT
   <213> Homo sapiens
<400> 178
   Asn Tyr Asp Lys Leu Ser Arg Ala Leu Arg Tyr Tyr Tyr Asp Lys Asn
   1               5                   10                  15
   Ile Met Thr Lys Val His Gly Lys Arg Tyr Ala Tyr Lys Phe Asp Phe
               20                  25                  30
   His Gly Ile Ala Gln Ala Leu Gln
           35                  40
<210> 179
   <211> 11
   <212> PRT
   <213> Homo sapiens
<400> 179
   Phe Asp Phe His Gly Ile Ala Gln Ala Leu Gln
   1               5                   10
<210> 180
   <211> 11
   <212> PRT
   <213> Homo sapiens
<400> 180
   Phe Asp Phe His Gly Ile Ala Gln Ala Leu Gln
   1               5                   10
<210> 181
   <211> 11
   <212> PRT
   <213> Homo sapiens
<400> 181
   Phe Asp Phe His Gly Ile Ala Gln Ala Leu Gln
   1               5                   10
<210> 182
   <211> 11
   <212> PRT
   <213> Homo sapiens
<400> 182
   Phe Asp Phe His Gly Ile Ala Gln Ala Leu Gln
   1               5                   10
<210> 183
   <211> 25
   <212> PRT
   <213> Homo sapiens
<400> 183
   Tyr Tyr Tyr Asp Lys Asn Ile Met Thr Lys Tyr Ala Tyr Lys Phe Asp
   1 5 10 15
   Phe His Gly Ile Ala Gln Ala Leu Gln
   20 25
<210> 184
   <211> 7
   <212> PRT
   <213> Homo sapiens
<400> 184
   Asn Tyr Asp Lys Leu Ser Arg
   1 5
<210> 185
   <211> 17
   <212> PRT
   <213> Homo sapiens
<400> 185
   Asn Tyr Asp Lys Leu Ser Arg Tyr Tyr Tyr Asp Lys Asn Ile Met Thr
   1 5 10 15
   Lys
<210> 186
   <211> 40
   <212> PRT
   <213> Homo sapiens
<400> 186
   Pro His Pro Pro Glu Ser Ser Leu Tyr Lys Tyr Pro Ser Asp Leu Pro
   1 5 10 15
   Tyr Met Gly Ser Tyr His Ala His Pro Gln Lys Met Asn Phe Val Ala
   20 25 30
   Pro His Pro Pro Ala Leu Pro Val
   35 40
<210> 187
   <211> 10
   <212> PRT
   <213> Homo sapiens
<400> 187
   Pro His Pro Pro Glu Ser Ser Leu Tyr Lys
   1 5 10
<210> 188
   <211> 24
   <212> PRT
   <213> Homo sapiens
<400> 188
   Pro His Pro Pro Glu Ser Ser Leu Tyr Lys Tyr Pro Ser Asp Leu Pro
   1 5 10 15
   Tyr Met Gly Ser Tyr His Ala His
   20
<210> 189
   <211> 27
   <212> PRT
   <213> Homo sapiens
<400> 189
   Pro His Pro Pro Glu Ser Ser Leu Tyr Lys Tyr Pro Ser Asp Leu Pro
   1 5 10 15
   Tyr Met Gly Ser Tyr His Ala His Pro Gln Lys
   20 25
<210> 190
   <211> 27
   <212> PRT
   <213> Homo sapiens
<400> 190
   Pro His Pro Pro Glu Ser Ser Leu Tyr Lys Tyr Pro Ser Asp Leu Pro
   1 5 10 15
   Tyr Met Gly Ser Tyr His Ala His Pro Gln Lys
   20 25
<210> 191
   <211> 17
   <212> PRT
   <213> Homo sapiens
<400> 191
   Tyr Pro Ser Asp Leu Pro Tyr Met Gly Ser Tyr His Ala His Pro Gln
   1 5 10 15
   Lys
<210> 192
   <211> 27
   <212> PRT
   <213> Homo sapiens
<400> 192
   Pro His Pro Pro Glu Ser Ser Leu Tyr Lys Tyr Pro Ser Asp Leu Pro
   1               5                   10                  15
   Tyr Met Gly Ser Tyr His Ala His Pro Gln Lys
               20                  25
<210> 193
   <211> 39
   <212> PRT
   <213> Homo sapiens
<400> 193
   Thr Ser Ser Ser Phe Phe Ala Ala Pro Asn Pro Tyr Trp Asn Ser Pro
   1               5                   10                  15
   Thr Gly Gly Ile Tyr Pro Asn Thr Arg Leu Pro Thr Ser His Met Pro
               20                  25                  30
   Ser His Leu Gly Thr Tyr Tyr
           35
<210> 194
   <211> 5
   <212> PRT
   <213> Homo sapiens
<400> 194
   Asn Ser Pro Thr Gly
   1               5
<210> 195
   <211> 11
   <212> PRT
   <213> Homo sapiens
<400> 195
   Ser Pro Thr Gly Gly Ile Tyr Pro Asn Thr Arg
   1               5                   10
<210> 196
   <211> 24
   <212> PRT
   <213> Homo sapiens
<400> 196
   Gly Gly Ala Ala Phe Ile Phe Pro Asn Thr Ser Val Tyr Pro Glu Ala
   1               5                   10                  15
   Thr Gln Arg Ile Thr Thr Arg Pro
               20
<210> 197
   <211> 10
   <212> PRT
   <213> Homo sapiens
<400> 197
   Val Pro Gln Gln Asp Trp Leu Ser Gln Pro
   1               5                   10
<210> 198
   <211> 10
   <212> PRT
   <213> Homo sapiens
<400> 198
   Val Pro Gln Gln Asp Trp Leu Ser Gln Pro
   1               5                   10
<210> 199
   <211> 12
   <212> PRT
   <213> Homo sapiens
<400> 199
   Met Ile Gln Thr Val Pro Asp Pro Ala Ala His Ile
   1               5                   10
<210> 200
   <211> 55
   <212> PRT
   <213> Homo sapiens
<400> 200
   Met Ala Ser Thr Ile Lys Glu Ala Leu Ser Val Val Ser Glu Asp Gln
   1               5                   10                  15
   Ser Leu Phe Glu Cys Ala Tyr Gly Thr Pro His Leu Ala Lys Thr Glu
               20                  25                  30
   Met Thr Ala Tyr Gly Gln Thr Ser Lys Met Ser Pro Arg Val Pro Gln
           35                  40                  45
   Gln Asp Trp Leu Ser Gln Pro
       50                  55
<210> 201
   <211> 12
   <212> PRT
   <213> Homo sapiens
<400> 201
   Met Ile Gln Thr Val Pro Asp Pro Ala Ala His Ile
   1 5 10
<210> 202
   <211> 23
   <212> DNA
   <213> Homo sapiens
<400> 202
   gctgcagact tggccaaatg gac 23
<210> 203
   <211> 23
   <212> DNA
   <213> Homo sapiens
<400> 203
   tcaccaccga cagagcctcc tta 23
<210> 204
   <211> 26
   <212> DNA
   <213> Homo sapiens
<400> 204
   accacatgaa tggatccagg gagtct 26
<210> 205
   <211> 24
   <212> DNA
   <213> Homo sapiens
<400> 205
   accagcttgc tgcatttgct aacg 24
<210> 206
   <211> 20
   <212> DNA
   <213> Homo sapiens
<400> 206
   ctccgcgcca ccaccctcta 20
<210> 207
   <211> 24
   <212> DNA
   <213> Homo sapiens
<400> 207
   ggccagcagt gaactttccc tgag 24
<210> 208
   <211> 23
   <212> DNA
   <213> Homo sapiens
<400> 208
   ctcctcccaa catgaccacc aac 23
<210> 209
   <211> 22
   <212> DNA
   <213> Homo sapiens
<400> 209
   gtctgcgggg acgatgactc tc 22
<210> 210
   <211> 23
   <212> DNA
   <213> Homo sapiens
<400> 210
   catgtgaggc aatggctgga gtg 23
<210> 211
   <211> 26
   <212> DNA
   <213> Homo sapiens
<400> 211
   ccatgttctg gaaaaaggat gtgtcg 26
<210> 212
   <211> 21
   <212> DNA
   <213> Homo sapiens
<400> 212
   ccttggaggg gcacaaacga t 21
<210> 213
   <211> 22
   <212> DNA
   <213> Homo sapiens
<400> 213
   ggtcgggccc aggatctgat ac 22
<210> 214
   <211> 21
   <212> DNA
   <213> Homo sapiens
<400> 214
   cgccaacgcc agctgtatca c 21
<210> 215
   <211> 19
   <212> DNA
   <213> Homo sapiens
<400> 215
   agcgcctggc cacctcatc 19
<210> 216
   <211> 30
   <212> DNA
   <213> Homo sapiens
<400> 216
   atgtttttat gaccaaagca gtttcttgtc 30
<210> 217
   <211> 27
   <212> DNA
   <213> Homo sapiens
<400> 217
   atgacgggtt aagtccatga ttctgtg 27
<210> 218
   <211> 21
   <212> DNA
   <213> Homo sapiens
<400> 218
   cgaaagctgc tcaaccatct c 21
<210> 219
   <211> 22
   <212> DNA
   <213> Homo sapiens
<400> 219
   taactgagga cgctggtctt ca 22
<210> 220
   <211> 15
   <212> DNA
   <213> Homo sapiens
<400> 220
   ccacagtgcc caaaa 15
<210> 221
<400> 221
   000
<210> 222
   <211> 25
   <212> DNA
   <213> Homo sapiens
<400> 222
   cggcaaaaga tatgcttaca aattt 25
<210> 223
   <211> 19
   <212> DNA
   <213> Homo sapiens
<400> 223
   gacgactcgg tcggatgtg 19
<210> 224
   <211> 14
   <212> DNA
   <213> Homo sapiens
<400> 224
   cacggcattg ccca 14
<210> 225
   <211> 17
   <212> DNA
   <213> Homo sapiens
<400> 225
   cgcggcagga agcctta 17
<210> 226
   <211> 22
   <212> DNA
   <213> Homo sapiens
<400> 226
   tccgtaggca cactcaaaca ac 22
<210> 227
   <211> 18
   <212> DNA
   <213> Homo sapiens
<400> 227
   cagttgtgag tgaggacc 18
<210> 228
   <211> 25
   <212> DNA
   <213> Homo sapiens
<400> 228
   gctcgctccg atactattat gagaa 25
<210> 229
   <211> 25
   <212> DNA
   <213> Homo sapiens
<400> 229
   cacacacaaa cttgtacacg taacg 25
<210> 230
   <211> 16
   <212> DNA
   <213> Homo sapiens
<400> 230
   accagccacc ttctgc 16
<210> 231
   <211> 25
   <212> DNA
   <213> Homo sapiens
<400> 231
   ggtcgaggca tggaatttaa actga 25
<210> 232
   <211> 22
   <212> DNA
   <213> Homo sapiens
<400> 232
   gctggcctgt ttttctgaat gc 22
<210> 233
   <211> 15
   <212> DNA
   <213> Homo sapiens
<400> 233
   tcgggccacc tcttc 15
<210> 234
   <211> 12
   <212> PRT
   <213> Homo sapiens
<400> 234
   Met Ile Gln Thr Val Pro Asp Pro Ala Ala His Ile
   1               5                   10
<210> 235
   <211> 23
   <212> PRT
   <213> Homo sapiens
<400> 235
   Ser Ala Trp Thr Gly His Gly His Pro Thr Pro Gln Ser Lys Ala Ala
   1               5                   10                  15
   Gln Pro Ser Pro Ser Thr Val
               20
<210> 236
   <211> 42
   <212> PRT
   <213> Homo sapiens
<220>
   <221> NON_CONS
   <222> (18)..(19)
<400> 236
   Met Ile Gln Thr Val Pro Asp Pro Ala Ala Ser His Ile Lys Glu Ala
   1               5                   10                  15
   Leu Ser Gly Gly Ala Ala Phe Ile Phe Pro Asn Thr Ser Val Tyr Pro
               20                  25                  30
   Glu Ala Thr Gln Arg Ile Thr Thr Arg Pro
           35                  40
<210> 237
   <211> 18
   <212> PRT
   <213> Homo sapiens
<400> 237
   Met Ile Gln Thr Val Pro Asp Pro Ala Ala Ser His Ile Lys Glu Ala
   1               5                   10                  15
   Leu Ser
<210> 238
   <211> 42
   <212> PRT
   <213> Homo sapiens
<220>
   <221> NON_CONS
   <222> (10)..(11)
<220>
   <221> NON_CONS
   <222> (14)..(15)
<220>
   <221> NON_CONS
   <222> (18)..(19)
<400> 238
   Met Ala Ser Thr Ile Lys Glu Ala Leu Ser Met Thr Ala Ser Met Ser
   1               5                   10                  15
   Pro Arg Gly Gly Ala Ala Phe Ile Phe Pro Asn Thr Ser Val Tyr Pro
               20                  25                  30
   Glu Ala Thr Gln Arg Ile Thr Thr Arg Pro
           35                  40
<210> 239
   <211> 18
   <212> PRT
   <213> Homo sapiens
<220>
   <221> NON_CONS
   <222> (10)..(11)
<220>
   <221> NON_CONS
   <222> (14)..(15)
<400> 239
   Met Ala Ser Thr Ile Lys Glu Ala Leu Ser Met Thr Ala Ser Met Ser
   1               5                   10                  15
   Pro Arg
<210> 240
   <211> 479
   <212> PRT
   <213> Homo sapiens
<400> 240
   Met Ala Ser Thr Ile Lys Glu Ala Leu Ser Val Val Ser Glu Asp Gln
   1               5                   10                  15
   Ser Leu Phe Glu Cys Ala Tyr Gly Thr Pro His Leu Ala Lys Thr Glu
               20                  25                  30
   Met Thr Ala Ser Ser Ser Ser Asp Tyr Gly Gln Thr Ser Lys Met Ser
           35                  40                  45
   Pro Arg Val Pro Gln Gln Asp Trp Leu Ser Gln Pro Pro Ala Arg Val
       50                  55                  60
   Thr Ile Lys Met Glu Cys Asn Pro Ser Gln Val Asn Gly Ser Arg Asn
   65                  70                  75                  80
   Ser Pro Asp Glu Cys Ser Val Ala Lys Gly Gly Lys Met Val Gly Ser
                   85                  90                  95
   Pro Asp Thr Val Gly Met Asn Tyr Gly Ser Tyr Met Glu Glu Lys His
               100                 105                 110
   Met Pro Pro Pro Asn Met Thr Thr Asn Glu Arg Arg Val Ile Val Pro
           115                 120                 125
   Ala Asp Pro Thr Leu Trp Ser Thr Asp His Val Arg Gln Trp Leu Glu
       130                 135                 140
   Trp Ala Val Lys Glu Tyr Gly Leu Pro Asp Val Asn Ile Leu Leu Phe
   145                 150                 155                 160
   Gln Asn Ile Asp Gly Lys Glu Leu Cys Lys Met Thr Lys Asp Asp Phe
                   165                 170                 175
   Gln Arg Leu Thr Pro Ser Tyr Asn Ala Asp Ile Leu Leu Ser His Leu
               180                 185                 190
   His Tyr Leu Arg Glu Thr Pro Leu Pro His Leu Thr Ser Asp Asp Val
           195                 200                 205
   Asp Lys Ala Leu Gln Asn Ser Pro Arg Leu Met His Ala Arg Asn Thr
       210                 215                 220
   Gly Gly Ala Ala Phe Ile Phe Pro Asn Thr Ser Val Tyr Pro Glu Ala
   225                 230                 235                 240
   Thr Gln Arg Ile Thr Thr Arg Pro Asp Leu Pro Tyr Glu Pro Pro Arg
                   245                 250                 255
   Arg Ser Ala Trp Thr Gly His Gly His Pro Thr Pro Gln Ser Lys Ala
               260                 265                 270
   Ala Gln Pro Ser Pro Ser Thr Val Pro Lys Thr Glu Asp Gln Arg Pro
           275                 280                 285
   Gln Leu Asp Pro Tyr Gln Ile Leu Gly Pro Thr Ser Ser Arg Leu Ala
       290                 295                 300
   Asn Pro Gly Ser Gly Gln Ile Gln Leu Trp Gln Phe Leu Leu Glu Leu
   305                 310                 315                 320
   Leu Ser Asp Ser Ser Asn Ser Ser Cys Ile Thr Trp Glu Gly Thr Asn
                   325                 330                 335
   Gly Glu Phe Lys Met Thr Asp Pro Asp Glu Val Ala Arg Arg Trp Gly
               340                 345                 350
   Glu Arg Lys Ser Lys Pro Asn Met Asn Tyr Asp Lys Leu Ser Arg Ala
           355                 360                 365
   Leu Arg Tyr Tyr Tyr Asp Lys Asn Ile Met Thr Lys Val His Gly Lys
       370                 375                 380
   Arg Tyr Ala Tyr Lys Phe Asp Phe His Gly Ile Ala Gln Ala Leu Gln
   385                 390                 395                 400
   Pro His Pro Pro Glu Ser Ser Leu Tyr Lys Tyr Pro Ser Asp Leu Pro
                   405                 410                 415
   Tyr Met Gly Ser Tyr His Ala His Pro Gln Lys Met Asn Phe Val Ala
               420                 425                 430
   Pro His Pro Pro Ala Leu Pro Val Thr Ser Ser Ser Phe Phe Ala Ala
           435                 440                 445
   Pro Asn Pro Tyr Trp Asn Ser Pro Thr Gly Gly Ile Tyr Pro Asn Thr
       450                 455                 460
   Arg Leu Pro Thr Ser His Met Pro Ser His Leu Gly Thr Tyr Tyr
   465                 470                 475
<210> 241
   <211> 13
   <212> PRT
   <213> Homo sapiens
<400> 241
   Gly Ala Ala Phe Ile Phe Pro Asn Thr Ser Val Tyr Pro
   1 5 10
<210> 242
   <211> 477
   <212> PRT
   <213> Homo sapiens
<400> 242
   Met Asp Gly Phe Tyr Asp Gln Gln Val Pro Tyr Met Val Thr Asn Ser
   1               5                   10                  15
   Gln Arg Gly Arg Asn Cys Asn Glu Lys Pro Thr Asn Val Arg Lys Arg
               20                  25                  30
   Lys Phe Ile Asn Arg Asp Leu Ala His Asp Ser Glu Glu Leu Phe Gln
           35                  40                  45
   Asp Leu Ser Gln Leu Gln Glu Thr Trp Leu Ala Glu Ala Gln Val Pro
       50                  55                  60
   Asp Asn Asp Glu Gln Phe Val Pro Asp Tyr Gln Ala Glu Ser Leu Ala
   65                  70                  75                  80
   Phe His Gly Leu Pro Leu Lys Ile Lys Lys Glu Pro His Ser Pro Cys
                   85                  90                  95
   Ser Glu Ile Ser Ser Ala Cys Ser Gln Glu Gln Pro Phe Lys Phe Ser
               100                 105                 110
   Tyr Gly Glu Lys Cys Leu Tyr Asn Val Ser Ala Tyr Asp Gln Lys Pro
           115                 120                 125
   Gln Val Gly Met Arg Pro Ser Asn Pro Pro Thr Pro Ser Ser Thr Pro
       130                 135                 140
   Val Ser Pro Leu His His Ala Ser Pro Asn Ser Thr His Thr Pro Lys
   145                 150                 155                 160
   Pro Asp Arg Ala Phe Pro Ala His Leu Pro Pro Ser Gln Ser Ile Pro
                   165                 170                 175
   Asp Ser Ser Tyr Pro Met Asp His Arg Phe Arg Arg Gln Leu Ser Glu
               180                 185                 190
   Pro Cys Asn Ser Phe Pro Pro Leu Pro Thr Met Pro Arg Glu Gly Arg
   195 200 205
   Pro Met Tyr Gln Arg Gln Met Ser Glu Pro Asn Ile Pro Phe Pro Pro
   210 215 220
   Gln Gly Phe Lys Gln Glu Tyr His Asp Pro Val Tyr Glu His Asn Thr
   225 230 235 240
   Met Val Gly Ser Ala Ala Ser Gln Ser Phe Pro Pro Pro Leu Met Ile
   245 250 255
   Lys Gln Glu Pro Arg Asp Phe Ala Tyr Asp Ser Glu Val Pro Ser Cys
   260 265 270
   His Ser Ile Tyr Met Arg Gln Glu Gly Phe Leu Ala His Pro Ser Arg
   275 280 285
   Thr Glu Gly Cys Met Phe Glu Lys Gly Pro Arg Gln Phe Tyr Asp Asp
   290 295 300
   Thr Cys Val Val Pro Glu Lys Phe Asp Gly Asp Ile Lys Gln Glu Pro
   305 310 315 320
   Gly Met Tyr Arg Glu Gly Pro Thr Tyr Gln Arg Arg Gly Ser Leu Gln
   325 330 335
   Leu Trp Gln Phe Leu Val Ala Leu Leu Asp Asp Pro Ser Asn Ser His
   340 345 350
   Phe Ile Ala Trp Thr Gly Arg Gly Met Glu Phe Lys Leu Ile Glu Pro
   355 360 365
   Glu Glu Val Ala Arg Arg Trp Gly Ile Gln Lys Asn Arg Pro Ala Met
   370 375 380
   Asn Tyr Asp Lys Leu Ser Arg Ser Leu Arg Tyr Tyr Tyr Glu Lys Gly
   385 390 395 400
   Ile Met Gln Lys Val Ala Gly Glu Arg Tyr Val Tyr Lys Phe Val Cys
   405 410 415
   Asp Pro Glu Ala Leu Phe Ser Met Ala Phe Pro Asp Asn Gln Arg Pro
   420 425 430
   Leu Leu Lys Thr Asp Met Glu Arg His Ile Asn Glu Glu Asp Thr Val
   435 440 445
   Pro Leu Ser His Phe Asp Glu Ser Met Ala Tyr Met Pro Glu Gly Gly
   450 455 460
   Cys Cys Asn Pro His Pro Tyr Asn Glu Gly Tyr Val Tyr
   465 470 475
<210> 243
   <211> 14
   <212> PRT
   <213> Homo sapiens
<400> 243
   Pro His Ser Pro Cys Ser Glu Ile Ser Ser Ala Cys Ser Gln
   1 5 10
<210> 244
   <211> 13
   <212> PRT
   <213> Homo sapiens
<400> 244
   Thr Pro Ser Ser Thr Pro Val Ser Pro Leu His His Ala
   1 5 10
<210> 245
   <211> 16
   <212> PRT
   <213> Homo sapiens
<400> 245
   Arg Ala Phe Pro Ala His Leu Pro Pro Ser Gln Ser Ile Pro Asp Ser
   1 5 10 15
<210> 246
   <211> 452
   <212> PRT
   <213> Homo sapiens
<400> 246
<210> 247
   <211> 14
   <212> PRT
   <213> Homo sapiens
<400> 247
   Ser Leu Phe Asp Ser Ala Tyr Gly Ala Ala Ala His Leu Pro
   1 5 10
<210> 248
   <211> 18
   <212> PRT
   <213> Homo sapiens
<400> 248
   His Lys Ile Asn Pro Leu Pro Pro Gln Ile Asn Gln Pro Val Arg Val
   1 5 10 15
   Asn Val
<210> 249
   <211> 17
   <212> PRT
   <213> Homo sapiens
<400> 249
   Arg Glu Ser Pro Val Asp Cys Ser Val Ser Lys Cys Ser Lys Leu Val
   1 5 10 15
   Gly
<210> 250
   <211> 7
   <212> PRT
   <213> Homo sapiens
<400> 250
   Thr Gln Glu His Val Arg Gln
   1 5
<210> 251
   <211> 8
   <212> PRT
   <213> Homo sapiens
<400> 251
   Ser Ser Arg Leu Ser Val Lys Glu
   1 5
<210> 252
   <211> 9
   <212> PRT
   <213> Homo sapiens
<400> 252
   Pro Asn Thr His Val Pro Ser His Leu
   1 5
<210> 253
   <211> 484
   <212> PRT
   <213> Homo sapiens
<400> 253
   Met Glu Arg Arg Met Lys Ala Gly Tyr Leu Asp Gln Gln Val Pro Tyr
   1               5                   10                  15
   Thr Phe Ser Ser Lys Ser Pro Gly Asn Gly Ser Leu Arg Glu Ala Leu
               20                  25                  30
   Ile Gly Pro Leu Gly Lys Leu Met Asp Pro Gly Ser Leu Pro Pro Leu
           35                  40                  45
   Asp Ser Glu Asp Leu Phe Gln Asp Leu Ser His Phe Gln Glu Thr Trp
       50                  55                  60
   Leu Ala Glu Ala Gln Val Pro Asp Ser Asp Glu Gln Phe Val Pro Asp
   65                  70                  75                  80
   Phe His Ser Glu Asn Leu Ala Phe His Ser Pro Thr Thr Arg Ile Lys
                   85                  90                  95
   Lys Glu Pro Gln Ser Pro Arg Thr Asp Pro Ala Leu Ser Cys Ser Arg
               100                 105                 110
   Lys Pro Pro Leu Pro Tyr His His Gly Glu Gln Cys Leu Tyr Ser Ser
           115                 120                 125
   Ala Tyr Asp Pro Pro Arg Gln Ile Ala Ile Lys Ser Pro Ala Pro Gly
       130                 135                 140
   Ala Leu Gly Gln Ser Pro Leu Gln Pro Phe Pro Arg Ala Glu Gln Arg
   145                 150                 155                 160
   Asn Phe Leu Arg Ser Ser Gly Thr Ser Gln Pro His Pro Gly His Gly
                   165                 170                 175
   Tyr Leu Gly Glu His Ser Ser Val Phe Gln Gln Pro Leu Asp Ile Cys
               180                 185                 190
   His Ser Phe Thr Ser Gln Gly Gly Gly Arg Glu Pro Leu Pro Ala Pro
           195                 200                 205
   Tyr Gln His Gln Leu Ser Glu Pro Cys Pro Pro Tyr Pro Gln Gln Ser
       210                 215                 220
   Phe Lys Gln Glu Tyr His Asp Pro Leu Tyr Glu Gln Ala Gly Gln Pro
   225                 230                 235                 240
   Ala Val Asp Gln Gly Gly Val Asn Gly His Arg Tyr Pro Gly Ala Gly
                   245                 250                 255
   Val Val Ile Lys Gln Glu Gln Thr Asp Phe Ala Tyr Asp Ser Asp Val
               260                 265                 270
   Thr Gly Cys Ala Ser Met Tyr Leu His Thr Glu Gly Phe Ser Gly Pro
           275                 280                 285
   Ser Pro Gly Asp Gly Ala Met Gly Tyr Gly Tyr Glu Lys Pro Leu Arg
       290                 295                 300
   Pro Phe Pro Asp Asp Val Cys Val Val Pro Glu Lys Phe Glu Gly Asp
   305                 310                 315                 320
   Ile Lys Gln Glu Gly Val Gly Ala Phe Arg Glu Gly Pro Pro Tyr Gln
   325 330 335
   Arg Arg Gly Ala Leu Gln Leu Trp Gln Phe Leu Val Ala Leu Leu Asp
   340 345 350
   Asp Pro Thr Asn Ala His Phe Ile Ala Trp Thr Gly Arg Gly Met Glu
   355 360 365
   Phe Lys Leu Ile Glu Pro Glu Glu Val Ala Arg Leu Trp Gly Ile Gln
   370 375 380
   Lys Asn Arg Pro Ala Met Asn Tyr Asp Lys Leu Ser Arg Ser Leu Arg
   385 390 395 400
   Tyr Tyr Tyr Glu Lys Gly Ile Met Gln Lys Val Ala Gly Glu Arg Tyr
   405 410 415
   Val Tyr Lys Phe Val Cys Glu Pro Glu Ala Leu Phe Ser Leu Ala Phe
   420 425 430
   Pro Asp Asn Gln Arg Pro Ala Leu Lys Ala Glu Phe Asp Arg Pro Val
   435 440 445
   Ser Glu Glu Asp Thr Val Pro Leu Ser His Leu Asp Glu Ser Pro Ala
   450 455 460
   Tyr Leu Pro Glu Leu Ala Gly Pro Ala Gln Pro Phe Gly Pro Lys Gly
   465 470 475 480
   Gly Tyr Ser Tyr
<210> 254
   <211> 12
   <212> PRT
   <213> Homo sapiens
<400> 254
   Gly Tyr Leu Asp Gln Gln Val Pro Tyr Thr Phe Ser
   1 5 10
<210> 255
   <211> 11
   <212> PRT
   <213> Homo sapiens
<400> 255
   Leu Arg Glu Ala Leu Ile Gly Pro Leu Gly Lys
   1 5 10
<210> 256
   <211> 7
   <212> PRT
   <213> Homo sapiens
<400> 256
   Leu Phe Gln Asp Leu Ser His
   1 5
<210> 257
   <211> 7
   <212> PRT
   <213> Homo sapiens
<400> 257
   Ser Glu Asn Leu Ala Phe His
   1 5
<210> 258
   <211> 7
   <212> PRT
   <213> Homo sapiens
<400> 258
   Thr Asp Pro Ala Leu Ser Cys
   1 5
<210> 259
   <211> 20
   <212> PRT
   <213> Homo sapiens
<400> 259
   Ser Arg Lys Pro Pro Leu Pro Tyr His His Gly Glu Gln Cys Leu Tyr
   1 5 10 15
   Ser Ser Ala Tyr
   20
<210> 260
   <211> 25
   <212> PRT
   <213> Homo sapiens
<400> 260
   Asp Pro Pro Arg Gln Ile Ala Ile Lys Ser Pro Ala Pro Gly Ala Leu
   1 5 10 15
   Gly Gln Ser Pro Leu Gln Pro Phe Pro
   20 25
<210> 261
   <211> 21
   <212> PRT
   <213> Homo sapiens
<400> 261
   Ser Gln Pro His Pro Gly His Tyr Leu Gly Glu His Ser Ser Val Phe
   1 5 10 15
   Gln Gln Pro Leu Asp
   20
<210> 262
   <211> 5
   <212> PRT
   <213> Homo sapiens
<400> 262
   Ile Cys His Ser Phe
   1 5
<210> 263
   <211> 21
   <212> PRT
   <213> Homo sapiens
<400> 263
   Glu Pro Leu Pro Ala Pro Tyr Gln His Gln Leu Ser Glu Pro Cys Pro
   1 5 10 15
   Pro Tyr Pro Gln Gln
   20
<210> 264
   <211> 15
   <212> PRT
   <213> Homo sapiens
<400> 264
   Tyr His Asp Pro Leu Tyr Glu Gln Gly Gln Pro Ala Val Asp Gln
   1 5 10 15
<210> 265
   <211> 10
   <212> PRT
   <213> Homo sapiens
<400> 265
   Arg Tyr Pro Gly Ala Gly Val Val Ile Lys
   1 5 10
<210> 266
   <211> 12
   <212> PRT
   <213> Homo sapiens
<400> 266
   Tyr Asp Ser Asp Val Thr Gly Cys Ala Ser Met Tyr
   1 5 10
<210> 267
   <211> 12
   <212> PRT
   <213> Homo sapiens
<400> 267
   Tyr Glu Lys Pro Leu Arg Pro Phe Pro Asp Asp Val
   1 5 10
<210> 268
   <211> 5
   <212> PRT
   <213> Homo sapiens
<400> 268
   Cys Val Val Pro Glu
   1 5
<210> 269
   <211> 71
   <212> DNA
   <213> Homo sapiens
<400> 269
   cgcgagctaa gcaggaggcg gaggcggagg cggagggcga ggggcgggga gcgccgcctg 60
   gagcgcggca g 71
<210> 270
   <211> 142
   <212> DNA
   <213> Homo sapiens
<400> 270
   cgcgagctaa gcaggaggcg gaggcggagg cggagggcga ggggcgggga gcgccgcctg 60
   gagcgcggca ggtcatattg aacattccag atacctatca ttactcgatg ctgttgataa 120
   cagcaagatg gctttgaact ca 142
<210> 271
   <211> 365
   <212> DNA
   <213> Homo sapiens
<400> 271
   cgcgagctaa gcaggaggcg gaggcggagg cggagggcga ggggcgggga gcgccgcctg 60
   gagcgcggca ggtcatattg aacattccag atacctatca ttactcgatg ctgttgataa 120
   cagcaagatg gctttgaact cagggtcacc accagctatt ggaccttact atgaaaacca 180
   tggataccaa ccggaaaacc cctatcccgc acagcccact gtggtcccca ctgtctacga 240
   ggtgcatccg gctcagtact acccgtcccc cgtgccccag tacgccccga gggtcctgac 300
   gcaggcttcc aaccccgtcg tctgcacgca gcccaaatcc ccatccggga cagtgtgcac 360
   ctcaa 365
<210> 272
   <211> 452
   <212> DNA
   <213> Homo sapiens
<400> 272
   cgcgagctaa gcaggaggcg gaggcggagg cggagggcga ggggcgggga gcgccgcctg 60
   gagcgcggca ggtcatattg aacattccag atacctatca ttactcgatg ctgttgataa 120
   cagcaagatg gctttgaact cagggtcacc accagctatt ggaccttact atgaaaacca 180
   tggataccaa ccggaaaacc cctatcccgc acagcccact gtggtcccca ctgtctacga 240
   ggtgcatccg gctcagtact acccgtcccc cgtgccccag tacgccccga gggtcctgac 300
   gcaggcttcc aaccccgtcg tctgcacgca gcccaaatcc ccatccggga cagtgtgcac 360
   ctcaaagact aagaaagcac tgtgcatcac cttgaccctg gggaccttcc tcgtgggagc 420
   tgcgctggcc gctggcctac tctggaagtt ca 452
<210> 273
   <211> 572
   <212> DNA
   <213> Homo sapiens
<400> 273
   cgcgagctaa gcaggaggcg gaggcggagg cggagggcga ggggcgggga gcgccgcctg 60
   gagcgcggca ggtcatattg aacattccag atacctatca ttactcgatg ctgttgataa 120
   cagcaagatg gctttgaact cagggtcacc accagctatt ggaccttact atgaaaacca 180
   tggataccaa ccggaaaacc cctatcccgc acagcccact gtggtcccca ctgtctacga 240
   ggtgcatccg gctcagtact acccgtcccc cgtgccccag tacgccccga gggtcctgac 300
   gcaggcttcc aaccccgtcg tctgcacgca gcccaaatcc ccatccggga cagtgtgcac 360
   ctcaaagact aagaaagcac tgtgcatcac cttgaccctg gggaccttcc tcgtgggagc 420
   tgcgctggcc gctggcctac tctggaagtt catgggcagc aagtgctcca actctgggat 480
   agagtgcgac tcctcaggta cctgcatcaa cccctctaac tggtgtgatg gcgtgtcaca 540
   ctgccccggc ggggaggacg agaatcggtg tg 572
<210> 274
   <211> 67
   <212> DNA
   <213> Homo sapiens
<400> 274
   ctttgataaa taagtttgta agaggagcct cagcatcgta aagagctttt ctccccgctt 60
   ctcgcag 67
<210> 275
   <211> 33
   <212> DNA
   <213> Homo sapiens
<400> 275
   atcgtaaaga gcttttctcc ccgcttctcg cag 33
<210> 276
   <211> 102
   <212> DNA
   <213> Homo sapiens
<400> 276
   gttattccag gatctttgga gacccgagga aagccgtgtt gaccaaaagc aagacaaatg 60
   actcacagag aaaaaagatg gcagaaccaa gggcaactaa ag 102
<210> 277
   <211> 86
   <212> DNA
   <213> Homo sapiens
<400> 277
   ccgtcaggtt ctgaacagct ggtagatggg ctggcttact gaaggacatg attcagactg 60
   tcccggaccc agcagctcat atcaag 86
<210> 278
   <211> 218
   <212> DNA
   <213> Homo sapiens
<400> 278
   gaagccttat cagttgtgag tgaggaccag tcgttgtttg agtgtgccta cggaacgcca 60
   cacctggcta agacagagat gaccgcgtcc tcctccagcg actatggaca gacttccaag 120
   atgagcccac gcgtccctca gcaggattgg ctgtctcaac ccccagccag ggtcaccatc 180
   aaaatggaat gtaaccctag ccaggtgaat ggctcaag 218
<210> 279
   <211> 152
   <212> DNA
   <213> Homo sapiens
<400> 279
   gaactctcct gatgaatgca gtgtggccaa aggcgggaag atggtgggca gcccagacac 60
   cgttgggatg aactacggca gctacatgga ggagaagcac atgccacccc caaacatgac 120
   cacgaacgag cgcagagtta tcgtgccagc ag 152
<210> 280
   <211> 204
   <212> DNA
   <213> Homo sapiens
<400> 280
   atcctacgct atggagtaca gaccatgtgc ggcagtggct ggagtgggcg gtgaaagaat 60
   atggccttcc agacgtcaac atcttgttat tccagaacat cgatgggaag gaactgtgca 120
   agatgaccaa ggacgacttc cagaggctca cccccagcta caacgccgac atccttctct 180
   cacatctcca ctacctcaga gaga 204
<210> 281
   <211> 81
   <212> DNA
   <213> Homo sapiens
<400> 281
   ctcctcttcc acatttgact tcagatgatg ttgataaagc cttacaaaac tctccacggt 60
   taatgcatgc tagaaacaca g 81
<210> 282
   <211> 72
   <212> DNA
   <213> Homo sapiens
<400> 282
   ggggtgcagc ttttattttc ccaaatactt cagtatatcc tgaagctacg caaagaatta 60
   caactaggcc ag 72
<210> 283
   <211> 69
   <212> DNA
   <213> Homo sapiens
<400> 283
   atttaccata tgagcccccc aggagatcag cctggaccgg tcacggccac cccacgcccc 60
   agtcgaaag 69
<210> 284
   <211> 57
   <212> DNA
   <213> Homo sapiens
<400> 284
   ctgctcaacc atctccttcc acagtgccca aaactgaaga ccagcgtcct cagttag 57
<210> 285
   <211> 48
   <212> DNA
   <213> Homo sapiens
<400> 285
   atccttatca gattcttgga ccaacaagta gccgccttgc aaatccag 48
<210> 286
   <211> 521
   <212> DNA
   <213> Homo sapiens
<400> 286
   gcagtggcca gatccagctt tggcagttcc tcctggagct cctgtcggac agctccaact 60
   ccagctgcat cacctgggaa ggcaccaacg gggagttcaa gatgacggat cccgacgagg 120
   tggcccggcg ctggggagag cggaagagca aacccaacat gaactacgat aagctcagcc 180
   gcgccctccg ttactactat gacaagaaca tcatgaccaa ggtccatggg aagcgctacg 240
   cctacaagtt cgacttccac gggatcgccc aggccctcca gccccacccc ccggagtcat 300
   ctctgtacaa gtacccctca gacctcccgt acatgggctc ctatcacgcc cacccacaga 360
   agatgaactt tgtggcgccc caccctccag ccctccccgt gacatcttcc agtttttttg 420
   ctgccccaaa cccatactgg aattcaccaa ctgggggtat ataccccaac actaggctcc 480
   ccaccagcca tatgccttct catctgggca cttactacta a 521
<210> 287
   <211> 54
   <212> DNA
   <213> Homo sapiens
<400> 287
   ctcaggtacc tgacaatgat gagcagtttg taccagacta tcaggctgaa agtt 54
<210> 288
   <211> 130
   <212> DNA
   <213> Homo sapiens
<400> 288
   tggcttttca tggcctgcca ctgaaaatca agaaagaacc ccacagtcca tgttcagaaa 60
   tcagctctgc ctgcagtcaa gaacagccct ttaaattcag ctatggagaa aagtgcctgt 120
   acaatgtcag 130
<210> 289
   <211> 189
   <212> DNA
   <213> Homo sapiens
<400> 289
   tgcctatgat cagaagccac aagtgggaat gaggccctcc aaccccccca caccatccag 60
   cacgccagtg tccccactgc atcatgcatc tccaaactca actcatacac cgaaacctga 120
   ccgggccttc ccagctcacc tccctccatc gcagtccata ccagatagca gctaccccat 180
   ggaccacag 189
<210> 290
   <211> 248
   <212> DNA
   <213> Homo sapiens
<400> 290
   atttcgccgc cagctttctg aaccctgtaa ctcctttcct cctttgccga cgatgccaag        60
   ggaaggacgt cctatgtacc aacgccagat gtctgagcca aacatcccct tcccaccaca       120
   aggctttaag caggagtacc acgacccagt gtatgaacac aacaccatgg ttggcagtgc       180
   ggccagccaa agctttcccc ctcctctgat gattaaacag gaacccagag attttgcata       240
   tgactcag                                                                248
<210> 291
   <211> 69
   <212> DNA
   <213> Homo sapiens
<400> 291
   aagtgcctag ctgccactcc atttatatga ggcaagaagg cttcctggct catcccagca        60
   gaacagaag                                                                69
<210> 292
   <211> 69
   <212> DNA
   <213> Homo sapiens
<400> 292
   gctgtatgtt tgaaaagggc cccaggcagt tttatgatga cacctgtgtt gtcccagaaa        60
   aattcgatg                                                                69
<210> 293
   <211> 170
   <212> DNA
   <213> Homo sapiens
<400> 293
   gagacatcaa acaagagcca ggaatgtatc gggaaggacc cacataccaa cggcgaggat        60
   cacttcagct ctggcagttt ttggtagctc ttctggatga cccttcaaat tctcatttta       120
   ttgcctggac tggtcgaggc atggaattta aactgattga gcctgaagag                  170
<210> 294
   <211> 102
   <212> DNA
   <213> Homo sapiens
<400> 294
   gtggcccgac gttggggcat tcagaaaaac aggccagcta tgaactatga taaacttagc 60
   cgttcactcc gctattacta tgagaaagga attatgcaaa ag 102
<210> 295
   <211> 222
   <212> DNA
   <213> Homo sapiens
<400> 295
   gtggctggag agagatatgt ctacaagttt gtgtgtgatc cagaagccct tttctccatg 60
   gcctttccag ataatcagcg tccactgctg aagacagaca tggaacgtca catcaacgag 120
   gaggacacag tgcctctttc tcactttgat gagagcatgg cctacatgcc ggaagggggc 180
   tgctgcaacc cccaccccta caacgaaggc tacgtgtatt aa 222
<210> 296
   <211> 93
   <212> DNA
   <213> Homo sapiens
<400> 296
   aaatcgcccg gaaatgggag cttgcgcgaa gcgctgatcg gcccgctggg gaagctcatg 60
   gacccgggct ccctgccgcc ctcgactctg aag 93
<210> 297
   <211> 48
   <212> DNA
   <213> Homo sapiens
<400> 297
   atctcttcca ggatctaagt cacttccagg agacgtggct cgctgaag 48
<210> 298
   <211> 54
   <212> DNA
   <213> Homo sapiens
<400> 298
   ctcaggtacc agacagtgat gagcagtttg ttcctgattt ccattcagaa aacc 54
<210> 299
   <211> 127
   <212> DNA
   <213> Homo sapiens
<400> 299
   tagctttcca cagccccacc accaggatca agaaggagcc ccagagtccc cgcacagacc 60
   cggccctgtc ctgcagcagg aagccgccac tcccctacca ccatggcgag cagtgccttt 120
   actccag 127
<210> 300
   <211> 162
   <212> DNA
   <213> Homo sapiens
<400> 300
   tgcctatgac ccccccagac aaatcgccat caagtcccct gcccctggtg cccttggaca 60
   gtcgccccta cagccctttc cccgggcaga gcaacggaat ttcctgagat cctctggcac 120
   ctcccagccc caccctggcc atgggtacct cggggaacat ag 162
<210> 301
   <211> 266
   <212> DNA
   <213> Homo sapiens
<400> 301
   ctccgtcttc cagcagcccc tggacatttg ccactccttc acatctcagg gagggggccg 60
   ggaacccctc ccagccccct accaacacca gctgtcggag ccctgcccac cctatcccca 120
   gcagagcttt aagcaagaat accatgatcc cctgtatgaa caggcgggcc agccagccgt 180
   ggaccagggt ggggtcaatg ggcacaggta cccaggggcg ggggtggtga tcaaacagga 240
   acagacggac ttcgcctacg actcag 266
<210> 302
   <211> 75
   <212> DNA
   <213> Homo sapiens
<400> 302
   atgtcaccgg gtgcgcatca atgtacctcc acacagaggg cttctctggg ccctctccag 60
   gtgacggggc catgg 75
<210> 303
   <211> 69
   <212> DNA
   <213> Homo sapiens
<400> 303
   gctatggcta tgagaaacct ctgcgaccat tcccagatga tgtctgcgtt gtccctgaga 60
   aatttgaag 69
<210> 304
   <211> 173
   <212> DNA
   <213> Homo sapiens
<400> 304
   gagacatcaa gcaggaaggg gtcggtgcat ttcgagaggg gccgccctac cagcgccggg 60
   gtgccctgca gctgtggcaa tttctggtgg ccttgctgga tgacccaaca aatgcccatt 120
   tcattgcctg gacgggccgg ggaatggagt tcaagctcat tgagcctgag gag 173
<210> 305
   <211> 102
   <212> DNA
   <213> Homo sapiens
<400> 305
   gtcgccaggc tctggggcat ccagaagaac cggccagcca tgaattacga caagctgagc 60
   cgctcgctcc gatactatta tgagaaaggc atcatgcaga ag 102
<210> 306
   <211> 225
   <212> DNA
   <213> Homo sapiens
<400> 306
   gtggctggtg agcgttacgt gtacaagttt gtgtgtgagc ccgaggccct cttctctttg 60
   gccttcccgg acaatcagcg tccagctctc aaggctgagt ttgaccggcc tgtcagtgag 120
   gaggacacag tccctttgtc ccacttggat gagagccccg cctacctccc agagctggct 180
   ggccccgccc agccatttgg ccccaagggt ggctactctt actag 225
<210> 307
   <211> 3226
   <212> DNA
   <213> Homo sapiens
<400> 307
   cgcgagctaa gcaggaggcg gaggcggagg cggagggcga ggggcgggga gcgccgcctg 60
   gagcgcggca ggtcatattg aacattccag atacctatca ttactcgatg ctgttgataa 120
   cagcaagatg gctttgaact cagggtcacc accagctatt ggaccttact atgaaaacca 180
   tggataccaa ccggaaaacc cctatcccgc acagcccact gtggtcccca ctgtctacga 240
   ggtgcatccg gctcagtact acccgtcccc cgtgccccag tacgccccga gggtcctgac 300
   gcaggcttcc aaccccgtcg tctgcacgca gcccaaatcc ccatccggga cagtgtgcac 360
   ctcaaagact aagaaagcac tgtgcatcac cttgaccctg gggaccttcc tcgtgggagc 420
   tgcgctggcc gctggcctac tctggaagtt catgggcagc aagtgctcca actctgggat 480
   agagtgcgac tcctcaggta cctgcatcaa cccctctaac tggtgtgatg gcgtgtcaca 540
   ctgccccggc ggggaggacg agaatcggtg tgttcgcctc tacggaccaa acttcatcct 600
   tcagatgtac tcatctcaga ggaagtcctg gcaccctgtg tgccaagacg actggaacga 660
   gaactacggg cgggcggcct gcagggacat gggctataag aataattttt actctagcca 720
   aggaatagtg gatgacagcg gatccaccag ctttatgaaa ctgaacacaa gtgccggcaa 780
   tgtcgatatc tataaaaaac tgtaccacag tgatgcctgt tcttcaaaag cagtggtttc 840
   tttacgctgt atagcctgcg gggtcaactt gaactcaagc cgccagagca ggatcgtggg 900
   cggtgagagc gcgctcccgg gggcctggcc ctggcaggtc agcctgcacg tccagaacgt 960
   ccacgtgtgc ggaggctcca tcatcacccc cgagtggatc gtgacagccg cccactgcgt 1020
   ggaaaaacct cttaacaatc catggcattg gacggcattt gcggggattt tgagacaatc 1080
   tttcatgttc tatggagccg gataccaagt agaaaaagtg atttctcatc caaattatga 1140
   ctccaagacc aagaacaatg acattgcgct gatgaagctg cagaagcctc tgactttcaa 1200
   cgacctagtg aaaccagtgt gtctgcccaa cccaggcatg atgctgcagc cagaacagct 1260
   ctgctggatt tccgggtggg ggccaccgga ggagaaaggg aagacctcag aagtgctgaa 1320
   cgctgccaag gtgcttctca ttgagacaca gagatgcaac agcagatatg tctatgacaa 1380
   cctgatcaca ccagccatga tctgtgccgg cttcctgcag gggaacgtcg attcttgcca 1440
   gggtgacagt ggagggcctc tggtcacttc gaagaacaat atctggtggc tgatagggga 1500
   tacaagctgg ggttctggct gtgccaaagc ttacagacca ggagtgtacg ggaatgtgat 1560
   ggtattcacg gactggattt atcgacaaat gagggcagac ggctaatcca catggtcttc 1620
   gtccttgacg tcgttttaca agaaaacaat ggggctggtt ttgcttcccc gtgcatgatt 1680
   tactcttaga gatgattcag aggtcacttc atttttatta aacagtgaac ttgtctggct 1740
   ttggcactct ctgccattct gtgcaggctg cagtggctcc cctgcccagc ctgctctccc 1800
   taaccccttg tccgcaaggg gtgatggccg gctggttgtg ggcactggcg gtcaagtgtg 1860
   gaggagaggg gtggaggctg ccccattgag atcttcctgc tgagtccttt ccaggggcca 1920
   attttggatg agcatggagc tgtcacctct cagctgctgg atgacttgag atgaaaaagg 1980
   agagacatgg aaagggagac agccaggtgg cacctgcagc ggctgccctc tggggccact 2040
   tggtagtgtc cccagcctac ctctccacaa ggggattttg ctgatgggtt cttagagcct 2100
   tagcagccct ggatggtggc cagaaataaa gggaccagcc cttcatgggt ggtgacgtgg 2160
   tagtcacttg taaggggaac agaaacattt ttgttcttat ggggtgagaa tatagacagt 2220
   gcccttggtg cgagggaagc aattgaaaag gaacttgccc tgagcactcc tggtgcaggt 2280
   ctccacctgc acattgggtg gggctcctgg gagggagact cagccttcct cctcatcctc 2340
   cctgaccctg ctcctagcac cctggagagt gcacatgccc cttggtcctg gcagggcgcc 2400
   aagtctggca ccatgttggc ctcttcaggc ctgctagtca ctggaaattg aggtccatgg 2460
   gggaaatcaa ggatgctcag tttaaggtac actgtttcca tgttatgttt ctacacattg 2520
   ctacctcagt gctcctggaa acttagcttt tgatgtctcc aagtagtcca ccttcattta 2580
   actctttgaa actgtatcac ctttgccaag taagagtggt ggcctatttc agctgctttg 2640
   acaaaatgac tggctcctga cttaacgttc tataaatgaa tgtgctgaag caaagtgccc 2700
   atggtggcgg cgaagaagag aaagatgtgt tttgttttgg actctctgtg gtcccttcca 2760
   atgctgtggg tttccaacca ggggaagggt cccttttgca ttgccaagtg ccataaccat 2820
   gagcactact ctaccatggt tctgcctcct ggccaagcag gctggtttgc aagaatgaaa 2880
   tgaatgattc tacagctagg acttaacctt gaaatggaaa gtcttgcaat cccatttgca 2940
   ggatccgtct gtgcacatgc ctctgtagag agcagcattc ccagggacct tggaaacagt 3000
   tggcactgta aggtgcttgc tccccaagac acatcctaaa aggtgttgta atggtgaaaa 3060
   cgtcttcctt ctttattgcc ccttcttatt tatgtgaaca actgtttgtc tttttttgta 3120
   tcttttttaa actgtaaagt tcaattgtga aaatgaatat catgcaaata aattatgcga 3180
   tttttttttc aaagcaaaaa aaaaaaaaaa aaaaaaaaaa aaaaaa 3226
<210> 308
   <211> 17
   <212> DNA
   <213> Artificial Sequence
<220>
   <223> Synthetic
<400> 308
   gatctttgtg acctact 17
<210> 309
   <211> 22
   <212> DNA
   <213> Artificial Sequence
<220>
   <223> Synthetic
<400> 309
   ctaccccatg gaccacagat tt 22
<210> 310
   <211> 22
   <212> DNA
   <213> Artificial Sequence
<220>
   <223> Synthetic
<400> 310
   cttaaagcct tgtggtggga ag 22
<210> 311
   <211> 24
   <212> DNA
   <213> Artificial Sequence
<220>
   <223> Synthetic
<400> 311
   cgcagagtta tcgtgccagc agat 24
<210> 312
   <211> 24
   <212> DNA
   <213> Artificial Sequence
<220>
   <223> Synthetic
<400> 312
   ccatattctt tcaccgccca ctcc 24
<210> 313
   <211> 23
   <212> DNA
   <213> Artificial Sequence
<220>
   <223> Synthetic
<400> 313
   cgactggagc acgaggacac tga 23
<210> 314
   <211> 24
   <212> DNA
   <213> Artificial Sequence
<220>
   <223> Synthetic
<400> 314
   catggactgt ggggttcttt cttg 24
<210> 315
   <211> 26
   <212> DNA
   <213> Artificial Sequence
<220>
   <223> Synthetic
<400> 315
   agacatctgg cgttggtaca taggac 26
<210> 316
   <211> 25
   <212> DNA
   <213> Artificial Sequence
<220>
   <223> Synthetic
<400> 316
   gagtcccaag tacgtccacg gtcag 25
<210> 317
   <211> 27
   <212> DNA
   <213> Artificial Sequence
<220>
   <223> Synthetic
<400> 317
   ggactgtggg gttctttctt gattttc 27
<210> 318
   <211> 20
   <212> DNA
   <213> Artificial Sequence
<220>
   <223> Synthetic
<400> 318
   tgcgggaaat cgggctgaag 20
<210> 319
   <211> 28
   <212> DNA
   <213> Artificial Sequence
<220>
   <223> Synthetic
<400> 319
   ttttcctgac atttgttggt ttctcgtt 28
<210> 320
   <211> 19
   <212> DNA
   <213> Artificial Sequence
<220>
   <223> Synthetic
<400> 320
   cgctggctcc gggtgacag 19
<210> 321
   <211> 27
   <212> DNA
   <213> Artificial Sequence
<220>
   <223> Synthetic
<400> 321
   gtggggttct ttcttgattt tcagtgg 27
<210> 322
   <211> 24
   <212> DNA
   <213> Artificial Sequence
<220>
   <223> Synthetic
<400> 322
   caactaacac tgcggcttcc tgag 24
<210> 323
   <211> 23
   <212> DNA
   <213> Artificial Sequence
<220>
   <223> Synthetic
<400> 323
   cattcccact tgtggcttct gat 23
<210> 324
   <211> 25
   <212> DNA
   <213> Artificial Sequence
<220>
   <223> Synthetic
<400> 324
   caggagtgta cgggaatgtg atggt 25
<210> 325
   <211> 24
   <212> DNA
   <213> Artificial Sequence
<220>
   <223> Synthetic
<400> 325
   gattagccgt ctgccctcat ttgt 24
<210> 326
   <211> 29
   <212> DNA
   <213> Artificial Sequence
<220>
   <223> Synthetic
<400> 326
   tgccatgaag atcagtacta cagcagaat 29
<210> 327
   <211> 25
   <212> DNA
   <213> Artificial Sequence
<220>
   <223> Synthetic
<400> 327
   gtggcccata aactcatgaa tcacc 25
<210> 328
   <211> 21
   <212> DNA
   <213> Artificial Sequence
<220>
   <223> Synthetic
<400> 328
   cagatcgtgg ccggtggttc t 21
<210> 329
   <211> 26
   <212> DNA
   <213> Artificial Sequence
<220>
   <223> Synthetic
<400> 329
   gggtgcatca ggcaaatttc tacaag 26
<210> 330
   <211> 27
   <212> DNA
   <213> Artificial Sequence
<220>
   <223> Synthetic
<400> 330
   caacaacaaa atgaggaact ggctact 27
<210> 331
   <211> 25
   <212> DNA
   <213> Artificial Sequence
<220>
   <223> Synthetic
<400> 331
   attccatatt tgctcgctct gtcag 25
<210> 332
   <211> 24
   <212> DNA
   <213> Artificial Sequence
<220>
   <223> Synthetic
<400> 332
   gaagatggaa gggcatgaaa ccag 24
<210> 333
   <211> 0
   <212> DNA
   <213> Artificial Sequence
<220>
   <223> Synthetic
<400> 333
   000
<210> 334
   <211> 20
   <212> DNA
   <213> Artificial Sequence
<220>
   <223> Synthetic
<400> 334
   gctgaccggg acggaggagt 20
<210> 335
   <211> 19
   <212> DNA
   <213> Artificial Sequence
<220>
   <223> Synthetic
<400> 335
   tcgtgggcga ggggctgta 19
<210> 336
   <211> 25
   <212> DNA
   <213> Artificial Sequence
<220>
   <223> Synthetic
<400> 336
   catccgaacg ccttcatcat agtgt 25
<210> 337
   <211> 27
   <212> DNA
   <213> Artificial Sequence
<220>
   <223> Synthetic
<400> 337
   cttttctcta gggtgaaggg actctcg 27
<210> 338
   <211> 26
   <212> DNA
   <213> Artificial Sequence
<220>
   <223> Synthetic
<400> 338
   cttcacccac aaggctcact gtagac 26
<210> 339
   <211> 27
   <212> DNA
   <213> Artificial Sequence
<220>
   <223> Synthetic
<400> 339
   gctttggctt tgttactttt gatgacc 27
<210> 340
   <211> 29
   <212> DNA
   <213> Artificial Sequence
<220>
   <223> Synthetic
<400> 340
   gcctttctta cttctgcatt atgaccatt 29
<210> 341
   <211> 25
   <212> DNA
   <213> Artificial Sequence
<220>
   <223> Synthetic
<400> 341
   aaggacgtgc aaggatgttt ttatt 25
<210> 342
   <211> 20
   <212> DNA
   <213> Artificial Sequence
<220>
   <223> Synthetic
<400> 342
   atgggaagat gggggctgtt 20
<210> 343
   <211> 30
   <212> DNA
   <213> Artificial Sequence
<220>
   <223> Synthetic
<400> 343
   gtcaatggct aaaagatgga taaaagtgga 30
<210> 344
   <211> 30
   <212> DNA
   <213> Artificial Sequence
<220>
   <223> Synthetic
<400> 344
   cagatagaag aggggttagc aaaatgtgtt 30
<210> 345
   <211> 26
   <212> DNA
   <213> Artificial Sequence
<220>
   <223> Synthetic
<400> 345
   cagaaggcaa atgtgagagg atagtc 26
<210> 346
   <211> 23
   <212> DNA
   <213> Artificial Sequence
<220>
   <223> Synthetic
<400> 346
   ctggatctgt aacacccgtg agc 23
<210> 347
   <211> 26
   <212> DNA
   <213> Artificial Sequence
<220>
   <223> Synthetic
<400> 347
   aaaaagcaaa gacaagaccg tggatt 26
<210> 348
   <211> 27
   <212> DNA
   <213> Artificial Sequence
<220>
   <223> Synthetic
<400> 348
   gaactacctg cgtgctgact tggagat 27
<210> 349
   <211> 30
   <212> DNA
   <213> Artificial Sequence
<220>
   <223> Synthetic
<400> 349
   aaaaggcaaa gaggggttaa aacatacata 30
<210> 350
   <211> 24
   <212> DNA
   <213> Artificial Sequence
<220>
   <223> Synthetic
<400> 350
   aacccctcct tccacttctc cact 24
<210> 351
   <211> 28
   <212> DNA
   <213> Artificial Sequence
<220>
   <223> Synthetic
<400> 351
   tggaggcata gaaaagctga gaaataag 28
<210> 352
   <211> 26
   <212> DNA
   <213> Artificial Sequence
<220>
   <223> Synthetic
<400> 352
   ttggtgctag aagaactggg agaaac 26
<210> 353
   <211> 28
   <212> DNA
   <213> Artificial Sequence
<220>
   <223> Synthetic
<400> 353
   gaaagtcagg ggcacatata gattagag 28
<210> 354
   <211> 24
   <212> DNA
   <213> Artificial Sequence
<220>
   <223> Synthetic
<400> 354
   gccttcccca tacagtttct cctt 24
<210> 355
   <211> 28
   <212> DNA
   <213> Artificial Sequence
<220>
   <223> Synthetic
<400> 355
   aagttcgtta agcccaggat cgtaggta 28
<210> 356
   <211> 25
   <212> DNA
   <213> Artificial Sequence
<220>
   <223> Synthetic
<400> 356
   atatgaagcc agcagccagg tagca 25
<210> 357
   <211> 28
   <212> DNA
   <213> Artificial Sequence
<220>
   <223> Synthetic
<400> 357
   ttagataaac tgaaagccga acctgaac 28
<210> 358
   <211> 25
   <212> DNA
   <213> Artificial Sequence
<220>
   <223> Synthetic
<400> 358
   caaactggca agcaatgtga actgt 25
<210> 359
   <211> 28
   <212> DNA
   <213> Artificial Sequence
<220>
   <223> Synthetic
<400> 359
   tcaccgacaa aacccataga gaaagagt 28
<210> 360
   <211> 27
   <212> DNA
   <213> Artificial Sequence
<220>
   <223> Synthetic
<400> 360
   ttaaatggtg aggcaatgag gaaagtg 27
<210> 361
   <211> 29
   <212> DNA
   <213> Artificial Sequence
<220>
   <223> Synthetic
<400> 361
   ttgctcattc tctttctccc ctacactaa 29
<210> 362
   <211> 21
   <212> DNA
   <213> Artificial Sequence
<220>
   <223> Synthetic
<400> 362
   tccccaccac caaccatcct c 21
<210> 363
   <211> 26
   <212> DNA
   <213> Artificial Sequence
<220>
   <223> Synthetic
<400> 363
   ctgggggaaa agcaagtagg aaagta 26
<210> 364
   <211> 27
   <212> DNA
   <213> Artificial Sequence
<220>
   <223> Synthetic
<400> 364
   acaagagtta gtcacggcaa aggagtt 27
<210> 365
   <211> 22
   <212> DNA
   <213> Artificial Sequence
<220>
   <223> Synthetic
<400> 365
   gccctttgcc catgagaact aa 22
<210> 366
   <211> 26
   <212> DNA
   <213> Artificial Sequence
<220>
   <223> Synthetic
<400> 366
   tcccagaaga gatgatatga ggtgtc 26
<210> 367
   <211> 24
   <212> DNA
   <213> Artificial Sequence
<220>
   <223> Synthetic
<400> 367
   tcagtcccat ctccccctaa acca 24
<210> 368
   <211> 24
   <212> DNA
   <213> Artificial Sequence
<220>
   <223> Synthetic
<400> 368
   caccattctc acccgaccac attg 24
<210> 369
   <211> 30
   <212> DNA
   <213> Artificial Sequence
<220>
   <223> Synthetic
<400> 369
   tgtaaactgc aatgaaaaga aaagaaaaag 30
<210> 370
   <211> 30
   <212> DNA
   <213> Artificial Sequence
<220>
   <223> Synthetic
<400> 370
   caagagatgg gagaggaaga atgaataata 30
<210> 371
   <211> 28
   <212> DNA
   <213> Artificial Sequence
<220>
   <223> Synthetic
<400> 371
   ctatctagtc ccttacgctt tccctgtg 28
<210> 372
   <211> 23
   <212> DNA
   <213> Artificial Sequence
<220>
   <223> Synthetic
<400> 372
   cattagcatt tggcctttgg tca 23
<210> 373
   <211> 25
   <212> DNA
   <213> Artificial Sequence
<220>
   <223> Synthetic
<400> 373
   tgcctcccca taagtcacca atctc 25
<210> 374
   <211> 30
   <212> DNA
   <213> Artificial Sequence
<220>
   <223> Synthetic
<400> 374
   cctgtattct aaccctggac ttctcatcaa 30
<210> 375
   <211> 28
   <212> DNA
   <213> Artificial Sequence
<220>
   <223> Synthetic
<400> 375
   cttgtttatt ggcctagtcc tttgtgct 28
<210> 376
   <211> 26
   <212> DNA
   <213> Artificial Sequence
<220>
   <223> Synthetic
<400> 376
   gctttgtggg tagtcctgtc tgagtg 26
<210> 377
   <211> 20
   <212> DNA
   <213> Artificial Sequence
<220>
   <223> Synthetic
<400> 377
   ggcccatccc ggtttgctaa 20
<210> 378
   <211> 27
   <212> DNA
   <213> Artificial Sequence
<220>
   <223> Synthetic
<400> 378
   gtttccccac cacttccttt ctatgtc 27
<210> 379
   <211> 25
   <212> DNA
   <213> Artificial Sequence
<220>
   <223> Synthetic
<400> 379
   gcacaagaca tacacgcaga tacac 25
<210> 380
   <211> 26
   <212> DNA
   <213> Artificial Sequence
<220>
   <223> Synthetic
<400> 380
   aacgctggac tatggaactt tacctg 26
<210> 381
   <211> 29
   <212> DNA
   <213> Artificial Sequence
<220>
   <223> Synthetic
<400> 381
   tcctctcatt cattttgcat tcgtgttag 29
<210> 382
   <211> 27
   <212> DNA
   <213> Artificial Sequence
<220>
   <223> Synthetic
<400> 382
   ggctttgagg gattactggg ttgttct 27
<210> 383
   <211> 21
   <212> DNA
   <213> Artificial Sequence
<220>
   <223> Synthetic
<400> 383
   gcagggcaaa gaagcagtag g 21
<210> 384
   <211> 26
   <212> DNA
   <213> Artificial Sequence
<220>
   <223> Synthetic
<400> 384
   ggatcccaat ttagtttcaa gttacg 26
<210> 385
   <211> 26
   <212> DNA
   <213> Artificial Sequence
<220>
   <223> Synthetic
<400> 385
   atgtgctggc tagattggac tgaaaa 26
<210> 386
   <211> 29
   <212> DNA
   <213> Artificial Sequence
<220>
   <223> Synthetic
<400> 386
   caataaagct ggaggggtga taaataaat 29
<210> 387
   <211> 25
   <212> DNA
   <213> Artificial Sequence
<220>
   <223> Synthetic
<400> 387
   ttagaaggag acaatcttat tccag 25
<210> 388
   <211> 24
   <212> DNA
   <213> Artificial Sequence
<220>
   <223> Synthetic
<400> 388
   ctcttaaaga gatgaagcag ggag 24
<210> 389
   <211> 24
   <212> DNA
   <213> Artificial Sequence
<220>
   <223> Synthetic
<400> 389
   ttggctagat acagggtgaa tatt 24
<210> 390
   <211> 24
   <212> DNA
   <213> Artificial Sequence
<220>
   <223> Synthetic
<400> 390
   tgaattcatg tgtgtagctg agcc 24
<210> 391
   <211> 24
   <212> DNA
   <213> Artificial Sequence
<220>
   <223> Synthetic
<400> 391
   tgacagcggg aataaagtac atgc 24
<210> 392
   <211> 24
   <212> DNA
   <213> Artificial Sequence
<220>
   <223> Synthetic
<400> 392
   gttgggaggt ttacttgcca atta 24
<210> 393
   <211> 26
   <212> DNA
   <213> Artificial Sequence
<220>
   <223> Synthetic
<400> 393
   acattttgct aacccctctt ctatct 26
<210> 394
   <211> 25
   <212> DNA
   <213> Artificial Sequence
<220>
   <223> Synthetic
<400> 394
   tcaacctcaa aaataaatgg catct 25
<210> 395
   <211> 19
   <212> DNA
   <213> Artificial Sequence
<220>
   <223> Synthetic
<400> 395
   gcatggcccc cgaggagat 19
<210> 396
   <211> 23
   <212> DNA
   <213> Artificial Sequence
<220>
   <223> Synthetic
<400> 396
   cttggcccat gaaaaggact gtt 23
<210> 397
   <211> 23
   <212> DNA
   <213> Artificial Sequence
<220>
   <223> Synthetic
<400> 397
   aggtacggga ccctgttcac gat 23
<210> 398
   <211> 26
   <212> DNA
   <213> Artificial Sequence
<220>
   <223> Synthetic
<400> 398
   ctaccagcat gctaaagcga ttgtct 26
<210> 399
   <211> 24
   <212> DNA
   <213> Artificial Sequence
<220>
   <223> Synthetic
<400> 399
   cgagtccaag cgggagacag aata 24
<210> 400
   <211> 24
   <212> DNA
   <213> Artificial Sequence
<220>
   <223> Synthetic
<400> 400
   tacacccctg ggactcagca catt 24
<210> 401
   <211> 26
   <212> DNA
   <213> Artificial Sequence
<220>
   <223> Synthetic
<400> 401
   ttcattttgg ccatctcttc cttcag 26
<210> 402
   <211> 26
   <212> DNA
   <213> Artificial Sequence
<220>
   <223> Synthetic
<400> 402
   tatccagctc gtacctcatt tcctct 26
<210> 403
   <211> 20
   <212> DNA
   <213> Artificial Sequence
<220>
   <223> Synthetic
<400> 403
   gcccaccagc tccaacacag 20
<210> 404
   <211> 24
   <212> DNA
   <213> Artificial Sequence
<220>
   <223> Synthetic
<400> 404
   gaagggtcaa gcaggaggtc atag 24
<210> 405
   <211> 26
   <212> DNA
   <213> Artificial Sequence
<220>
   <223> Synthetic
<400> 405
   ccctgtggat gactgagtac ctgaac 26
<210> 406
   <211> 21
   <212> DNA
   <213> Artificial Sequence
<220>
   <223> Synthetic
<400> 406
   ggcatcccag cctccgttat c 21
<210> 407
   <211> 24
   <212> DNA
   <213> Artificial Sequence
<220>
   <223> Synthetic
<400> 407
   aattttgtgc tgcccgatga tgac 24
<210> 408
   <211> 23
   <212> DNA
   <213> Artificial Sequence
<220>
   <223> Synthetic
<400> 408
   ggaacagaag gccgggaggt agt 23
<210> 409
   <211> 22
   <212> DNA
   <213> Artificial Sequence
<220>
   <223> Synthetic
<400> 409
   gaaggccaag tggtccgtgt ga 22
<210> 410
   <211> 26
   <212> DNA
   <213> Artificial Sequence
<220>
   <223> Synthetic
<400> 410
   cagctgttgt actccttgcc attgaa 26
<210> 411
   <211> 20
   <212> DNA
   <213> Artificial Sequence
<220>
   <223> Synthetic
<400> 411
   gcctatgccc cctgagagtg 20
<210> 412
   <211> 26
   <212> DNA
   <213> Artificial Sequence
<220>
   <223> Synthetic
<400> 412
   gcttgagttg gcctagtttg ttgttc 26
<210> 413
   <211> 21
   <212> DNA
   <213> Artificial Sequence
<220>
   <223> Synthetic
<400> 413
   tgcccggacc aaggatacag t 21
<210> 414
   <211> 19
   <212> DNA
   <213> Artificial Sequence
<220>
   <223> Synthetic
<400> 414
   agcgcgtggc cgaactcat 19
<210> 415
   <211> 26
   <212> DNA
   <213> Artificial Sequence
<220>
   <223> Synthetic
<400> 415
   tacggctctg aagtcaccac caaaat 26
<210> 416
   <211> 23
   <212> DNA
   <213> Artificial Sequence
<220>
   <223> Synthetic
<400> 416
   ccccagctca caattccagt caa 23
<210> 417
   <211> 28
   <212> DNA
   <213> Artificial Sequence
<220>
   <223> Synthetic
<400> 417
   ccgaaggctt tgcttactaa gtttctga 28
<210> 418
   <211> 23
   <212> DNA
   <213> Artificial Sequence
<220>
   <223> Synthetic
<400> 418
   cactgccctt gtttgcctga atg 23
<210> 419
   <211> 23
   <212> DNA
   <213> Artificial Sequence
<220>
   <223> Synthetic
<400> 419
   agctcccgtt tgatcttggt tgg 23
<210> 420
   <211> 25
   <212> DNA
   <213> Artificial Sequence
<220>
   <223> Synthetic
<400> 420
   catggtaggc tcctgtttga ctttg 25
<210> 421
   <211> 417
   <212> DNA
   <213> Homo sapiens
<400> 421
   agtagcagca gcgccgggtc ccgtgcggag gtgctcctcg cagagttgtt tctcgagcag 60
   cggcagttct cactacagcg ccaggacgag tccggttcgt gttcgtccgc ggagatctct 120
   ctcatctcgc tcggctgcgg gaaatcgggc tgaagcgact gagtccgcga tggagagtca 180
   gcgtgggaga aattgtaacg agaaaccaac aaatgtcagg aaaagaaaat tcattaacag 240
   agatctggct catgattcag aagaactctt tcaagatcta agtcaattac aggaaacatg 300
   gcttgcagaa gctcaggtac ctgacaatga tgagcagttt gtaccagact atcaggctga 360
   aagtttggct tttcatggcc tgccactgaa aatcaagaaa gaaccccaca gtccatg 417
<210> 422
   <211> 413
   <212> DNA
   <213> Homo sapiens
<400> 422
   aaatactgag ggaactcaga gaccggtgcg gcgcgggtcc tccatatgct gagcgccggt 60
   cccctgggcc cacttttctt tctctatact ttgtctctgt tgtctttctt ttctcaagtc 120
   tctcgttcca cctgaggaga aatgcccaca gctgtggagg cgcaggccac tccatctggt 180
   gcccaacgtg gatgcttttc tctagggtga agggactctc gagtgtggtc attgaggaca 240
   agtcaacgag agattcccga gtacgtctac agtgagcctt gtgggtgaag gtactctaca 300
   gtgtggtcat tgaggacaag ttgacgagag agtcccaagt acgtccacgg tcagccttgc 360
   gtggcttttc atggcctgcc actgaaaatc aagaaagaac cccacagtcc atg 413
<210> 423
   <211> 278
   <212> DNA
   <213> Homo sapiens
<400> 423
   ccctgggtac cagccccacc cctctatccc ccctcgcagc accccccacc tccgtcccgc 60
   ctcgccccgc ccccctcctg gcagtcccag cgcgcatgct ctctcccccc gcggagtaac 120
   ctggagattt aaaagccgcc ggctggcgcg cgtggggggc aaggaagggg gggcggaacc 180
   agcctgcacg cgctggctcc gggtgacagc cgcgcgcctc ggccagtggc ttttcatggc 240
   ctgccactga aaatcaagaa agaaccccac agtccatg 278
<210> 424
   <211> 627
   <212> DNA
   <213> Homo sapiens
<400> 424
   aactacctac ctaagcgatg aaatccaaag gtgaatcttt tggttggtga ttttgatgat 60
   agccacaaag caagcaggct gtatggtagc cttgaaaacc aacaggccac aatcacagtg 120
   aaaacctcag gttggcagcc actggaggat acaaaatgag tttggatctc cttcaatgcc 180
   ccattcccag agaactgtca ttatttgacc tgctggtagt tccctaaagg acgtgcaagg 240
   atgtttttat ttgactggac acttgaagct tgccctgtat gaacagcccc catcttccca 300
   tgcttgtcaa aaatcagagg caactaacac tgcggcttcc tgagtgccta tgatcagaag 360
   ccacaagtgg gaatgaggcc ctccaacccc cccacatcat ccagcacgcc agtgtcccca 420
   ctgcatcatg catctccaaa ctcaactcat acaccgaaac ctgaccgggc cttcccagct 480
   cacctccctc catcgcagtc cataccagat agcagctacc ccatggacca cagatttcgc 540
   cgccagcttt ctgaaccctg taactccttt cctcctttgc cgacgatgcc aagggaagga 600
   cgtcctatgt accaacgcca gatgtct 627
<210> 425
   <211> 645
   <212> DNA
   <213> Homo sapiens
<400> 425
   cttcccctgg gtaccagccc cacccctcta tcccccctcg cagcaccccc cacctccgtc 60
   ccgcctcgcc ccgcccccct cctggcagtc ccagcgcgca tgctctctcc ccccgcggag 120
   taacctggag atttaaaagc cgccggctgg cgcgcgtggg gggcaaggaa gggggggcgg 180
   aaccagcctg cacgcgctgg ctccgggtga cagccgcgcg cctcggccag atttcagaga 240
   caactgtctg aaccctgcca ccccttccct cctcagccag gagttcctgg agataatcgc 300
   cccagttacc atcggcaaat gtcagaacct attgtccctg cagctccccc gccccctcag 360
   ggattcaaac aagaatacca tgacccactc tatgaacatg gggtcccggg catgccaggg 420
   cccccagcac acgggttcca gtcaccaatg ggaatcaagc aggagcctcg ggattactgc 480
   gtcgattcag aagtgcctaa ctgccagtca tcctacatga gagggggtta tttctccagc 540
   agccatgaag gtttttcata tgaaaaagat ccccgattat actttgacga cacttgtgtt 600
   gtgcctgaga gactggaagg caaagtcaaa caggagccta ccatg 645
<210> 426
   <211> 425
   <212> DNA
   <213> Homo sapiens
<400> 426
   gtaggcgcga gctaagcagg aggcggaggc ggaggcggag ggcgaggggc ggggagcgcc 60
   gcctggagcg cggcaggagg atcccttttc ccccagaaat tactcaatgc tgaaacctct 120
   caaagtggta ttagagacgc tgaaagcacc atggacgggt tttatgatca gcaagtccct 180
   tttatggtcc cagggaaatc tcgatctgag gaatgcagag ggcggcctgt gattgacaga 240
   aagaggaagt ttttggacac agatctggct cacgattctg aagagctatt tcaggatctc 300
   agtcaacttc aagaggcttg gttagctgaa gcacaagttc ctgatgatga acagtttgtc 360
   ccagattttc agtctgataa cctggtgctt catgccccac ctccaaccaa gatcaaacgg 420
   gagct 425
<210> 427
   <211> 721
   <212> DNA
   <213> Homo sapiens
<400> 427
   gagtaggcgc gagctaagca ggaggcggag gcggaggcgg agggcgaggg gcggggagcg 60
   ccgcctggag cgcggcaggt catattgaac attccagata cctatcatta ctcgatgctg 120
   ttgataacag caagatggct ttgaactcag ggtcaccacc agctattgga ccttactatg 180
   aaaaccatgg ataccaaccg gaaaacccct atcccgcaca gcccactgtg gtccccactg 240
   tctacgaggt gcatccggct cagtactacc cgtcccccgt gccccagtac gccccgaggg 300
   tcctgacgca ggcttccaac cccgtcgtct gcacgcagcc caaatcccca tccgggacag 360
   tgtgcacctc aagaggatcc cttttccccc agaaattact caatgctgaa acctctcaaa 420
   gtggtattag agacgctgaa agcaccatgg acgggtttta tgatcagcaa gtccctttta 480
   tggtcccagg gaaatctcga tctgaggaat gcagagggcg gcctgtgatt gacagaaaga 540
   ggaagttttt ggacacagat ctggctcacg attctgaaga gctatttcag gatctcagtc 600
   aacttcaaga ggcttggtta gctgaagcac aagttcctga tgatgaacag tttgtcccag 660
   attttcagtc tgataacctg gtgcttcacg ccccacctcc aaccaagatc aaacgggagc 720
   t 721
<210> 428
   <211> 803
   <212> DNA
   <213> Homo sapiens
<400> 428
   ctggctcctg agctgctgct gtgaagggag gacctggtgg gctaggtaac cgggagctcc      60
   cccagaaagt cactggaggt tgctcaggac ttccccacct ttactgagtg cttttgaaga     120
   agtaggacct gccctgctcc gctacagaac tactggacag gtcatattga acattccaga     180
   tacctatcat tactcgatgc cgttgataac agcaagatgg ctttgaactc agggtcacca     240
   ccagctattg gaccttacta tgaaaaccat ggataccaac cggaaaaccc ctatcccgca     300
   cagcccactg tggtccccac tgtctacgag gtgcatccgg ctcagtacta cccgtccccc     360
   gtgccccagt acgccccgag ggtcctgacg caggcttcca accccgtcgt ctgcacgcag     420
   cccaaatccc catccgggac agtgtgcacc tcaagaggat cccttttccc ccagaaatta     480
   ctcaatgctg aaacctctca aagtggtatt agagacgctg aaagcaccat ggacgggttt     540
   tatgatcagc aagtcccttt tatggtccca gggaaatctc gatctgagga atgcagaggg     600
   cggcctgtga ttgacagaaa gaggaagttt ttggacacag atctggctca cgattctgaa     660
   gagctatttc aggatctcag tcaacttcaa gaggcttggt tagctgaagc acaagttcct     720
   gatgatgaac agtttgtccc agattttcag tctgataacc tggtgcttca tgccccacct     780
   ccaaccaaga tcaaacggga gct                                             803